# EUROPEAN PATENT APPLICATION

(11) **EP 1 493 750 A2**
(43) Date of publication of application: **05.01.2005**
(21) Application number: 04015098.9
(22) Date of filing: 28.06.2004
(51) Int. Cl.: C07K 14/47

(54) **HCV regulated proteins**

(30) Priority: 30.06.2003 GB 0315248
(71) Applicant: F. HOFFMANN-LA ROCHE AG, 4070 Basel (CH)
(72) Inventor: Berndt, Peter, 4056 Basel (CH); Kilby, Peter Michael, Berkshire RG41 3JB (GB); Rugman, Paul, Leicestershire LE12 8AH (GB)

(57) **Abstract**

The present invention relates to novel host cell targets for antiviral, preferably anti-hepatitis C virus (HCV), compounds identified by analysis of HCV replicon-regulated polypeptide expression, to prognostic markers for the prediction of the outcome of a viral infection, to in vitro methods for the prediction of the outcome of a HCV infection in a subject, to screening methods for identifying compounds which interact with and/or modulate the activity of the novel host cell targets or which modulate the expression of said novel host cell targets.

## Description

The present invention relates to novel host cell targets for anti-hepatitis C virus (HCV) drugs identified by analysis of HCV replicon-regulated polypeptide expression, to prognostic markers for the prediction of the outcome of a viral infection, to in vitro methods for the prediction of the outcome of a HCV infection in a subject, to screening methods for identifying compounds which interact with and/or modulate the activity of the novel host cell targets or which modulate the expression of said novel host cell targets.

Hepatitis C virus (HCV) infection is a rapidly increasing public health problem, with an estimated 200 million chronically infected patients worldwide. No vaccines are currently available (Hagedom, C. H. & Rice, C. M. eds. (1999) Current Topics in Microbiology and Immunology (Springer, Berlin)) for HCV, and only a subset of patients responds to current treatments. The mechanisms of viral infection, persistence and clearance are not well understood, mainly because of lack of adequate animal models and cell culture systems.

As the propagation of the virus in cell culture has been unsuccessful (Tan, S.-L. et al. (2002), Hepatitis C Therapeutics: Current Status and Emerging Strategies, Nature Reviews, Vol. 1, 867 - 881), a HCV-replicon system, in which expression of the HCV non-structural proteins drives the replication of a subgenomic HCV RNA (Bartenschläger, R. (2002), Hepatitis C virus replicons: potential role for drug development, Nature Reviews, Vol. 1, 911 - 916) has been generated. With this system in hand, HCV replication and host-all response can be studied in greater detail. Although most of the current antiviral efforts are aimed at key viral enzymes that are essential to HCV replication, cellular genes that are required for HCV replication represent an attractive class of genes for targeting to control viral replication, e.g., with small molecules.

mRNA viruses are totally dependent on the host cell for protein synthesis. They have to subvert the host cell protein synthesis machinery to a significant degree in order to express their viral proteins. Not surprisingly, parts of this process are already described in the art. Thus, Krüger et al. (Krüger, M. et al., Identification of eIF2Bγ and eIF2γ as cofactors of HCV IRES-mediated translation using a functional genomics approach, PNAS, 2000, vol. 97, 8566 - 8571) used a functional genomics approach to identify eIF2Bγ and eIF2γ cellular cofactors involved in HCV IRES function.

During viral assembly, mammalian viruses are dependent on the host-cell glycosylation machinery for morphogenesis and secretion. The first step in the N-linked glycosylation pathway is accomplished by the ER-localized glucosidases, which may be targeted at a low level to treat viral infections without compromising the host. Su A. J. (Su AI, Pezacki JP et al, PNAS, 2002, 99(24), 15669-15674) showed that among the host genes induced in HCV infection were several involved in the immune response and induced by IFN-γ as CD8, MHC Class II components, chemokines as RANTES and MIG. Other genes identified that were differentially induced or repressed during early HCV infection were associated with lipid metabolism (prognostic markers of outcome).

However, it is well known that regulation on the transcriptional level is not necessarily indicative of a similar regulation of the expression of the respective gene on the translational level. Several studies have shown that mRNA expression levels and actual protein expression are only poorly correlated (Gygi SP, Rochon Y, Franza BR, Aebersold R., Mol Cell Biol., 1999, 19(3), 1720-30). Thus, only by demonstrating that the polypeptides as listed in Tables 1 and 2 are down- or up-regulated by the presence of HCV replicon-derived RNA is it possible to use them as novel targets for viral drugs, and as prognostic markers for the prediction of the disease outcome.

Therefore, there is a need in the art for novel host cell targets in the treatment of a HCV infection, as well as for markers to study and to predict the outcome of a HCV infection.

The present invention relates to novel host cell targets for antiviral compounds, preferably anti-HCV compounds, identified by analysis of HCV replicon-regulated polypeptide expression, to prognostic markers for the prediction of the outcome of a viral infection, to in vitro methods for the prediction of the outcome of a HCV infection in a subject, to screening methods for identifying compounds which interact with and/or modulate the activity of the novel host cell targets or which modulate the expression of said novel host cell targets.

The present invention provides a target for an antiviral compound comprising at least one polypeptide selected from the group of polypeptides as listed in Tables 1 and 2. Preferably, a target for an antiviral compound comprising at least one polypeptide selected from the group of polypeptides as listed in Table 3 is provided. The polypeptides as listed in Table 3 comprise up- or down-regulated mRNA chaperones, mRNA-binding proteins as, e.g., PTB. Also preferably, a target for an antiviral compound comprising at least one polypeptide selected from the group of polypeptides as listed in Table 4 is provided. The polypeptides as listed in Table 4 comprise up- or down-regulated translation initiation proteins as, e.g., eIF2γ and eIF2Bγ. In further preferred embodiment, a target for an antiviral compound comprising at least one polypeptide selected from the group of polypeptides as listed in Table 5 is provided. The polypeptides as listed in Table 5 comprise up- or down-regulated polypeptides involved in lipid metabolism, e.g., polypeptides involved in cholesterol synthesis and β-oxidation. In another preferred embodiment, a target for an antiviral compound comprising at least one polypeptide selected from the group of polypeptides as listed in Table 6 is provided. The polypeptides as listed in Table 6 comprise up- or down-regulated polypeptides involved in signal transduction pathways, e.g., MAP kinases as MK01 or oncogenes as SRC8. Preferred are the above-described targets as anti-HCV targets. The target for an antiviral compound, preferably an anti-HCV compound may comprise two, three, four, five, six or even more of the polypeptides as listed in Table 1, Table 2, preferably in Table 3, in Table 4, in Table 5, and in Table 6. Especially preferred as targets for an anti-HCV compound are the polypeptides of SEQ ID NO: 70 (PTB_HUMAN), 101 (NR54_HUMAN), 72 (ACDB_HUMAN), 193 (CHR1-O14805), or 166 (SCR8_HUMAN). The antiviral compound as used herein may comprise compounds against any virus e.g. DAN and RNA virus, preferably they comprise compounds against RNA viruses e.g. HIV, poliovirus. Most preferably, the antiviral compounds are compounds against HCV.

The term "polypeptide" as used herein, refers to a polymer of amino acids, and not to a specific length. Thus, peptides, oligopeptides and proteins are included within the definition of polypeptide.

The present invention also provides a prognostic marker for the prediction of the outcome of a viral infection comprising at least one polypeptide selected from group of polypeptides as listed in Tables 1 and 2. Preferably, a prognostic marker for the prediction of the outcome of a viral infection comprising at least one polypeptide selected from the group of polypeptides as listed in Tables 3 to 6 is provided. The marker may comprise two, three, four, five six, seven, eight, nine, ten or more of the polypeptides selected from the polypeptides as listed in Tables 1 and 2 or as listed in Tables 3 to 6. Preferably, the invention provides a prognostic marker for the prediction of the outcome of a HCV infection. The outcome of a HCV infection may comprise hepatic failure, hepatocellular carcinoma and liver cirrhosis (Hofnagle, J.H., Hepatitis C - the clinical spectrum of disease Hepatology 26, 1997, 15 - 20).

The present invention also provides an in vitro method for the prediction of the outcome of a HCV infection in a subject comprising the steps of obtaining a biological sample from a HCV-infected subject; and determining the expression level of at least one polypeptide from the group of polypeptides as listed in Tables 1 to 6. The term "determining the expression level" as used herein refers to the quantitative determination of the expression of polypeptides in biological samples from an HCV-infected subject. Moreover, the in vitro method further relates to comparing the expression level of the at least one polypeptides from the group of polypeptides as listed in Tables 1 to 6 to its expression level from different time points in the course of the HCV infection and/or to the differences in expression to biological samples from uninfected subjects. Methods for determining the expression level of polypeptides in biological samples are well known in the art and include, but are not limited to, Western-blotting, ELISA or RIA. Antibodies recognizing the polypeptides listed in Tables 1 to 6 can either be generated for the purpose of detecting said polypeptides, e.g., by immunizing rabbits with purified proteins, or known antibodies recognizing said polypeptides can be used. For example, an antibody capable of binding to the denatured proteins, such as a polyclonal antibody, can be used to detect the polypeptides of this invention in a Western Blot. An example for such a method to determine the expression level of a prognostic marker is an ELISA. This type of protein quantification is based on an antibody capable of capturing a specific antigen, and a second antibody capable of detecting the captured antigen. A further method for the determination of the expression of a marker for the prediction of the outcome of a HCV infection comprises analysing biopsy specimens for the presence or absence of the markers of this invention. Methods for the detection of these markers are well known in the art and include, but are not limited to, immunohistochemistry or immunofluorescent detection of the presence or absence of the polypeptides of this invention. Methods for preparation and use of antibodies, and the assays mentioned hereinbefore are described in Harlow, E. and Lane, D. Antibodies: A Laboratory Manual, (1988), Cold Spring Harbor Laboratory Press.

For the methods for the prediction of the outcome of a HCV infection of the present invention, suitable biological samples need to be analysed for the expression of a marker. Said biological samples can be serum, plasma, or liver tissue. Cells from liver tissue can be obtained by, e.g., large-bore needle biopsy.

In the present invention, the term subject comprises any mammal, which may be infected with HCV. Preferably, the subject is a human being.

The present invention also provides a screening method for identifying and/or obtaining a compound which interacts with a polypeptide selected from the polypeptides as listed in Tables 1 to 6 whose expression is up-regulated or down-regulated in HCV-infected tissue comprising the steps of contacting said polypeptide with a compound or a plurality of compounds under conditions which allow interaction of said compound with said polypeptide; and by detecting the interaction between said compound or plurality of compounds with said polypeptide.

The "interaction" in the screening methods as disclosed herein may be measured by conventional methods. The type of conventional method for testing the interaction of a compound with a polypeptide that is soluble, as opposed to membrane associated, can be an in vitro method using either purified recombinant polypeptide, or native polypeptide purified from cells that endogenously express the polypeptide. As a non-limiting example, a polypeptide of the invention can be bound to beads or immobilized on plastic or other surfaces, and interaction of a compound with the polypeptide can be measured by either using a labeled compound and measuring the label bound to the polypeptide, or by displacement of a labeled known ligand from said polypeptide.

For polypeptides that are associated with the cell membrane on the cell surface, or which are expressed as transmembrane or integral membrane polypeptides, the interaction of a compound with said polypeptides can be detected with different methods which include, but are not limited to, methods using cells that either normally express the polypeptide or in which the polypeptide is overexpressed, e.g., by detecting displacement of a known ligand which is labeled by the compound to be screened. Alternatively, membrane preparations may be used to test for interaction of a compound with such a polypeptide.

Interaction assays to be employed in the method disclosed herein may comprise FRET-assays (fluorescence resonance energy transfer; as described, inter alia, in Ng, Science 283 (1999), 2085-2089 or Ubarretxena-Belandia, Biochem. 38 (1999), 7398-7405), TR-FRETs and biochemical assays as disclosed herein. Furthermore, commercial assays like "Amplified Luminescent Proximity Homogenous Assay™" (BioSignal Packard) may be employed. Further methods are well known in the art and, inter alia, described in Fernandez, Curr. Opin. Chem. Biol. 2 (1998), 597-603.

The method for identifying and/or obtaining interacting compounds may also be carried out by specific immunological and/or biochemical assays which are well known in the art and which comprise, e.g., homogenous and heterogeneous assays as described herein below. Said interaction assays employing read-out systems are well known in the art and comprise, inter alia, two-hybrid screenings (as, described, inter alia, in EP-0 963 376, WO 98/25947, WO 00/02911; GST-pull-down columns, co-precipitation assays from cell extracts as described, inter alia, in Kasus-Jacobi, Oncogene 19 (2000), 2052-2059, "interaction-trap" systems (as described, inter alia, in US 6,004,746) expression cloning (e.g. lamda gt11), phage display (as described, inter alia, in US 5,541,109), in vitro binding assays and the like. Further interaction assay methods and corresponding read out systems are, inter alia, described in US 5,525,490, WO 99/51741, WO 00/17221, WO 00/14271 or WO 00/05410. Vidal and Legrain (Nucleic Acids Research 27 (1999), 919-929) describe, review and summarize further interaction assays known in the art which may be employed in accordance with the present invention.

Homogeneous (interaction) assays comprise assays wherein the binding partners remain in solution and comprise assays, e.g., agglutination assays. Heterogeneous assays comprise assays like, inter alia, immuno assays, e.g., Enzyme Linked Immunosorbent Assays (ELISA), Radioactive Immunoassays (RIA), Immuno Radiometric Assays (IRMA), Flow Injection Analysis (FIA), Flow Activated Cell Sorting (FACS), Chemiluminescent Immuno Assays (CLIA) or Electrogenerated Chemiluminescent (ECL) reporting.

The present invention further provides a screening method for identifying and/or obtaining a compound which is an inhibitor or an antagonist of a polypeptide as listed in Tables 2 to 6 whose expression is up-regulated in HCV-infected tissue comprising the steps of a) contacting said polypeptide with a compound or a plurality of compounds under conditions which allow interaction of said compound with said polypeptide; b) determining the activity of said polypeptide; c) comparing the activity of said polypeptide in the presence of the compound or the plurality of compounds to the activity determined in the absence of the compound or the plurality of compounds; and identifying and/or obtaining an inhibitor or an antagonist, wherein a decreased activity determined in (c) is indicative for an inhibitor or antagonist. The terms inhibitors and antagonists as used herein are used interchangeably. The screening method can be performed either as an in vitro assay, or as a host-based assay. The host to be employed in the screening methods of the present invention and comprising and/or expressing a polypeptide listed as in Tables 2 to 6 may comprise prokaryotic as well as eukaryotic cells. Said cells may comprise bacterial cells, yeast cells, as well as cultured (tissue) cell lines, inter alia, derived from mammals. Furthermore animals may also be employed as hosts, for example non-human transgenic animals. Accordingly, said host (cell) may be transfected or transformed with the vector comprising a nucleic acid molecule encoding a polypeptide which is differentially expressed in HCV-infected tissue as disclosed herein. Said host cell or host may therefore be genetically modified with a nucleic acid molecule encoding such a polypeptide or with a vector comprising such a nucleic acid molecule. The present invention further provides a screening method for identifying and/or obtaining a compound which is an activator or agonist of a polypeptide as listed in Tables 1, 3 to 6 whose expression is down-regulated in HCV-infected tissue comprising the steps of a) contacting said polypeptide with a compound or a plurality of compounds under conditions which allow interaction of said compound with said polypeptide; b) determining the activity of said polypeptide; c) comparing the activity of said polypeptide in the presence of the compound or the plurality of compounds to the activity determined in the absence of the compound or the plurality of compounds; and identifying and/or obtaining an activator or agonist, wherein an increased activity determined in (c) is indicative for an activator or agonist. The terms activator and agonist as used herein are used interchangeably. The screening method can be performed either as an in vitro assay, or as a host-based assay. The host to be employed in the screening methods of the present invention and comprising and/or expressing a polypeptide listed as in Tables 1, 3 to 6 may comprise prokaryotic as well as eukaryotic cells. Said cells may comprise bacterial cells, yeast cells, as well as cultured (tissue) cell lines, inter alia, derived from mammals. Furthermore animals may also be employed as hosts, for example non-human transgenic animals. Accordingly, said host (cell) may be transfected or transformed with the vector comprising a nucleic acid molecule encoding a polypeptide which is differentially expressed in HCV-infected tissue as disclosed herein. Said host cell or host may therefore be genetically modified with a nucleic acid molecule encoding such a polypeptide or with a vector comprising such a nucleic acid molecule.
The term "genetically modified" means that the host cell or host comprises in addition to its natural genome a nucleic acid molecule or vector coding for a polypeptide as listed in Tables 2 to 6 or at least a fragment thereof. Said additional genetic material may be introduced into the host (cell) or into one of its predecessors/parents. The nucleic acid molecule or vector may be present in the genetically modified host cell or host either as an independent molecule outside the genome, preferably as a molecule which is capable of replication, or it may be stably integrated into the genome of the host cell or host.

As mentioned herein above, the host cell of the present invention may be any prokaryotic or eukaryotic cell. Suitable prokaryotic cells are those generally used for cloning like *E. coli* or *Bacillus subtilis.* Yet, these prokaryotic host cells are also envisaged in the screening methods disclosed herein. Furthermore, eukaryotic cells comprise, for example, fungal or animal cells. Examples for suitable fungal cells are yeast cells, preferably those of the genus Saccharomyces and most preferably those of the species Saccharomyces cerevisiae. Suitable animal cells are, for instance, insect cells, vertebrate cells, preferably mammalian cells, such as e.g. CHO, HeLa, NIH3T3 or MOLT-4. Further suitable cell lines known in the art are obtainable from cell line depositories, like the American Type Culture Collection (ATCC).

The hosts may also be selected from non-human mammals, most preferably mice, rats, sheep, calves, dogs, monkeys or apes. As described herein above, said animals/mammals also comprise non-human transgenic animals, which preferably express at least one polypeptide differentially expressed in HCV-infected cells or tissue as disclosed herein. Preferably, said polypeptide is a polypeptide, which is up-regulated in tissue derived from HCV-infected subjects. Yet it is also envisaged that non-human transgenic animals be produced which do not express the polypeptides as disclosed herein or which only express limited amounts of said polypeptides. Said animals are preferably related to polypeptides, which are down-regulated in HCV-infected tissue. Transgenic non-human animals comprising and/or expressing the up-regulated polypeptides of the present invention or alternatively, which comprise silenced or less efficient versions of down-regulated polypeptides are useful models for studying the course and/or outcome of a HCV infection and provide for useful models for testing drugs and therapeutics for treatment of HCV infection.

A compound which inhibits or antagonizes or which activates or agonizes a polypeptide as listed in Tables 1 to 6 is identified by determining the activity of said polypeptide in the presence of said compound.

The term "activity" as used herein relates to the functional property or properties of a specific polypeptide. For the polypeptides as listed in Tables 1 to 6 know as enzymes, the term "activity" relates to the enzymatic activity of the specific polypeptide. Activity assays for the enzymes as listed in Tables 1 to 6 are well known in the art.

For the polypeptides as listed in Tables 1 to 6 know as adhesion molecules, the term "activity" relates to the adhesive properties of a polypeptide and may be determined using assays such as, but not limited to, adhesion assays, cell spreading assays, or in vitro interaction assays of the adhesion molecule with a known ligand. Such assays are well known in the art.

For the polypeptides as listed in Tables 1 to 6 known as cytoskeletal proteins, the term "activity" relates to the regulation of the cytoskeleton by such polypeptides, or to their incorporation into the cytoskeleton. As a non-limiting example, the ability of Gelsolin to regulate actin polymerization, or of Filamin A to promote orthogonal branching of actin filaments, may be determined using in vitro actin polymerization assays. Activity in relation to the regulation of cytoskeletal structures may further be determined by, as non-limiting examples, cell spreading assays, cell migration assays, cell proliferation assays or immunofluorecence assays, or by staining actin filaments with fluorescently labeled phalloidin. All of these assays are well known to the person skilled in the art.

For the polypeptides as listed in Tables 1 to 6 known as ion channels the term "activity" relates to ion flux across the membrane. Methods to determine ion flux across membranes are well known to the person skilled in the art.

For polypeptides as listed in Tables 1 to 6 known as transcription factors, the term "activity" relates to their ability to regulate gene transcription. The transcriptional activity of a polypeptide can be determined using assays know in the art, such as a reporter gene assay.

For polypeptides as listed in Tables 1 to 6 known as growth factors and hormones or their receptors, the term "activity" relates to their ability to bind to their receptors or ligands, respectively, and to induce receptor activation and subsequent signaling cascades, and/or it relates to the ability of the factor or receptor to mediate the cellular function or functions eventually caused by growth factor or hormone mediated receptor activation. Growth factor or hormone binding to receptors can be determined by commonly known ligand binding assays. Receptor activation can be determined by testing for receptor autophosphorylation, or by assaying for modification or recruitment of downstream signaling mediators to the receptors (by immunoprecipitation and Western Blotting of signaling complexes). Cellular functions regulated by growth factors or hormones and their receptors can be cell proliferation (e.g. determined by using thymidine incorporation or cell counts), cell migration assays (e.g. determined by using modified Boyden chambers), cell survival or apoptosis assays (e.g. determined by using DAPI staining), angiogenesis assays (e.g. in vitro assays to measure endothelial tube formation that are commercially available). In addition to these assays, other assays may be used as well to determine these and other cellular functions.

Inhibitors or antagonists of a polypeptide as listed in Tables 2 to 6 are identified by the screening method described above when there is a decreased activity determined in the presence of the compound in comparison to the activity in the absence of the compound in the screening method.

Activators or agonists of a polypeptides as listed in Tables 1, 3 to 6 are identified by the described screening method of the invention when there is an increased activity determined in the presence of the compound as compared to the activity in the absence of the compound in the screening method.

Further to the screening methods disclosed above, this invention provides a screening method for identifying and/or obtaining a compound which is an inhibitor of the expression of a polypeptide selected from the polypeptides as to listed in Tables 2 to 6 whose expression is up-regulated in HCV-infected tissue, comprising the steps of a) contacting a host which expresses said polypeptide with a compound or a plurality of compounds; b) determining the expression level of said polypeptide; c) comparing the expression level of said polypeptide in the presence of the compound or the plurality of compounds to the expression level of said polypeptide determined in the absence of the compound or plurality of compounds; and d), identifying and/or obtaining an inhibitor of the expression of a polypeptide, wherein a decreased expression level determined in (c) is indicative for an inhibitor of the expression of said polypeptide.

Further to the screening methods disclosed above, this invention provides a screening method for identifying and/or obtaining a compound which is an activator of the expression of a polypeptide selected from the polypeptides as to listed in Tables 1, 3 to 6 whose expression is down-regulated in HCV-infected tissue, comprising the steps of a) contacting a host which expresses said polypeptide with a compound or a plurality of compounds; b) determining the expression level of said polypeptide; c) comparing the expression level of said polypeptide in the presence of the compound or the plurality of compounds to the expression level of said polypeptide determined in the absence of the compound or plurality of compounds; and d), identifying and/or obtaining an activator of the expression of a polypeptide, wherein a increased expression level determined in (c) is indicative for an activator of the expression of said polypeptide.

An inhibitor of the expression of a polypeptide as listed in Tables 2 to 6 is identified by the screening method described hereinbefore, when a decreased expression of the protein is determined in the presence of the compound in comparison to the absence of the compound in the screening method, which is indicative for an inhibitor of the expression of a polypeptide.

The term "expression" as used herein relates to amount determined of a polypeptide as listed in Tables 2 to 6 which is up-regulated or down-regulated in HCV-infected tissue, in HCV-infected cells, which is elevated or reduced as compared to the amount of the same polypeptide in uninfected tissue or uninfected cells. Preferably, expression levels are at least 2 fold, more preferably at least 3 fold, even more preferably at least 4 fold, most preferably at least 5 fold higher or lower than in uninfected tissue or cells.

Furthermore, the present invention provides a compound identified and/or obtained by any of the screening methods hereinbefore described. The compounds identified and/or obtained by any of the screening methods of the present invention comprise small molecules, polypeptides, hormones, polynucleotides comprising antisense nucleotides and antibodies. A pharmaceutical composition comprising said compound and pharmaceutically acceptable carrier or diluent is further provided. A method for the preparation of said pharmaceutical composition comprising formulating said compound in a pharmaceutically acceptable carrier or diluent is also claimed. Any conventional carrier material can be utilized. The carrier material can be an organic or inorganic one suitable for enteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, stabilizers, emulsifying agents, buffers and the like may be added in accordance with accepted practices of pharmaceutical compounding.

Said compound may be used for the preparation of a medicament for the treatment or prevention of HCV infection. Preferably, said compound is selected from the group comprising an antibody, an antibody-derivative, an antibody fragment, a peptide or an antisense construct.

Within the scope of the present invention, antibodies against the polypeptides as listed in Tables 1 to 6, or antigen-binding fragments thereof, for the use in an in vitro method for the prediction of the outcome of a HCV infection in a subject are provided.

In order to efficiently perform the prognostic method, the present invention provides a kit for the prediction of the outcome of a HCV infection in a subject comprising one or more of the antibodies, or antigen-binding fragments thereof as described above. Another kit provided by the invention is a kit for screening of compounds that activate or inhibit the activity, or activate or inhibit the expression, of any one of the polypeptides as listed in Tables 1 and 2.
Having now generally described this invention, the same will become better understood by reference to the specific examples, which are included herein for purpose of illustration only and are not intended to be limiting unless otherwise specified.

**Table 1**

| SEQ ID NO | Down-regulated polypeptides |
|---|---|
| 1 | HSUGP:006101-3-0 |
| 2 | HUMANGP:CHR11-Q9UMS4 |
| 3 | HUMANGP:CHR17-Q8TCD5 |
| 4 | HUMANGP:CHR19-Q9BVL0 |
| 5 | HUMANGP:CHR1-CAC22475 |
| 6 | HUMANGP:CHR1-Q9HB71 |
| 7 | HUMANGP:CHR4-Q96S43 |
| 8 | HUMANGP:CHR4-Q9Z130 |
| 9 | HUMANGP:CHR7-Q13122 |
| 10 | HUMANGP:CHR9-O60376 |
| 11 | HUMANGP:CHR9-Q9BS26 |
| 12 | SW_HUM:AKC1_HUMAN |
| 13 | SW_HUM:AKC2_HUMAN |
| 14 | SW_HUM:ALFC_HUMAN |
| 15 | SW_HUM:APT_HUMAN |
| 16 | SW_HUM:ARGI_HUMAN |
| 17 | SW_HUM:B53A_HUMAN |
| 18 | SW_HUM:CATB_HUMAN |
| 19 | SW_HUM:CN03_HUMAN |
| 20 | SW_HUM:COF1_HUMAN |
| 21 | SW_HUM:COPE_HUMAN |
| 22 | SW_HUM:DEST_HUMAN |
| 23 | SW_HUM:DHA5_HUMAN |
| 24 | SW_HUM:DHQU_HUMAN |
| 25 | SW_HUM:DPP3_HUMAN |
| 26 | SW_HUM:ECH1_HUMAN |
| 27 | SW_HUM:EF1D_HUMAN |
| 28 | SW_HUM:ENOL_HUMAN |
| 29 | SW_HUM:FIBG_HUMAN |
| 30 | SW_HUM:G3B2_HUMAN |
| 31 | SW_HUM:G3BP_HUMAN |
| 32 | SW_HUM:G6PD_HUMAN |
| 33 | SW_HUM:GAMT_HUMAN |
| 34 | SW_HUM:GRF1_HUMAN |
| 35 | SW_HUM:GTO1_HUMAN |
| 36 | SW_HUM:H105_HUMAN |
| 37 | SW_HUM:HDGF_HUMAN |
| 38 | SW_HUM:HMCS_HUMAN |
| 39 | SW_HUM:IF2A_HUMAN |
| 40 | SW_HUM:IF33_HUMAN |
| 41 | SW_HUM:IF41_HUMAN |
| 42 | SW_HUM:LAM1_HUMAN |
| 43 | SW_HUM:LAM2_HUMAN |
| 44 | SW_HUM:LAMA_HUMAN |
| 45 | SW_HUM:LDHA_HUMAN |
| 46 | SW_HUM:MAE1_HUMAN |
| 47 | SW_HUM:MAOX_HUMAN |
| 48 | SW_HUM:MDHC_HUMAN |
| 49 | SW_HUM:NDKA_HUMAN |
| 50 | SW_HUM:NDR1_HUMAN |
| 51 | SW_HUM:PCNA_HUMAN |
| 52 | SW_HUM:PGK1_HUMAN |
| 53 | SW_HUM:PRS6_HUMAN |
| 54 | SW_HUM:PSB7_HUMAN |
| 55 | SW_HUM:R23B_HUMAN |
| 56 | SW_HUM:ROC_HUMAN |
| 57 | SW_HUM:ROH2_HUMAN |
| 58 | SW_HUM:ROH3_HUMAN |
| 59 | SW_HUM:SC13_HUMAN |
| 60 | SW_HUM:SNAA_HUMAN |
| 61 | SW_HUM:SPEE_HUMAN |
| 62 | SW_HUM:TKT_HUMAN |
| 63 | SW_HUM:UBA1_HUMAN |
| 64 | SW_HUM:UBC1_HUMAN |
| 65 | SW_HUM:UBPE_HUMAN |
| 66 | SW_HUM:UNRI_HUMAN |
| 67 | SW_HUM:VAA1_HUMAN |
| 68 | SW_HUM:VIL1_HUMAN |
| 69 | TR_HUM:Q96HX0 |

**Table 2**

| SEQ ID NO | Up-regulated polypeptides |
|---|---|
| 70 | SW_HUM:PTB_HUMAN |
| 71 | SW_HUM:KPY2_HUMAN |
| 72 | SW_HUM:ACDB_HUMAN |
| 73 | SW_HUM:RS4_HUMAN |
| 74 | SW_HUM:CRKL_HUMAN |
| 75 | SW_HUM:DDX5_HUMAN |
| 76 | SW_HUM:LAS1_HUMAN |
| 77 | SW_HUM:SYY_HUMAN |
| 78 | SW_HUM:SEP7_HUMAN |
| 79 | SW_HUM:ATPO_HUMAN |
| 80 | SW_HUM:MCM3_HUMAN |
| 81 | SW_HUM:RL12_HUMAN |
| 82 | TR_HUM:Q9Y280 |
| 83 | SW_HUM:AMRP_HUMAN |
| 84 | SW_HUM:ARG2_HUMAN |
| 85 | SW_HUM:IF2B_HUMAN |
| 86 | SW_HUM:ROG_HUMAN |
| 87 | HUMANGP:CHR11-075932 |
| 88 | SW_HUM:TCPD_HUMAN |
| 89 | HSUGP:075546-8-0 |
| 90 | HUMANGP:CHR3-075191 |
| 91 | SW_HUM:DUS9_HUMAN |
| 92 | SW_HUM:GTM4_HUMAN |
| 93 | SW_HUM:RS8_HUMAN |
| 94 | SW_HUM:SM30_HUMAN |
| 95 | HSUGP:013561-2-0 |
| 96 | HUMANGP:CHR2-Q9UQE6 |
| 97 | HUMANGP:CHR7-Q9UNH0 |
| 98 | SW_HUM:PEBB_HUMAN |
| 99 | HSUGP:075309-23-0 |
| 100 | HSUGP:075309-24-0 |
| 101 | SW_HUM:NR54_HUMAN |
| 102 | SW_HUM:DDXY_HUMAN |
| 103 | HUMANGP:CHR8-094905 |
| 104 | SW_HUM:COPD_HUMAN |
| 105 | SW_HUM:CAPB_HUMAN |
| 106 | SW_HUM:VATE_HUMAN |
| 107 | SW_HUM:ACLY_HUMAN |
| 108 | HSUGP:008768-9-0 |
| 109 | HSUGP:075546-6-0 |
| 110 | SW_HUM:DD17_HUMAN |
| 111 | SW_HUM:CAPG_HUMAN |
| 112 | SW_HUM:VAB1_HUMAN |
| 113 | SW_HUM:UBP5_HUMAN |
| 114 | TR_HUM:Q9BVH9 |
| 115 | SW_HUM:CGL1_HUMAN |
| 116 | SW_HUM:VIME_HUMAN |
| 117 | SW_HUM:PRO1_HUMAN |
| 118 | SW_HUM:RS3_HUMAN |
| 119 | SW_HUM:NADC_HUMAN |
| 120 | HUMANGP:CHR5-Q96NM0 |
| 121 | HUMANGP:CHR19-O95717 |
| 122 | SW_HUM:GNPI_HUMAN |
| 123 | SW_HUM:NAGK_HUMAN |
| 124 | SW_HUM:SODM_HUMAN |
| 125 | SW_HUM:ESTD_HUMAN |
| 126 | SW_HUM:GLYM_HUMAN |
| 127 | SW_HUM:IDHP_HUMAN |
| 128 | SW_HUM:PSA1_HUMAN |
| 129 | SW_HUM:K6A3_HUMAN |
| 130 | HUMANGP:CHR20-O14878 |
| 131 | SW_HUM:PAK2_HUMAN |
| 132 | SW_HUM:MEPD_HUMAN |
| 133 | SW_HUM:PSB5_HUMAN |
| 134 | SW_HUM:MK01_HUMAN |
| 135 | SW_HUM:MCCB_HUMAN |
| 136 | SW_HUM:RRB1_HUMAN |
| 137 | TR_HUM:Q96E67 |
| 138 | SW_HUM:HGD_HUMAN |
| 139 | SW_HUM:6PGD_HUMAN |
| 140 | SW_HUM:IF4N_HUMAN |
| 141 | SW_HUM:UCR2_HUMAN |
| 142 | SW_HUM:143Z_HUMAN |
| 143 | SW_HUM:TAL1_HUMAN |
| 144 | SW_HUM:NPS1_HUMAN |
| 145 | SW_HUM:DCUP_HUMAN |
| 146 | SW_HUM:AATM_HUMAN |
| 147 | SW_HUM:PSA2_HUMAN |
| 148 | SW_HUM:KPY1_HUMAN |
| 149 | SW_HUM:AATC_HUMAN |
| 150 | SW_HUM:ATPA_HUMAN |
| 151 | SW_HUM:E2BG_HUMAN |
| 152 | SW_HUM:GLPK_HUMAN |
| 153 | SW_HUM:RS2_HUMAN |
| 154 | SW_HUM:SYFA_HUMAN |
| 155 | SW_HUM:DJA1_HUMAN |
| 156 | SW_HUM:SYSM_HUMAN |
| 157 | SW_HUM:HAX1_HUMAN |
| 158 | HUMANGP:CHR19-Q96G16 |
| 159 | HUMANGP:CHR10-Q9HBK9 |
| 160 | SW_HUM:SPS1_HUMAN |
| 161 | SW_HUM:IF39_HUMAN |
| 162 | SW_HUM:NCB1_HUMAN |
| 163 | SW_HUM:Y33K_HUMAN |
| 164 | SW_HUM:HEM6_HUMAN |
| 165 | SW_HUM:THIM_HUMAN |
| 166 | SW_HUM:SRC8_HUMAN |
| 167 | SW_HUM:KAD4_HUMAN |
| 168 | HUMANGP:CHR12-Q9NRV9 |
| 169 | HUMANGP:CHR12-Q14221 |
| 170 | SW_HUM:MYG1_HUMAN |
| 171 | SW_HUM:MPPA_HUMAN |
| 172 | SW_HUM:PSDB_HUMAN |
| 173 | SW_HUM:ACDM_HUMAN |
| 174 | SW_HUM:GIPC_HUMAN |
| 175 | HUMANGP:CHR1-AAH15403 |
| 176 | SW_HUM:GATM_HUMAN |
| 177 | SW_HUM:PSE3_HUMAN |
| 178 | SW_HUM:CALU_HUMAN |
| 179 | SW_HUM:DCT2_HUMAN |
| 180 | SW_HUM:HCD2_HUMAN |
| 181 | SW_HUM:PDXK_HUMAN |
| 182 | SW_HUM:IF6_HUMAN |
| 183 | SW_ HUM:PDX5_ HUMAN |
| 184 | SW_ HUM:PP1G HUMAN |
| 185 | SW_HUM:PIMT_HUMAN |
| 186 | SW_ HUM:TCPQ_HUMAN |
| 187 | HSUGP:333417-11-0 |
| 188 | SW_HUM:PSB3_HUMAN |
| 189 | HUMANGP:CHR6-Q8TDM4 |
| 190 | SW_HUM:BIEA_HUMAN |
| 191 | SW_HUM:RAN_HUMAN |
| 192 | SW_HUM:PRS8_HUMAN |
| 193 | HUMANGP:CHR1-014805 |
| 194 | SW_HUM:PSA6_HUMAN |
| 195 | SW_HUM:6PGL_HUMAN |
| 196 | HUMANGP:CHR19-Q8WVXO |
| 197 | SW_HUM:ACON_HUMAN |
| 198 | SW_ HUM:UCR1_HUMAN |
| 199 | SW_HUM:HS27_HUMAN |

**Table 3**

| SEQ ID NO | Up- or downregulated m_RNA binding poteins and mRNA chaperones (selected from Tables 1 and 2) |
|---|---|
| 70 | PTB_HUMAN |
| 101 | NR54_HUMAN |
| 75 | DDX5_HUMAN |
| 110 | DD17_HUMAN |
| 102 | DDXY_HUMAN |
| 86 | ROG_HUMAN |
| 82 | Q9Y280 |

**Table 4**

| SEQ ID NO | Up- or downregulated proteins involved in the regulation of protein synthesis (selected from Tables 1 and 2) |
|---|---|
| 85 | IF2B_HUMAN |
| 151 | E2BG_HUMAN |
| 161 | IF39_HUMAN |
| 140 | IF4N_HUMAN |
| 182 | IF6_HUMAN |
| 136 | RRB1_HUMAN |
| 129 | K6A3_HUMAN |

**Table 5**

| SEQ ID NO | Up- or downregulated proteins involved in lipid metabolism (selected from Tables 1 and 2) |
|---|---|
| 180 | HDC2_HUMAN |
| 173 | ACDM_HUMAN |
| 72 | ACDB_HUMAN |
| 135 | MCCB_HUMAN |
| 165 | THIM_HUMAN |

**Table 6**

| SEQ ID NO | Up- or downregulated proteins involved in signal transduction pathways (selected from Tables 1 and 2) |
|---|---|
| 91 | DUS9_HUMAN |
| 98 | PEBB_HUMAN |
| 166 | SCR8_HUMAN |
| 157 | HAX1_HUMAN |
| 131 | PAK2_HUMAN |
| 193 | O14805 |

### Examples

Commercially available reagents referred to in the examples were used according to manufacturer's instructions unless otherwise indicated.

### Example 1

Huh 8.1 cell line was grown under standard conditions (control) and compared to Huh 9.13 (replicon). The Huh 9.13 cell line was prepared according to Lohmann, V. et al., Science 1999, 285, 110-113. The growth experiment was repeated 3 times, different cell lysis conditions were employed each time (Lohmann, V, et al., (1999), Science 285, 110 - 113; Bartenschläger, R. (2002), Nature Reviews, Hepatitis C virus replicons: potential role for drug development, Vol. 1, 911 - 916).

Several standard cell fractions using differential centrifugation (800g, 12000g, 60000g) were generated. In addition, a particle fraction enriched in replicon protein (replicon particle) was isolated by differential centrifugation/density gradient centrifugation. For all fractions, sample/control pairs of 2D PAGE were run under identical conditions using IPG ranges from 3-10 and 4-7/6-10, and all accessible protein spots were identified using MALDI MS peptide fingerprinting.

Full Extract
Replicon Particle
P1 (800g)
P2 (12000g)
P3 (60000g)
S (>60000g)

For comparison, the results of all experiments were combined and counts of protein identifications were generated. A protein was assigned as "up-regulated" ("down-regulated") if it was found in at least two samples and if it was either totally absent in the control (respective replicon sample) or the quotient between the count of mass spectra identified as the protein in the sample and control was larger than 2 (smaller than 0.5).

Polypeptides down-regulated by the presence of HCV replicon-derived RNA are depicted in Table 1, and polypeptides up-regulated by the presence of HCV replicon derived RNA are depicted in Table 2, comprising a number of interesting mRNA binding proteins and dysregulated components of the protein synthesis machinery.

## Claims

1. A target for an antiviral compound comprising at least one polypeptide selected from the group of polypeptides as listed in Tables 1 and 2.

2. A target for an antiviral compound comprising at least one polypeptide selected from the group of polypeptides as listed in Table 3.

3. A target for an antiviral compound comprising at least one polypeptide selected from the group of polypeptides as listed in Table 4.

4. A target for an antiviral compound comprising at least one polypeptide selected from the group of polypeptides as listed in Table 5.

5. A target for an antiviral compound comprising at least one polypeptide selected from the group of polypeptides as listed in Table 6.

6. The target according to any one of claims 1 to 5, wherein the antiviral compound is an anti-HCV compound.

7. A marker for the prediction of the outcome of a viral infection comprising at least one polypeptide selected from group of polypeptides as listed in Tables 1 and 2.

8. A marker for the prediction of the outcome of a viral infection comprising at least one polypeptide selected from the group of polypeptides as listed in Tables 3 to 6.

9. The marker according to any one of claims 7 and 8, wherein the viral infection is a HCV infection.

10. An in vitro method for the prediction of the outcome of a HCV infection in a subject comprising obtaining a biological sample from a HCV-infected subject and determining the expression level of at least one polypeptide from the group of polypeptides as listed in Tables 1 and 2.

11. An in vitro method for the prediction of the outcome of a HCV infection in a subject comprising determining the expression level of at least one polypeptide from the group of polypeptides as listed in Tables 3 to 6.

12. The method according to any one of claims 10 and 11, further comprising comparing the expression level of the at least one polypeptide to its expression level determined at another time point in the course of a HCV infection or to its expression level in a healthy subject.

13. A screening method for identifying and/or obtaining a compound which interacts with a polypeptide selected from the polypeptides as listed in Tables 1 to 6 whose expression is up-regulated or down-regulated in HCV-infected tissue comprising the steps of
a) contacting said polypeptide with a compound or a plurality of compounds under conditions which allow interaction of said compound with said polypeptide; and
b) detecting the interaction of said compound or plurality of compounds with said polypeptide.

14. A screening method for identifying and/or obtaining a compound which is an inhibitor or an antagonist of a polypeptide as listed in Tables 2 to 6 whose expression is up-regulated in HCV-infected tissue, comprising the steps of
a) contacting said polypeptide with a compound or a plurality of compounds under conditions which allow interaction of said compound with said polypeptide;
b) determining the activity of said polypeptide;
c) comparing the activity of said polypeptide in the presence of the compound or the plurality of compounds to the activity determined in the absence of the compound or the plurality of compounds; and
d) identifying and/or obtaining an inhibitor or an antagonist, wherein a decreased activity determined in (c) is indicative for an inhibitor or an antagonist.

15. A screening method for identifying and/or obtaining a compound which is an activator or an agonist of a polypeptide as listed in Tables 1, 3 to 6 whose expression is down-regulated in HCV-infected tissue, comprising the steps of
a) contacting said polypeptide with a compound or a plurality of compounds under conditions which allow interaction of said compound with said polypeptide;
b) determining the activity of said polypeptide;
c) comparing the activity of said polypeptide in the presence of the compound or the plurality of compounds to the activity determined in the absence of the compound or the plurality of compounds; and
d) identifying and/or obtaining an activator or an agonist, wherein an increased activity determined in (c) is indicative for an activator or an agonist.

16. A screening method for identifying and/or obtaining a compound which is an inhibitor of the expression of a polypeptide selected from the polypeptides as listed in Tables 2 to 6 whose expression is up-regulated in HCV-infected tissue, comprising the steps of
a) contacting a host which expresses said polypeptide with a compound or a plurality of compounds;
b) determining the expression level of said polypeptide;
c) comparing the expression level determined in the presence of the compound or the plurality of compounds to the expression level determined in the absence of the compound or the plurality of compounds; and
d) identifying and/or obtaining an inhibitor of the expression of a polypeptide, wherein a decreased expression level determined in (c) is indicative for an inhibitor of the expression of said polypeptide.

17. A screening method for identifying and/or obtaining a compound which is an activator of the expression of a polypeptide selected from the polypeptides as listed in Tables 1, 3 to 6 whose expression is down-regulated in HCV-infected tissue, comprising the steps of
a) contacting a host which expresses said polypeptide with a compound or a plurality of compounds;
b) determining the expression level of said polypeptide;
c) comparing the expression level determined in the presence of the compound or the plurality of compounds to the expression level determined in the absence of the compound or the plurality of compounds; and
d) identifying and/or obtaining an activator of the expression of a polypeptide, wherein an increased expression level determined in (c) is indicative for an activator of the expression of said polypeptide.

18. A compound identified and/or obtained by the screening methods of any one of claims 13 to 17.

19. A pharmaceutical composition comprising the compound of claim 18 and a pharmaceutically acceptable carrier or diluent.

20. A method for the preparation of the pharmaceutical composition of claim 19 comprising formulating the compound of claim 18 in a pharmaceutically acceptable carrier or diluent.

21. Use of a compound of claim 18 for the preparation of a medicament for the treatment or prevention of HCV infection.

22. The use of claim 21, wherein said compound is selected from the group comprising an antibody, an antibody-derivative, an antibody fragment, a peptide or an antisense construct.

23. Antibodies against the polypeptides as listed in Tables 1 and 2, or antigen-binding fragments thereof, for the use in an in vitro method for the prediction of the outcome of a HCV infection in a subject.

24. A kit for the prediction of the outcome of a HCV infection in a subject comprising one or more of the antibodies, or antigen-binding fragments thereof, of claim 23.

25. A kit for screening of compounds that activate or inhibit the activity, or activate or inhibit the expression, of any one of the polypeptides as listed in Tables 1 and 2.

26. The targets, markers, methods, compounds, compositions, uses, and kits substantially as herein before described especially with reference to the foregoing Examples.


Sequence Listing

SEQUENCE LISTING

<110>  F. Hoffmann-La Roche AG

<120>  HCV regulated protein expression

<130>  21762

<160>  199

<170>  PatentIn version 3.1

<210>  1
<211>  414
<212>  PRT
<213>  Homo sapiens

<220>
<221>  MISC_FEATURE
<222>  (307)..(307)
<223>  x

<300>
<308>  hsugp:006101-3-0
<309>  2003-02-16

<400>  1

Leu Pro Leu Leu Ala Arg Pro Ala Ala Leu Thr Ala Leu Leu Leu Leu
1               5                   10                  15

Leu Leu Ala Met Ala Ala Ala Asp Gly Pro Pro Ala Ala Asp Gly Glu
            20                  25                  30

Asp Gly Gln Asp Pro His Ser Lys His Leu Tyr Thr Ala Asp Met Phe
        35                  40                  45

Thr His Gly Ile Gln Ser Ala Ala His Phe Val Met Phe Phe Ala Pro
    50                  55                  60

Glu Met Gly Ser Cys Ser Val Thr Gln Pro Glu Thr Ser Gly Thr Gln
65                  70                  75                  80

Pro Gln Leu Thr Val Ala Ser Asn Ser Trp Ala Gln Val Ile Leu Leu

85                  90                  95

Pro Gln Pro Leu Lys Tyr Cys Asp Tyr Arg Arg Arg Cys Gly His Cys
            100                 105                 110

Gln Arg Leu Gln Pro Thr Trp Asn Asp Leu Gly Asp Lys Tyr Asn Ser
        115                 120                 125

Met Glu Asp Ala Lys Val Tyr Val Ala Lys Val Asp Cys Thr Ala His
    130                 135                 140

Ser Asp Val Cys Ser Ala Gln Gly Val Arg Gly Tyr Pro Thr Leu Lys
145                 150                 155                 160

Leu Phe Lys Pro Gly Gln Glu Ala Val Lys Tyr Gln Gly Pro Arg Asp
                165                 170                 175

Phe Gln Thr Leu Glu Asn Trp Met Leu Gln Thr Leu Asn Glu Glu Pro
            180                 185                 190

Val Thr Pro Glu Pro Glu Val Glu Pro Pro Ser Ala Pro Glu Leu Lys
        195                 200                 205

Gln Gly Leu Tyr Glu Leu Ser Ala Ser Asn Phe Glu Leu His Val Ala
    210                 215                 220

Gln Gly Asp His Phe Ile Lys Phe Phe Ala Pro Trp Cys Gly His Cys
225                 230                 235                 240

Lys Ala Leu Ala Pro Thr Trp Glu Gln Leu Ala Leu Gly Leu Glu His
                245                 250                 255

Ser Glu Thr Val Lys Ile Gly Lys Val Asp Cys Thr Gln His Tyr Glu
            260                 265                 270

Leu Cys Ser Gly Asn Gln Val Arg Gly Tyr Pro Thr Leu Leu Trp Phe
        275                 280                 285

Arg Asp Gly Lys Lys Val Asp Gln Tyr Lys Gly Lys Arg Asp Leu Glu
    290                 295                 300

Ser Gln Xaa Glu Pro Glu Ala Asp Lys Gly Thr Val Leu Ala Leu Thr
305                 310                 315                 320

Glu Asn Asn Phe Asp Asp Thr Ile Ala Glu Gly Ile Thr Phe Ile Lys
                325                 330                 335

Phe Tyr Ala Pro Trp Cys Gly His Cys Lys Thr Leu Ala Pro Thr Trp
            340                 345                 350

Glu Glu Leu Ser Lys Lys Glu Phe Pro Gly Leu Ala Gly Val Lys Ile
        355                 360                 365

Ala Glu Val Asp Cys Thr Leu Asn Gly Ile Ser Ala Ala Ser Ile Arg
    370                 375                 380

Tyr Glu Ala Thr Pro Arg Tyr Cys Phe Ser Glu Glu Gly Arg Lys Ser
385                 390                 395                 400

Val Ser Thr Val Glu Ala Glu Thr Leu Thr Arg Tyr Thr Ala
                405                 410

<210>  2

<211>  503

<212>  PRT

<213>  Homo sapiens

<300>

<308>  humangp:chr11-q9ums4

<309>  2003-04-22

<400>  2
   Ser Leu Phe Ser Thr Val Ser Asn Glu Val Pro Glu His Pro Cys Val
   1               5                   10                  15
   Ser Pro Val Ser Asn His Val Tyr Glu Arg Arg Leu Ile Glu Lys Tyr
               20                  25                  30
   Ile Ala Glu Asn Gly Thr Asp Pro Ile Asn Asn Gln Pro Leu Ser Glu
           35                  40                  45
   Glu Gln Leu Ile Asp Ile Lys Val Ala His Pro Ile Arg Pro Lys Pro
       50                  55                  60
   Pro Ser Ala Thr Ser Ile Pro Ala Ile Leu Lys Ala Leu Gln Asp Glu
   65                  70                  75                  80
   Trp Asp Ala Val Met Leu His Ser Phe Thr Leu Arg Gln Gln Leu Gln
                   85                  90                  95
   Thr Thr Arg Gln Glu Leu Ser His Ala Leu Tyr Gln His Asp Ala Ala
               100                 105                 110
   Cys Arg Val Ile Ala Arg Leu Thr Lys Glu Val Thr Ala Ala Arg Glu
           115                 120                 125
   Ala Leu Ala Thr Leu Lys Pro Gln Ala Gly Leu Ile Val Pro Gln Ala
       130                 135                 140
   Val Pro Ser Ser Gln Pro Ser Val Val Gly Ala Gly Glu Pro Met Asp
   145                 150                 155                 160
   Leu Gly Glu Leu Val Gly Met Thr Pro Glu Ile Ile Gln Lys Leu Gln
                   165                 170                 175
   Asp Lys Ala Thr Val Leu Thr Thr Glu Arg Lys Lys Arg Gly Lys Thr
               180                 185                 190
   Val Pro Glu Glu Leu Val Lys Pro Glu Glu Leu Ser Lys Tyr Arg Gln
           195                 200                 205
   Val Ala Ser His Val Gly Leu His Ser Ala Ser Ile Pro Gly Ile Leu
       210                 215                 220
   Ala Leu Asp Leu Cys Pro Ser Asp Thr Asn Lys Ile Leu Thr Gly Gly
   225                 230                 235                 240
   Ala Asp Lys Asn Val Val Val Phe Asp Lys Ser Ser Glu Gln Ile Leu
                   245                 250                 255
   Ala Thr Leu Lys Gly His Thr Lys Lys Val Thr Ser Val Val Phe His
               260                 265                 270
   Pro Ser Gln Asp Leu Val Phe Ser Ala Ser Pro Asp Ala Thr Ile Arg
           275                 280                 285
   Ile Trp Ser Val Pro Asn Ala Ser Cys Val Gln Val Val Arg Ala His
       290                 295                 300
   Glu Ser Ala Val Thr Gly Leu Ser Leu His Ala Thr Gly Asp Tyr Leu
   305                 310                 315                 320
   Leu Ser Ser Ser Asp Asp Gln Tyr Trp Ala Phe Ser Asp Ile Gln Thr
                   325                 330                 335
   Gly Arg Val Leu Thr Lys Val Thr Asp Glu Thr Ser Gly Cys Ser Leu
               340                 345                 350
   Thr Cys Ala Gln Phe His Pro Asp Gly Leu Ile Phe Gly Thr Gly Thr
           355                 360                 365
   Met Asp Ser Gln Ile Lys Ile Trp Asp Leu Lys Glu Arg Thr Asn Val
       370                 375                 380
   Ala Asn Phe Pro Gly His Ser Gly Pro Ile Thr Ser Ile Ala Phe Ser
   385                 390                 395                 400
   Glu Asn Gly Tyr Tyr Leu Ala Thr Ala Ala Asp Asp Ser Ser Val Lys
                   405                 410                 415
   Leu Trp Asp Leu Arg Lys Leu Lys Asn Phe Lys Thr Leu Gln Leu Asp
               420                 425                 430
   Asn Asn Phe Glu Val Lys Ser Leu Ile Phe Asp Gln Ser Gly Thr Tyr
           435                 440                 445
   Leu Ala Leu Gly Gly Thr Asp Val Gln Ile Tyr Ile Cys Lys Gln Trp
       450                 455                 460
   Thr Glu Ile Leu His Phe Thr Glu His Ser Gly Leu Thr Thr Gly Val
   465                 470                 475                 480
   Ala Phe Gly His His Ala Lys Phe Ile Ala Ser Thr Gly Met Asp Arg
                   485                 490                 495
   Ser Leu Lys Phe Tyr Ser Leu
               500

<210>  3

<211>  201

<212>  PRT

<213>  Homo sapiens


<300>

<308>  humangp/chr17-q8tcd5

<309>  2003-04-22


<400>  3

Met Ala Arg Ser Val Arg Val Leu Val Asp Met Asp Gly Val Leu Ala
1               5                   10                  15

Asp Phe Glu Ala Gly Leu Leu Arg Gly Phe Arg Arg Arg Phe Pro Glu
            20                  25                  30

Glu Pro His Val Pro Leu Glu Gln Arg Arg Gly Phe Leu Ala Arg Glu
        35                  40                  45

Gln Tyr Arg Ala Leu Arg Pro Asp Leu Ala Asp Lys Val Ala Ser Val
    50                  55                  60

Tyr Glu Ala Pro Gly Phe Phe Leu Asp Leu Glu Pro Ile Pro Gly Ala
65                  70                  75                  80

Leu Asp Ala Val Arg Glu Met Asn Asp Leu Pro Asp Thr Gln Val Phe
                85                  90                  95

Ile Cys Thr Ser Pro Leu Leu Lys Tyr His His Cys Val Gly Glu Lys
            100                 105                 110

Tyr Arg Trp Val Glu Gln His Leu Gly Pro Gln Phe Val Glu Arg Ile
        115                 120                 125

Ile Leu Thr Arg Asp Lys Thr Val Val Leu Gly Asp Leu Leu Ile Asp
    130                 135                 140

Asp Lys Asp Thr Val Arg Gly Gln Glu Glu Thr Pro Ser Trp Glu His
145                 150                 155                 160

Ile Leu Phe Thr Cys Cys His Asn Arg His Leu Val Leu Pro Pro Thr
                165                 170                 175

Arg Arg Arg Leu Leu Ser Trp Ser Asp Asn Trp Arg Glu Ile Leu Asp
            180                 185                 190

Ser Lys Arg Gly Ala Ala Gln Arg Glu
        195                 200

<210>  4

<211>  277

<212>  PRT

<213>  Homo sapiens

<300>

<308>  humangp/chr19-q9bvl0

<309>  2003-04-22

<400>  4

Met Ala Asn Pro Lys Leu Leu Gly Met Gly Leu Ser Glu Ala Glu Ala
1               5                   10                  15

Ile Gly Ala Asp Ser Ala Arg Phe Glu Glu Leu Leu Leu Gln Ala Ser
            20                  25                  30

Lys Glu Leu Gln Gln Ala Gln Thr Thr Arg Pro Glu Ser Thr Gln Ile
        35                  40                  45

Gln Pro Gln Pro Gly Phe Cys Ile Lys Thr Asn Ser Ser Glu Gly Lys
    50                  55                  60

Val Phe Ile Asn Ile Cys His Ser Pro Ser Ile Pro Pro Pro Ala Asp
65                  70                  75                  80

Val Thr Glu Glu Glu Leu Leu Gln Met Leu Glu Glu Asp Gln Ala Gly
                85                  90                  95

Phe Arg Ile Pro Met Ser Leu Gly Glu Pro His Ala Glu Leu Asp Ala
            100                 105                 110

Lys Gly Gln Gly Cys Thr Ala Tyr Asp Val Ala Val Asn Ser Asp Phe
        115                 120                 125

Tyr Arg Arg Met Gln Asn Ser Asp Phe Leu Arg Glu Leu Val Ile Thr
    130                 135                 140

Ile Ala Arg Glu Gly Leu Glu Asp Lys Tyr Asn Leu Gln Leu Asn Pro
145                 150                 155                 160

Glu Trp Arg Met Met Lys Asn Arg Pro Phe Met Gly Ser Ile Ser Gln
                165                 170                 175

Gln Asn Ile Arg Ser Glu Gln Arg Pro Arg Ile Gln Glu Leu Gly Asp
            180                 185                 190

Leu Tyr Thr Pro Ala Pro Gly Arg Ala Glu Ser Gly Pro Glu Lys Pro
        195                 200                 205

His Leu Asn Leu Trp Leu Glu Ala Pro Asp Leu Leu Leu Ala Glu Val
    210                 215                 220

Asp Leu Pro Lys Leu Asp Gly Ala Leu Gly Leu Ser Leu Glu Ile Gly
225                 230                 235                 240

Glu Asn Arg Leu Val Met Gly Gly Pro Gln Gln Leu Tyr His Leu Asp

                245                 250                 255

Ala Tyr Ile Pro Leu Gln Ile Asn Ser His Glu Ser Lys Ala Ala Phe
            260                 265                 270

His Arg Lys Arg Lys
        275

<210> 5

<211> 224

<212> PRT

<213> Homo sapiens

<300>

<308> humangp/chr1cac22475

<309> 2003-04-22

<400> 5

Gln Leu Gln Lys Asp Leu Glu Glu Val Lys Val Leu Leu Glu Lys Ala
1               5                   10                  15

Thr Arg Lys Arg Val Arg Asp Ala Leu Thr Ala Glu Lys Ser Lys Ile
            20                  25                  30

Glu Thr Glu Ile Lys Asn Lys Met Gln Gln Lys Ser Gln Lys Lys Ala
        35                  40                  45

Glu Leu Leu Asp Asn Glu Lys Pro Ala Ala Val Val Ala Pro Ile Thr
    50                  55                  60

Thr Gly Tyr Thr Val Lys Ile Ser Asn Tyr Gly Trp Asp Gln Ser Asp
65                  70                  75                  80

Lys Phe Val Lys Ile Tyr Ile Thr Leu Thr Gly Val His Gln Val Pro
                85                  90                  95

Thr Glu Asn Val Gln Val His Phe Thr Glu Arg Ser Phe Asp Leu Leu
            100                 105                 110

Val Lys Asn Leu Asn Gly Lys Ser Tyr Ser Met Ile Val Asn Asn Leu
        115                 120                 125

Leu Lys Pro Ile Ser Val Glu Gly Ser Ser Lys Lys Val Lys Thr Asp
    130                 135                 140

Thr Val Leu Ile Leu Cys Arg Lys Lys Val Glu Asn Thr Arg Trp Asp
145                 150                 155                 160

Tyr Leu Thr Gln Val Glu Lys Glu Cys Lys Glu Lys Glu Lys Pro Ser
                165                 170                 175

Tyr Asp Thr Glu Thr Asp Pro Ser Glu Gly Leu Met Asn Val Leu Lys
            180                 185                 190

Lys Ile Tyr Glu Asp Gly Asp Asp Asp Met Lys Arg Thr Ile Asn Lys
        195                 200                 205

Ala Trp Val Glu Ser Arg Glu Lys Gln Ala Lys Gly Asp Thr Glu Phe
    210                 215                 220

<210>  6

<211>  198

<212>  PRT

<213>  Homo sapiens

<300>

<308>  humangp/chr1q9hb71

<309>  2003-04-22

<400>  6

Gln Leu Gln Lys Asp Leu Glu Glu Val Lys Val Leu Leu Glu Lys Ala
1               5                   10                  15

Thr Arg Lys Arg Val Arg Asp Ala Leu Thr Ala Glu Lys Ser Lys Ile
            20                  25                  30

Glu Thr Glu Ile Lys Asn Lys Met Gln Gln Lys Ser Gln Lys Lys Ala
        35                  40                  45

Glu Leu Leu Asp Asn Glu Lys Pro Ala Ala Val Val Ala Pro Ile Thr
    50                  55                  60

Thr Gly Tyr Thr Val Lys Ile Ser Asn Tyr Gly Trp Asp Gln Ser Asp
65                  70                  75                  80

Lys Phe Val Lys Ile Tyr Ile Thr Leu Thr Gly Val His Gln Val Pro
                85                  90                  95

Thr Glu Asn Val Gln Val His Phe Thr Glu Arg Ser Phe Asp Leu Leu
            100                 105                 110

Val Lys Asn Leu Asn Gly Lys Ser Tyr Ser Met Ile Val Asn Asn Leu
        115                 120                 125

Leu Lys Pro Ile Ser Val Glu Gly Ser Ser Lys Lys Val Lys Thr Asp
    130                 135                 140

Thr Val Leu Ile Leu Cys Arg Lys Lys Val Glu Asn Thr Arg Trp Asp
145                 150                 155                 160

Tyr Leu Thr Gln Val Glu Lys Glu Cys Lys Glu Lys Glu Lys Pro Ser
                165                 170                 175

Tyr Asp Thr Glu Thr Asp Pro Ser Glu Gly Leu Met Asn Val Leu Lys
            180                 185                 190

Lys Ile Tyr Glu Asp Gly
        195

<210>  7

<211>  244

<212>  PRT

<213>  Homo sapiens

<300>

<308>  humangp/chr4-q96s43

<309>  2003-04-22

<400>  7

Met Glu Asp Met Asn Glu Tyr Ser Asn Ile Glu Glu Phe Ala Glu Gly
1               5                   10                  15

Ser Lys Ile Asn Ala Ser Lys Asn Gln Gln Asp Asp Gly Lys Met Phe
            20                  25                  30

Ile Gly Gly Leu Ser Trp Asp Thr Ser Lys Lys Asp Leu Thr Glu Tyr
        35                  40                  45

Leu Ser Arg Phe Gly Glu Val Val Asp Cys Thr Ile Lys Thr Asp Pro
    50                  55                  60

Val Thr Gly Arg Ser Arg Gly Phe Gly Phe Val Leu Phe Lys Asp Ala
65                  70                  75                  80

Ala Ser Val Asp Lys Val Leu Glu Leu Lys Glu His Lys Leu Asp Gly
                85                  90                  95

Lys Leu Ile Asp Pro Lys Arg Ala Lys Ala Leu Lys Gly Lys Glu Pro
            100                 105                 110

Pro Lys Lys Val Phe Val Gly Gly Leu Ser Pro Asp Thr Ser Glu Glu
        115                 120                 125

Gln Ile Lys Glu Tyr Phe Gly Ala Phe Gly Glu Ile Glu Asn Ile Glu
    130                 135                 140

Leu Pro Met Asp Thr Lys Thr Asn Glu Arg Arg Gly Phe Cys Phe Ile
145                 150                 155                 160

Thr Tyr Thr Asp Glu Glu Pro Val Lys Lys Leu Leu Glu Ser Arg Tyr
                165                 170                 175

His Gln Ile Gly Ser Gly Lys Cys Glu Ile Lys Val Ala Gln Pro Lys
            180                 185                 190

Glu Val Tyr Arg Gln Gln Gln Gln Gln Gln Lys Gly Gly Arg Gly Ala
        195                 200                 205

Ala Ala Gly Gly Arg Gly Gly Thr Arg Gly Arg Gly Arg Gly Gln Gln
    210                 215                 220

Ser Thr Tyr Gly Lys Ala Ser Arg Gly Gly Gly Asn His Gln Asn Asn
225                 230                 235                 240

Tyr Gln Pro Tyr

<210>  8

<211>  301

<212>  PRT

<213>  Homo sapiens

<300>

<308>  humangp/chr4-q9z130

<309>  2003-04-22

<400>  8

Met Glu Asp Met Asn Glu Tyr Ser Asn Ile Glu Glu Phe Ala Glu Gly
1               5                   10                  15

Ser Lys Ile Asn Ala Ser Lys Asn Gln Gln Asp Asp Gly Lys Met Phe
            20                  25                  30

Ile Gly Gly Leu Ser Trp Asp Thr Ser Lys Lys Asp Leu Thr Glu Tyr
        35                  40                  45

Leu Ser Arg Phe Gly Glu Val Val Asp Cys Thr Ile Lys Thr Asp Pro
    50                  55                  60

Val Thr Gly Arg Ser Arg Gly Phe Gly Phe Val Leu Phe Lys Asp Ala
65                  70                  75                  80

Ala Ser Val Asp Lys Val Leu Glu Leu Lys Glu His Lys Leu Asp Gly
                85                  90                  95

Lys Leu Ile Asp Pro Lys Arg Ala Lys Ala Leu Lys Gly Lys Glu Pro
            100                 105                 110

Pro Lys Lys Val Phe Val Gly Gly Leu Ser Pro Asp Thr Ser Glu Glu
        115                 120                 125

Gln Ile Lys Glu Tyr Phe Gly Ala Phe Gly Glu Ile Glu Asn Ile Glu
    130                 135                 140

Leu Pro Met Asp Thr Lys Thr Asn Glu Arg Arg Gly Phe Cys Phe Ile
145                 150                 155                 160

Thr Tyr Thr Asp Glu Glu Pro Val Lys Lys Leu Leu Glu Ser Arg Tyr
                165                 170                 175

His Gln Ile Gly Ser Gly Lys Cys Glu Ile Lys Val Ala Gln Pro Lys

180                 185                 190

Glu Val Tyr Arg Gln Gln Gln Gln Gln Gln Lys Gly Gly Arg Gly Ala
        195                 200                 205

Ala Ala Gly Gly Arg Gly Gly Thr Arg Gly Arg Gly Arg Gly Gln Gly
    210                 215                 220

Gln Asn Trp Asn Gln Gly Phe Asn Asn Tyr Tyr Asp Gln Gly Tyr Gly
225                 230                 235                 240

Asn Tyr Asn Ser Ala Tyr Gly Gly Asp Gln Asn Tyr Ser Gly Tyr Gly
                245                 250                 255

Gly Tyr Asp Tyr Thr Gly Tyr Asn Tyr Gly Asn Tyr Gly Tyr Gly Gln
            260                 265                 270

Gly Tyr Ala Asp Tyr Ser Gly Gln Gln Ser Thr Tyr Gly Lys Ala Ser
        275                 280                 285

Arg Gly Gly Gly Asn His Gln Asn Asn Tyr Gln Pro Tyr
    290                 295                 300

<210>  9

<211>  884

<212>  PRT

<213>  Homo sapiens

<300>

<308>  chr7-q13122

<309>  2003-04-22

<400>  9

Val Leu Ser Gly Cys Ala Ile Ile Val Arg Gly Gln Pro Arg Gly Gly
1               5                   10                  15

Pro Pro Pro Glu Arg Gln Ile Asn Leu Ser Asn Ile Arg Ala Gly Asn
            20                  25                  30

Leu Ala Arg Arg Ala Ala Ala Thr Gln Pro Asp Ala Lys Asp Thr Pro
        35                  40                  45

Asp Glu Pro Trp Ala Phe Pro Ala Arg Glu Phe Leu Arg Lys Lys Leu
    50                  55                  60

Ile Gly Lys Glu Val Cys Phe Thr Ile Glu Asn Lys Thr Pro Gln Gly
65                  70                  75                  80

Arg Glu Tyr Gly Met Ile Tyr Leu Gly Lys Asp Thr Asn Gly Glu Asn
                85                  90                  95

Ile Ala Glu Ser Leu Val Ala Glu Gly Leu Ala Thr Arg Arg Glu Gly
            100                 105                 110

Met Arg Ala Asn Asn Pro Glu Gln Asn Arg Leu Ser Glu Cys Glu Glu
        115                 120                 125

Gln Ala Lys Ala Ala Lys Lys Gly Met Trp Ser Glu Gly Asn Gly Ser
    130                 135                 140

His Thr Ile Arg Asp Leu Lys Tyr Thr Ile Glu Asn Pro Arg His Phe
145                 150                 155                 160

Val Asp Ser His His Gln Lys Pro Val Asn Ala Ile Ile Glu His Val
                165                 170                 175

Arg Asp Gly Ser Val Val Arg Ala Leu Leu Leu Pro Asp Tyr Tyr Leu
            180                 185                 190

Val Thr Val Met Leu Ser Gly Ile Lys Cys Pro Thr Phe Arg Arg Glu
        195                 200                 205

Ala Asp Gly Ser Glu Thr Pro Glu Pro Phe Ala Ala Glu Ala Lys Phe
    210                 215                 220

Phe Thr Glu Ser Arg Leu Leu Gln Arg Asp Val Gln Ile Ile Leu Glu
225                 230                 235                 240

Ser Cys His Asn Gln Asn Ile Leu Gly Thr Ile Leu His Pro Asn Gly
                245                 250                 255

Asn Ile Thr Glu Leu Leu Leu Lys Glu Gly Phe Ala Arg Cys Val Asp
            260                 265                 270

Trp Ser Ile Ala Val Tyr Thr Arg Gly Ala Glu Lys Leu Arg Ala Ala
        275                 280                 285

Glu Arg Phe Ala Lys Glu Arg Arg Leu Arg Ile Trp Arg Asp Tyr Val
    290                 295                 300

Ala Pro Thr Ala Asn Leu Asp Gln Lys Asp Lys Gln Phe Val Ala Lys
305                 310                 315                 320

Val Met Gln Val Leu Asn Ala Asp Ala Ile Val Val Lys Leu Asn Ser
                325                 330                 335

Gly Asp Tyr Lys Thr Ile His Leu Ser Ser Ile Arg Pro Pro Arg Leu
            340                 345                 350

Glu Gly Glu Asn Thr Gln Asp Lys Asn Lys Lys Leu Arg Pro Leu Tyr
        355                 360                 365

Asp Ile Pro Tyr Met Phe Glu Ala Arg Glu Phe Leu Arg Lys Lys Leu
    370                 375                 380

Ile Gly Lys Lys Val Asn Val Thr Val Asp Tyr Ile Arg Pro Ala Ser
385                 390                 395                 400

Pro Ala Thr Glu Thr Val Pro Ala Phe Ser Glu Arg Thr Cys Ala Thr
                405                 410                 415

Val Thr Ile Gly Gly Ile Asn Ile Ala Glu Ala Leu Val Ser Lys Gly
            420                 425                 430

Leu Ala Thr Val Ile Arg Tyr Arg Gln Asp Asp Asp Gln Arg Ser Ser
        435                 440                 445

His Tyr Asp Glu Leu Leu Ala Ala Glu Ala Arg Ala Ile Lys Asn Gly
    450                 455                 460

Lys Gly Leu His Ser Lys Lys Glu Val Pro Ile His Arg Val Ala Asp
465                 470                 475                 480

Ile Ser Gly Asp Thr Gln Lys Ala Lys Gln Phe Leu Pro Phe Leu Gln
                485                 490                 495

Arg Ala Gly Arg Ser Glu Ala Val Val Glu Tyr Val Phe Ser Gly Ser
            500                 505                 510

Arg Leu Lys Leu Tyr Leu Pro Lys Glu Thr Cys Leu Ile Thr Phe Leu
        515                 520                 525

Leu Ala Gly Ile Glu Cys Pro Arg Gly Ala Arg Asn Leu Pro Gly Leu
    530                 535                 540

Val Gln Glu Gly Glu Pro Phe Ser Glu Glu Ala Thr Leu Phe Thr Lys
545                 550                 555                 560

Glu Leu Val Leu Gln Arg Glu Val Glu Val Glu Val Glu Ser Met Asp
                565                 570                 575

Lys Ala Gly Asn Phe Ile Gly Trp Leu His Ile Asp Gly Ala Asn Leu
            580                 585                 590

Ser Val Leu Leu Val Glu His Ala Leu Ser Lys Val His Phe Thr Ala
        595                 600                 605

Glu Arg Ser Ser Tyr Tyr Lys Ser Leu Leu Ser Ala Glu Glu Ala Ala
    610                 615                 620

Lys Gln Lys Lys Glu Lys Val Trp Ala His Tyr Glu Glu Gln Pro Val
625                 630                 635                 640

Glu Glu Val Met Pro Val Leu Glu Glu Lys Glu Arg Ser Ala Ser Tyr
                645                 650                 655

Lys Pro Val Phe Val Thr Glu Ile Thr Asp Asp Leu His Phe Tyr Val
            660                 665                 670

Gln Asp Val Glu Thr Gly Thr Gln Leu Glu Lys Leu Met Glu Asn Met
        675                 680                 685

Arg Asn Asp Ile Ala Ser His Pro Pro Val Glu Gly Ser Tyr Ala Pro
    690                 695                 700

Arg Arg Gly Glu Phe Cys Ile Ala Lys Phe Val Asp Gly Glu Trp Tyr
705                 710                 715                 720

Arg Ala Arg Val Glu Lys Val Glu Ser Pro Ala Lys Ile His Val Phe
                725                 730                 735

Tyr Ile Asp Tyr Gly Asn Arg Glu Val Leu Pro Ser Thr Arg Leu Gly
            740                 745                 750

Thr Leu Ser Pro Ala Phe Ser Thr Arg Val Leu Pro Ala Gln Ala Thr
        755                 760                 765

Glu Tyr Ala Phe Ala Phe Ile Gln Val Pro Gln Asp Asp Asp Ala Arg
    770                 775                 780

Thr Asp Ala Val Asp Ser Val Val Arg Asp Ile Gln Asn Thr Gln Cys
785                 790                 795                 800

Leu Leu Asn Val Glu His Leu Ser Ala Gly Cys Pro His Val Thr Leu
                805                 810                 815

Gln Phe Ala Asp Ser Lys Gly Asp Val Gly Leu Gly Leu Val Lys Glu
            820                 825                 830

Gly Leu Val Met Val Glu Val Arg Lys Glu Lys Gln Phe Gln Lys Val
        835                 840                 845

Ile Thr Glu Tyr Leu Asn Ala Gln Glu Ser Ala Lys Ser Ala Arg Leu
    850                 855                 860

Asn Leu Trp Arg Tyr Gly Asp Phe Arg Ala Asp Asp Ala Asp Glu Phe
865                 870                 875                 880

Gly Tyr Ser Arg

<210>  10

<211>  355

<212>  PRT

<213>  Homo sapiens

<300>

<308>  humangp/chr9-060376

<309>  2003-04-22

<400>  10

Leu Gln Gly Ser Leu Leu Ala Ser Gly Arg Ala Pro Arg Arg Ala Ser
1               5                   10                  15

Ser Gly Leu Pro Arg Asn Thr Val Val Leu Phe Val Pro Gln Gln Glu
            20                  25                  30

Ala Trp Val Val Glu Arg Met Gly Arg Phe His Arg Ile Leu Glu Pro
        35                  40                  45

Gly Leu Asn Ile Leu Ile Pro Val Leu Asp Arg Ile Arg Tyr Val Gln

    50                  55                  60

Ser Leu Lys Glu Ile Val Ile Asn Val Pro Glu Gln Ser Ala Val Thr
65                  70                  75                  80

Leu Asp Asn Val Thr Leu Gln Ile Asp Gly Val Leu Tyr Leu Arg Ile
                85                  90                  95

Met Asp Pro Tyr Lys Ala Ser Tyr Gly Val Glu Asp Pro Glu Tyr Ala
            100                 105                 110

Val Thr Gln Leu Ala Gln Thr Thr Met Arg Ser Glu Leu Gly Lys Leu
        115                 120                 125

Ser Leu Asp Lys Val Phe Arg Glu Arg Glu Ser Leu Asn Ala Ser Ile
    130                 135                 140

Val Asp Ala Ile Asn Gln Ala Ala Asp Cys Trp Gly Ile Arg Cys Leu
145                 150                 155                 160

Arg Tyr Glu Ile Lys Asp Ile His Val Pro Pro Arg Val Lys Glu Ser
                165                 170                 175

Met Gln Met Gln Val Gly Ala Lys Glu Gly Trp Glu Lys Gly Leu Arg
            180                 185                 190

Val Glu Ala Glu Arg Arg Lys Arg Ala Thr Val Leu Glu Ser Glu Gly
        195                 200                 205

Thr Arg Glu Ser Ala Ile Asn Val Ala Glu Gly Lys Lys Gln Ala Gln
    210                 215                 220

Ile Leu Ala Ser Glu Ala Glu Lys Ala Glu Gln Ile Asn Gln Ala Ala
225                 230                 235                 240

Gly Glu Ala Ser Ala Val Leu Ala Lys Ala Lys Ala Lys Ala Glu Ala
                245                 250                 255

Ile Arg Ile Leu Ala Ala Ala Leu Thr Gln His Asn Gly Asp Ala Ala
            260                 265                 270

Ala Ser Leu Thr Val Ala Glu Gln Tyr Val Ser Ala Phe Ser Lys Leu
        275                 280                 285

Ala Lys Asp Ser Asn Thr Ile Leu Leu Pro Ser Asn Pro Gly Asp Val
    290                 295                 300

Thr Ser Met Val Ala Gln Ala Met Gly Val Tyr Gly Ala Leu Thr Lys
305                 310                 315                 320

Ala Pro Val Pro Gly Thr Pro Asp Ser Leu Ser Ser Gly Ser Ser Arg
                325                 330                 335

Asp Val Gln Gly Thr Asp Ala Ser Leu Asp Glu Glu Leu Asp Arg Val
            340                 345                 350

Lys Met Ser
        355

<210>  11

<211>  406

<212>  PRT

<213>  Homo sapiens


<300>

<308>  humangp/chr9-q9bs26

<309>  2003-04-22


<400>  11

Met His Pro Ala Val Phe Leu Ser Leu Pro Asp Leu Arg Cys Ser Leu
1               5                   10                  15

Leu Leu Leu Val Thr Trp Val Phe Thr Pro Val Thr Thr Glu Ile Thr
            20                  25                  30

Ser Leu Asp Thr Glu Asn Ile Asp Glu Ile Leu Asn Asn Ala Asp Val
        35                  40                  45

Ala Leu Val Asn Phe Tyr Ala Asp Trp Cys Arg Phe Ser Gln Met Leu
    50                  55                  60

His Pro Ile Phe Glu Glu Ala Ser Asp Val Ile Lys Glu Glu Phe Pro
65                  70                  75                  80

Asn Glu Asn Gln Val Val Phe Ala Arg Val Asp Cys Asp Gln His Ser
                85                  90                  95

Asp Ile Ala Gln Arg Tyr Arg Ile Ser Lys Tyr Pro Thr Leu Lys Leu
            100                 105                 110

Phe Arg Asn Gly Met Met Met Lys Arg Glu Tyr Arg Gly Gln Arg Ser
        115                 120                 125

Val Lys Ala Leu Ala Asp Tyr Ile Arg Gln Gln Lys Ser Asp Pro Ile
    130                 135                 140

Gln Glu Ile Arg Asp Leu Ala Glu Ile Thr Thr Leu Asp Arg Ser Lys
145                 150                 155                 160

Arg Asn Ile Ile Gly Tyr Phe Glu Gln Lys Asp Ser Asp Asn Tyr Arg
                165                 170                 175

Val Phe Glu Arg Val Ala Asn Ile Leu His Asp Asp Cys Ala Phe Leu
            180                 185                 190

Ser Ala Phe Gly Asp Val Ser Lys Pro Glu Arg Tyr Ser Gly Asp Asn
        195                 200                 205

Ile Ile Tyr Lys Pro Pro Gly His Ser Ala Pro Asp Met Val Tyr Leu
    210                 215                 220

Gly Ala Met Thr Asn Phe Asp Val Thr Tyr Asn Trp Ile Gln Asp Lys
225                 230                 235                 240

Cys Val Pro Leu Val Arg Glu Ile Thr Phe Glu Asn Gly Glu Glu Leu
                245                 250                 255

Thr Glu Glu Gly Leu Pro Phe Leu Ile Leu Phe His Met Lys Glu Asp
            260                 265                 270

Thr Glu Ser Leu Glu Ile Phe Gln Asn Glu Val Ala Arg Gln Leu Ile
        275                 280                 285

Ser Glu Lys Gly Thr Ile Asn Phe Leu His Ala Asp Cys Asp Lys Phe
    290                 295                 300

Arg His Pro Leu Leu His Ile Gln Lys Thr Pro Ala Asp Cys Pro Val
305                 310                 315                 320

Ile Ala Ile Asp Ser Phe Arg His Met Tyr Val Phe Gly Asp Phe Lys
                325                 330                 335

Asp Val Leu Ile Pro Gly Lys Leu Lys Gln Phe Val Phe Asp Leu His
            340                 345                 350

Ser Gly Lys Leu His Arg Glu Phe His His Gly Pro Asp Pro Thr Asp
        355                 360                 365

Thr Ala Pro Gly Glu Gln Ala Gln Asp Val Ala Ser Ser Pro Pro Glu
    370                 375                 380

Ser Ser Phe Gln Lys Leu Ala Pro Ser Glu Tyr Arg Tyr Thr Leu Leu
385                 390                 395                 400

Arg Asp Arg Asp Glu Leu
                405

<210>  12

<211>  323

<212>  PRT

<213>  Homo sapiens

<300>

<308>  sw_hum/akc1_human

<309>  1993-10-01

<400>  12

Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Thr Lys Leu Ala Ile Glu Ala Gly Phe Arg His
        35                  40                  45

Ile Asp Ser Ala His Leu Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Cys Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Val Glu Lys Cys Lys Asp Ala Gly Leu Ala
145                 150                 155                 160

Lys Ser Ile Gly Val Ser Asn Phe Asn Arg Arg Gln Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val
225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr

<210>  13

<211>  323

<212>  PRT

<213>  Homo sapiens

<300>

<308>  sw_hum/ack2_human

<309>  1996-10-01

<400>  13

Met Asp Ser Lys Tyr Gln Cys Val Lys Leu Asn Asp Gly His Phe Met
1               5                   10                  15

Pro Val Leu Gly Phe Gly Thr Tyr Ala Pro Ala Glu Val Pro Lys Ser
            20                  25                  30

Lys Ala Leu Glu Ala Val Lys Leu Ala Ile Glu Ala Gly Phe His His
        35                  40                  45

Ile Asp Ser Ala His Val Tyr Asn Asn Glu Glu Gln Val Gly Leu Ala
    50                  55                  60

Ile Arg Ser Lys Ile Ala Asp Gly Ser Val Lys Arg Glu Asp Ile Phe
65                  70                  75                  80

Tyr Thr Ser Lys Leu Trp Ser Asn Ser His Arg Pro Glu Leu Val Arg
                85                  90                  95

Pro Ala Leu Glu Arg Ser Leu Lys Asn Leu Gln Leu Asp Tyr Val Asp
            100                 105                 110

Leu Tyr Leu Ile His Phe Pro Val Ser Val Lys Pro Gly Glu Glu Val
        115                 120                 125

Ile Pro Lys Asp Glu Asn Gly Lys Ile Leu Phe Asp Thr Val Asp Leu
    130                 135                 140

Cys Ala Thr Trp Glu Ala Met Glu Lys Cys Lys Asp Ala Gly Leu Ala
145                 150                 155                 160

Lys Ser Ile Gly Val Ser Asn Phe Asn His Arg Leu Leu Glu Met Ile
                165                 170                 175

Leu Asn Lys Pro Gly Leu Lys Tyr Lys Pro Val Cys Asn Gln Val Glu
            180                 185                 190

Cys His Pro Tyr Phe Asn Gln Arg Lys Leu Leu Asp Phe Cys Lys Ser
        195                 200                 205

Lys Asp Ile Val Leu Val Ala Tyr Ser Ala Leu Gly Ser His Arg Glu
    210                 215                 220

Glu Pro Trp Val Asp Pro Asn Ser Pro Val Leu Leu Glu Asp Pro Val

225                 230                 235                 240

Leu Cys Ala Leu Ala Lys Lys His Lys Arg Thr Pro Ala Leu Ile Ala
                245                 250                 255

Leu Arg Tyr Gln Leu Gln Arg Gly Val Val Val Leu Ala Lys Ser Tyr
            260                 265                 270

Asn Glu Gln Arg Ile Arg Gln Asn Val Gln Val Phe Glu Phe Gln Leu
        275                 280                 285

Thr Ser Glu Glu Met Lys Ala Ile Asp Gly Leu Asn Arg Asn Val Arg
    290                 295                 300

Tyr Leu Thr Leu Asp Ile Phe Ala Gly Pro Pro Asn Tyr Pro Phe Ser
305                 310                 315                 320

Asp Glu Tyr

<210>  14

<211>  363

<212>  PRT

<213>  Homo sapiens

<300>

<308>  sw_hum/alfc_human

<309>  1989-03-01

<400>  14

Pro His Ser Tyr Pro Ala Leu Ser Ala Glu Gln Lys Lys Glu Leu Ser
1               5                   10                  15

Asp Ile Ala Leu Arg Ile Val Ala Pro Gly Lys Gly Ile Leu Ala Ala
            20                  25                  30

Asp Glu Ser Val Gly Ser Met Ala Lys Arg Leu Ser Gln Ile Gly Val
        35                  40                  45

Glu Asn Thr Glu Glu Asn Arg Arg Leu Tyr Arg Gln Val Leu Phe Ser
    50                  55                  60

Ala Asp Asp Arg Val Lys Lys Cys Ile Gly Gly Val Ile Phe Phe His
65                  70                  75                  80

Glu Thr Leu Tyr Gln Lys Asp Asp Asn Gly Val Pro Phe Val Arg Thr
                85                  90                  95

Ile Gln Asp Lys Gly Ile Val Val Gly Ile Lys Val Asp Lys Gly Val
            100                 105                 110

Val Pro Leu Ala Gly Thr Asp Gly Glu Thr Thr Thr Gln Gly Leu Asp
        115                 120                 125

Gly Leu Ser Glu Arg Cys Ala Gln Tyr Lys Lys Asp Gly Ala Asp Phe
    130                 135                 140

Ala Lys Trp Arg Cys Val Leu Lys Ile Ser Glu Arg Thr Pro Ser Ala
145                 150                 155                 160

Leu Ala Ile Leu Glu Asn Ala Asn Val Leu Ala Arg Tyr Ala Ser Ile
                165                 170                 175

Cys Gln Gln Asn Gly Ile Val Pro Ile Val Glu Pro Glu Ile Leu Pro
            180                 185                 190

Asp Gly Asp His Asp Leu Lys Arg Cys Gln Tyr Val Thr Glu Lys Val
        195                 200                 205

Leu Ala Ala Val Tyr Lys Ala Leu Ser Asp His His Val Tyr Leu Glu
    210                 215                 220

Gly Thr Leu Leu Lys Pro Asn Met Val Thr Pro Gly His Ala Cys Pro
225                 230                 235                 240

Ile Lys Tyr Thr Pro Glu Glu Ile Ala Met Ala Thr Val Thr Ala Leu
                245                 250                 255

Arg Arg Thr Val Pro Pro Ala Val Pro Gly Val Thr Phe Leu Ser Gly
            260                 265                 270

Gly Gln Ser Glu Glu Glu Ala Ser Phe Asn Leu Asn Ala Ile Asn Arg
        275                 280                 285

Cys Pro Leu Pro Arg Pro Trp Ala Leu Thr Phe Ser Tyr Gly Arg Ala
    290                 295                 300

Leu Gln Ala Ser Ala Leu Asn Ala Trp Arg Gly Gln Arg Asp Asn Ala
305                 310                 315                 320

Gly Ala Ala Thr Glu Glu Phe Ile Lys Arg Ala Glu Val Asn Gly Leu
                325                 330                 335

Ala Ala Gln Gly Lys Tyr Glu Gly Ser Gly Glu Asp Gly Gly Ala Ala
            340                 345                 350

Ala Gln Ser Leu Tyr Ile Ala Asn His Ala Tyr
        355                 360

<210>  15

<211>  179

<212>  PRT

<213>  Homo sapiens


<300>

<308>  sw_hum/apt_human

<309>  1988-08-01

<400>  15

Ala Asp Ser Glu Leu Gln Leu Val Glu Gln Arg Ile Arg Ser Phe Pro
1               5                   10                  15

Asp Phe Pro Thr Pro Gly Val Val Phe Arg Asp Ile Ser Pro Val Leu
            20                  25                  30

Lys Asp Pro Ala Ser Phe Arg Ala Ala Ile Gly Leu Leu Ala Arg His
        35                  40                  45

Leu Lys Ala Thr His Gly Gly Arg Ile Asp Tyr Ile Ala Gly Leu Asp
    50                  55                  60

Ser Arg Gly Phe Leu Phe Gly Pro Ser Leu Ala Gln Glu Leu Gly Leu
65                  70                  75                  80

Gly Cys Val Leu Ile Arg Lys Arg Gly Lys Leu Pro Gly Pro Thr Leu
                85                  90                  95

Trp Ala Ser Tyr Ser Leu Glu Tyr Gly Lys Ala Glu Leu Glu Ile Gln
            100                 105                 110

Lys Asp Ala Leu Glu Pro Gly Gln Arg Val Val Val Val Asp Asp Leu
        115                 120                 125

Leu Ala Thr Gly Gly Thr Met Asn Ala Ala Cys Glu Leu Leu Gly Arg
    130                 135                 140

Leu Gln Ala Glu Val Leu Glu Cys Val Ser Leu Val Glu Leu Thr Ser
145                 150                 155                 160

Leu Lys Gly Arg Glu Lys Leu Ala Pro Val Pro Phe Phe Ser Leu Leu
                165                 170                 175

Gln Tyr Glu

<210>  16

<211>  322

<212>  PRT

<213>  Homo sapiens

<300>

<308>  sw_hum/argi_human

<309>  1987-08-13

<400>  16

<210> 17

<211> 429

<212> PRT

<213> Homo sapiens

<300>

<308> sw_hum/b53a_human

<309> 2003-02-28

<400> 17

Met Ser Gly Gly Val Tyr Gly Gly Asp Glu Val Gly Ala Leu Val Phe
1               5                   10                  15

Asp Ile Gly Ser Tyr Thr Val Arg Ala Gly Tyr Ala Gly Glu Asp Cys
            20                  25                  30

Pro Lys Val Asp Phe Pro Thr Ala Ile Gly Met Val Val Glu Arg Asp
        35                  40                  45

Asp Gly Ser Thr Leu Met Glu Ile Asp Gly Asp Lys Gly Lys Gln Gly
    50                  55                  60

Gly Pro Thr Tyr Tyr Ile Asp Thr Asn Ala Leu Arg Val Pro Arg Glu
65                  70                  75                  80

Asn Met Glu Ala Ile Ser Pro Leu Lys Asn Gly Met Val Glu Asp Trp
                85                  90                  95

Asp Ser Phe Gln Ala Ile Leu Asp His Thr Tyr Lys Met His Val Lys
            100                 105                 110

Ser Glu Ala Ser Leu His Pro Val Leu Met Ser Glu Ala Pro Trp Asn
        115                 120                 125

Thr Arg Ala Lys Arg Glu Lys Leu Thr Glu Leu Met Phe Glu His Tyr
    130                 135                 140

Asn Ile Pro Ala Phe Phe Leu Cys Lys Thr Ala Val Leu Thr Ala Phe
145                 150                 155                 160

Ala Asn Gly Arg Ser Thr Gly Leu Ile Leu Asp Ser Gly Ala Thr His
                165                 170                 175

Thr Thr Ala Ile Pro Val His Asp Gly Tyr Val Leu Gln Gln Gly Ile
            180                 185                 190

Val Lys Ser Pro Leu Ala Gly Asp Phe Ile Thr Met Gln Cys Arg Glu
        195                 200                 205

Leu Phe Gln Glu Met Asn Ile Glu Leu Val Pro Pro Tyr Met Ile Ala

210                 215                 220

Ser Lys Glu Ala Val Arg Glu Gly Ser Pro Ala Asn Trp Lys Arg Lys
225                 230                 235                 240

Glu Lys Leu Pro Gln Val Thr Arg Ser Trp His Asn Tyr Met Cys Asn
                245                 250                 255

Cys Val Ile Gln Asp Phe Gln Ala Ser Val Leu Gln Val Ser Asp Ser
            260                 265                 270

Thr Tyr Asp Glu Gln Val Ala Ala Gln Met Pro Thr Val His Tyr Glu
        275                 280                 285

Phe Pro Asn Gly Tyr Asn Cys Asp Phe Gly Ala Glu Arg Leu Lys Ile
    290                 295                 300

Pro Glu Gly Leu Phe Asp Pro Ser Asn Val Lys Gly Leu Ser Gly Asn
305                 310                 315                 320

Thr Met Leu Gly Val Ser His Val Val Thr Thr Ser Val Gly Met Cys
                325                 330                 335

Asp Ile Asp Ile Arg Pro Gly Leu Tyr Gly Ser Val Ile Val Ala Gly
            340                 345                 350

Gly Asn Thr Leu Ile Gln Ser Phe Thr Asp Arg Leu Asn Arg Glu Leu
        355                 360                 365

Ser Gln Lys Thr Pro Pro Ser Met Arg Leu Lys Leu Ile Ala Asn Asn
    370                 375                 380

Thr Thr Val Glu Arg Arg Phe Ser Ser Trp Ile Gly Gly Ser Ile Leu
385                 390                 395                 400

Ala Ser Leu Gly Thr Phe Gln Gln Met Trp Ile Ser Lys Gln Glu Tyr
                405                 410                 415

Glu Glu Gly Gly Lys Gln Cys Val Glu Arg Lys Cys Pro
            420                 425

<210>  18

<211>  339

<212>  PRT

<213>  Homo sapiens


<300>

<308>  sw_human/catb_human

<309>  1988-08-01


<400>  18

<210>  19

<211>  338

<212>  PRT

<213>  Homo sapiens

<300>

<308>  sw_hum/cn03_human

<309>  2001-10-16

<400>  19

Met Ala Lys Trp Gly Glu Gly Asp Pro Arg Trp Ile Val Glu Glu Arg
1               5                   10                  15

Ala Asp Ala Thr Asn Val Asn Asn Trp His Trp Thr Glu Arg Asp Ala
            20                  25                  30

Ser Asn Trp Ser Thr Asp Lys Leu Lys Thr Leu Phe Leu Ala Val Gln
        35                  40                  45

Val Gln Asn Glu Glu Gly Lys Cys Glu Val Thr Glu Val Ser Lys Leu
    50                  55                  60

Asp Gly Glu Ala Ser Ile Asn Asn Arg Lys Gly Lys Leu Ile Phe Phe
65                  70                  75                  80

Tyr Glu Trp Ser Val Lys Leu Asn Trp Thr Gly Thr Ser Lys Ser Gly
                85                  90                  95

Val Gln Tyr Lys Gly His Val Glu Ile Pro Asn Leu Ser Asp Glu Asn
            100                 105                 110

Ser Val Asp Glu Val Glu Ile Ser Val Ser Leu Ala Lys Asp Glu Pro
        115                 120                 125

Asp Thr Asn Leu Val Ala Leu Met Lys Glu Glu Gly Val Lys Leu Leu
    130                 135                 140

Arg Glu Ala Met Gly Ile Tyr Ile Ser Thr Leu Lys Thr Glu Phe Thr
145                 150                 155                 160

Gln Gly Met Ile Leu Pro Thr Met Asn Gly Glu Ser Val Asp Pro Val
                165                 170                 175

Gly Gln Pro Ala Leu Lys Thr Glu Glu Arg Lys Ala Lys Pro Ala Pro
            180                 185                 190

Ser Lys Thr Gln Ala Arg Pro Val Gly Val Lys Ile Pro Thr Cys Lys
        195                 200                 205

Ile Thr Leu Lys Glu Thr Phe Leu Thr Ser Pro Glu Glu Leu Tyr Arg
    210                 215                 220

Val Phe Thr Thr Gln Glu Leu Val Gln Ala Phe Thr His Ala Pro Ala
225                 230                 235                 240

Thr Leu Glu Ala Asp Arg Gly Gly Lys Phe His Met Val Asp Gly Asn
                245                 250                 255

Val Ser Gly Glu Phe Thr Asp Leu Val Pro Glu Lys His Ile Val Met
            260                 265                 270

Lys Trp Arg Phe Lys Ser Trp Pro Glu Gly His Phe Ala Thr Ile Thr
        275                 280                 285

Leu Thr Phe Ile Asp Lys Asn Gly Glu Thr Glu Leu Cys Met Glu Gly
    290                 295                 300

Arg Gly Ile Pro Ala Pro Glu Glu Glu Arg Thr Arg Gln Gly Trp Gln
305                 310                 315                 320

Arg Tyr Tyr Phe Glu Gly Ile Lys Gln Thr Phe Gly Tyr Gly Ala Arg
                325                 330                 335

Leu Phe

<210>  20

<211>  166

<212>  PRT

<213>  Homo sapiens

<300>

<308>  sw_hum/cof1_human

<309>  1991-11-01

<400>  20

Met Ala Ser Gly Val Ala Val Ser Asp Gly Val Ile Lys Val Phe Asn
1               5                   10                  15

Asp Met Lys Val Arg Lys Ser Ser Thr Pro Glu Glu Val Lys Lys Arg
            20                  25                  30

Lys Lys Ala Val Leu Phe Cys Leu Ser Glu Asp Lys Lys Asn Ile Ile
        35                  40                  45

Leu Glu Glu Gly Lys Glu Ile Leu Val Gly Asp Val Gly Gln Thr Val
    50                  55                  60

Asp Asp Pro Tyr Ala Thr Phe Val Lys Met Leu Pro Asp Lys Asp Cys

65                  70                  75                  80

Arg Tyr Ala Leu Tyr Asp Ala Thr Tyr Glu Thr Lys Glu Ser Lys Lys
                85                  90                  95

Glu Asp Leu Val Phe Ile Phe Trp Ala Pro Glu Ser Ala Pro Leu Lys
            100                 105                 110

Ser Lys Met Ile Tyr Ala Ser Ser Lys Asp Ala Ile Lys Lys Lys Leu
        115                 120                 125

Thr Gly Ile Lys His Glu Leu Gln Ala Asn Cys Tyr Glu Glu Val Lys
    130                 135                 140

Asp Arg Cys Thr Leu Ala Glu Lys Leu Gly Gly Ser Ala Val Ile Ser
145                 150                 155                 160

Leu Glu Gly Lys Pro Leu
                165

<210>  21

<211>  307

<212>  PRT

<213>  Homo sapiens

<300>

<308>  sw_hum/cope_human

<309>  2000-05-30

<400>  21

Ala Pro Pro Ala Pro Gly Pro Ala Ser Gly Gly Ser Gly Glu Val Asp
1               5                   10                  15

Glu Leu Phe Asp Val Lys Asn Ala Phe Tyr Ile Gly Ser Tyr Gln Gln
            20                  25                  30

Cys Ile Asn Glu Ala Gln Arg Val Lys Leu Ser Ser Pro Glu Arg Asp
        35                  40                  45

Val Glu Arg Asp Val Phe Leu Tyr Arg Ala Tyr Leu Ala Gln Arg Lys
    50                  55                  60

Phe Gly Val Val Leu Asp Glu Ile Lys Pro Ser Ser Ala Pro Glu Leu
65                  70                  75                  80

Gln Ala Val Arg Met Phe Ala Asp Tyr Leu Ala His Glu Ser Arg Arg
                85                  90                  95

Asp Ser Ile Val Ala Glu Leu Asp Arg Glu Met Ser Arg Ser Val Asp
            100                 105                 110

Val Thr Asn Thr Thr Phe Leu Leu Met Ala Ala Ser Ile Tyr Leu His
        115                 120                 125

Asp Gln Asn Pro Asp Ala Ala Leu Arg Ala Leu His Gln Gly Asp Ser
    130                 135                 140

Leu Glu Cys Thr Ala Met Thr Val Gln Ile Leu Leu Lys Leu Asp Arg
145                 150                 155                 160

Leu Asp Leu Ala Arg Lys Glu Leu Lys Arg Met Gln Asp Leu Asp Glu
                165                 170                 175

Asp Ala Thr Leu Thr Gln Leu Ala Thr Ala Trp Val Ser Leu Ala Thr
            180                 185                 190

Gly Gly Glu Lys Leu Gln Asp Ala Tyr Tyr Ile Phe Gln Glu Met Ala
        195                 200                 205

Asp Lys Cys Ser Pro Thr Leu Leu Leu Leu Asn Gly Gln Ala Ala Cys
    210                 215                 220

His Met Ala Gln Gly Arg Trp Glu Ala Ala Glu Gly Leu Leu Gln Glu
225                 230                 235                 240

Ala Leu Asp Lys Asp Ser Gly Tyr Pro Glu Thr Leu Val Asn Leu Ile
                245                 250                 255

Val Leu Ser Gln His Leu Gly Lys Pro Pro Glu Val Thr Asn Arg Tyr
            260                 265                 270

Leu Ser Gln Leu Lys Asp Ala His Arg Ser His Pro Phe Ile Lys Glu
        275                 280                 285

Tyr Gln Ala Lys Glu Asn Asp Phe Asp Arg Leu Val Leu Gln Tyr Ala
    290                 295                 300

Pro Ser Ala
305

<210>  22

<211>  165

<212>  PRT

<213>  Homo sapiens

<300>

<308>  sw_hum/dest_human

<309>  1990-11-01

<400>  22

Met Ala Ser Gly Val Gln Val Ala Asp Glu Val Cys Arg Ile Phe Tyr
1               5                   10                  15

Asp Met Lys Val Arg Lys Cys Ser Thr Pro Glu Glu Ile Lys Lys Arg
            20                  25                  30

Lys Lys Ala Val Ile Phe Cys Leu Ser Ala Asp Lys Lys Cys Ile Ile
        35                  40                  45

Val Glu Glu Gly Lys Glu Ile Leu Val Gly Asp Val Gly Val Thr Ile
    50                  55                  60

Thr Asp Pro Phe Lys His Phe Val Gly Met Leu Pro Glu Lys Asp Cys
65                  70                  75                  80

Arg Tyr Ala Leu Tyr Asp Ala Ser Phe Glu Thr Lys Glu Ser Arg Lys
                85                  90                  95

Glu Glu Leu Met Phe Phe Leu Trp Ala Pro Glu Leu Ala Pro Leu Lys
            100                 105                 110

Ser Lys Met Ile Tyr Ala Ser Ser Lys Asp Ala Ile Lys Lys Lys Phe
        115                 120                 125

Gln Gly Ile Lys His Glu Cys Gln Ala Asn Gly Pro Glu Asp Leu Asn
    130                 135                 140

Arg Ala Cys Ile Ala Glu Lys Leu Gly Gly Ser Leu Ile Val Ala Phe
145                 150                 155                 160

Glu Gly Cys Pro Val
                165

<210>  23

<211>  517

<212>  PRT

<213>  Homo sapiens


<300>

<308>  sw_hum/dha5_human

<309>  1993-07-01


<400>  23

Met Leu Arg Phe Leu Ala Pro Arg Leu Leu Ser Leu Gln Gly Arg Thr
1               5                   10                  15

Ala Leu Tyr Ser Ser Ala Ala Ala Leu Pro Ser Pro Ile Leu Asn Pro
            20                  25                  30

Asp Ile Pro Tyr Asn Gln Leu Phe Ile Asn Asn Glu Trp Gln Asp Ala
        35                  40                  45

Val Ser Lys Lys Thr Phe Pro Thr Val Asn Pro Thr Thr Gly Glu Val
    50                  55                  60

Ile Gly His Val Ala Glu Gly Asp Arg Ala Asp Val Asp Arg Ala Val
65                  70                  75                  80

Lys Ala Ala Arg Glu Ala Phe Arg Leu Gly Ser Pro Trp Arg Arg Met
                85                  90                  95

Asp Ala Ser Glu Arg Gly Arg Leu Leu Asn Leu Leu Ala Asp Leu Val
            100                 105                 110

Glu Arg Asp Arg Val Tyr Leu Ala Ser Leu Glu Thr Leu Asp Asn Gly
        115                 120                 125

Lys Pro Phe Gln Glu Ser Tyr Ala Leu Asp Leu Asp Glu Val Ile Lys
    130                 135                 140

Val Tyr Arg Tyr Phe Ala Gly Trp Ala Asp Lys Trp His Gly Lys Thr
145                 150                 155                 160

Ile Pro Met His Gly Gln His Phe Cys Phe Thr Arg His Glu Pro Val
                165                 170                 175

Gly Val Cys Gly Gln Ile Ile Pro Trp Asn Phe Pro Leu Val Met Gln
            180                 185                 190

Gly Trp Lys Leu Ala Pro Ala Leu Ala Thr Gly Asn Thr Val Val Met
        195                 200                 205

Lys Val Ala Glu Gln Thr Pro Leu Ser Ala Leu Tyr Leu Ala Ser Leu
    210                 215                 220

Ile Lys Glu Ala Gly Phe Pro Pro Gly Val Val Asn Ile Ile Thr Gly
225                 230                 235                 240

Tyr Gly Pro Thr Ala Gly Ala Ala Ile Ala Gln His Met Asp Val Asp
                245                 250                 255

Lys Val Ala Phe Thr Gly Ser Thr Glu Val Gly His Leu Ile Gln Lys
            260                 265                 270

Ala Ala Gly Asp Ser Asn Leu Lys Arg Val Thr Leu Glu Leu Gly Gly
        275                 280                 285

Lys Ser Pro Ser Ile Val Leu Ala Asp Ala Asp Met Glu His Ala Val
    290                 295                 300

Glu Gln Cys His Glu Ala Leu Phe Phe Asn Met Gly Gln Cys Cys Cys
305                 310                 315                 320

Ala Gly Ser Arg Thr Phe Val Glu Glu Ser Ile Tyr Asn Glu Phe Leu
                325                 330                 335

Glu Arg Thr Val Glu Lys Ala Lys Gln Arg Lys Val Gly Asn Pro Phe
            340                 345                 350

Glu Leu Asp Thr Gln Gln Gly Pro Gln Val Asp Lys Glu Gln Phe Glu
        355                 360                 365

Arg Val Leu Gly Tyr Ile Gln Leu Gly Gln Lys Glu Gly Ala Lys Leu
    370                 375                 380

Leu Cys Gly Gly Glu Arg Phe Gly Glu Arg Gly Phe Phe Ile Lys Pro

385                 390                 395                 400

Thr Val Phe Gly Gly Val Gln Asp Asp Met Arg Ile Ala Lys Glu Glu
                405                 410                 415

Ile Phe Gly Pro Val Gln Pro Leu Phe Lys Phe Lys Lys Ile Glu Glu
            420                 425                 430

Val Val Glu Arg Ala Asn Asn Thr Arg Tyr Gly Leu Ala Ala Ala Val
        435                 440                 445

Phe Thr Arg Asp Leu Asp Lys Ala Met Tyr Phe Thr Gln Ala Leu Gln
    450                 455                 460

Ala Gly Thr Val Trp Val Asn Thr Tyr Asn Ile Val Thr Cys His Thr
465                 470                 475                 480

Pro Phe Gly Gly Phe Lys Glu Ser Gly Asn Gly Arg Glu Leu Gly Glu
                485                 490                 495

Asp Gly Leu Lys Ala Tyr Thr Glu Val Lys Thr Val Thr Ile Lys Val
            500                 505                 510

Pro Gln Lys Asn Ser
        515

<210>  24

<211>  274

<212>  PRT

<213>  Homo sapiens

<300>

<308>  sw_hum/dhqu_human

<309>  1990-04-01

<400>  24

<210>  25

<211>  737

<212>  PRT

<213>  Homo sapiens

<300>

<308>  sw_hum/dpp3_human

<309>  2001-10-16

<400>  25

Met Ala Asp Thr Gln Tyr Ile Leu Pro Asn Asp Ile Gly Val Ser Ser
1               5                   10                  15

Leu Asp Cys Arg Glu Ala Phe Arg Leu Leu Ser Pro Thr Glu Arg Leu
            20                  25                  30

Tyr Ala Tyr His Leu Ser Arg Ala Ala Trp Tyr Gly Gly Leu Ala Val
        35                  40                  45

Leu Leu Gln Thr Ser Pro Glu Ala Pro Tyr Ile Tyr Ala Leu Leu Ser
    50                  55                  60

Arg Leu Phe Arg Ala Gln Asp Pro Asp Gln Leu Arg Gln His Ala Leu
65                  70                  75                  80

Ala Glu Gly Leu Thr Glu Glu Glu Tyr Gln Ala Phe Leu Val Tyr Ala
                85                  90                  95

Ala Gly Val Tyr Ser Asn Met Gly Asn Tyr Lys Ser Phe Gly Asp Thr
            100                 105                 110

Lys Phe Val Pro Asn Leu Pro Lys Glu Lys Leu Glu Arg Val Ile Leu
        115                 120                 125

Gly Ser Glu Ala Ala Gln Gln His Pro Glu Glu Val Arg Gly Leu Trp
    130                 135                 140

Gln Thr Cys Gly Glu Leu Met Phe Ser Leu Glu Pro Arg Leu Arg His
145                 150                 155                 160

Leu Gly Leu Gly Lys Glu Gly Ile Thr Thr Tyr Phe Ser Gly Asn Cys
                165                 170                 175

Thr Met Glu Asp Ala Lys Leu Ala Gln Asp Phe Leu Asp Ser Gln Asn
            180                 185                 190

Leu Ser Ala Tyr Asn Thr Arg Leu Phe Lys Glu Val Asp Gly Glu Gly
        195                 200                 205

Lys Pro Tyr Tyr Glu Val Arg Leu Ala Ser Val Leu Gly Ser Glu Pro
    210                 215                 220

Ser Leu Asp Ser Glu Val Thr Ser Lys Leu Lys Ser Tyr Glu Phe Arg
225                 230                 235                 240

Gly Ser Pro Phe Gln Val Thr Arg Gly Asp Tyr Ala Pro Ile Leu Gln
                245                 250                 255

Lys Val Val Glu Gln Leu Glu Lys Ala Lys Ala Tyr Ala Ala Asn Ser
            260                 265                 270

His Gln Gly Gln Met Leu Ala Gln Tyr Ile Glu Ser Phe Thr Gln Gly
        275                 280                 285

Ser Ile Glu Ala His Lys Arg Gly Ser Arg Phe Trp Ile Gln Asp Lys
    290                 295                 300

Gly Pro Ile Val Glu Ser Tyr Ile Gly Phe Ile Glu Ser Tyr Arg Asp
305                 310                 315                 320

Pro Phe Gly Ser Arg Gly Glu Phe Glu Gly Phe Val Ala Val Val Asn
                325                 330                 335

Lys Ala Met Ser Ala Lys Phe Glu Arg Leu Val Ala Ser Ala Glu Gln
            340                 345                 350

Leu Leu Lys Glu Leu Pro Trp Pro Pro Thr Phe Glu Lys Asp Lys Phe
        355                 360                 365

Leu Thr Pro Asp Phe Thr Ser Leu Asp Val Leu Thr Phe Ala Gly Ser
    370                 375                 380

Gly Ile Pro Ala Gly Ile Asn Ile Pro Asn Tyr Asp Asp Leu Arg Gln
385                 390                 395                 400

Thr Glu Gly Phe Lys Asn Val Ser Leu Gly Asn Val Leu Ala Val Ala
                405                 410                 415

Tyr Ala Thr Gln Arg Glu Lys Leu Thr Phe Leu Glu Glu Asp Asp Lys
            420                 425                 430

Asp Leu Tyr Ile Leu Trp Lys Gly Pro Ser Phe Asp Val Gln Val Gly
        435                 440                 445

Leu His Glu Leu Leu Gly His Gly Ser Gly Lys Leu Phe Val Gln Asp
    450                 455                 460

Glu Lys Gly Ala Phe Asn Phe Asp Gln Glu Thr Val Ile Asn Pro Glu
465                 470                 475                 480

Thr Gly Glu Gln Ile Gln Ser Trp Tyr Arg Ser Gly Glu Thr Trp Asp
                485                 490                 495

Ser Lys Phe Ser Thr Ile Ala Ser Ser Tyr Glu Glu Cys Arg Ala Glu
            500                 505                 510

Ser Val Gly Leu Tyr Leu Cys Leu His Pro Gln Val Leu Glu Ile Phe
        515                 520                 525

Gly Phe Glu Gly Ala Asp Ala Glu Asp Val Ile Tyr Val Asn Trp Leu
    530                 535                 540

Asn Met Val Arg Ala Gly Leu Leu Ala Leu Glu Phe Tyr Thr Pro Glu
545                 550                 555                 560

Ala Phe Asn Trp Arg Gln Ala His Met Gln Ala Arg Phe Val Ile Leu
                565                 570                 575

Arg Val Leu Leu Glu Ala Gly Glu Gly Leu Val Thr Ile Thr Pro Thr
            580                 585                 590

Thr Gly Ser Asp Gly Arg Pro Asp Ala Arg Val Arg Leu Asp Arg Ser
        595                 600                 605

Lys Ile Arg Ser Val Gly Lys Pro Ala Leu Glu Arg Phe Leu Arg Arg
    610                 615                 620

Leu Gln Val Leu Lys Ser Thr Gly Asp Val Ala Gly Gly Arg Ala Leu
625                 630                 635                 640

Tyr Glu Gly Tyr Ala Thr Val Thr Asp Ala Pro Pro Glu Cys Phe Leu

                645                 650                 655

Thr Leu Arg Asp Thr Val Leu Leu Arg Lys Glu Ser Arg Lys Leu Ile
            660                 665                 670

Val Gln Pro Asn Thr Arg Leu Glu Gly Ser Asp Val Gln Leu Leu Glu
        675                 680                 685

Tyr Glu Ala Ser Ala Ala Gly Leu Ile Arg Ser Phe Ser Glu Arg Phe
    690                 695                 700

Pro Glu Asp Gly Pro Glu Leu Glu Glu Ile Leu Thr Gln Leu Ala Thr
705                 710                 715                 720

Ala Asp Ala Arg Phe Trp Lys Gly Pro Ser Glu Ala Pro Ser Gly Gln
                725                 730                 735

Ala

<210> 26

<211> 328

<212> PRT

<213> Homo sapiens

<300>

<308> sw_hum/ech1_human

<309> 1997-11-01

<400> 26

Met Ala Ala Gly Ile Val Ala Ser Arg Arg Leu Arg Asp Leu Leu Thr
1               5                   10                  15

Arg Arg Leu Thr Gly Ser Asn Tyr Pro Gly Leu Ser Ile Ser Leu Arg
            20                  25                  30

Leu Thr Gly Ser Ser Ala Gln Glu Glu Ala Ser Gly Val Ala Leu Gly
        35                  40                  45

Glu Ala Pro Asp His Ser Tyr Glu Ser Leu Arg Val Thr Ser Ala Gln
    50                  55                  60

Lys His Val Leu His Val Gln Leu Asn Arg Pro Asn Lys Arg Asn Ala
65                  70                  75                  80

Met Asn Lys Val Phe Trp Arg Glu Met Val Glu Cys Phe Asn Lys Ile
                85                  90                  95

Ser Arg Asp Ala Asp Cys Arg Ala Val Val Ile Ser Gly Ala Gly Lys
            100                 105                 110

Met Phe Thr Ala Gly Ile Asp Leu Met Asp Met Ala Ser Asp Ile Leu
        115                 120                 125

Gln Pro Lys Gly Asp Asp Val Ala Arg Ile Ser Trp Tyr Leu Arg Asp
    130                 135                 140

Ile Ile Thr Arg Tyr Gln Glu Thr Phe Asn Val Ile Glu Arg Cys Pro
145                 150                 155                 160

Lys Pro Val Ile Ala Ala Val His Gly Gly Cys Ile Gly Gly Gly Val
                165                 170                 175

Asp Leu Val Thr Ala Cys Asp Ile Arg Tyr Cys Ala Gln Asp Ala Phe
            180                 185                 190

Phe Gln Val Lys Glu Val Asp Val Gly Leu Ala Ala Asp Val Gly Thr
        195                 200                 205

Leu Glu Arg Leu Pro Lys Val Ile Gly Asn Gln Ser Leu Val Asn Glu
    210                 215                 220

Leu Ala Phe Thr Ala His Lys Met Met Ala Asp Glu Ala Leu Asp Ser
225                 230                 235                 240

Gly Leu Val Ser Arg Val Phe Pro Asp Lys Glu Val Met Leu Asp Ala
                245                 250                 255

Ala Leu Pro Leu Ala Pro Glu Ile Ser Ser Lys Thr Thr Val Leu Val
            260                 265                 270

Gln Ser Thr Lys Val Asn Leu Leu Tyr Ser Arg Asp His Ser Val Ala
        275                 280                 285

Glu Ser Leu Asn Tyr Val Ala Ser Trp Asn Met Ser Met Leu Gln Thr
    290                 295                 300

Gln Asp Leu Val Lys Ser Val Gln Pro Thr Thr Glu Asn Lys Glu Leu
305                 310                 315                 320

Lys Thr Val Thr Phe Ser Lys Leu
                325

<210>  27

<211>  281

<212>  PRT

<213>  Homo sapiens


<300>

<308>  sw_hum/ef1d_human

<309>  1993-04-01


<400>  27

Met Ala Thr Asn Phe Leu Ala His Glu Lys Ile Trp Phe Asp Lys Phe
1               5                   10                  15

Lys Tyr Asp Asp Ala Glu Arg Arg Phe Tyr Glu Gln Met Asn Gly Pro
            20                  25                  30

Val Ala Gly Ala Ser Arg Gln Glu Asn Gly Ala Ser Val Ile Leu Arg
        35                  40                  45

Asp Ile Ala Arg Ala Arg Glu Asn Ile Gln Lys Ser Leu Ala Gly Ser
    50                  55                  60

Ser Gly Pro Gly Ala Ser Ser Gly Thr Ser Gly Asp His Gly Glu Leu
65                  70                  75                  80

Val Val Arg Ile Ala Ser Leu Glu Val Glu Asn Gln Ser Leu Arg Gly
                85                  90                  95

Val Val Gln Glu Leu Gln Gln Ala Ile Ser Lys Leu Glu Ala Arg Leu
            100                 105                 110

Asn Val Leu Glu Lys Ser Ser Pro Gly His Arg Ala Thr Ala Pro Gln
        115                 120                 125

Thr Gln His Val Ser Pro Met Arg Gln Val Glu Pro Pro Ala Lys Lys
    130                 135                 140

Pro Ala Thr Pro Ala Glu Asp Asp Glu Asp Asp Asp Ile Asp Leu Phe
145                 150                 155                 160

Gly Ser Asp Asn Glu Glu Glu Asp Lys Glu Ala Ala Gln Leu Arg Glu
                165                 170                 175

Glu Arg Leu Arg Gln Tyr Ala Glu Lys Lys Ala Lys Lys Pro Ala Leu
            180                 185                 190

Val Ala Lys Ser Ser Ile Leu Leu Asp Val Lys Pro Trp Asp Asp Glu
        195                 200                 205

Thr Asp Met Ala Gln Leu Glu Ala Cys Val Arg Ser Ile Gln Leu Asp
    210                 215                 220

Gly Leu Val Trp Gly Ala Ser Lys Leu Val Pro Val Gly Tyr Gly Ile
225                 230                 235                 240

Arg Lys Leu Gln Ile Gln Cys Val Val Glu Asp Asp Lys Val Gly Thr
                245                 250                 255

Asp Leu Leu Glu Glu Glu Ile Thr Lys Phe Glu Glu His Val Gln Ser
            260                 265                 270

Val Asp Ile Ala Ala Phe Asn Lys Ile
        275                 280

<210>  28

<211>  458

<212>  PRT

<213>  Homo sapiens

<300>

<308>  sw_hum/enol_human

<309>  1995-02-01

<400>  28

<210> 29

<211> 453

<212> PRT

<213> Homo sapiens

<300>

<308> sw_hum/fibg_human

<309> 1986-07-21

<400> 29

Met Ser Trp Ser Leu His Pro Arg Asn Leu Ile Leu Tyr Phe Tyr Ala
1               5                   10                  15

Leu Leu Phe Leu Ser Ser Thr Cys Val Ala Tyr Val Ala Thr Arg Asp
            20                  25                  30

Asn Cys Cys Ile Leu Asp Glu Arg Phe Gly Ser Tyr Cys Pro Thr Thr
        35                  40                  45

Cys Gly Ile Ala Asp Phe Leu Ser Thr Tyr Gln Thr Lys Val Asp Lys
    50                  55                  60

Asp Leu Gln Ser Leu Glu Asp Ile Leu His Gln Val Glu Asn Lys Thr
65                  70                  75                  80

Ser Glu Val Lys Gln Leu Ile Lys Ala Ile Gln Leu Thr Tyr Asn Pro
                85                  90                  95

Asp Glu Ser Ser Lys Pro Asn Met Ile Asp Ala Ala Thr Leu Lys Ser
            100                 105                 110

Arg Lys Met Leu Glu Glu Ile Met Lys Tyr Glu Ala Ser Ile Leu Thr
        115                 120                 125

His Asp Ser Ser Ile Arg Tyr Leu Gln Glu Ile Tyr Asn Ser Asn Asn
    130                 135                 140

Gln Lys Ile Val Asn Leu Lys Glu Lys Val Ala Gln Leu Glu Ala Gln
145                 150                 155                 160

Cys Gln Glu Pro Cys Lys Asp Thr Val Gln Ile His Asp Ile Thr Gly
                165                 170                 175

Lys Asp Cys Gln Asp Ile Ala Asn Lys Gly Ala Lys Gln Ser Gly Leu
            180                 185                 190

Tyr Phe Ile Lys Pro Leu Lys Ala Asn Gln Gln Phe Leu Val Tyr Cys
        195                 200                 205

Glu Ile Asp Gly Ser Gly Asn Gly Trp Thr Val Phe Gln Lys Arg Leu
    210                 215                 220

Asp Gly Ser Val Asp Phe Lys Lys Asn Trp Ile Gln Tyr Lys Glu Gly
225                 230                 235                 240

Phe Gly His Leu Ser Pro Thr Gly Thr Thr Glu Phe Trp Leu Gly Asn
                245                 250                 255

Glu Lys Ile His Leu Ile Ser Thr Gln Ser Ala Ile Pro Tyr Ala Leu
            260                 265                 270

Arg Val Glu Leu Glu Asp Trp Asn Gly Arg Thr Ser Thr Ala Asp Tyr
        275                 280                 285

Ala Met Phe Lys Val Gly Pro Glu Ala Asp Lys Tyr Arg Leu Thr Tyr
    290                 295                 300

Ala Tyr Phe Ala Gly Gly Asp Ala Gly Asp Ala Phe Asp Gly Phe Asp
305                 310                 315                 320

Phe Gly Asp Asp Pro Ser Asp Lys Phe Phe Thr Ser His Asn Gly Met
                325                 330                 335

Gln Phe Ser Thr Trp Asp Asn Asp Asn Asp Lys Phe Glu Gly Asn Cys
            340                 345                 350

Ala Glu Gln Asp Gly Ser Gly Trp Trp Met Asn Lys Cys His Ala Gly
        355                 360                 365

His Leu Asn Gly Val Tyr Tyr Gln Gly Gly Thr Tyr Ser Lys Ala Ser
    370                 375                 380

Thr Pro Asn Gly Tyr Asp Asn Gly Ile Ile Trp Ala Thr Trp Lys Thr
385                 390                 395                 400

Arg Trp Tyr Ser Met Lys Lys Thr Thr Met Lys Ile Ile Pro Phe Asn
                405                 410                 415

Arg Leu Thr Ile Gly Glu Gly Gln Gln His His Leu Gly Gly Ala Lys
            420                 425                 430

Gln Val Arg Pro Glu His Pro Ala Glu Thr Glu Tyr Asp Ser Leu Tyr
        435                 440                 445

Pro Glu Asp Asp Leu
    450

<210>  30

<211>  482

<212>  PRT

<213>  Homo sapiens

<300>

<308>  sw_humg3b2_human

<309>  2001-10-16

<400>  30

<210>  31

<211>  466

<212>  PRT

<213>  Homo sapiens

<300>

<308>  sw_humg3bp_human

<309>  2001-10-16

<400>  31

Met Val Met Glu Lys Pro Ser Pro Leu Leu Val Gly Arg Glu Phe Val
1               5                   10                  15

Arg Gln Tyr Tyr Thr Leu Leu Asn Gln Ala Pro Asp Met Leu His Arg
            20                  25                  30

Phe Tyr Gly Lys Asn Ser Ser Tyr Val His Gly Gly Leu Asp Ser Asn
        35                  40                  45

Gly Lys Pro Ala Asp Ala Val Tyr Gly Gln Lys Glu Ile His Arg Lys
    50                  55                  60

Val Met Ser Gln Asn Phe Thr Asn Cys His Thr Lys Ile Arg His Val
65                  70                  75                  80

Asp Ala His Ala Thr Leu Asn Asp Gly Val Val Val Gln Val Met Gly
                85                  90                  95

Leu Leu Ser Asn Asn Asn Gln Ala Leu Arg Arg Phe Met Gln Thr Phe
            100                 105                 110

Val Leu Ala Pro Glu Gly Ser Val Ala Asn Lys Phe Tyr Val His Asn
        115                 120                 125

Asp Ile Phe Arg Tyr Gln Asp Glu Val Phe Gly Gly Phe Val Thr Glu
    130                 135                 140

Pro Gln Glu Glu Ser Glu Glu Glu Val Glu Glu Pro Glu Glu Arg Gln
145                 150                 155                 160

Gln Thr Pro Glu Val Val Pro Asp Asp Ser Gly Thr Phe Tyr Asp Gln
                165                 170                 175

Ala Val Val Ser Asn Asp Met Glu Glu His Leu Glu Glu Pro Val Ala
            180                 185                 190

Glu Pro Glu Pro Asp Pro Glu Pro Glu Pro Glu Gln Glu Pro Val Ser
        195                 200                 205

Glu Ile Gln Glu Glu Lys Pro Glu Pro Val Leu Glu Glu Thr Ala Pro
    210                 215                 220

Glu Asp Ala Gln Lys Ser Ser Ser Pro Ala Pro Ala Asp Ile Ala Gln
225                 230                 235                 240

Thr Val Gln Glu Asp Leu Arg Thr Phe Ser Trp Ala Ser Val Thr Ser
                245                 250                 255

Lys Asn Leu Pro Pro Ser Gly Ala Val Pro Val Thr Gly Ile Pro Pro
            260                 265                 270

His Val Val Lys Val Pro Ala Ser Gln Pro Arg Pro Glu Ser Lys Pro
        275                 280                 285

Glu Ser Gln Ile Pro Pro Gln Arg Pro Gln Arg Asp Gln Arg Val Arg
    290                 295                 300

Glu Gln Arg Ile Asn Ile Pro Pro Gln Arg Gly Pro Arg Pro Ile Arg
305                 310                 315                 320

Glu Ala Gly Glu Gln Gly Asp Ile Glu Pro Arg Arg Met Val Arg His
                325                 330                 335

Pro Asp Ser His Gln Leu Phe Ile Gly Asn Leu Pro His Glu Val Asp
            340                 345                 350

Lys Ser Glu Leu Lys Asp Phe Phe Gln Ser Tyr Gly Asn Val Val Glu
        355                 360                 365

Leu Arg Ile Asn Ser Gly Gly Lys Leu Pro Asn Phe Gly Phe Val Val
    370                 375                 380

Phe Asp Asp Ser Glu Pro Val Gln Lys Val Leu Ser Asn Arg Pro Ile
385                 390                 395                 400

Met Phe Arg Gly Glu Val Arg Leu Asn Val Glu Glu Lys Lys Thr Arg
                405                 410                 415

Ala Ala Arg Glu Gly Asp Arg Arg Asp Asn Arg Leu Arg Gly Pro Gly
            420                 425                 430

Gly Pro Arg Gly Gly Leu Gly Gly Gly Met Arg Gly Pro Pro Arg Gly
        435                 440                 445

Gly Met Val Gln Lys Pro Gly Phe Gly Val Gly Arg Gly Leu Ala Pro
    450                 455                 460

Arg Gln
465

<210>  32

<211>  514

<212>  PRT

<213>  Homo sapiens

<300>

<308>  sw_hum/g6pd_human

<309>  1989-10-01

<400>  32

Ala Glu Gln Val Ala Leu Ser Arg Thr His Val Cys Gly Ile Leu Arg
1               5                   10                  15

Glu Glu Leu Phe Gln Gly Asp Ala Phe His Gln Ser Asp Thr His Ile
            20                  25                  30

Phe Ile Ile Met Gly Ala Ser Gly Asp Leu Ala Lys Lys Lys Ile Tyr
        35                  40                  45

Pro Thr Ile Trp Trp Leu Phe Arg Asp Gly Leu Leu Pro Glu Asn Thr
    50                  55                  60

Phe Ile Val Gly Tyr Ala Arg Ser Arg Leu Thr Val Ala Asp Ile Arg
65                  70                  75                  80

Lys Gln Ser Glu Pro Phe Phe Lys Ala Thr Pro Glu Glu Lys Leu Lys
                85                  90                  95

Leu Glu Asp Phe Phe Ala Arg Asn Ser Tyr Val Ala Gly Gln Tyr Asp
            100                 105                 110

Asp Ala Ala Ser Tyr Gln Arg Leu Asn Ser His Met Asn Ala Leu His
        115                 120                 125

Leu Gly Ser Gln Ala Asn Arg Leu Phe Tyr Leu Ala Leu Pro Pro Thr
    130                 135                 140

Val Tyr Glu Ala Val Thr Lys Asn Ile His Glu Ser Cys Met Ser Gln
145                 150                 155                 160

Ile Gly Trp Asn Arg Ile Ile Val Glu Lys Pro Phe Gly Arg Asp Leu
                165                 170                 175

Gln Ser Ser Asp Arg Leu Ser Asn His Ile Ser Ser Leu Phe Arg Glu
            180                 185                 190

Asp Gln Ile Tyr Arg Ile Asp His Tyr Leu Gly Lys Glu Met Val Gln
        195                 200                 205

Asn Leu Met Val Leu Arg Phe Ala Asn Arg Ile Phe Gly Pro Ile Trp
    210                 215                 220

Asn Arg Asp Asn Ile Ala Cys Val Ile Leu Thr Phe Lys Glu Pro Phe
225                 230                 235                 240

Gly Thr Glu Gly Arg Gly Gly Tyr Phe Asp Glu Phe Gly Ile Ile Arg
                245                 250                 255

Asp Val Met Gln Asn His Leu Leu Gln Met Leu Cys Leu Val Ala Met
            260                 265                 270

Glu Lys Pro Ala Ser Thr Asn Ser Asp Asp Val Arg Asp Glu Lys Val
        275                 280                 285

Lys Val Leu Lys Cys Ile Ser Glu Val Gln Ala Asn Asn Val Val Leu
    290                 295                 300

Gly Gln Tyr Val Gly Asn Pro Asp Gly Glu Gly Glu Ala Thr Lys Gly
305                 310                 315                 320

Tyr Leu Asp Asp Pro Thr Val Pro Arg Gly Ser Thr Thr Ala Thr Phe
                325                 330                 335

Ala Ala Val Val Leu Tyr Val Glu Asn Glu Arg Trp Asp Gly Val Pro
            340                 345                 350

Phe Ile Leu Arg Cys Gly Lys Ala Leu Asn Glu Arg Lys Ala Glu Val
        355                 360                 365

Arg Leu Gln Phe His Asp Val Ala Gly Asp Ile Phe His Gln Gln Cys
    370                 375                 380

Lys Arg Asn Glu Leu Val Ile Arg Val Gln Pro Asn Glu Ala Val Tyr
385                 390                 395                 400

Thr Lys Met Met Thr Lys Lys Pro Gly Met Phe Phe Asn Pro Glu Glu
                405                 410                 415

Ser Glu Leu Asp Leu Thr Tyr Gly Asn Arg Tyr Lys Asn Val Lys Leu
            420                 425                 430

Pro Asp Ala Tyr Glu Arg Leu Ile Leu Asp Val Phe Cys Gly Ser Gln
        435                 440                 445

Met His Phe Val Arg Ser Asp Glu Leu Arg Glu Ala Trp Arg Ile Phe
    450                 455                 460

Thr Pro Leu Leu His Gln Ile Glu Leu Glu Lys Pro Lys Pro Ile Pro
465                 470                 475                 480

Tyr Ile Tyr Gly Ser Arg Gly Pro Thr Glu Ala Asp Glu Leu Met Lys
                485                 490                 495

Arg Val Gly Phe Gln Tyr Glu Gly Thr Tyr Lys Trp Val Asn Pro His

500                 505                 510

Lys Leu

<210>  33

<211>  236

<212>  PRT

<213>  Homo sapiens

<300>

<308>  sw_hum/gamt_human

<309>  1997-11-01

<400>  33

<210>  34

<211>  424

<212>  PRT

<213>  Homo sapiens

<300>

<308>  sw_hum/grf1_human

<309>  1997-11-01

<400>  34

Met Ala Gly Thr Arg Trp Val Leu Gly Ala Leu Leu Arg Gly Cys Gly
1               5                   10                  15

Cys Asn Cys Ser Ser Cys Arg Arg Thr Gly Ala Ala Cys Leu Pro Phe
            20                  25                  30

Tyr Ser Ala Ala Ser Tyr Pro Ala Leu Arg Ala Ser Leu Leu Pro Gln
        35                  40                  45

Ser Leu Ala Ala Ala Ala Ala Val Pro Thr Arg Ser Tyr Ser Gln Glu
    50                  55                  60

Ser Lys Thr Thr Tyr Leu Glu Asp Leu Pro Pro Pro Pro Glu Tyr Glu
65                  70                  75                  80

Leu Ala Pro Ser Lys Leu Glu Glu Glu Val Asp Asp Val Phe Leu Ile
                85                  90                  95

Arg Ala Gln Gly Leu Pro Trp Ser Cys Thr Met Glu Asp Val Leu Asn
            100                 105                 110

Phe Phe Ser Asp Cys Arg Ile Arg Asn Gly Glu Asn Gly Ile His Phe
        115                 120                 125

Leu Leu Asn Arg Asp Gly Lys Arg Arg Gly Asp Ala Leu Ile Glu Met
    130                 135                 140

Glu Ser Glu Gln Asp Val Gln Lys Ala Leu Glu Lys His Arg Met Tyr
145                 150                 155                 160

Met Gly Gln Arg Tyr Val Glu Val Tyr Glu Ile Asn Asn Glu Asp Val
                165                 170                 175

Asp Ala Leu Met Lys Ser Leu Gln Val Lys Ser Ser Pro Val Val Asn
            180                 185                 190

Asp Gly Val Val Arg Leu Arg Gly Leu Pro Tyr Ser Cys Asn Glu Lys
        195                 200                 205

Asp Ile Val Asp Phe Phe Ala Gly Leu Asn Ile Val Asp Ile Thr Phe
    210                 215                 220

Val Met Asp Tyr Arg Gly Arg Arg Lys Thr Gly Glu Ala Tyr Val Gln
225                 230                 235                 240

Phe Glu Glu Pro Glu Met Ala Asn Gln Ala Leu Leu Lys His Arg Glu
                245                 250                 255

Glu Ile Gly Asn Arg Tyr Ile Glu Ile Phe Pro Ser Arg Arg Asn Glu
            260                 265                 270

Val Arg Thr His Val Gly Ser Tyr Lys Gly Lys Lys Ile Ala Ser Phe
        275                 280                 285

Pro Thr Ala Lys Tyr Ile Thr Glu Pro Glu Met Val Phe Glu Glu His
    290                 295                 300

Glu Val Asn Glu Val Phe Gln Pro Met Thr Ala Phe Glu Ser Glu Lys
305                 310                 315                 320

Glu Ile Glu Leu Pro Lys Glu Val Pro Glu Lys Leu Pro Glu Ala Ala
                325                 330                 335

Asp Phe Gly Thr Thr Ser Ser Leu His Phe Val His Met Arg Gly Leu
            340                 345                 350

Pro Phe Gln Ala Asn Ala Gln Asp Ile Ile Asn Phe Phe Ala Pro Leu
        355                 360                 365

Lys Pro Val Arg Ile Thr Met Glu Tyr Ser Ser Ser Gly Lys Ala Thr
    370                 375                 380

Gly Glu Ala Asp Val His Phe Glu Thr His Glu Asp Ala Val Ala Ala
385                 390                 395                 400

Met Leu Lys Asp Arg Ser His Val His His Arg Tyr Ile Glu Leu Phe
                405                 410                 415

Leu Asn Ser Cys Pro Lys Gly Lys
            420

<210>  35

<211>  241

<212>  PRT

<213>  Homo sapiens


<300>

<308>  sw_hum/gto1_hum

<309>  1999-07-15

<400>  35

Met Ser Gly Glu Ser Ala Arg Ser Leu Gly Lys Gly Ser Ala Pro Pro
1               5                   10                  15

Gly Pro Val Pro Glu Gly Ser Ile Arg Ile Tyr Ser Met Arg Phe Cys
            20                  25                  30

Pro Phe Ala Glu Arg Thr Arg Leu Val Leu Lys Ala Lys Gly Ile Arg
        35                  40                  45

His Glu Val Ile Asn Ile Asn Leu Lys Asn Lys Pro Glu Trp Phe Phe
    50                  55                  60

Lys Lys Asn Pro Phe Gly Leu Val Pro Val Leu Glu Asn Ser Gln Gly
65                  70                  75                  80

Gln Leu Ile Tyr Glu Ser Ala Ile Thr Cys Glu Tyr Leu Asp Glu Ala
                85                  90                  95

Tyr Pro Gly Lys Lys Leu Leu Pro Asp Asp Pro Tyr Glu Lys Ala Cys
            100                 105                 110

Gln Lys Met Ile Leu Glu Leu Phe Ser Lys Val Pro Ser Leu Val Gly
        115                 120                 125

Ser Phe Ile Arg Ser Gln Asn Lys Glu Asp Tyr Ala Gly Leu Lys Glu
    130                 135                 140

Glu Phe Arg Lys Glu Phe Thr Lys Leu Glu Glu Val Leu Thr Asn Lys
145                 150                 155                 160

Lys Thr Thr Phe Phe Gly Gly Asn Ser Ile Ser Met Ile Asp Tyr Leu
                165                 170                 175

Ile Trp Pro Trp Phe Glu Arg Leu Glu Ala Met Lys Leu Asn Glu Cys
            180                 185                 190

Val Asp His Thr Pro Lys Leu Lys Leu Trp Met Ala Ala Met Lys Glu
        195                 200                 205

Asp Pro Thr Val Ser Ala Leu Leu Thr Ser Glu Lys Asp Trp Gln Gly
    210                 215                 220

Phe Leu Glu Leu Tyr Leu Gln Asn Ser Pro Glu Ala Cys Asp Tyr Gly
225                 230                 235                 240

Leu


<210>  36

<211>  858

<212>  PRT

<213>  Homo sapiens

<300>

<308>  sw_hum/h105_human

<309>  1997-11-01

<400>  36

Met Ser Val Val Gly Leu Asp Val Gly Ser Gln Ser Cys Tyr Ile Ala
1               5                   10                  15

Val Ala Arg Ala Gly Gly Ile Glu Thr Ile Ala Asn Glu Phe Ser Asp
            20                  25                  30

Arg Cys Thr Pro Ser Val Ile Ser Phe Gly Ser Lys Asn Arg Thr Ile
        35                  40                  45

Gly Val Ala Ala Lys Asn Gln Gln Ile Thr His Ala Asn Asn Thr Val
    50                  55                  60

Ser Asn Phe Lys Arg Phe His Gly Arg Ala Phe Asn Asp Pro Phe Ile
65                  70                  75                  80

Gln Lys Glu Lys Glu Asn Leu Ser Tyr Asp Leu Val Pro Leu Lys Asn
                85                  90                  95

Gly Gly Val Gly Ile Lys Val Met Tyr Met Gly Glu Glu His Leu Phe
            100                 105                 110

Ser Val Glu Gln Ile Thr Ala Met Leu Leu Thr Lys Leu Lys Glu Thr
        115                 120                 125

Ala Glu Asn Ser Leu Lys Lys Pro Val Thr Asp Cys Val Ile Ser Val
    130                 135                 140

Pro Ser Phe Phe Thr Asp Ala Glu Arg Arg Ser Val Leu Asp Ala Ala
145                 150                 155                 160

Gln Ile Val Gly Leu Asn Cys Leu Arg Leu Met Asn Asp Met Thr Ala
                165                 170                 175

Val Ala Leu Asn Tyr Gly Ile Tyr Lys Gln Asp Leu Pro Ser Leu Asp
            180                 185                 190

Glu Lys Pro Arg Ile Val Val Phe Val Asp Met Gly His Ser Ala Phe
        195                 200                 205

Gln Val Ser Ala Cys Ala Phe Asn Lys Gly Lys Leu Lys Val Leu Gly
    210                 215                 220

Thr Ala Phe Asp Pro Phe Leu Gly Gly Lys Asn Phe Asp Glu Lys Leu
225                 230                 235                 240

Val Glu His Phe Cys Ala Glu Phe Lys Thr Lys Tyr Lys Leu Asp Ala
                245                 250                 255

Lys Ser Lys Ile Arg Ala Leu Leu Arg Leu Tyr Gln Glu Cys Glu Lys

            260                 265                 270

Leu Lys Lys Leu Met Ser Ser Asn Ser Thr Asp Leu Pro Leu Asn Ile
        275                 280                 285

Glu Cys Phe Met Asn Asp Lys Asp Val Ser Gly Lys Met Asn Arg Ser
    290                 295                 300

Gln Phe Glu Glu Leu Cys Ala Glu Leu Leu Gln Lys Ile Glu Val Pro
305                 310                 315                 320

Leu Tyr Ser Leu Leu Glu Gln Thr His Leu Lys Val Glu Asp Val Ser
                325                 330                 335

Ala Val Glu Ile Val Gly Gly Ala Thr Arg Ile Pro Ala Val Lys Glu
            340                 345                 350

Arg Ile Ala Lys Phe Phe Gly Lys Asp Ile Ser Thr Thr Leu Asn Ala
        355                 360                 365

Asp Glu Ala Val Ala Arg Gly Cys Ala Leu Gln Cys Ala Ile Leu Ser
    370                 375                 380

Pro Ala Phe Lys Val Arg Glu Phe Ser Val Thr Asp Ala Val Pro Phe
385                 390                 395                 400

Pro Ile Ser Leu Ile Trp Asn His Asp Ser Glu Asp Thr Glu Gly Val
                405                 410                 415

His Glu Val Phe Ser Arg Asn His Ala Ala Pro Phe Ser Lys Val Leu
            420                 425                 430

Thr Phe Leu Arg Arg Gly Pro Phe Glu Leu Glu Ala Phe Tyr Ser Asp
        435                 440                 445

Pro Gln Gly Val Pro Tyr Pro Glu Ala Lys Ile Gly Arg Phe Val Val
    450                 455                 460

Gln Asn Val Ser Ala Gln Lys Asp Gly Glu Lys Ser Arg Val Lys Val
465                 470                 475                 480

Lys Val Arg Val Asn Thr His Gly Ile Phe Thr Ile Ser Thr Ala Ser
                485                 490                 495

Met Val Glu Lys Val Pro Thr Glu Glu Asn Glu Met Ser Ser Glu Ala
            500                 505                 510

Asp Met Glu Cys Leu Asn Gln Arg Pro Pro Glu Asn Pro Asp Thr Asp
        515                 520                 525

Lys Asn Val Gln Gln Asp Asn Ser Glu Ala Gly Thr Gln Pro Gln Val
    530                 535                 540

Gln Thr Asp Ala Gln Gln Thr Ser Gln Ser Pro Pro Ser Pro Glu Leu
545                 550                 555                 560

Thr Ser Glu Glu Asn Lys Ile Pro Asp Ala Asp Lys Ala Asn Glu Lys
                565                 570                 575

Lys Val Asp Gln Pro Pro Glu Ala Lys Lys Pro Lys Ile Lys Val Val
            580                 585                 590

Asn Val Glu Leu Pro Ile Glu Ala Asn Leu Val Trp Gln Leu Gly Lys
        595                 600                 605

Asp Leu Leu Asn Met Tyr Ile Glu Thr Glu Gly Lys Met Ile Met Gln
    610                 615                 620

Asp Lys Leu Glu Lys Glu Arg Asn Asp Ala Lys Asn Ala Val Glu Glu
625                 630                 635                 640

Tyr Val Tyr Glu Phe Arg Asp Lys Leu Cys Gly Pro Tyr Glu Lys Phe
                645                 650                 655

Ile Cys Glu Gln Asp His Gln Asn Phe Leu Arg Leu Leu Thr Glu Thr
            660                 665                 670

Glu Asp Trp Leu Tyr Glu Glu Gly Glu Asp Gln Ala Lys Gln Ala Tyr
        675                 680                 685

Val Asp Lys Leu Glu Glu Leu Met Lys Ile Gly Thr Pro Val Lys Val
    690                 695                 700

Arg Phe Gln Glu Ala Glu Glu Arg Pro Lys Met Phe Glu Glu Leu Gly
705                 710                 715                 720

Gln Arg Leu Gln His Tyr Ala Lys Ile Ala Ala Asp Phe Arg Asn Lys
                725                 730                 735

Asp Glu Lys Tyr Asn His Ile Asp Glu Ser Glu Met Lys Lys Val Glu
            740                 745                 750

Lys Ser Val Asn Glu Val Met Glu Trp Met Asn Asn Val Met Asn Ala
        755                 760                 765

Gln Ala Lys Lys Ser Leu Asp Gln Asp Pro Val Val Arg Ala Gln Glu
    770                 775                 780

Ile Lys Thr Lys Ile Lys Glu Leu Asn Asn Thr Cys Glu Pro Val Val
785                 790                 795                 800

Thr Gln Pro Lys Pro Lys Ile Glu Ser Pro Lys Leu Glu Arg Thr Pro
                805                 810                 815

Asn Gly Pro Asn Ile Asp Lys Lys Glu Glu Asp Leu Glu Asp Lys Asn
            820                 825                 830

Asn Phe Gly Ala Glu Pro Pro His Gln Asn Gly Glu Cys Tyr Pro Asn
        835                 840                 845

Glu Lys Asn Ser Val Asn Met Asp Leu Asp
    850                 855

<210>  37

<211>  240

<212>  PRT

<213>  Homo sapiens

<300>

<308>  sw_hum/hdgf_human

<309>  1996-10-01

<400>  37

Met Ser Arg Ser Asn Arg Gln Lys Glu Tyr Lys Cys Gly Asp Leu Val
1               5                   10                  15

Phe Ala Lys Met Lys Gly Tyr Pro His Trp Pro Ala Arg Ile Asp Glu
            20                  25                  30

Met Pro Glu Ala Ala Val Lys Ser Thr Ala Asn Lys Tyr Gln Val Phe
        35                  40                  45

Phe Phe Gly Thr His Glu Thr Ala Phe Leu Gly Pro Lys Asp Leu Phe
    50                  55                  60

Pro Tyr Glu Glu Ser Lys Glu Lys Phe Gly Lys Pro Asn Lys Arg Lys
65                  70                  75                  80

Gly Phe Ser Glu Gly Leu Trp Glu Ile Glu Asn Asn Pro Thr Val Lys
                85                  90                  95

Ala Ser Gly Tyr Gln Ser Ser Gln Lys Lys Ser Cys Val Glu Glu Pro
            100                 105                 110

Glu Pro Glu Pro Glu Ala Ala Glu Gly Asp Gly Asp Lys Lys Gly Asn
        115                 120                 125

Ala Glu Gly Ser Ser Asp Glu Glu Gly Lys Leu Val Ile Asp Glu Pro
    130                 135                 140

Ala Lys Glu Lys Asn Glu Lys Gly Ala Leu Lys Arg Arg Ala Gly Asp
145                 150                 155                 160

Leu Leu Glu Asp Ser Pro Lys Arg Pro Lys Glu Ala Glu Asn Pro Glu
                165                 170                 175

Gly Glu Glu Lys Glu Ala Ala Thr Leu Glu Val Glu Arg Pro Leu Pro
            180                 185                 190

Met Glu Val Glu Lys Asn Ser Thr Pro Ser Glu Pro Gly Ser Gly Arg
        195                 200                 205

Gly Pro Pro Gln Glu Glu Glu Glu Glu Glu Asp Glu Glu Glu Glu Ala
    210                 215                 220

Thr Lys Glu Asp Ala Glu Ala Pro Gly Ile Arg Asp His Glu Ser Leu
225                 230                 235                 240

<210>  38

<211>  520

<212>  PRT

<213>  Homo sapiens

<300>

<308>  sw_hum/hmcs_human

<309>  1993-07-01

<400>  38

Met Pro Gly Ser Leu Pro Leu Asn Ala Glu Ala Cys Trp Pro Lys Asp
1               5                   10                  15

Val Gly Ile Val Ala Leu Glu Ile Tyr Phe Pro Ser Gln Tyr Val Asp
            20                  25                  30

Gln Ala Glu Leu Glu Lys Tyr Asp Gly Val Asp Ala Gly Lys Tyr Thr
        35                  40                  45

Ile Gly Leu Gly Gln Ala Lys Met Gly Phe Cys Thr Asp Arg Glu Asp
    50                  55                  60

Ile Asn Ser Leu Cys Met Thr Val Val Gln Asn Leu Met Glu Arg Asn
65                  70                  75                  80

Asn Leu Ser Tyr Asp Cys Ile Gly Arg Leu Glu Val Gly Thr Glu Thr
                85                  90                  95

Ile Ile Asp Lys Ser Lys Ser Val Lys Thr Asn Leu Met Gln Leu Phe
            100                 105                 110

Glu Glu Ser Gly Asn Thr Asp Ile Glu Gly Ile Asp Thr Thr Asn Ala
        115                 120                 125

Cys Tyr Gly Gly Thr Ala Ala Val Phe Asn Ala Val Asn Trp Ile Glu
    130                 135                 140

Ser Ser Ser Trp Asp Gly Arg Tyr Ala Leu Val Val Ala Gly Asp Ile
145                 150                 155                 160

Ala Val Tyr Ala Thr Gly Asn Ala Arg Pro Thr Gly Gly Val Gly Ala
                165                 170                 175

Val Ala Leu Leu Ile Gly Pro Asn Ala Pro Leu Ile Phe Glu Arg Gly
            180                 185                 190

Leu Arg Gly Thr His Met Gln His Ala Tyr Asp Phe Tyr Lys Pro Asp
        195                 200                 205

Met Leu Ser Glu Tyr Pro Ile Val Asp Gly Lys Leu Ser Ile Gln Cys
    210                 215                 220

Tyr Leu Ser Ala Leu Asp Arg Cys Tyr Ser Val Tyr Cys Lys Lys Ile
225                 230                 235                 240

His Ala Gln Trp Gln Lys Glu Gly Asn Asp Lys Asp Phe Thr Leu Asn
                245                 250                 255

Asp Phe Gly Phe Met Ile Phe His Ser Pro Tyr Cys Lys Leu Val Gln
            260                 265                 270

Lys Ser Leu Ala Arg Met Leu Leu Asn Asp Phe Leu Asn Asp Gln Asn
        275                 280                 285

Arg Asp Lys Asn Ser Ile Tyr Ser Gly Leu Glu Ala Phe Gly Asp Val
    290                 295                 300

Lys Leu Glu Asp Thr Tyr Phe Asp Arg Asp Val Glu Lys Ala Phe Met
305                 310                 315                 320

Lys Ala Ser Ser Glu Leu Phe Ser Gln Lys Thr Lys Ala Ser Leu Leu
                325                 330                 335

Val Ser Asn Gln Asn Gly Asn Met Tyr Thr Ser Ser Val Tyr Gly Ser
            340                 345                 350

Leu Ala Ser Val Leu Ala Gln Tyr Ser Pro Gln Gln Leu Ala Gly Lys
        355                 360                 365

Arg Ile Gly Val Phe Ser Tyr Gly Ser Gly Leu Ala Ala Thr Leu Tyr
    370                 375                 380

Ser Leu Lys Val Thr Gln Asp Ala Thr Pro Gly Ser Ala Leu Asp Lys
385                 390                 395                 400

Ile Thr Ala Ser Leu Cys Asp Leu Lys Ser Arg Leu Asp Ser Arg Thr
                405                 410                 415

Gly Val Ala Pro Asp Val Phe Ala Glu Asn Met Lys Leu Arg Glu Asp
            420                 425                 430

Thr His His Leu Val Asn Tyr Ile Pro Gln Gly Ser Ile Asp Ser Leu
        435                 440                 445

Phe Glu Gly Thr Trp Tyr Leu Val Arg Val Asp Glu Lys His Arg Arg
    450                 455                 460

Thr Tyr Ala Arg Arg Pro Thr Pro Asn Asp Asp Thr Leu Asp Glu Gly
465                 470                 475                 480

Val Gly Leu Val His Ser Asn Ile Ala Thr Glu His Ile Pro Ser Pro
                485                 490                 495

Ala Lys Lys Val Pro Arg Leu Pro Ala Thr Ala Ala Glu Pro Glu Ala
            500                 505                 510

Ala Val Ile Ser Asn Gly Glu His
        515                 520

<210>  39

<211>  314

<212>  PRT

<213>  Homo sapiens

<300>

<308>  sw_hum/if2a_human

<309>  1987-08-13

<400>  39

Pro Gly Leu Ser Cys Arg Phe Tyr Gln His Lys Phe Pro Glu Val Glu
1               5                   10                  15

Asp Val Val Met Val Asn Val Arg Ser Ile Ala Glu Met Gly Ala Tyr
            20                  25                  30

Val Ser Leu Leu Glu Tyr Asn Asn Ile Glu Gly Met Ile Leu Leu Ser
        35                  40                  45

Glu Leu Ser Arg Arg Arg Ile Arg Ser Ile Asn Lys Leu Ile Arg Ile
    50                  55                  60

Gly Arg Asn Glu Cys Val Val Val Ile Arg Val Asp Lys Glu Lys Gly
65                  70                  75                  80

Tyr Ile Asp Leu Ser Lys Arg Arg Val Ser Pro Glu Glu Ala Ile Lys
                85                  90                  95

Cys Glu Asp Lys Phe Thr Lys Ser Lys Thr Val Tyr Ser Ile Leu Arg
            100                 105                 110

His Val Ala Glu Val Leu Glu Tyr Thr Lys Asp Glu Gln Leu Glu Ser
        115                 120                 125

Leu Phe Gln Arg Thr Ala Trp Val Phe Asp Asp Lys Tyr Lys Arg Pro
    130                 135                 140

Gly Tyr Gly Ala Tyr Asp Ala Phe Lys His Ala Val Ser Asp Pro Ser
145                 150                 155                 160

Ile Leu Asp Ser Leu Asp Leu Asn Glu Asp Glu Arg Glu Val Leu Ile
                165                 170                 175

Asn Asn Ile Asn Arg Arg Leu Thr Pro Gln Ala Val Lys Ile Arg Ala
            180                 185                 190

Asp Ile Glu Val Ala Cys Tyr Gly Tyr Glu Gly Ile Asp Ala Val Lys
        195                 200                 205

Glu Ala Leu Arg Ala Gly Leu Asn Cys Ser Thr Glu Asn Met Pro Ile
    210                 215                 220

Lys Ile Asn Leu Ile Ala Pro Pro Arg Tyr Val Met Thr Thr Thr Thr

225                 230                 235                 240

Leu Glu Arg Thr Glu Gly Leu Ser Val Leu Ser Gln Ala Met Ala Val
                245                 250                 255

Ile Lys Glu Lys Ile Glu Glu Lys Arg Gly Val Phe Asn Val Gln Met
            260                 265                 270

Glu Pro Lys Val Val Thr Asp Thr Asp Glu Thr Glu Leu Ala Arg Gln
        275                 280                 285

Met Glu Arg Leu Glu Arg Glu Asn Ala Glu Val Asp Gly Asp Asp Asp
    290                 295                 300

Ala Glu Glu Met Glu Ala Lys Ala Glu Asp
305                 310

<210>  40

<211>  352

<212>  PRT

<213>  Homo sapiens


<300>

<308>  sw_hum/if33_human

<309>  2000-05-30


<400>  40

Met Ala Ser Arg Lys Glu Gly Thr Gly Ser Thr Ala Thr Ser Ser Ser
1               5                   10                  15

Ser Thr Ala Gly Ala Ala Gly Lys Gly Lys Gly Lys Gly Gly Ser Gly
            20                  25                  30

Asp Ser Ala Val Lys Gln Val Gln Ile Asp Gly Leu Val Val Leu Lys
        35                  40                  45

Ile Ile Lys His Tyr Gln Glu Glu Gly Gln Gly Thr Glu Val Val Gln
    50                  55                  60

Gly Val Leu Leu Gly Leu Val Val Glu Asp Arg Leu Glu Ile Thr Asn
65                  70                  75                  80

Cys Phe Pro Phe Pro Gln His Thr Glu Asp Asp Ala Asp Phe Asp Glu
                85                  90                  95

Val Gln Tyr Gln Met Glu Met Met Arg Ser Leu Arg His Val Asn Ile
            100                 105                 110

Asp His Leu His Val Gly Trp Tyr Gln Ser Thr Tyr Tyr Gly Ser Phe
        115                 120                 125

Val Thr Arg Ala Leu Leu Asp Ser Gln Phe Ser Tyr Gln His Ala Ile
    130                 135                 140

Glu Glu Ser Val Val Leu Ile Tyr Asp Pro Ile Lys Thr Ala Gln Gly
145                 150                 155                 160

Ser Leu Ser Leu Lys Ala Tyr Arg Leu Thr Pro Lys Leu Met Glu Val
                165                 170                 175

Cys Lys Glu Lys Asp Phe Ser Pro Glu Ala Leu Lys Lys Ala Asn Ile
            180                 185                 190

Thr Phe Glu Tyr Met Phe Glu Glu Val Pro Ile Val Ile Lys Asn Ser
        195                 200                 205

His Leu Ile Asn Val Leu Met Trp Glu Leu Glu Lys Lys Ser Ala Val
    210                 215                 220

Ala Asp Lys His Glu Leu Leu Ser Leu Ala Ser Ser Asn His Leu Gly
225                 230                 235                 240

Lys Asn Leu Gln Leu Leu Met Asp Arg Val Asp Glu Met Ser Gln Asp
                245                 250                 255

Ile Val Lys Tyr Asn Thr Tyr Met Arg Asn Thr Ser Lys Gln Gln Gln
            260                 265                 270

Gln Lys His Gln Tyr Gln Gln Arg Arg Gln Gln Glu Asn Met Gln Arg
        275                 280                 285

Gln Ser Arg Gly Glu Pro Pro Leu Pro Glu Glu Asp Leu Ser Lys Leu
    290                 295                 300

Phe Lys Pro Pro Gln Pro Pro Ala Arg Met Asp Ser Leu Leu Ile Ala
305                 310                 315                 320

Gly Gln Ile Asn Thr Tyr Cys Gln Asn Ile Lys Glu Phe Thr Ala Gln
                325                 330                 335

Asn Leu Gly Lys Leu Phe Met Ala Gln Ala Leu Gln Glu Tyr Asn Asn
            340                 345                 350

<210>  41

<211>  406

<212>  PRT

<213>  Homo sapiens

<300>

<308>  sw_hum/if41_human

<309>  1987-08-13

<400>  41

Met Ser Ala Ser Gln Asp Ser Arg Ser Arg Asp Asn Gly Pro Asp Gly
1               5                   10                  15

Met Glu Pro Glu Gly Val Ile Glu Ser Asn Trp Asn Glu Ile Val Asp
            20                  25                  30

Ser Phe Asp Asp Met Asn Leu Ser Glu Ser Leu Leu Arg Gly Ile Tyr
        35                  40                  45

Ala Tyr Gly Phe Glu Lys Pro Ser Ala Ile Gln Gln Arg Ala Ile Leu
    50                  55                  60

Pro Cys Ile Lys Gly Tyr Asp Val Ile Ala Gln Ala Gln Ser Gly Thr
65                  70                  75                  80

Gly Lys Thr Ala Thr Phe Ala Ile Ser Ile Leu Gln Gln Ile Glu Leu
                85                  90                  95

Asp Leu Lys Ala Thr Gln Ala Leu Val Leu Ala Pro Thr Arg Glu Leu
            100                 105                 110

Ala Gln Gln Ile Gln Lys Val Val Met Ala Leu Gly Asp Tyr Met Gly
        115                 120                 125

Ala Ser Cys His Ala Cys Ile Gly Gly Thr Asn Val Arg Ala Glu Val
    130                 135                 140

Gln Lys Leu Gln Met Glu Ala Pro His Ile Ile Val Gly Thr Pro Gly
145                 150                 155                 160

Arg Val Phe Asp Met Leu Asn Arg Arg Tyr Leu Ser Pro Lys Tyr Ile
                165                 170                 175

Lys Met Phe Val Leu Asp Glu Ala Asp Glu Met Leu Ser Arg Gly Phe
            180                 185                 190

Lys Asp Gln Ile Tyr Asp Ile Phe Gln Lys Leu Asn Ser Asn Thr Gln
        195                 200                 205

Val Val Leu Leu Ser Ala Thr Met Pro Ser Asp Val Leu Glu Val Thr
    210                 215                 220

Lys Lys Phe Met Arg Asp Pro Ile Arg Ile Leu Val Lys Lys Glu Glu
225                 230                 235                 240

Leu Thr Leu Glu Gly Ile Arg Gln Phe Tyr Ile Asn Val Glu Arg Glu
                245                 250                 255

Glu Trp Lys Leu Asp Thr Leu Cys Asp Leu Tyr Glu Thr Leu Thr Ile
            260                 265                 270

Thr Gln Ala Val Ile Phe Ile Asn Thr Arg Arg Lys Val Asp Trp Leu
        275                 280                 285

Thr Glu Lys Met His Ala Arg Asp Phe Thr Val Ser Ala Met His Gly
    290                 295                 300

Asp Met Asp Gln Lys Glu Arg Asp Val Ile Met Arg Glu Phe Arg Ser
305                 310                 315                 320

Gly Ser Ser Arg Val Leu Ile Thr Thr Asp Leu Leu Ala Arg Gly Ile
                325                 330                 335

Asp Val Gln Gln Val Ser Leu Val Ile Asn Tyr Asp Leu Pro Thr Asn
            340                 345                 350

Arg Glu Asn Tyr Ile His Arg Ile Gly Arg Gly Gly Arg Phe Gly Arg
        355                 360                 365

Lys Gly Val Ala Ile Asn Met Val Thr Glu Glu Asp Lys Arg Thr Leu
    370                 375                 380

Arg Asp Ile Glu Thr Phe Tyr Asn Thr Ser Ile Glu Glu Met Pro Leu
385                 390                 395                 400

Asn Val Ala Asp Leu Ile
                405

<210>  42

<211>  585

<212>  PRT

<213>  Homo sapiens

<300>

<308>  sw_hum/lam1_human

<309>  1991-02-01

<400>  42

Ala Thr Ala Thr Pro Val Pro Pro Arg Met Gly Ser Arg Ala Gly Gly
1               5                   10                  15

Pro Thr Thr Pro Leu Ser Pro Thr Arg Leu Ser Arg Leu Gln Glu Lys
            20                  25                  30

Glu Glu Leu Arg Glu Leu Asn Asp Arg Leu Ala Val Tyr Ile Asp Lys
        35                  40                  45

Val Arg Ser Leu Glu Thr Glu Asn Ser Ala Leu Gln Leu Gln Val Thr
    50                  55                  60

Glu Arg Glu Glu Val Arg Gly Arg Glu Leu Thr Gly Leu Lys Ala Leu
65                  70                  75                  80

Tyr Glu Thr Glu Leu Ala Asp Ala Arg Arg Ala Leu Asp Asp Thr Ala
                85                  90                  95

Arg Glu Arg Ala Lys Leu Gln Ile Glu Leu Gly Lys Cys Lys Ala Glu
            100                 105                 110

His Asp Gln Leu Leu Leu Asn Tyr Ala Lys Lys Glu Ser Asp Leu Asn
        115                 120                 125

Gly Ala Gln Ile Lys Leu Arg Glu Tyr Glu Ala Ala Leu Asn Ser Lys

130                 135                 140

Asp Ala Ala Leu Ala Thr Ala Leu Gly Asp Lys Lys Ser Leu Glu Gly
145                 150                 155                 160

Asp Leu Glu Asp Leu Lys Asp Gln Ile Ala Gln Leu Glu Ala Ser Leu
                165                 170                 175

Ala Ala Ala Lys Lys Gln Leu Ala Asp Glu Thr Leu Leu Lys Val Asp
            180                 185                 190

Leu Glu Asn Arg Cys Gln Ser Leu Thr Glu Asp Leu Glu Phe Arg Lys
        195                 200                 205

Ser Met Tyr Glu Glu Glu Ile Asn Glu Thr Arg Arg Lys His Glu Thr
    210                 215                 220

Arg Leu Val Glu Val Asp Ser Gly Arg Gln Ile Glu Tyr Glu Tyr Lys
225                 230                 235                 240

Leu Ala Gln Ala Leu His Glu Met Arg Glu Gln His Asp Ala Gln Val
                245                 250                 255

Arg Leu Tyr Lys Glu Glu Leu Glu Gln Thr Tyr His Ala Lys Leu Glu
            260                 265                 270

Asn Ala Arg Leu Ser Ser Glu Met Asn Thr Ser Thr Val Asn Ser Ala
        275                 280                 285

Arg Glu Glu Leu Met Glu Ser Arg Met Arg Ile Glu Ser Leu Ser Ser
    290                 295                 300

Gln Leu Ser Asn Leu Gln Lys Glu Ser Arg Ala Cys Leu Glu Arg Ile
305                 310                 315                 320

Gln Glu Leu Glu Asp Leu Leu Ala Lys Glu Lys Asp Asn Ser Arg Arg
                325                 330                 335

Met Leu Thr Asp Lys Glu Arg Glu Met Ala Glu Ile Arg Asp Gln Met
            340                 345                 350

Gln Gln Gln Leu Asn Asp Tyr Glu Gln Leu Leu Asp Val Lys Leu Ala
        355                 360                 365

Leu Asp Met Glu Ile Ser Ala Tyr Arg Lys Leu Leu Glu Gly Glu Glu
    370                 375                 380

Glu Arg Leu Lys Leu Ser Pro Ser Pro Ser Ser Arg Val Thr Val Ser
385                 390                 395                 400

Arg Ala Ser Ser Ser Arg Ser Val Arg Thr Thr Arg Gly Lys Arg Lys
                405                 410                 415

Arg Val Asp Val Glu Glu Ser Glu Ala Ser Ser Ser Val Ser Ile Ser
            420                 425                 430

His Ser Ala Ser Ala Thr Gly Asn Val Cys Ile Glu Glu Ile Asp Val
        435                 440                 445

Asp Gly Lys Phe Ile Arg Leu Lys Asn Thr Ser Glu Gln Asp Gln Pro
    450                 455                 460

Met Gly Gly Trp Glu Met Ile Arg Lys Ile Gly Asp Thr Ser Val Ser
465                 470                 475                 480

Tyr Lys Tyr Thr Ser Arg Tyr Val Leu Lys Ala Gly Gln Thr Val Thr
                485                 490                 495

Ile Trp Ala Ala Asn Ala Gly Val Thr Ala Ser Pro Pro Thr Asp Leu
            500                 505                 510

Ile Trp Lys Asn Gln Asn Ser Trp Gly Thr Gly Glu Asp Val Lys Val
        515                 520                 525

Ile Leu Lys Asn Ser Gln Gly Glu Glu Val Ala Gln Arg Ser Thr Val
    530                 535                 540

Phe Lys Thr Thr Ile Pro Glu Glu Glu Glu Glu Glu Glu Glu Ala Ala
545                 550                 555                 560

Gly Val Val Val Glu Glu Glu Leu Phe His Gln Gln Gly Thr Pro Arg
                565                 570                 575

Ala Ser Asn Arg Ser Cys Ala Ile Met
            580                 585

<210>  43

<211>  600

<212>  PRT

<213>  Homo sapiens


<300>

<308>  sw_hum/lam2_human

<309>  1994-06-01


<400>  43
   Met Ala Thr Pro Leu Pro Gly Arg Ala Gly Gly Pro Ala Thr Pro Leu
   1               5                   10                  15
   Ser Pro Thr Arg Leu Ser Arg Leu Gln Glu Lys Glu Glu Leu Arg Glu
               20                  25                  30
   Leu Asn Asp Arg Leu Ala His Tyr Ile Asp Arg Val Arg Ala Leu Glu
           35                  40                  45
   Leu Glu Asn Asp Arg Leu Leu Leu Lys Ile Ser Glu Lys Glu Glu Val
       50                  55                  60
   Thr Thr Arg Glu Val Ser Gly Ile Lys Ala Leu Tyr Glu Ser Glu Leu
   65                  70                  75                  80
   Ala Asp Ala Arg Arg Val Leu Asp Glu Thr Ala Arg Glu Arg Ala Arg
   85 90 95
   Leu Gln Ile Glu Ile Gly Lys Leu Arg Ala Glu Leu Asp Glu Val Asn
   100 105 110
   Lys Ser Ala Lys Lys Arg Glu Gly Glu Leu Thr Val Ala Gln Gly Arg
   115 120 125
   Val Lys Asp Leu Glu Ser Leu Phe His Arg Ser Glu Val Glu Leu Ala
   130 135 140
   Ala Ala Leu Ser Asp Lys Arg Gly Leu Glu Ser Asp Val Ala Glu Leu
   145 150 155 160
   Arg Ala Gln Leu Ala Lys Ala Glu Asp Gly His Ala Val Ala Lys Lys
   165 170 175
   Gln Leu Glu Lys Glu Thr Leu Met Arg Val Asp Leu Glu Asn Arg Cys
   180 185 190
   Gln Ser Leu Gln Glu Glu Leu Asp Phe Arg Lys Ser Val Phe Glu Glu
   195 200 205
   Glu Val Arg Glu Thr Arg Arg Arg His Glu Arg Arg Leu Val Glu Val
   210 215 220
   Asp Ser Ser Arg Gln Gln Glu Tyr Asp Phe Lys Met Ala Gln Ala Leu
   225 230 235 240
   Glu Glu Leu Arg Ser Gln His Asp Glu Gln Val Arg Leu Tyr Lys Leu
   245 250 255
   Glu Leu Glu Gln Thr Tyr Gln Ala Lys Leu Asp Ser Ala Lys Leu Ser
   260 265 270
   Ser Asp Gln Asn Asp Lys Ala Ala Ser Ala Ala Arg Glu Glu Leu Lys
   275 280 285
   Glu Ala Arg Met Arg Leu Glu Ser Leu Ser Tyr Gln Leu Ser Gly Leu
   290 295 300
   Gln Lys Gln Ala Ser Ala Ala Glu Asp Arg Ile Arg Glu Leu Glu Glu
   305 310 315 320
   Ala Met Ala Gly Glu Arg Asp Lys Phe Arg Lys Met Leu Asp Ala Lys
   325 330 335
   Glu Gln Glu Met Thr Glu Met Arg Asp Val Met Gln Gln Gln Leu Ala
   340 345 350
   Glu Tyr Gln Glu Leu Leu Asp Val Lys Leu Ala Leu Asp Met Glu Ile
   355 360 365
   Asn Ala Tyr Arg Lys Leu Leu Glu Gly Glu Glu Glu Arg Leu Lys Leu
   370 375 380
   Ser Pro Ser Pro Ser Ser Arg Val Thr Val Ser Arg Ala Thr Ser Ser
   385                 390                 395                 400
   Ser Ser Gly Ser Leu Ser Ala Thr Gly Arg Leu Gly Arg Ser Lys Arg
                   405                 410                 415
   Lys Arg Leu Glu Val Glu Glu Pro Leu Gly Ser Gly Pro Ser Val Leu
               420                 425                 430
   Gly Thr Gly Thr Gly Gly Ser Gly Gly Phe His Leu Ala Gln Gln Ala
           435                 440                 445
   Ser Ala Ser Gly Ser Val Ser Ile Glu Glu Ile Asp Leu Glu Gly Lys
       450                 455                 460
   Phe Val Gln Leu Lys Asn Asn Ser Asp Lys Asp Gln Ser Leu Gly Asn
   465                 470                 475                 480
   Trp Arg Ile Lys Arg Gln Val Leu Glu Gly Glu Glu Ile Ala Tyr Lys
                   485                 490                 495
   Phe Thr Pro Lys Tyr Ile Leu Arg Ala Gly Gln Met Val Thr Val Trp
               500                 505                 510
   Ala Ala Gly Ala Gly Val Ala His Ser Pro Pro Ser Thr Leu Val Trp
           515                 520                 525
   Lys Gly Gln Ser Ser Trp Gly Thr Gly Glu Ser Phe Arg Thr Val Leu
       530                 535                 540
   Val Asn Ala Asp Gly Glu Glu Val Ala Met Arg Thr Val Lys Lys Ser
   545                 550                 555                 560
   Ser Val Met Arg Glu Asn Glu Asn Gly Glu Glu Glu Glu Glu Glu Ala
                   565                 570                 575
   Glu Phe Gly Glu Glu Asp Leu Phe His Gln Gln Gly Asp Pro Arg Thr
               580                 585                 590
   Thr Ser Arg Gly Cys Tyr Val Met
           595                 600

<210>  44

<211>  664

<212>  PRT

<213>  Homo sapiens

<300>

<308>  sw_hum/lama_human

<309>  1986-07-21

<400>  44

Met Glu Thr Pro Ser Gln Arg Arg Ala Thr Arg Ser Gly Ala Gln Ala

1               5                   10                  15

Ser Ser Thr Pro Leu Ser Pro Thr Arg Ile Thr Arg Leu Gln Glu Lys
            20                  25                  30

Glu Asp Leu Gln Glu Leu Asn Asp Arg Leu Ala Val Tyr Ile Asp Arg
        35                  40                  45

Val Arg Ser Leu Glu Thr Glu Asn Ala Gly Leu Arg Leu Arg Ile Thr
    50                  55                  60

Glu Ser Glu Glu Val Val Ser Arg Glu Val Ser Gly Ile Lys Ala Ala
65                  70                  75                  80

Tyr Glu Ala Glu Leu Gly Asp Ala Arg Lys Thr Leu Asp Ser Val Ala
                85                  90                  95

Lys Glu Arg Ala Arg Leu Gln Leu Glu Leu Ser Lys Val Arg Glu Glu
            100                 105                 110

Phe Lys Glu Leu Lys Ala Arg Asn Thr Lys Lys Glu Gly Asp Leu Ile
        115                 120                 125

Ala Ala Gln Ala Arg Leu Lys Asp Leu Glu Ala Leu Leu Asn Ser Lys
    130                 135                 140

Glu Ala Ala Leu Ser Thr Ala Leu Ser Glu Lys Arg Thr Leu Glu Gly
145                 150                 155                 160

Glu Leu His Asp Leu Arg Gly Gln Val Ala Lys Leu Glu Ala Ala Leu
                165                 170                 175

Gly Glu Ala Lys Lys Gln Leu Gln Asp Glu Met Leu Arg Arg Val Asp
            180                 185                 190

Ala Glu Asn Arg Leu Gln Thr Met Lys Glu Glu Leu Asp Phe Gln Lys
        195                 200                 205

Asn Ile Tyr Ser Glu Glu Leu Arg Glu Thr Lys Arg Arg His Glu Thr
    210                 215                 220

Arg Leu Val Glu Ile Asp Asn Gly Lys Gln Arg Glu Phe Glu Ser Arg
225                 230                 235                 240

Leu Ala Asp Ala Leu Gln Glu Leu Arg Ala Gln His Glu Asp Gln Val
                245                 250                 255

Glu Gln Tyr Lys Lys Glu Leu Glu Lys Thr Tyr Ser Ala Lys Leu Asp
            260                 265                 270

Asn Ala Arg Gln Ser Ala Glu Arg Asn Ser Asn Leu Val Gly Ala Ala
        275                 280                 285

His Glu Glu Leu Gln Gln Ser Arg Ile Arg Ile Asp Ser Leu Ser Ala
    290                 295                 300

Gln Leu Ser Gln Leu Gln Lys Gln Leu Ala Ala Lys Glu Ala Lys Leu
305                 310                 315                 320

Arg Asp Leu Glu Asp Ser Leu Ala Arg Glu Arg Asp Thr Ser Arg Arg
                325                 330                 335

Leu Leu Ala Glu Lys Glu Arg Glu Met Ala Glu Met Arg Ala Arg Met
            340                 345                 350

Gln Gln Gln Leu Asp Glu Tyr Gln Glu Leu Leu Asp Ile Lys Leu Ala
        355                 360                 365

Leu Asp Met Glu Ile His Ala Tyr Arg Lys Leu Leu Glu Gly Glu Glu
    370                 375                 380

Glu Arg Leu Arg Leu Ser Pro Ser Pro Thr Ser Gln Arg Ser Arg Gly
385                 390                 395                 400

Arg Ala Ser Ser His Ser Ser Gln Thr Gln Gly Gly Gly Ser Val Thr
                405                 410                 415

Lys Lys Arg Lys Leu Glu Ser Thr Glu Ser Arg Ser Ser Phe Ser Gln
            420                 425                 430

His Ala Arg Thr Ser Gly Arg Val Ala Val Glu Glu Val Asp Glu Glu
        435                 440                 445

Gly Lys Phe Val Arg Leu Arg Asn Lys Ser Asn Glu Asp Gln Ser Met
    450                 455                 460

Gly Asn Trp Gln Ile Lys Arg Gln Asn Gly Asp Asp Pro Leu Leu Thr
465                 470                 475                 480

Tyr Arg Phe Pro Pro Lys Phe Thr Leu Lys Ala Gly Gln Val Val Thr
                485                 490                 495

Ile Trp Ala Ala Gly Ala Gly Ala Thr His Ser Pro Pro Thr Asp Leu
            500                 505                 510

Val Trp Lys Ala Gln Asn Thr Trp Gly Cys Gly Asn Ser Leu Arg Thr
        515                 520                 525

Ala Leu Ile Asn Ser Thr Gly Glu Glu Val Ala Met Arg Lys Leu Val
    530                 535                 540

Arg Ser Val Thr Val Val Glu Asp Asp Glu Asp Glu Asp Gly Asp Asp
545                 550                 555                 560

Leu Leu His His His His Gly Ser His Cys Ser Ser Ser Gly Asp Pro
                565                 570                 575

Ala Glu Tyr Asn Leu Arg Ser Arg Thr Val Leu Cys Gly Thr Cys Gly
            580                 585                 590

Gln Pro Ala Asp Lys Ala Ser Ala Ser Gly Ser Gly Ala Gln Val Gly
        595                 600                 605

Gly Pro Ile Ser Ser Gly Ser Ser Ala Ser Ser Val Thr Val Thr Arg
    610                 615                 620

Ser Tyr Arg Ser Val Gly Gly Ser Gly Gly Gly Ser Phe Gly Asp Asn
625                 630                 635                 640

Leu Val Thr Arg Ser Tyr Leu Leu Gly Asn Ser Ser Pro Arg Thr Gln
                645                 650                 655

Ser Pro Gln Asn Cys Ser Ile Met
            660

<210>  45

<211>  331

<212>  PRT

<213>  Homo sapiens

<300>

<308>  sw_hum/ldha_human

<309>  1986-07-21

<400>  45

Ala Thr Leu Lys Asp Gln Leu Ile Tyr Asn Leu Leu Lys Glu Glu Gln
1               5                   10                  15

Thr Pro Gln Asn Lys Ile Thr Val Val Gly Val Gly Ala Val Gly Met
            20                  25                  30

Ala Cys Ala Ile Ser Ile Leu Met Lys Asp Leu Ala Asp Glu Leu Ala
        35                  40                  45

Leu Val Asp Val Ile Glu Asp Lys Leu Lys Gly Glu Met Met Asp Leu
    50                  55                  60

Gln His Gly Ser Leu Phe Leu Arg Thr Pro Lys Ile Val Ser Gly Lys
65                  70                  75                  80

Asp Tyr Asn Val Thr Ala Asn Ser Lys Leu Val Ile Ile Thr Ala Gly
                85                  90                  95

Ala Arg Gln Gln Glu Gly Glu Ser Arg Leu Asn Leu Val Gln Arg Asn
            100                 105                 110

Val Asn Ile Phe Lys Phe Ile Ile Pro Asn Val Val Lys Tyr Ser Pro
        115                 120                 125

Asn Cys Lys Leu Leu Ile Val Ser Asn Pro Val Asp Ile Leu Thr Tyr
    130                 135                 140

Val Ala Trp Lys Ile Ser Gly Phe Pro Lys Asn Arg Val Ile Gly Ser
145                 150                 155                 160

Gly Cys Asn Leu Asp Ser Ala Arg Phe Arg Tyr Leu Met Gly Glu Arg
                165                 170                 175

Leu Gly Val His Pro Leu Ser Cys His Gly Trp Val Leu Gly Glu His
            180                 185                 190

Gly Asp Ser Ser Val Pro Val Trp Ser Gly Met Asn Val Ala Gly Val
        195                 200                 205

Ser Leu Lys Thr Leu His Pro Asp Leu Gly Thr Asp Lys Asp Lys Glu
    210                 215                 220

Gln Trp Lys Glu Val His Lys Gln Val Val Glu Ser Ala Tyr Glu Val
225                 230                 235                 240

Ile Lys Leu Lys Gly Tyr Thr Ser Trp Ala Ile Gly Leu Ser Val Ala
                245                 250                 255

Asp Leu Ala Glu Ser Ile Met Lys Asn Leu Arg Arg Val His Pro Val
            260                 265                 270

Ser Thr Met Ile Lys Gly Leu Tyr Gly Ile Lys Asp Asp Val Phe Leu
        275                 280                 285

Ser Val Pro Cys Ile Leu Gly Gln Asn Gly Ile Ser Asp Leu Val Lys
    290                 295                 300

Val Thr Leu Thr Ser Glu Glu Glu Ala Arg Leu Lys Lys Ser Ala Asp
305                 310                 315                 320

Thr Leu Trp Gly Ile Gln Lys Glu Leu Gln Phe
                325                 330

<210> 46

<211> 268

<212> PRT

<213> Homo sapiens

<300>

<308> sw_hum/mae1_human

<309> 2003-02-28

<400> 46

Met Ala Val Asn Val Tyr Ser Thr Ser Val Thr Ser Asp Asn Leu Ser
1               5                   10                  15

Arg His Asp Met Leu Ala Trp Ile Asn Glu Ser Leu Gln Leu Asn Leu
            20                  25                  30

Thr Lys Ile Glu Gln Leu Cys Ser Gly Ala Ala Tyr Cys Gln Phe Met
        35                  40                  45

Asp Met Leu Phe Pro Gly Ser Ile Ala Leu Lys Lys Val Lys Phe Gln
    50                  55                  60

Ala Lys Leu Glu His Glu Tyr Ile Gln Asn Phe Lys Ile Leu Gln Ala

65                  70                  75                  80

Gly Phe Lys Arg Met Gly Val Asp Lys Ile Ile Pro Val Asp Lys Leu
                85                  90                  95

Val Lys Gly Lys Phe Gln Asp Asn Phe Glu Phe Val Gln Trp Phe Lys
            100                 105                 110

Lys Phe Phe Asp Ala Asn Tyr Asp Gly Lys Asp Tyr Asp Pro Val Ala
        115                 120                 125

Ala Arg Gln Gly Gln Glu Thr Ala Val Ala Pro Ser Leu Val Ala Pro
    130                 135                 140

Ala Leu Asn Lys Pro Lys Lys Pro Leu Thr Ser Ser Ser Ala Ala Pro
145                 150                 155                 160

Gln Arg Pro Ile Ser Thr Gln Arg Thr Ala Ala Ala Pro Lys Ala Gly
                165                 170                 175

Pro Gly Val Val Arg Lys Asn Pro Gly Val Gly Asn Gly Asp Asp Glu
            180                 185                 190

Ala Ala Glu Leu Met Gln Gln Val Asn Val Leu Lys Leu Thr Val Glu
        195                 200                 205

Asp Leu Glu Lys Glu Arg Asp Phe Tyr Phe Gly Lys Leu Arg Asn Ile
    210                 215                 220

Glu Leu Ile Cys Gln Glu Asn Glu Gly Glu Asn Asp Pro Val Leu Gln
225                 230                 235                 240

Arg Ile Val Asp Ile Leu Tyr Ala Thr Asp Glu Gly Phe Val Ile Pro
                245                 250                 255

Asp Glu Gly Gly Pro Gln Glu Glu Gln Glu Glu Tyr
            260                 265

<210>  47

<211>  572

<212>  PRT

<213>  Homo sapiens

<300>

<308>  sw_hum/maox_human

<309>  1996-02-01

<400>  47

Met Glu Pro Glu Ala Pro Arg Arg Arg His Thr His Gln Arg Gly Tyr
1               5                   10                  15

Leu Leu Thr Arg Asn Pro His Leu Asn Lys Asp Leu Ala Phe Thr Leu
            20                  25                  30

Glu Glu Arg Gln Gln Leu Asn Ile His Gly Leu Leu Pro Pro Ser Phe
        35                  40                  45

Asn Ser Gln Glu Ile Gln Val Leu Arg Val Val Lys Asn Phe Glu His
    50                  55                  60

Leu Asn Ser Asp Phe Asp Arg Tyr Leu Leu Leu Met Asp Leu Gln Asp
65                  70                  75                  80

Arg Asn Glu Lys Leu Phe Tyr Arg Val Leu Thr Ser Asp Ile Glu Lys
                85                  90                  95

Phe Met Pro Ile Val Tyr Thr Pro Thr Val Gly Leu Ala Cys Gln Gln
            100                 105                 110

Tyr Ser Leu Val Phe Arg Lys Pro Arg Gly Leu Phe Ile Thr Ile His
        115                 120                 125

Asp Arg Gly His Ile Ala Ser Val Leu Asn Ala Trp Pro Glu Asp Val
    130                 135                 140

Ile Lys Ala Ile Val Val Thr Asp Gly Glu Arg Ile Leu Gly Leu Gly
145                 150                 155                 160

Asp Leu Gly Cys Asn Gly Met Gly Ile Pro Val Gly Lys Leu Ala Leu
                165                 170                 175

Tyr Thr Ala Cys Gly Gly Met Asn Pro Gln Glu Cys Leu Pro Val Ile
            180                 185                 190

Leu Asp Val Gly Thr Glu Asn Glu Glu Leu Leu Lys Asp Pro Leu Tyr
        195                 200                 205

Ile Gly Leu Arg Gln Arg Arg Val Arg Gly Ser Glu Tyr Asp Asp Phe
    210                 215                 220

Leu Asp Glu Phe Met Glu Ala Val Ser Ser Lys Tyr Gly Met Asn Cys
225                 230                 235                 240

Leu Ile Gln Phe Glu Asp Phe Ala Asn Val Asn Ala Phe Arg Leu Leu
                245                 250                 255

Asn Lys Tyr Arg Asn Gln Tyr Cys Thr Phe Asn Asp Asp Ile Gln Gly
            260                 265                 270

Thr Ala Ser Val Ala Val Ala Gly Leu Leu Ala Ala Leu Arg Ile Thr
        275                 280                 285

Lys Asn Lys Leu Ser Asp Gln Thr Ile Leu Phe Gln Gly Ala Gly Glu
    290                 295                 300

Ala Ala Leu Gly Ile Ala His Leu Ile Val Met Ala Leu Glu Lys Glu
305                 310                 315                 320

Gly Leu Pro Lys Glu Lys Ala Ile Lys Lys Ile Trp Leu Val Asp Ser
                325                 330                 335

Lys Gly Leu Ile Val Lys Gly Arg Ala Ser Leu Thr Gln Glu Lys Glu
            340                 345                 350

Lys Phe Ala His Glu His Glu Glu Met Lys Asn Leu Glu Ala Ile Val
        355                 360                 365

Gln Glu Ile Lys Pro Thr Ala Leu Ile Gly Val Ala Ala Ile Gly Gly
    370                 375                 380

Ala Phe Ser Glu Gln Ile Leu Lys Asp Met Ala Ala Phe Asn Glu Arg
385                 390                 395                 400

Pro Ile Ile Phe Ala Leu Ser Asn Pro Thr Ser Lys Ala Glu Cys Ser
                405                 410                 415

Ala Glu Gln Cys Tyr Lys Ile Thr Lys Gly Arg Ala Ile Phe Ala Ser
            420                 425                 430

Gly Ser Pro Phe Asp Pro Val Thr Leu Pro Asn Gly Gln Thr Leu Tyr
        435                 440                 445

Pro Gly Gln Gly Asn Asn Ser Tyr Val Phe Pro Gly Val Ala Leu Gly
    450                 455                 460

Val Val Ala Cys Gly Leu Arg Gln Ile Thr Asp Asn Ile Phe Leu Thr
465                 470                 475                 480

Thr Ala Glu Val Ile Ala Gln Gln Val Ser Asp Lys His Leu Glu Glu
                485                 490                 495

Gly Arg Leu Tyr Pro Pro Leu Asn Thr Ile Arg Asp Val Ser Leu Lys
            500                 505                 510

Ile Ala Glu Lys Ile Val Lys Asp Ala Tyr Gln Glu Lys Thr Ala Thr
        515                 520                 525

Val Tyr Pro Glu Pro Gln Asn Lys Glu Ala Phe Val Arg Ser Gln Met
    530                 535                 540

Tyr Ser Thr Asp Tyr Asp Gln Ile Leu Pro Asp Cys Tyr Ser Trp Pro
545                 550                 555                 560

Glu Glu Val Gln Lys Ile Gln Thr Lys Val Asp Gln
                565                 570

<210>  48

<211>  333

<212>  PRT

<213>  Homo sapiens


<300>

<308>  sw_hum/mdhc_human

<309>  1995-02-01

<400> 48

Ser Glu Pro Ile Arg Val Leu Val Thr Gly Ala Ala Gly Gln Ile Ala
1               5                   10                  15

Tyr Ser Leu Leu Tyr Ser Ile Gly Asn Gly Ser Val Phe Gly Lys Asp
            20                  25                  30

Gln Pro Ile Ile Leu Val Leu Leu Asp Ile Thr Pro Met Met Gly Val
        35                  40                  45

Leu Asp Gly Val Leu Met Glu Leu Gln Asp Cys Ala Leu Pro Leu Leu
    50                  55                  60

Lys Asp Val Ile Ala Thr Asp Lys Glu Asp Val Ala Phe Lys Asp Leu
65                  70                  75                  80

Asp Val Ala Ile Leu Val Gly Ser Met Pro Arg Arg Glu Gly Met Glu
                85                  90                  95

Arg Lys Asp Leu Leu Lys Ala Asn Val Lys Ile Phe Lys Ser Gln Gly
            100                 105                 110

Ala Ala Leu Asp Lys Tyr Ala Lys Lys Ser Val Lys Val Ile Val Val
        115                 120                 125

Gly Asn Pro Ala Asn Thr Asn Cys Leu Thr Ala Ser Lys Ser Ala Pro
    130                 135                 140

Ser Ile Pro Lys Glu Asn Phe Ser Cys Leu Thr Arg Leu Asp His Asn
145                 150                 155                 160

Arg Ala Lys Ala Gln Ile Ala Leu Lys Leu Gly Val Thr Ala Asn Asp
                165                 170                 175

Val Lys Asn Val Ile Ile Trp Gly Asn His Ser Ser Thr Gln Tyr Pro
            180                 185                 190

Asp Val Asn His Ala Lys Val Lys Leu Gln Gly Lys Glu Val Gly Val
        195                 200                 205

Tyr Glu Ala Leu Lys Asp Asp Ser Trp Leu Lys Gly Glu Phe Val Thr
    210                 215                 220

Thr Val Gln Gln Arg Gly Ala Ala Val Ile Lys Ala Arg Lys Leu Ser
225                 230                 235                 240

Ser Ala Met Ser Ala Ala Lys Ala Ile Cys Asp His Val Arg Asp Ile
                245                 250                 255

Trp Phe Gly Thr Pro Glu Gly Glu Phe Val Ser Met Gly Val Ile Ser
            260                 265                 270

Asp Gly Asn Ser Tyr Gly Val Pro Asp Asp Leu Leu Tyr Ser Phe Pro
        275                 280                 285

Val Val Ile Lys Asn Lys Thr Trp Lys Phe Val Glu Gly Leu Pro Ile

290                 295                 300

Asn Asp Phe Ser Arg Glu Lys Met Asp Leu Thr Ala Lys Glu Leu Thr
305                 310                 315                 320

Glu Glu Lys Glu Ser Ala Phe Glu Phe Leu Ser Ser Ala
                325                 330

<210>  49

<211>  152

<212>  PRT

<213>  Homo sapiens

<300>

<308>  sw_hum/ndka_human

<309>  1990-04-01

<400>  49

Met Ala Asn Cys Glu Arg Thr Phe Ile Ala Ile Lys Pro Asp Gly Val
1               5                   10                  15

Gln Arg Gly Leu Val Gly Glu Ile Ile Lys Arg Phe Glu Gln Lys Gly
            20                  25                  30

Phe Arg Leu Val Gly Leu Lys Phe Met Gln Ala Ser Glu Asp Leu Leu
        35                  40                  45

Lys Glu His Tyr Val Asp Leu Lys Asp Arg Pro Phe Phe Ala Gly Leu
    50                  55                  60

Val Lys Tyr Met His Ser Gly Pro Val Val Ala Met Val Trp Glu Gly
65                  70                  75                  80

Leu Asn Val Val Lys Thr Gly Arg Val Met Leu Gly Glu Thr Asn Pro
                85                  90                  95

Ala Asp Ser Lys Pro Gly Thr Ile Arg Gly Asp Phe Cys Ile Gln Val
            100                 105                 110

Gly Arg Asn Ile Ile His Gly Ser Asp Ser Val Glu Ser Ala Glu Lys
        115                 120                 125

Glu Ile Gly Leu Trp Phe His Pro Glu Glu Leu Val Asp Tyr Thr Ser
    130                 135                 140

Cys Ala Gln Asn Trp Ile Tyr Glu
145                 150

<210>  50

<211>  394

<212>  PRT

<213>  Homo sapiens

<300>

<308>  sw_hum/ndr1_human

<309>  1999-07-15

<400>  50

Met Ser Arg Glu Met Gln Asp Val Asp Leu Ala Glu Val Lys Pro Leu
1               5                   10                  15

Val Glu Lys Gly Glu Thr Ile Thr Gly Leu Leu Gln Glu Phe Asp Val
            20                  25                  30

Gln Glu Gln Asp Ile Glu Thr Leu His Gly Ser Val His Val Thr Leu
        35                  40                  45

Cys Gly Thr Pro Lys Gly Asn Arg Pro Val Ile Leu Thr Tyr His Asp
    50                  55                  60

Ile Gly Met Asn His Lys Thr Cys Tyr Asn Pro Leu Phe Asn Tyr Glu
65                  70                  75                  80

Asp Met Gln Glu Ile Thr Gln His Phe Ala Val Cys His Val Asp Ala
                85                  90                  95

Pro Gly Gln Gln Asp Gly Ala Ala Ser Phe Pro Ala Gly Tyr Met Tyr
            100                 105                 110

Pro Ser Met Asp Gln Leu Ala Glu Met Leu Pro Gly Val Leu Gln Gln
        115                 120                 125

Phe Gly Leu Lys Ser Ile Ile Gly Met Gly Thr Gly Ala Gly Ala Tyr
    130                 135                 140

Ile Leu Thr Arg Phe Ala Leu Asn Asn Pro Glu Met Val Glu Gly Leu
145                 150                 155                 160

Val Leu Ile Asn Val Asn Pro Cys Ala Glu Gly Trp Met Asp Trp Ala
                165                 170                 175

Ala Ser Lys Ile Ser Gly Trp Thr Gln Ala Leu Pro Asp Met Val Val
            180                 185                 190

Ser His Leu Phe Gly Lys Glu Glu Met Gln Ser Asn Val Glu Val Val
        195                 200                 205

His Thr Tyr Arg Gln His Ile Val Asn Asp Met Asn Pro Gly Asn Leu
    210                 215                 220

His Leu Phe Ile Asn Ala Tyr Asn Ser Arg Arg Asp Leu Glu Ile Glu
225                 230                 235                 240

Arg Pro Met Pro Gly Thr His Thr Val Thr Leu Gln Cys Pro Ala Leu
                245                 250                 255

Leu Val Val Gly Asp Ser Ser Pro Ala Val Asp Ala Val Val Glu Cys
            260                 265                 270

Asn Ser Lys Leu Asp Pro Thr Lys Thr Thr Leu Leu Lys Met Ala Asp
        275                 280                 285

Cys Gly Gly Leu Pro Gln Ile Ser Gln Pro Ala Lys Leu Ala Glu Ala
    290                 295                 300

Phe Lys Tyr Phe Val Gln Gly Met Gly Tyr Met Pro Ser Ala Ser Met
305                 310                 315                 320

Thr Arg Leu Met Arg Ser Arg Thr Ala Ser Gly Ser Ser Val Thr Ser
                325                 330                 335

Leu Asp Gly Thr Arg Ser Arg Ser His Thr Ser Glu Gly Thr Arg Ser
            340                 345                 350

Arg Ser His Thr Ser Glu Gly Thr Arg Ser Arg Ser His Thr Ser Glu
        355                 360                 365

Gly Ala His Leu Asp Ile Thr Pro Asn Ser Gly Ala Ala Gly Asn Ser
    370                 375                 380

Ala Gly Pro Lys Ser Met Glu Val Ser Cys
385                 390

<210>  51

<211>  261

<212>  PRT

<213>  Homo sapiens

<300>

<308>  sw_hum/pcna_human

<309>  1989-10-01

<400>  51

Met Phe Glu Ala Arg Leu Val Gln Gly Ser Ile Leu Lys Lys Val Leu
1               5                   10                  15

Glu Ala Leu Lys Asp Leu Ile Asn Glu Ala Cys Trp Asp Ile Ser Ser
            20                  25                  30

Ser Gly Val Asn Leu Gln Ser Met Asp Ser Ser His Val Ser Leu Val
        35                  40                  45

Gln Leu Thr Leu Arg Ser Glu Gly Phe Asp Thr Tyr Arg Cys Asp Arg
    50                  55                  60

Asn Leu Ala Met Gly Val Asn Leu Thr Ser Met Ser Lys Ile Leu Lys
65                  70                  75                  80

Cys Ala Gly Asn Glu Asp Ile Ile Thr Leu Arg Ala Glu Asp Asn Ala
                85                  90                  95

Asp Thr Leu Ala Leu Val Phe Glu Ala Pro Asn Gln Glu Lys Val Ser
            100                 105                 110

Asp Tyr Glu Met Lys Leu Met Asp Leu Asp Val Glu Gln Leu Gly Ile
        115                 120                 125

Pro Glu Gln Glu Tyr Ser Cys Val Val Lys Met Pro Ser Gly Glu Phe
    130                 135                 140

Ala Arg Ile Cys Arg Asp Leu Ser His Ile Gly Asp Ala Val Val Ile
145                 150                 155                 160

Ser Cys Ala Lys Asp Gly Val Lys Phe Ser Ala Ser Gly Glu Leu Gly
                165                 170                 175

Asn Gly Asn Ile Lys Leu Ser Gln Thr Ser Asn Val Asp Lys Glu Glu
            180                 185                 190

Glu Ala Val Thr Ile Glu Met Asn Glu Pro Val Gln Leu Thr Phe Ala
        195                 200                 205

Leu Arg Tyr Leu Asn Phe Phe Thr Lys Ala Thr Pro Leu Ser Ser Thr
    210                 215                 220

Val Thr Leu Ser Met Ser Ala Asp Val Pro Leu Val Val Glu Tyr Lys
225                 230                 235                 240

Ile Ala Asp Met Gly His Leu Lys Tyr Tyr Leu Ala Pro Lys Ile Glu
                245                 250                 255

Asp Glu Glu Gly Ser
            260

<210>  52

<211>  417

<212>  PRT

<213>  Homo sapiens


<300>

<308>  sw_hum/pgk1_human

<309>  1986-07-21


<400>  52

Ser Leu Ser Asn Lys Leu Thr Leu Asp Lys Leu Asp Val Lys Gly Lys
1               5                   10                  15

Arg Val Val Met Arg Val Asp Phe Asn Val Pro Met Lys Asn Asn Gln
            20                  25                  30

Ile Thr Asn Asn Gln Arg Ile Lys Ala Ala Val Pro Ser Ile Lys Phe

Ala Leu Met Asp Glu Val Val Lys Ala Thr Ser Arg Gly Cys Ile Thr
        355                 360                 365

Ile Ile Gly Gly Gly Asp Thr Ala Thr Cys Cys Ala Lys Trp Asn Thr
    370                 375                 380

Glu Asp Lys Val Ser His Val Ser Thr Gly Gly Gly Ala Ser Leu Glu
385                 390                 395                 400

Leu Leu Glu Gly Lys Val Leu Pro Gly Val Asp Ala Leu Ser Asn Ile
                405                 410                 415

Leu

<210>  53

<211>  418

<212>  PRT

<213>  Homo sapiens

<300>

<308>  sw_hum/prs6_human

<309>  1995-11-01

<400>  53

Met Glu Glu Ile Gly Ile Leu Val Glu Lys Ala Gln Asp Glu Ile Pro
1               5                   10                  15

Ala Leu Ser Val Ser Arg Pro Gln Thr Gly Leu Ser Phe Leu Gly Pro
            20                  25                  30

Glu Pro Glu Asp Leu Glu Asp Leu Tyr Ser Arg Tyr Lys Lys Leu Gln
        35                  40                  45

Gln Glu Leu Glu Phe Leu Glu Val Gln Glu Glu Tyr Ile Lys Asp Glu
    50                  55                  60

Gln Lys Asn Leu Lys Lys Glu Phe Leu His Ala Gln Glu Glu Val Lys
65                  70                  75                  80

Arg Ile Gln Ser Ile Pro Leu Val Ile Gly Gln Phe Leu Glu Ala Val
                85                  90                  95

Asp Gln Asn Thr Ala Ile Val Gly Ser Thr Thr Gly Ser Asn Tyr Tyr
            100                 105                 110

Val Arg Ile Leu Ser Thr Ile Asp Arg Glu Leu Leu Lys Pro Asn Ala
        115                 120                 125

Ser Val Ala Leu His Lys His Ser Asn Ala Leu Val Asp Val Leu Pro
    130                 135                 140

Pro Glu Ala Asp Ser Ser Ile Met Met Leu Thr Ser Asp Gln Lys Pro
145                 150                 155                 160

Asp Val Met Tyr Ala Asp Ile Gly Gly Met Asp Ile Gln Lys Gln Glu
                165                 170                 175

Val Arg Glu Ala Val Glu Leu Pro Leu Thr His Phe Glu Leu Tyr Lys
            180                 185                 190

Gln Ile Gly Ile Asp Pro Pro Arg Gly Val Leu Met Tyr Gly Pro Pro
        195                 200                 205

Gly Cys Gly Lys Thr Met Leu Ala Lys Ala Val Ala His His Thr Thr
    210                 215                 220

Ala Ala Phe Ile Arg Val Val Gly Ser Glu Phe Val Gln Lys Tyr Leu
225                 230                 235                 240

Gly Glu Gly Pro Arg Met Val Arg Asp Val Phe Arg Leu Ala Lys Glu
                245                 250                 255

Asn Ala Pro Ala Ile Ile Phe Ile Asp Glu Ile Asp Ala Ile Ala Thr
            260                 265                 270

Lys Arg Phe Asp Ala Gln Thr Gly Ala Asp Arg Glu Val Gln Arg Ile
        275                 280                 285

Leu Leu Glu Leu Leu Asn Gln Met Asp Gly Phe Asp Gln Asn Val Asn
    290                 295                 300

Val Lys Val Ile Met Ala Thr Asn Arg Ala Asp Thr Leu Asp Pro Ala
305                 310                 315                 320

Leu Leu Arg Pro Gly Arg Leu Asp Arg Lys Ile Glu Phe Pro Leu Pro
                325                 330                 335

Asp Arg Arg Gln Lys Arg Leu Ile Phe Ser Thr Ile Thr Ser Lys Met
            340                 345                 350

Asn Leu Ser Glu Glu Val Asp Leu Glu Asp Tyr Val Ala Arg Pro Asp
        355                 360                 365

Lys Ile Ser Gly Ala Asp Ile Asn Ser Ile Cys Gln Glu Ser Gly Met
    370                 375                 380

Leu Ala Val Arg Glu Asn Arg Tyr Ile Val Leu Ala Lys Asp Phe Glu
385                 390                 395                 400

Lys Ala Tyr Lys Thr Val Ile Lys Lys Asp Glu Gln Glu His Glu Phe
                405                 410                 415

Tyr Lys

<210>  54

<211>  277

<212>  PRT

<213>  Homo sapiens

<300>

<308>  sw_hum/psb7_human

<309>  2001-10-16

<400>  54

Met Ala Ala Val Ser Val Tyr Ala Pro Pro Val Gly Gly Phe Ser Phe
1               5                   10                  15

Asp Asn Cys Arg Arg Asn Ala Val Leu Glu Ala Asp Phe Ala Lys Arg
            20                  25                  30

Gly Tyr Lys Leu Pro Lys Val Arg Lys Thr Gly Thr Thr Ile Ala Gly
        35                  40                  45

Val Val Tyr Lys Asp Gly Ile Val Leu Gly Ala Asp Thr Arg Ala Thr
    50                  55                  60

Glu Gly Met Val Val Ala Asp Lys Asn Cys Ser Lys Ile His Phe Ile
65                  70                  75                  80

Ser Pro Asn Ile Tyr Cys Cys Gly Ala Gly Thr Ala Ala Asp Thr Asp
                85                  90                  95

Met Thr Thr Gln Leu Ile Ser Ser Asn Leu Glu Leu His Ser Leu Ser
            100                 105                 110

Thr Gly Arg Leu Pro Arg Val Val Thr Ala Asn Arg Met Leu Lys Gln
        115                 120                 125

Met Leu Phe Arg Tyr Gln Gly Tyr Ile Gly Ala Ala Leu Val Leu Gly
    130                 135                 140

Gly Val Asp Val Thr Gly Pro His Leu Tyr Ser Ile Tyr Pro His Gly
145                 150                 155                 160

Ser Thr Asp Lys Leu Pro Tyr Val Thr Met Gly Ser Gly Ser Leu Ala
                165                 170                 175

Ala Met Ala Val Phe Glu Asp Lys Phe Arg Pro Asp Met Glu Glu Glu
            180                 185                 190

Glu Ala Lys Asn Leu Val Ser Glu Ala Ile Ala Ala Gly Ile Phe Asn
        195                 200                 205

Asp Leu Gly Ser Gly Ser Asn Ile Asp Leu Cys Val Ile Ser Lys Asn
    210                 215                 220

Lys Leu Asp Phe Leu Arg Pro Tyr Thr Val Pro Asn Lys Lys Gly Thr
225                 230                 235                 240

Arg Leu Gly Arg Tyr Arg Cys Glu Lys Gly Thr Thr Ala Val Leu Thr

245                 250                 255

Glu Lys Ile Thr Pro Leu Glu Ile Glu Val Leu Glu Glu Thr Val Gln
        260                 265                 270

Thr Met Asp Thr Ser
        275

<210>  55

<211>  409

<212>  PRT

<213>  Homo sapiens

<300>

<308>  sw_hum/r23b_human

<309>  1996-10-01

<400>  55

Met Gln Val Thr Leu Lys Thr Leu Gln Gln Gln Thr Phe Lys Ile Asp
1               5                   10                  15

Ile Asp Pro Glu Glu Thr Val Lys Ala Leu Lys Glu Lys Ile Glu Ser
            20                  25                  30

Glu Lys Gly Lys Asp Ala Phe Pro Val Ala Gly Gln Lys Leu Ile Tyr
        35                  40                  45

Ala Gly Lys Ile Leu Asn Asp Asp Thr Ala Leu Lys Glu Tyr Lys Ile
    50                  55                  60

Asp Glu Lys Asn Phe Val Val Val Met Val Thr Lys Pro Lys Ala Val
65                  70                  75                  80

Ser Thr Pro Ala Pro Ala Thr Thr Gln Gln Ser Ala Pro Ala Ser Thr
                85                  90                  95

Thr Ala Val Thr Ser Ser Thr Thr Thr Thr Val Ala Gln Ala Pro Thr
            100                 105                 110

Pro Val Pro Ala Leu Ala Pro Thr Ser Thr Pro Ala Ser Ile Thr Pro
        115                 120                 125

Ala Ser Ala Thr Ala Ser Ser Glu Pro Ala Pro Ala Ser Ala Ala Lys
    130                 135                 140

Gln Glu Lys Pro Ala Glu Lys Pro Ala Glu Thr Pro Val Ala Thr Ser
145                 150                 155                 160

Pro Thr Ala Thr Asp Ser Thr Ser Gly Asp Ser Ser Arg Ser Asn Leu
                165                 170                 175

Phe Glu Asp Ala Thr Ser Ala Leu Val Thr Gly Gln Ser Tyr Glu Asn
            180                 185                 190

Met Val Thr Glu Ile Met Ser Met Gly Tyr Glu Arg Glu Gln Val Ile
        195                 200                 205

Ala Ala Leu Arg Ala Ser Phe Asn Asn Pro Asp Arg Ala Val Glu Tyr
    210                 215                 220

Leu Leu Met Gly Ile Pro Gly Asp Arg Glu Ser Gln Ala Val Val Asp
225                 230                 235                 240

Pro Pro Gln Ala Ala Ser Thr Gly Ala Pro Gln Ser Ser Ala Val Ala
                245                 250                 255

Ala Ala Ala Ala Thr Thr Thr Ala Thr Thr Thr Thr Thr Ser Ser Gly
            260                 265                 270

Gly His Pro Leu Glu Phe Leu Arg Asn Gln Pro Gln Phe Gln Gln Met
        275                 280                 285

Arg Gln Ile Ile Gln Gln Asn Pro Ser Leu Leu Pro Ala Leu Leu Gln
    290                 295                 300

Gln Ile Gly Arg Glu Asn Pro Gln Leu Leu Gln Gln Ile Ser Gln His
305                 310                 315                 320

Gln Glu His Phe Ile Gln Met Leu Asn Glu Pro Val Gln Glu Ala Gly
                325                 330                 335

Gly Gln Gly Gly Gly Gly Gly Gly Gly Ser Gly Gly Ile Ala Glu Ala
            340                 345                 350

Gly Ser Gly His Met Asn Tyr Ile Gln Val Thr Pro Gln Glu Lys Glu
        355                 360                 365

Ala Ile Glu Arg Leu Lys Ala Leu Gly Phe Pro Glu Gly Leu Val Ile
    370                 375                 380

Gln Ala Tyr Phe Ala Cys Glu Lys Asn Glu Asn Leu Ala Ala Asn Phe
385                 390                 395                 400

Leu Leu Gln Gln Asn Phe Asp Glu Asp
                405

<210>  56

<211>  306

<212>  PRT

<213>  Homo sapiens


<300>

<308>  sw_hum/roc_human

<309>  1988-08-01


<400>  56

Met Ala Ser Asn Val Thr Asn Lys Thr Asp Pro Arg Ser Met Asn Ser
1               5                   10                  15

Arg Val Phe Ile Gly Asn Leu Asn Thr Leu Val Val Lys Lys Ser Asp
            20                  25                  30

Val Glu Ala Ile Phe Ser Lys Tyr Gly Lys Ile Val Gly Cys Ser Val
        35                  40                  45

His Lys Gly Phe Ala Phe Val Gln Tyr Val Asn Glu Arg Asn Ala Arg
    50                  55                  60

Ala Ala Val Ala Gly Glu Asp Gly Arg Met Ile Ala Gly Gln Val Leu
65                  70                  75                  80

Asp Ile Asn Leu Ala Ala Glu Pro Lys Val Asn Arg Gly Lys Ala Gly
                85                  90                  95

Val Lys Arg Ser Ala Ala Glu Met Tyr Gly Ser Val Thr Glu His Pro
            100                 105                 110

Ser Pro Ser Pro Leu Leu Ser Ser Ser Phe Asp Leu Asp Tyr Asp Phe
        115                 120                 125

Gln Arg Asp Tyr Tyr Asp Arg Met Tyr Ser Tyr Pro Ala Arg Val Pro
    130                 135                 140

Pro Pro Pro Pro Ile Ala Arg Ala Val Val Pro Ser Lys Arg Gln Arg
145                 150                 155                 160

Val Ser Gly Asn Thr Ser Arg Arg Gly Lys Ser Gly Phe Asn Ser Lys
                165                 170                 175

Ser Gly Gln Arg Gly Ser Ser Lys Ser Gly Lys Leu Lys Gly Asp Asp
            180                 185                 190

Leu Gln Ala Ile Lys Lys Glu Leu Thr Gln Ile Lys Gln Lys Val Asp
        195                 200                 205

Ser Leu Leu Glu Asn Leu Glu Lys Met Glu Lys Glu Gln Ser Lys Gln
    210                 215                 220

Ala Val Glu Met Lys Asn Asp Lys Ser Glu Glu Glu Gln Ser Ser Ser
225                 230                 235                 240

Ser Val Lys Lys Asp Glu Thr Asn Val Lys Met Glu Ser Glu Gly Gly
                245                 250                 255

Ala Asp Asp Ser Ala Glu Glu Gly Asp Leu Leu Asp Asp Asp Asp Asn
            260                 265                 270

Glu Asp Arg Gly Asp Asp Gln Leu Glu Leu Ile Lys Asp Asp Glu Lys
        275                 280                 285

Glu Ala Glu Glu Gly Glu Asp Asp Arg Asp Ser Ala Asn Gly Glu Asp
    290                 295                 300

Asp Ser
305

<210> 57

<211> 449

<212> PRT

<213> Homo sapiens

<300>

<308> sw_hum/roh2_human

<309> 1997-11-01

<400> 57

<210>  58

<211>  346

<212>  PRT

<213>  Homo sapiens

<300>

<308>  sw_hum/roh3_human

<309>  1993-07-01

<400>  58

Met Asp Trp Val Met Lys His Asn Gly Pro Asn Asp Ala Ser Asp Gly
1               5                   10                  15

Thr Val Arg Leu Arg Gly Leu Pro Phe Gly Cys Ser Lys Glu Glu Ile
            20                  25                  30

Val Gln Phe Phe Gln Gly Leu Glu Ile Val Pro Asn Gly Ile Thr Leu
        35                  40                  45

Thr Met Asp Tyr Gln Gly Arg Ser Thr Gly Glu Ala Phe Val Gln Phe
    50                  55                  60

Ala Ser Lys Glu Ile Ala Glu Asn Ala Leu Gly Lys His Lys Glu Arg
65                  70                  75                  80

Ile Gly His Arg Tyr Ile Glu Ile Phe Arg Ser Ser Arg Ser Glu Ile
                85                  90                  95

Lys Gly Phe Tyr Asp Pro Pro Arg Arg Leu Leu Gly Gln Arg Pro Gly
            100                 105                 110

Pro Tyr Asp Arg Pro Ile Gly Gly Arg Gly Gly Tyr Tyr Gly Ala Gly
        115                 120                 125

Arg Gly Ser Met Tyr Asp Arg Met Arg Arg Gly Gly Asp Gly Tyr Asp
    130                 135                 140

Gly Gly Tyr Gly Gly Phe Asp Asp Tyr Gly Gly Tyr Asn Asn Tyr Gly
145                 150                 155                 160

Tyr Gly Asn Asp Gly Phe Asp Asp Arg Met Arg Asp Gly Arg Gly Met
                165                 170                 175

Gly Gly His Gly Tyr Gly Gly Ala Gly Asp Ala Ser Ser Gly Phe His
            180                 185                 190

Gly Gly His Phe Val His Met Arg Gly Leu Pro Phe Arg Ala Thr Glu
        195                 200                 205

Asn Asp Ile Ala Asn Phe Phe Ser Pro Leu Asn Pro Ile Arg Val His
    210                 215                 220

Ile Asp Ile Gly Ala Asp Gly Arg Ala Thr Gly Glu Ala Asp Val Glu
225                 230                 235                 240

Phe Val Thr His Glu Asp Ala Val Ala Ala Met Ser Lys Asp Lys Asn
                245                 250                 255

Asn Met Gln His Arg Tyr Ile Glu Leu Phe Leu Asn Ser Thr Pro Gly
            260                 265                 270

Gly Gly Ser Gly Met Gly Gly Ser Gly Met Gly Gly Tyr Gly Arg Asp
        275                 280                 285

Gly Met Asp Asn Gln Gly Gly Tyr Gly Ser Val Gly Arg Met Gly Met
    290                 295                 300

Gly Asn Asn Tyr Ser Gly Gly Tyr Gly Thr Pro Asp Gly Leu Gly Gly
305                 310                 315                 320

Tyr Gly Arg Gly Gly Gly Gly Ser Gly Gly Tyr Tyr Gly Gln Gly Gly
                325                 330                 335

Met Ser Gly Gly Gly Trp Arg Gly Met Tyr
            340                 345

<210>  59

<211>  322

<212>  PRT

<213>  Homo sapiens

<300>

<308>  sw_hum/sc13_human

<309>  1997-11-01

<400>  59

Met Val Ser Val Ile Asn Thr Val Asp Thr Ser His Glu Asp Met Ile
1               5                   10                  15

His Asp Ala Gln Met Asp Tyr Tyr Gly Thr Arg Leu Ala Thr Cys Ser
            20                  25                  30

Ser Asp Arg Ser Val Lys Ile Phe Asp Val Arg Asn Gly Gly Gln Ile
        35                  40                  45

Leu Ile Val Asp Leu Arg Gly His Glu Gly Pro Val Trp Gln Val Ala
    50                  55                  60

Trp Ala His Pro Met Tyr Gly Asn Ile Leu Ala Ser Cys Ser Tyr Asp
65                  70                  75                  80

Arg Lys Val Ile Ile Trp Arg Glu Glu Asn Gly Thr Trp Glu Lys Ser
                85                  90                  95

His Glu His Ala Gly His Asp Ser Ser Val Asn Ser Val Cys Trp Ala
            100                 105                 110

Pro His Asp Tyr Gly Leu Ile Leu Ala Cys Gly Ser Ser Asp Gly Ala
        115                 120                 125

Ile Ser Leu Leu Thr Tyr Thr Gly Glu Gly Gln Trp Glu Val Lys Lys
    130                 135                 140

Ile Asn Asn Ala His Thr Ile Gly Cys Asn Ala Val Ser Trp Ala Pro
145                 150                 155                 160

Ala Val Val Pro Gly Ser Leu Ile Asp His Pro Ser Gly Gln Lys Pro
                165                 170                 175

Asn Tyr Ile Lys Arg Phe Ala Ser Gly Gly Cys Asp Asn Leu Ile Lys
            180                 185                 190

Leu Trp Lys Glu Glu Glu Asp Gly Gln Trp Lys Glu Glu Gln Lys Leu
        195                 200                 205

Glu Ala His Ser Asp Trp Val Arg Asp Val Ala Trp Ala Pro Ser Ile
    210                 215                 220

Gly Leu Pro Thr Ser Thr Ile Ala Ser Cys Ser Gln Asp Gly Arg Val
225                 230                 235                 240

Phe Ile Trp Thr Cys Asp Asp Ala Ser Ser Asn Thr Trp Ser Pro Lys
                245                 250                 255

Leu Leu His Lys Phe Asn Asp Val Val Trp His Val Ser Trp Ser Ile
            260                 265                 270

Thr Ala Asn Ile Leu Ala Val Ser Gly Gly Asp Asn Lys Val Thr Leu
        275                 280                 285

Trp Lys Glu Ser Val Asp Gly Gln Trp Val Cys Ile Ser Asp Val Asn
    290                 295                 300

Lys Gly Gln Gly Ser Val Ser Ala Ser Val Thr Glu Gly Gln Gln Asn
305                 310                 315                 320

Glu Gln

<210>  60

<211>  295

<212>  PRT

<213>  Homo sapiens

<300>

<308>  sw_hum/snaa_human

<309>  1996-10-01

<400>  60

Met Asp Asn Ser Gly Lys Glu Ala Glu Ala Met Ala Leu Leu Ala Glu
1               5                   10                  15

Ala Glu Arg Lys Val Lys Asn Ser Gln Ser Phe Phe Ser Gly Leu Phe
            20                  25                  30

Gly Gly Ser Ser Lys Ile Glu Glu Ala Cys Glu Ile Tyr Ala Arg Ala
        35                  40                  45

Ala Asn Met Phe Lys Met Ala Lys Asn Trp Ser Ala Ala Gly Asn Ala

50                  55                  60

Phe Cys Gln Ala Ala Gln Leu His Leu Gln Leu Gln Ser Lys His Asp
65                  70                  75                  80

Ala Ala Thr Cys Phe Val Asp Ala Gly Asn Ala Phe Lys Lys Ala Asp
                85                  90                  95

Pro Gln Glu Ala Ile Asn Cys Leu Met Arg Ala Ile Glu Ile Tyr Thr
            100                 105                 110

Asp Met Gly Arg Phe Thr Ile Ala Ala Lys His His Ile Ser Ile Ala
        115                 120                 125

Glu Ile Tyr Glu Thr Glu Leu Val Asp Ile Glu Lys Ala Ile Ala His
    130                 135                 140

Tyr Glu Gln Ser Ala Asp Tyr Tyr Lys Gly Glu Glu Ser Asn Ser Ser
145                 150                 155                 160

Ala Asn Lys Cys Leu Leu Lys Val Ala Gly Tyr Ala Ala Leu Leu Glu
                165                 170                 175

Gln Tyr Gln Lys Ala Ile Asp Ile Tyr Glu Gln Val Gly Thr Asn Ala
            180                 185                 190

Met Asp Thr Pro Leu Leu Lys Tyr Ser Ala Lys Asp Tyr Phe Phe Lys
        195                 200                 205

Ala Ala Leu Cys His Phe Cys Ile Asp Met Leu Asn Ala Lys Leu Ala
    210                 215                 220

Val Gln Lys Tyr Glu Glu Leu Phe Pro Ala Phe Ser Asp Ser Arg Glu
225                 230                 235                 240

Cys Lys Leu Met Lys Lys Leu Leu Glu Ala His Glu Glu Gln Asn Val
                245                 250                 255

Asp Ser Tyr Thr Glu Ser Val Lys Glu Tyr Asp Ser Ile Ser Arg Leu
            260                 265                 270

Asp Gln Trp Leu Thr Thr Met Leu Leu Arg Ile Lys Lys Thr Ile Gln
        275                 280                 285

Gly Asp Glu Glu Asp Leu Arg
    290                 295

<210>  61

<211>  302

<212>  PRT

<213>  Homo sapiens


<300>

<308>  sw_hum/spee_human

<309>  1991-02-01

<400>  61

Met Glu Pro Gly Pro Asp Gly Pro Ala Ala Ser Gly Pro Ala Ala Ile
1               5                   10                  15

Arg Glu Gly Trp Phe Arg Glu Thr Cys Ser Leu Trp Pro Gly Gln Ala
            20                  25                  30

Leu Ser Leu Gln Val Glu Gln Leu Leu His His Arg Arg Ser Arg Tyr
        35                  40                  45

Gln Asp Ile Leu Val Phe Arg Ser Lys Thr Tyr Gly Asn Val Leu Val
    50                  55                  60

Leu Asp Gly Val Ile Gln Cys Thr Glu Arg Asp Glu Phe Ser Tyr Gln
65                  70                  75                  80

Glu Met Ile Ala Asn Leu Pro Leu Cys Ser His Pro Asn Pro Arg Lys
                85                  90                  95

Val Leu Ile Ile Gly Gly Gly Asp Gly Gly Val Leu Arg Glu Val Val
            100                 105                 110

Lys His Pro Ser Val Glu Ser Val Val Gln Cys Glu Ile Asp Glu Asp
        115                 120                 125

Val Ile Gln Val Ser Lys Lys Phe Leu Pro Gly Met Ala Ile Gly Tyr
    130                 135                 140

Ser Ser Ser Lys Leu Thr Leu His Val Gly Asp Gly Phe Glu Phe Met
145                 150                 155                 160

Lys Gln Asn Gln Asp Ala Phe Asp Val Ile Ile Thr Asp Ser Ser Asp
                165                 170                 175

Pro Met Gly Pro Ala Glu Ser Leu Phe Lys Glu Ser Tyr Tyr Gln Leu
            180                 185                 190

Met Lys Thr Ala Leu Lys Glu Asp Gly Val Leu Cys Cys Gln Gly Glu
        195                 200                 205

Cys Gln Trp Leu His Leu Asp Leu Ile Lys Glu Met Arg Gln Phe Cys
    210                 215                 220

Gln Ser Leu Phe Pro Val Val Ala Tyr Ala Tyr Cys Thr Ile Pro Thr
225                 230                 235                 240

Tyr Pro Ser Gly Gln Ile Gly Phe Met Leu Cys Ser Lys Asn Pro Ser
                245                 250                 255

Thr Asn Phe Gln Glu Pro Val Gln Pro Leu Thr Gln Gln Gln Val Ala
            260                 265                 270

Gln Met Gln Leu Lys Tyr Tyr Asn Ser Asp Val His Arg Ala Ala Phe
        275                 280                 285

Val Leu Pro Glu Phe Ala Arg Lys Ala Leu Asn Asp Val Ser
    290                 295                 300

<210>  62

<211>  623

<212>  PRT

<213>  Homo sapiens

<300>

<308>  sw_hum/tkt_human

<309>  1992-12-01

<400>  62

Met Glu Ser Tyr His Lys Pro Asp Gln Gln Lys Leu Gln Ala Leu Lys
1               5                   10                  15

Asp Thr Ala Asn Arg Leu Arg Ile Ser Ser Ile Gln Ala Thr Thr Ala
            20                  25                  30

Ala Gly Ser Gly His Pro Thr Ser Cys Cys Ser Ala Ala Glu Ile Met
        35                  40                  45

Ala Val Leu Phe Phe His Thr Met Arg Tyr Lys Ser Gln Asp Pro Arg
    50                  55                  60

Asn Pro His Asn Asp Arg Phe Val Leu Ser Lys Gly His Ala Ala Pro
65                  70                  75                  80

Ile Leu Tyr Ala Val Trp Ala Glu Ala Gly Phe Leu Ala Glu Ala Glu
                85                  90                  95

Leu Leu Asn Leu Arg Lys Ile Ser Ser Asp Leu Asp Gly His Pro Val
            100                 105                 110

Pro Lys Gln Ala Phe Thr Asp Val Ala Thr Gly Ser Leu Gly Gln Gly
        115                 120                 125

Leu Gly Ala Ala Cys Gly Met Ala Tyr Thr Gly Lys Tyr Phe Asp Lys
    130                 135                 140

Ala Ser Tyr Arg Val Tyr Cys Leu Leu Gly Asp Gly Glu Leu Ser Glu
145                 150                 155                 160

Gly Ser Val Trp Glu Ala Met Ala Phe Ala Ser Ile Tyr Lys Leu Asp
                165                 170                 175

Asn Leu Val Ala Ile Leu Asp Ile Asn Arg Leu Gly Gln Ser Asp Pro
            180                 185                 190

Ala Pro Leu Gln His Gln Met Asp Ile Tyr Gln Lys Arg Cys Glu Ala
        195                 200                 205

Phe Gly Trp His Ala Ile Ile Val Asp Gly His Ser Val Glu Glu Leu
    210                 215                 220

Cys Lys Ala Phe Gly Gln Ala Lys His Gln Pro Thr Ala Ile Ile Ala
225                 230                 235                 240

Lys Thr Phe Lys Gly Arg Gly Ile Thr Gly Val Glu Asp Lys Glu Ser
                245                 250                 255

Trp His Gly Lys Pro Leu Pro Lys Asn Met Ala Glu Gln Ile Ile Gln
            260                 265                 270

Glu Ile Tyr Ser Gln Ile Gln Ser Lys Lys Lys Ile Leu Ala Thr Pro
        275                 280                 285

Pro Gln Glu Asp Ala Pro Ser Val Asp Ile Ala Asn Ile Arg Met Pro
    290                 295                 300

Ser Leu Pro Ser Tyr Lys Val Gly Asp Lys Ile Ala Thr Arg Lys Ala
305                 310                 315                 320

Tyr Gly Gln Ala Leu Ala Lys Leu Gly His Ala Ser Asp Arg Ile Ile
                325                 330                 335

Ala Leu Asp Gly Asp Thr Lys Asn Ser Thr Phe Ser Glu Ile Phe Lys
            340                 345                 350

Lys Glu His Pro Asp Arg Phe Ile Glu Cys Tyr Ile Ala Glu Gln Asn
        355                 360                 365

Met Val Ser Ile Ala Val Gly Cys Ala Thr Arg Asn Arg Thr Val Pro
    370                 375                 380

Phe Cys Ser Thr Phe Ala Ala Phe Phe Thr Arg Ala Phe Asp Gln Ile
385                 390                 395                 400

Arg Met Ala Ala Ile Ser Glu Ser Asn Ile Asn Leu Cys Gly Ser His
                405                 410                 415

Cys Gly Val Ser Ile Gly Glu Asp Gly Pro Ser Gln Met Ala Leu Glu
            420                 425                 430

Asp Leu Ala Met Phe Arg Ser Val Pro Thr Ser Thr Val Phe Tyr Pro
        435                 440                 445

Ser Asp Gly Val Ala Thr Glu Lys Ala Val Glu Leu Ala Ala Asn Thr
    450                 455                 460

Lys Gly Ile Cys Phe Ile Arg Thr Ser Arg Pro Glu Asn Ala Ile Ile
465                 470                 475                 480

Tyr Asn Asn Asn Glu Asp Phe Gln Val Gly Gln Ala Lys Val Val Leu
                485                 490                 495

Lys Ser Lys Asp Asp Gln Val Thr Val Ile Gly Ala Gly Val Thr Leu
            500                 505                 510

His Glu Ala Leu Ala Ala Ala Glu Leu Leu Lys Lys Glu Lys Ile Asn

515                    520                    525

Ile Arg Val Leu Asp Pro Phe Thr Ile Lys Pro Leu Asp Arg Lys Leu
    530                    535                    540

Ile Leu Asp Ser Ala Arg Ala Thr Lys Gly Arg Ile Leu Thr Val Glu
545                    550                    555                    560

Asp His Tyr Tyr Glu Gly Gly Ile Gly Glu Ala Val Ser Ser Ala Val
                565                    570                    575

Val Gly Glu Pro Gly Ile Thr Val Thr His Leu Ala Val Asn Arg Val
            580                    585                    590

Pro Arg Ser Gly Lys Pro Ala Glu Leu Leu Lys Met Phe Gly Ile Asp
        595                    600                    605

Arg Asp Ala Ile Ala Gln Ala Val Arg Gly Leu Ile Thr Lys Ala
    610                    615                    620

<210>  63

<211>  1058

<212>  PRT

<213>  Homo sapiens

<300>

<308>  w_hum/uba1_human

<309>  1991-08-01

<400>  63

<210>  64

<211>  200

<212>  PRT

<213>  Homo sapiens

<300>

<308>  sw_hum/ubc1_human

<309>  1992-08-01

<400>  64

Met Ala Asn Ile Ala Val Gln Arg Ile Lys Arg Glu Phe Lys Glu Val
1               5                   10                  15

Leu Lys Ser Glu Glu Thr Ser Lys Asn Gln Ile Lys Val Asp Leu Val
            20                  25                  30

Asp Glu Asn Phe Thr Glu Leu Arg Gly Glu Ile Ala Gly Pro Pro Asp
        35                  40                  45

Thr Pro Tyr Glu Gly Gly Arg Tyr Gln Leu Glu Ile Lys Ile Pro Glu
    50                  55                  60

Thr Tyr Pro Phe Asn Pro Pro Lys Val Arg Phe Ile Thr Lys Ile Trp
65                  70                  75                  80

His Pro Asn Ile Ser Ser Val Thr Gly Ala Ile Cys Leu Asp Ile Leu
                85                  90                  95

Lys Asp Gln Trp Ala Ala Ala Met Thr Leu Arg Thr Val Leu Leu Ser
            100                 105                 110

Leu Gln Ala Leu Leu Ala Ala Ala Glu Pro Asp Asp Pro Gln Asp Ala
        115                 120                 125

Val Val Ala Asn Gln Tyr Lys Gln Asn Pro Glu Met Phe Lys Gln Thr
    130                 135                 140

Ala Arg Leu Trp Ala His Val Tyr Ala Gly Ala Pro Val Ser Ser Pro
145                 150                 155                 160

Glu Tyr Thr Lys Lys Ile Glu Asn Leu Cys Ala Met Gly Phe Asp Arg
                165                 170                 175

Asn Ala Val Ile Val Ala Leu Ser Ser Lys Ser Trp Asp Val Glu Thr
            180                 185                 190

Ala Thr Glu Leu Leu Leu Ser Asn
        195                 200

<210> 65

<211> 493

<212> PRT

<213> Homo sapiens

<300>

<308> sw_hum/ubpe_human

<309> 1996-10-01

<400> 65

Pro Leu Tyr Ser Val Thr Val Lys Trp Gly Lys Glu Lys Phe Glu Gly
1               5                   10                  15

Val Glu Leu Asn Thr Asp Glu Pro Pro Met Val Phe Lys Ala Gln Leu
            20                  25                  30

Phe Ala Leu Thr Gly Val Gln Pro Ala Arg Gln Lys Val Met Val Lys
        35                  40                  45

Gly Gly Thr Leu Lys Asp Asp Asp Trp Gly Asn Ile Lys Ile Lys Asn
    50                  55                  60

Gly Met Thr Leu Leu Met Met Gly Ser Ala Asp Ala Leu Pro Glu Glu
65                  70                  75                  80

Pro Ser Ala Lys Thr Val Phe Val Glu Asp Met Thr Glu Glu Gln Leu
                85                  90                  95

Ala Ser Ala Met Glu Leu Pro Cys Gly Leu Thr Asn Leu Gly Asn Thr
            100                 105                 110

Cys Tyr Met Asn Ala Thr Val Gln Cys Ile Arg Ser Val Pro Glu Leu
        115                 120                 125

Lys Asp Ala Leu Lys Arg Tyr Ala Gly Ala Leu Arg Ala Ser Gly Glu
    130                 135                 140

Met Ala Ser Ala Gln Tyr Ile Thr Ala Ala Leu Arg Asp Leu Phe Asp
145                 150                 155                 160

Ser Met Asp Lys Thr Ser Ser Ser Ile Pro Pro Ile Ile Leu Leu Gln
                165                 170                 175

Phe Leu His Met Ala Phe Pro Gln Phe Ala Glu Lys Gly Glu Gln Gly
            180                 185                 190

Gln Tyr Leu Gln Gln Asp Ala Asn Glu Cys Trp Ile Gln Met Met Arg
        195                 200                 205

Val Leu Gln Gln Lys Leu Glu Ala Ile Glu Asp Asp Ser Val Lys Glu
    210                 215                 220

Thr Asp Ser Ser Ser Ala Ser Ala Ala Thr Pro Ser Lys Lys Lys Ser
225                 230                 235                 240

Leu Ile Asp Gln Phe Phe Gly Val Glu Phe Glu Thr Thr Met Lys Cys
                245                 250                 255

Thr Glu Ser Glu Glu Glu Glu Val Thr Lys Gly Lys Glu Asn Gln Leu
            260                 265                 270

Gln Leu Ser Cys Phe Ile Asn Gln Glu Val Lys Tyr Leu Phe Thr Gly
        275                 280                 285

Leu Lys Leu Arg Leu Gln Glu Glu Ile Thr Lys Gln Ser Pro Thr Leu
    290                 295                 300

Gln Arg Asn Ala Leu Tyr Ile Lys Ser Ser Lys Ile Ser Arg Leu Pro
305                 310                 315                 320

Ala Tyr Leu Thr Ile Gln Met Val Arg Phe Phe Tyr Lys Glu Lys Glu
                325                 330                 335

Ser Val Asn Ala Lys Val Leu Lys Asp Val Lys Phe Pro Leu Met Leu
            340                 345                 350

Asp Met Tyr Glu Leu Cys Thr Pro Glu Leu Gln Glu Lys Met Val Ser
        355                 360                 365

Phe Arg Ser Lys Phe Lys Asp Leu Glu Asp Lys Lys Val Asn Gln Gln
    370                 375                 380

Pro Asn Thr Ser Asp Lys Lys Ser Ser Pro Gln Lys Glu Val Lys Tyr
385                 390                 395                 400

Glu Pro Phe Ser Phe Ala Asp Asp Ile Gly Ser Asn Asn Cys Gly Tyr
                405                 410                 415

Tyr Asp Leu Gln Ala Val Leu Thr His Gln Gly Arg Ser Ser Ser Ser
            420                 425                 430

Gly His Tyr Val Ser Trp Val Lys Arg Lys Gln Asp Glu Trp Ile Lys
        435                 440                 445

Phe Asp Asp Asp Lys Val Ser Ile Val Thr Pro Glu Asp Ile Leu Arg
    450                 455                 460

Leu Ser Gly Gly Gly Asp Trp His Ile Ala Tyr Val Leu Leu Tyr Gly
465                 470                 475                 480

Pro Arg Arg Val Glu Ile Met Glu Glu Glu Ser Glu Gln
                485                 490

<210>  66

<211>  350

<212>  PRT

<213>  Homo sapiens

<300>

<308>  sw_hum/unri_human

<309>  2001-10-16

<400>  66

Met Ala Met Arg Gln Thr Pro Leu Thr Cys Ser Gly His Thr Arg Pro
1               5                   10                  15

Val Val Asp Leu Ala Phe Ser Gly Ile Thr Pro Tyr Gly Tyr Phe Leu
            20                  25                  30

Ile Ser Ala Cys Lys Asp Gly Lys Pro Met Leu Arg Gln Gly Asp Thr
        35                  40                  45

Gly Asp Trp Ile Gly Thr Phe Leu Gly His Lys Gly Ala Val Trp Gly
    50                  55                  60

Ala Thr Leu Asn Lys Asp Ala Thr Lys Ala Ala Thr Ala Ala Ala Asp
65                  70                  75                  80

Phe Thr Ala Lys Val Trp Asp Ala Val Ser Gly Asp Glu Leu Met Thr
                85                  90                  95

Leu Ala His Lys His Ile Val Lys Thr Val Asp Phe Thr Gln Asp Ser
            100                 105                 110

Asn Tyr Leu Leu Thr Gly Gly Gln Asp Lys Leu Leu Arg Ile Tyr Asp
        115                 120                 125

Leu Asn Lys Pro Glu Ala Glu Pro Lys Glu Ile Ser Gly His Thr Ser
    130                 135                 140

Gly Ile Lys Lys Ala Leu Trp Cys Ser Glu Asp Lys Gln Ile Leu Ser
145                 150                 155                 160

Ala Asp Asp Lys Thr Val Arg Leu Trp Asp His Ala Thr Met Thr Glu
                165                 170                 175

Val Lys Ser Leu Asn Phe Asn Met Ser Val Ser Ser Met Glu Tyr Ile
            180                 185                 190

Pro Glu Gly Glu Ile Leu Val Ile Thr Tyr Gly Arg Ser Ile Ala Phe
        195                 200                 205

His Ser Ala Val Ser Leu Asp Pro Ile Lys Ser Phe Glu Ala Pro Ala
    210                 215                 220

Thr Ile Asn Ser Ala Ser Leu His Pro Glu Lys Glu Phe Leu Val Ala
225                 230                 235                 240

Gly Gly Glu Asp Phe Lys Leu Tyr Lys Tyr Asp Tyr Asn Ser Gly Glu
                245                 250                 255

Glu Leu Glu Ser Tyr Lys Gly His Phe Gly Pro Ile His Cys Val Arg

260                 265                 270

Phe Ser Pro Asp Gly Glu Leu Tyr Ala Ser Gly Ser Glu Asp Gly Thr
        275                 280                 285

Leu Arg Leu Trp Gln Thr Val Val Gly Lys Thr Tyr Gly Leu Trp Lys
    290                 295                 300

Cys Val Leu Pro Glu Glu Asp Ser Gly Glu Leu Ala Lys Pro Lys Ile
305                 310                 315                 320

Gly Phe Pro Glu Thr Thr Glu Glu Glu Leu Glu Glu Ile Ala Ser Glu
                325                 330                 335

Asn Ser Asp Cys Ile Phe Pro Ser Ala Pro Asp Val Lys Ala
            340                 345                 350

<210>  67

<211>  617

<212>  PRT

<213>  Homo sapiens

<300>

<308>  sw_hum/vaa1_human

<309>  1994-10-01

<400>  67

<210>  68

<211>  826

<212>  PRT

<213>  Homo sapiens


<300>

<308>  sw_hum/vil1_human

<309>  1989-03-01


<400>  68

Thr Lys Leu Ser Ala Gln Val Lys Gly Ser Leu Asn Ile Thr Thr Pro
1               5                   10                  15

Gly Leu Gln Ile Trp Arg Ile Glu Ala Met Gln Met Val Pro Val Pro
            20                  25                  30

Ser Ser Thr Phe Gly Ser Phe Phe Asp Gly Asp Cys Tyr Ile Ile Leu
        35                  40                  45

Ala Ile His Lys Thr Ala Ser Ser Leu Ser Tyr Asp Ile His Tyr Trp
    50                  55                  60

Ile Gly Gln Asp Ser Ser Leu Asp Glu Gln Gly Ala Ala Ala Ile Tyr
65                  70                  75                  80

Thr Thr Gln Met Asp Asp Phe Leu Lys Gly Arg Ala Val Gln His Arg
                85                  90                  95

Glu Val Gln Gly Asn Glu Ser Glu Ala Phe Arg Gly Tyr Phe Lys Gln
            100                 105                 110

Gly Leu Val Ile Arg Lys Gly Gly Val Ala Ser Gly Met Lys His Val
        115                 120                 125

Glu Thr Asn Ser Tyr Asp Val Gln Arg Leu Leu His Val Lys Gly Lys
    130                 135                 140

Arg Asn Val Val Ala Gly Glu Val Glu Met Ser Trp Lys Ser Phe Asn
145                 150                 155                 160

Arg Gly Asp Val Phe Leu Leu Asp Leu Gly Lys Leu Ile Ile Gln Trp
                165                 170                 175

Asn Gly Pro Glu Ser Thr Arg Met Glu Arg Leu Arg Gly Met Thr Leu
            180                 185                 190

Ala Lys Glu Ile Arg Asp Gln Glu Arg Gly Gly Arg Thr Tyr Val Gly
        195                 200                 205

Val Val Asp Gly Glu Asn Glu Leu Ala Ser Pro Lys Leu Met Glu Val
    210                 215                 220

Met Asn His Val Leu Gly Lys Arg Arg Glu Leu Lys Ala Ala Val Pro
225                 230                 235                 240

Asp Thr Val Val Glu Pro Ala Leu Lys Ala Ala Leu Lys Leu Tyr His
                245                 250                 255

Val Ser Asp Ser Glu Gly Asn Leu Val Val Arg Glu Val Ala Thr Arg
            260                 265                 270

Pro Leu Thr Gln Asp Leu Leu Ser His Glu Asp Cys Tyr Ile Leu Asp
        275                 280                 285

Gln Gly Gly Leu Lys Ile Tyr Val Trp Lys Gly Lys Lys Ala Asn Glu
    290                 295                 300

Gln Glu Lys Lys Gly Ala Met Ser His Ala Leu Asn Phe Ile Lys Ala
305                 310                 315                 320

Lys Gln Tyr Pro Pro Ser Thr Gln Val Glu Val Gln Asn Asp Gly Ala
                325                 330                 335

Glu Ser Ala Val Phe Gln Gln Leu Phe Gln Lys Trp Thr Ala Ser Asn
            340                 345                 350

Arg Thr Ser Gly Leu Gly Lys Thr His Thr Val Gly Ser Val Ala Lys

        355                 360                 365

Val Glu Gln Val Lys Phe Asp Ala Thr Ser Met His Val Lys Pro Gln
    370                 375                 380

Val Ala Ala Gln Gln Lys Met Val Asp Asp Gly Ser Gly Glu Val Gln
385                 390                 395                 400

Val Trp Arg Ile Glu Asn Leu Glu Leu Val Pro Val Asp Ser Lys Trp
                405                 410                 415

Leu Gly His Phe Tyr Gly Gly Asp Cys Tyr Leu Leu Leu Tyr Thr Tyr
            420                 425                 430

Leu Ile Gly Glu Lys Gln His Tyr Leu Leu Tyr Val Trp Gln Gly Ser
        435                 440                 445

Gln Ala Ser Gln Asp Glu Ile Thr Ala Ser Ala Tyr Gln Ala Val Ile
    450                 455                 460

Leu Asp Gln Lys Tyr Asn Gly Glu Pro Val Gln Ile Arg Val Pro Met
465                 470                 475                 480

Gly Lys Glu Pro Pro His Leu Met Ser Ile Phe Lys Gly Arg Met Val
                485                 490                 495

Val Tyr Gln Gly Gly Thr Ser Arg Thr Asn Asn Leu Glu Thr Gly Pro
            500                 505                 510

Ser Thr Arg Leu Phe Gln Val Gln Gly Thr Gly Ala Asn Asn Thr Lys
        515                 520                 525

Ala Phe Glu Val Pro Ala Arg Ala Asn Phe Leu Asn Ser Asn Asp Val
    530                 535                 540

Phe Val Leu Lys Thr Gln Ser Cys Cys Tyr Leu Trp Cys Gly Lys Gly
545                 550                 555                 560

Cys Ser Gly Asp Glu Arg Glu Met Ala Lys Met Val Ala Asp Thr Ile
                565                 570                 575

Ser Arg Thr Glu Lys Gln Val Val Val Glu Gly Gln Glu Pro Ala Asn
            580                 585                 590

Phe Trp Met Ala Leu Gly Gly Lys Ala Pro Tyr Ala Asn Thr Lys Arg
        595                 600                 605

Leu Gln Glu Glu Asn Leu Val Ile Thr Pro Arg Leu Phe Glu Cys Ser
    610                 615                 620

Asn Lys Thr Gly Arg Phe Leu Ala Thr Glu Ile Pro Asp Phe Asn Gln
625                 630                 635                 640

Asp Asp Leu Glu Glu Asp Asp Val Phe Leu Leu Asp Val Trp Asp Gln
                645                 650                 655

Val Phe Phe Trp Ile Gly Lys His Ala Asn Glu Glu Glu Lys Lys Ala
            660                 665                 670

Ala Ala Thr Thr Ala Gln Glu Tyr Leu Lys Thr His Pro Ser Gly Arg
        675                 680                 685

Asp Pro Glu Thr Pro Ile Ile Val Val Lys Gln Gly His Glu Pro Pro
    690                 695                 700

Thr Phe Thr Gly Trp Phe Leu Ala Trp Asp Pro Phe Lys Trp Ser Asn
705                 710                 715                 720

Thr Lys Ser Tyr Glu Asp Leu Lys Ala Glu Ser Gly Asn Leu Arg Asp
                725                 730                 735

Trp Ser Gln Ile Thr Ala Glu Val Thr Ser Pro Lys Val Asp Val Phe
            740                 745                 750

Asn Ala Asn Ser Asn Leu Ser Ser Gly Pro Leu Pro Ile Phe Pro Leu
        755                 760                 765

Glu Gln Leu Val Asn Lys Pro Val Glu Glu Leu Pro Glu Gly Val Asp
    770                 775                 780

Pro Ser Arg Lys Glu Glu His Leu Ser Ile Glu Asp Phe Thr Gln Ala
785                 790                 795                 800

Phe Gly Met Thr Pro Ala Ala Phe Ser Ala Leu Pro Arg Trp Lys Gln
                805                 810                 815

Gln Asn Leu Lys Lys Glu Lys Gly Leu Phe
            820                 825

<210>  69

<211>  227

<212>  PRT

<213>  Homo sapiens

<300>

<308>  tr_hum/q96hx0

<309>  2001-12-01

<400>  69

Thr Pro Thr Tyr Gly Asp Leu Asn His Leu Val Ser Ala Thr Met Ser
1               5                   10                  15

Gly Val Thr Thr Cys Leu Arg Phe Pro Gly Gln Leu Asn Ala Asp Leu
            20                  25                  30

Arg Lys Leu Ala Val Asn Met Val Pro Phe Pro Arg Leu His Phe Phe
        35                  40                  45

Met Pro Gly Phe Ala Pro Leu Thr Ser Arg Gly Ser Gln Gln Tyr Arg
    50                  55                  60

Ala Leu Thr Val Pro Glu Leu Thr Gln Gln Met Phe Asp Ala Lys Asn
65                  70                  75                  80

Met Met Ala Ala Cys Asp Pro Arg His Gly Arg Tyr Leu Thr Val Ala
                85                  90                  95

Ala Val Phe Arg Gly Arg Met Ser Met Lys Glu Val Asp Glu Gln Met
            100                 105                 110

Leu Asn Val Gln Asn Lys Asn Ser Ser Tyr Phe Val Glu Trp Ile Pro
        115                 120                 125

Asn Asn Val Lys Thr Ala Val Cys Asp Ile Pro Pro Arg Gly Leu Lys
    130                 135                 140

Met Ser Ala Thr Phe Ile Gly Asn Ser Thr Ala Ile Gln Glu Leu Phe
145                 150                 155                 160

Lys Arg Ile Ser Glu Gln Phe Thr Ala Met Phe Arg Arg Lys Ala Phe
                165                 170                 175

Leu His Trp Tyr Thr Gly Glu Gly Met Asp Glu Met Glu Phe Thr Glu
            180                 185                 190

Ala Glu Ser Asn Met Asn Asp Leu Val Ser Glu Tyr Gln Gln Tyr Gln
        195                 200                 205

Asp Ala Thr Ala Glu Glu Glu Gly Glu Phe Glu Glu Glu Ala Glu Glu
    210                 215                 220

Glu Val Ala
225

<210>  70

<211>  531

<212>  PRT

<213>  Homo sapiens

<300>

<308>  sw_hum/ptb_human

<309>  1992-08-01

<400>  70

Met Asp Gly Ile Val Pro Asp Ile Ala Val Gly Thr Lys Arg Gly Ser
1               5                   10                  15

Asp Glu Leu Phe Ser Thr Cys Val Thr Asn Gly Pro Phe Ile Met Ser
            20                  25                  30

Ser Asn Ser Ala Ser Ala Ala Asn Gly Asn Asp Ser Lys Lys Phe Lys
        35                  40                  45

Gly Asp Ser Arg Ser Ala Gly Val Pro Ser Arg Val Ile His Ile Arg
    50                  55                  60

Lys Leu Pro Ile Asp Val Thr Glu Gly Glu Val Ile Ser Leu Gly Leu
65                  70                  75                  80

Pro Phe Gly Lys Val Thr Asn Leu Leu Met Leu Lys Gly Lys Asn Gln
                85                  90                  95

Ala Phe Ile Glu Met Asn Thr Glu Glu Ala Ala Asn Thr Met Val Asn
            100                 105                 110

Tyr Tyr Thr Ser Val Thr Pro Val Leu Arg Gly Gln Pro Ile Tyr Ile
        115                 120                 125

Gln Phe Ser Asn His Lys Glu Leu Lys Thr Asp Ser Ser Pro Asn Gln
    130                 135                 140

Ala Arg Ala Gln Ala Ala Leu Gln Ala Val Asn Ser Val Gln Ser Gly
145                 150                 155                 160

Asn Leu Ala Leu Ala Ala Ser Ala Ala Ala Val Asp Ala Gly Met Ala
                165                 170                 175

Met Ala Gly Gln Ser Pro Val Leu Arg Ile Ile Val Glu Asn Leu Phe
            180                 185                 190

Tyr Pro Val Thr Leu Asp Val Leu His Gln Ile Phe Ser Lys Phe Gly
        195                 200                 205

Thr Val Leu Lys Ile Ile Thr Phe Thr Lys Asn Asn Gln Phe Gln Ala
    210                 215                 220

Leu Leu Gln Tyr Ala Asp Pro Val Ser Ala Gln His Ala Lys Leu Ser
225                 230                 235                 240

Leu Asp Gly Gln Asn Ile Tyr Asn Ala Cys Cys Thr Leu Arg Ile Asp
                245                 250                 255

Phe Ser Lys Leu Thr Ser Leu Asn Val Lys Tyr Asn Asn Asp Lys Ser
            260                 265                 270

Arg Asp Tyr Thr Arg Pro Asp Leu Pro Ser Gly Asp Ser Gln Pro Ser
        275                 280                 285

Leu Asp Gln Thr Met Ala Ala Ala Phe Gly Leu Ser Val Pro Asn Val
    290                 295                 300

His Gly Ala Leu Ala Pro Leu Ala Ile Pro Ser Ala Ala Ala Ala Ala
305                 310                 315                 320

Ala Ala Ala Gly Arg Ile Ala Ile Pro Gly Leu Ala Gly Ala Gly Asn
                325                 330                 335

Ser Val Leu Leu Val Ser Asn Leu Asn Pro Glu Arg Val Thr Pro Gln
            340                 345                 350

Ser Leu Phe Ile Leu Phe Gly Val Tyr Gly Asp Val Gln Arg Val Lys

        355                 360                 365

Ile Leu Phe Asn Lys Lys Glu Asn Ala Leu Val Gln Met Ala Asp Gly
    370                 375                 380

Asn Gln Ala Gln Leu Ala Met Ser His Leu Asn Gly His Lys Leu His
385                 390                 395                 400

Gly Lys Pro Ile Arg Ile Thr Leu Ser Lys His Gln Asn Val Gln Leu
                405                 410                 415

Pro Arg Glu Gly Gln Glu Asp Gln Gly Leu Thr Lys Asp Tyr Gly Asn
            420                 425                 430

Ser Pro Leu His Arg Phe Lys Lys Pro Gly Ser Lys Asn Phe Gln Asn
        435                 440                 445

Ile Phe Pro Pro Ser Ala Thr Leu His Leu Ser Asn Ile Pro Pro Ser
    450                 455                 460

Val Ser Glu Glu Asp Leu Lys Val Leu Phe Ser Ser Asn Gly Gly Val
465                 470                 475                 480

Val Lys Gly Phe Lys Phe Phe Gln Lys Asp Arg Lys Met Ala Leu Ile
                485                 490                 495

Gln Met Gly Ser Val Glu Glu Ala Val Gln Ala Leu Ile Asp Leu His
            500                 505                 510

Asn His Asp Leu Gly Glu Asn His His Leu Arg Val Ser Phe Ser Lys
        515                 520                 525

Ser Thr Ile
    530

<210> 71

<211> 530

<212> PRT

<213> Homo sapiens

<300>

<308> sw_hum/kpy2_human

<309> 1990-04-01

<400> 71

Ser Lys Pro His Ser Glu Ala Gly Thr Ala Phe Ile Gln Thr Gln Gln
1               5                   10                  15

Leu His Ala Ala Met Ala Asp Thr Phe Leu Glu His Met Cys Arg Leu
            20                  25                  30

Asp Ile Asp Ser Pro Pro Ile Thr Ala Arg Asn Thr Gly Ile Ile Cys
        35                  40                  45

Thr Ile Gly Pro Ala Ser Arg Ser Val Glu Thr Leu Lys Glu Met Ile
    50                  55                  60

Lys Ser Gly Met Asn Val Ala Arg Leu Asn Phe Ser His Gly Thr His
65                  70                  75                  80

Glu Tyr His Ala Glu Thr Ile Lys Asn Val Arg Thr Ala Thr Glu Ser
                85                  90                  95

Phe Ala Ser Asp Pro Ile Leu Tyr Arg Pro Val Ala Val Ala Leu Asp
            100                 105                 110

Thr Lys Gly Pro Glu Ile Arg Thr Gly Leu Ile Lys Gly Ser Gly Thr
        115                 120                 125

Ala Glu Val Glu Leu Lys Lys Gly Ala Thr Leu Lys Ile Thr Leu Asp
    130                 135                 140

Asn Ala Tyr Met Glu Lys Cys Asp Glu Asn Ile Leu Trp Leu Asp Tyr
145                 150                 155                 160

Lys Asn Ile Cys Lys Val Val Glu Val Gly Ser Lys Ile Tyr Val Asp
                165                 170                 175

Asp Gly Leu Ile Ser Leu Gln Val Lys Gln Lys Gly Ala Asp Phe Leu
            180                 185                 190

Val Thr Glu Val Glu Asn Gly Gly Ser Leu Gly Ser Lys Lys Gly Val
        195                 200                 205

Asn Leu Pro Gly Ala Ala Val Asp Leu Pro Ala Val Ser Glu Lys Asp
    210                 215                 220

Ile Gln Asp Leu Lys Phe Gly Val Glu Gln Asp Val Asp Met Val Phe
225                 230                 235                 240

Ala Ser Phe Ile Arg Lys Ala Ser Asp Val His Glu Val Arg Lys Val
                245                 250                 255

Leu Gly Glu Lys Gly Lys Asn Ile Lys Ile Ile Ser Lys Ile Glu Asn
            260                 265                 270

His Glu Gly Val Arg Arg Phe Asp Glu Ile Leu Glu Ala Ser Asp Gly
        275                 280                 285

Ile Met Val Ala Arg Gly Asp Leu Gly Ile Glu Ile Pro Ala Glu Lys
    290                 295                 300

Val Phe Leu Ala Gln Lys Met Met Ile Gly Arg Cys Asn Arg Ala Gly
305                 310                 315                 320

Lys Pro Val Ile Cys Ala Thr Gln Met Leu Glu Ser Met Ile Lys Lys
                325                 330                 335

Pro Arg Pro Thr Arg Ala Glu Gly Ser Asp Val Ala Asn Ala Val Leu
            340                 345                 350

Asp Gly Ala Asp Cys Ile Met Leu Ser Gly Glu Thr Ala Lys Gly Asp
        355                 360                 365

Tyr Pro Leu Glu Ala Val Arg Met Gln Asn Leu Ile Ala Arg Glu Ala
    370                 375                 380

Glu Ala Ala Ile Tyr His Leu Gln Leu Phe Glu Glu Leu Arg Arg Leu
385                 390                 395                 400

Ala Pro Ile Thr Ser Asp Pro Thr Glu Ala Thr Ala Val Gly Ala Val
                405                 410                 415

Glu Ala Ser Phe Lys Cys Cys Ser Gly Ala Ile Ile Val Leu Thr Lys
            420                 425                 430

Ser Gly Arg Ser Ala His Gln Val Ala Arg Tyr Arg Pro Arg Ala Pro
        435                 440                 445

Ile Ile Ala Val Thr Arg Asn Pro Gln Thr Ala Arg Gln Ala His Leu
    450                 455                 460

Tyr Arg Gly Ile Phe Pro Val Leu Cys Lys Asp Pro Val Gln Glu Ala
465                 470                 475                 480

Trp Ala Glu Asp Val Asp Leu Arg Val Asn Phe Ala Met Asn Val Gly
                485                 490                 495

Lys Ala Arg Gly Phe Phe Lys Lys Gly Asp Val Val Ile Val Leu Thr
            500                 505                 510

Gly Trp Arg Pro Gly Ser Gly Phe Thr Asn Thr Met Arg Val Val Pro
        515                 520                 525

Val Pro
    530

<210>  72

<211>  432

<212>  PRT

<213>  Homo sapiens


<300>

<308>  sw_hum/acdb_human

<309>  1995-11-01


<400>  72

<210>  73

<211>  262

<212>  PRT

<213>  Homo sapiens

<300>

<308>  sw_hum/rs4_human

<309>  1989-10-01

<400>  73

Ala Arg Gly Pro Lys Lys His Leu Lys Arg Val Ala Ala Pro Lys His
1               5                   10                  15

Trp Met Leu Asp Lys Leu Thr Gly Val Phe Ala Pro Arg Pro Ser Thr
            20                  25                  30

Gly Pro His Lys Leu Arg Glu Cys Leu Pro Leu Ile Ile Phe Leu Arg
        35                  40                  45

Asn Arg Leu Lys Tyr Ala Leu Thr Gly Asp Glu Val Lys Lys Ile Cys
    50                  55                  60

Met Gln Arg Phe Ile Lys Ile Asp Gly Lys Val Arg Thr Asp Ile Thr
65                  70                  75                  80

Tyr Pro Ala Gly Phe Met Asp Val Ile Ser Ile Asp Lys Thr Gly Glu
                85                  90                  95

Asn Phe Arg Leu Ile Tyr Asp Thr Lys Gly Arg Phe Ala Val His Arg
            100                 105                 110

Ile Thr Pro Glu Glu Ala Lys Tyr Lys Leu Cys Lys Val Arg Lys Ile
        115                 120                 125

Phe Val Gly Thr Lys Gly Ile Pro His Leu Val Thr His Asp Ala Arg
    130                 135                 140

Thr Ile Arg Tyr Pro Asp Pro Leu Ile Lys Val Asn Asp Thr Ile Gln
145                 150                 155                 160

Ile Asp Leu Glu Thr Gly Lys Ile Thr Asp Phe Ile Lys Phe Asp Thr
                165                 170                 175

Gly Asn Leu Cys Met Val Thr Gly Gly Ala Asn Leu Gly Arg Ile Gly
            180                 185                 190

Val Ile Thr Asn Arg Glu Arg His Pro Gly Ser Phe Asp Val Val His
        195                 200                 205

Val Lys Asp Ala Asn Gly Asn Ser Phe Ala Thr Arg Leu Ser Asn Ile
    210                 215                 220

Phe Val Ile Gly Lys Gly Asn Lys Pro Trp Ile Ser Leu Pro Arg Gly
225                 230                 235                 240

Lys Gly Ile Arg Leu Thr Ile Ala Glu Glu Arg Asp Lys Arg Leu Ala
                245                 250                 255

Ala Lys Gln Ser Ser Gly
            260

<210>  74

<211>  303

<212>  PRT

<213>  Homo sapiens


<300>

<308>  sw_hum/crkl_human

<309>  1995-11-01


<400>  74

Met Ser Ser Ala Arg Phe Asp Ser Ser Asp Arg Ser Ala Trp Tyr Met
1               5                   10                  15

Gly Pro Val Ser Arg Gln Glu Ala Gln Thr Arg Leu Gln Gly Gln Arg
            20                  25                  30

His Gly Met Phe Leu Val Arg Asp Ser Ser Thr Cys Pro Gly Asp Tyr
        35                  40                  45

Val Leu Ser Val Ser Glu Asn Ser Arg Val Ser His Tyr Ile Ile Asn
    50                  55                  60

Ser Leu Pro Asn Arg Arg Phe Lys Ile Gly Asp Gln Glu Phe Asp His
65                  70                  75                  80

Leu Pro Ala Leu Leu Glu Phe Tyr Lys Ile His Tyr Leu Asp Thr Thr
                85                  90                  95

Thr Leu Ile Glu Pro Ala Pro Arg Tyr Pro Ser Pro Pro Met Gly Ser
            100                 105                 110

Val Ser Ala Pro Asn Leu Pro Thr Ala Glu Asp Asn Leu Glu Tyr Val
        115                 120                 125

Arg Thr Leu Tyr Asp Phe Pro Gly Asn Asp Ala Glu Asp Leu Pro Phe
    130                 135                 140

Lys Lys Gly Glu Ile Leu Val Ile Ile Glu Lys Pro Glu Glu Gln Trp
145                 150                 155                 160

Trp Ser Ala Arg Asn Lys Asp Gly Arg Val Gly Met Ile Pro Val Pro
                165                 170                 175

Tyr Val Glu Lys Leu Val Arg Ser Ser Pro His Gly Lys His Gly Asn
            180                 185                 190

Arg Asn Ser Asn Ser Tyr Gly Ile Pro Glu Pro Ala His Ala Tyr Ala
        195                 200                 205

Gln Pro Gln Thr Thr Thr Pro Leu Pro Ala Val Ser Gly Ser Pro Gly
    210                 215                 220

Ala Ala Ile Thr Pro Leu Pro Ser Thr Gln Asn Gly Pro Val Phe Ala
225                 230                 235                 240

Lys Ala Ile Gln Lys Arg Val Pro Cys Ala Tyr Asp Lys Thr Ala Leu
                245                 250                 255

Ala Leu Glu Val Gly Asp Ile Val Lys Val Thr Arg Met Asn Ile Asn
            260                 265                 270

Gly Gln Trp Glu Gly Glu Val Asn Gly Arg Lys Gly Leu Phe Pro Phe
        275                 280                 285

Thr His Val Lys Ile Phe Asp Pro Gln Asn Pro Asp Glu Asn Glu
    290                 295                 300

<210>  75

<211>  614

<212>  PRT

<213>  Homo sapiens


<300>

<308>  sw_hum/ddx5_human

<309>  1990-08-01


<400>  75

Met Ser Gly Tyr Ser Ser Asp Arg Asp Arg Gly Arg Asp Arg Gly Phe
1               5                   10                  15

Gly Ala Pro Arg Phe Gly Gly Ser Arg Ala Gly Pro Leu Ser Gly Lys
            20                  25                  30

Lys Phe Gly Asn Pro Gly Glu Lys Leu Val Lys Lys Lys Trp Asn Leu
        35                  40                  45

Asp Glu Leu Pro Lys Phe Glu Lys Asn Phe Tyr Gln Glu His Pro Asp
    50                  55                  60

Leu Ala Arg Arg Thr Ala Gln Glu Val Glu Thr Tyr Arg Arg Ser Lys
65                  70                  75                  80

Glu Ile Thr Val Arg Gly His Asn Cys Pro Lys Pro Val Leu Asn Phe
                85                  90                  95

Tyr Glu Ala Asn Phe Pro Ala Asn Val Met Asp Val Ile Ala Arg Gln
            100                 105                 110

Asn Phe Thr Glu Pro Thr Ala Ile Gln Ala Gln Gly Trp Pro Val Ala
        115                 120                 125

Leu Ser Gly Leu Asp Met Val Gly Val Ala Gln Thr Gly Ser Gly Lys
    130                 135                 140

Thr Leu Ser Tyr Leu Leu Pro Ala Ile Val His Ile Asn His Gln Pro
145                 150                 155                 160

Phe Leu Glu Arg Gly Asp Gly Pro Ile Cys Leu Val Leu Ala Pro Thr
                165                 170                 175

Arg Glu Leu Ala Gln Gln Val Gln Gln Val Ala Ala Glu Tyr Cys Arg
            180                 185                 190

Ala Cys Arg Leu Lys Ser Thr Cys Ile Tyr Gly Gly Ala Pro Lys Gly
        195                 200                 205

Pro Gln Ile Arg Asp Leu Glu Arg Gly Val Glu Ile Cys Ile Ala Thr
    210                 215                 220

Pro Gly Arg Leu Ile Asp Phe Leu Glu Cys Gly Lys Thr Asn Leu Arg
225                 230                 235                 240

Arg Thr Thr Tyr Leu Val Leu Asp Glu Ala Asp Arg Met Leu Asp Met
                245                 250                 255

Gly Phe Glu Pro Gln Ile Arg Lys Ile Val Asp Gln Ile Arg Pro Asp
            260                 265                 270

Arg Gln Thr Leu Met Trp Ser Ala Thr Trp Pro Lys Glu Val Arg Gln
        275                 280                 285

Leu Ala Glu Asp Phe Leu Lys Asp Tyr Ile His Ile Asn Ile Gly Ala
    290                 295                 300

Leu Glu Leu Ser Ala Asn His Asn Ile Leu Gln Ile Val Asp Val Cys
305                 310                 315                 320

His Asp Val Glu Lys Asp Glu Lys Leu Ile Arg Leu Met Glu Glu Ile

                325                 330                 335

Met Ser Glu Lys Glu Asn Lys Thr Ile Val Phe Val Glu Thr Lys Arg
            340                 345                 350

Arg Cys Asp Glu Leu Thr Arg Lys Met Arg Arg Asp Gly Trp Pro Ala
        355                 360                 365

Met Gly Ile His Gly Asp Lys Ser Gln Gln Glu Arg Asp Trp Val Leu
    370                 375                 380

Asn Glu Phe Lys His Gly Lys Ala Pro Ile Leu Ile Ala Thr Asp Val
385                 390                 395                 400

Ala Ser Arg Gly Leu Asp Val Glu Asp Val Lys Phe Val Ile Asn Tyr
                405                 410                 415

Asp Tyr Pro Asn Ser Ser Glu Asp Tyr Ile His Arg Ile Gly Arg Thr
            420                 425                 430

Ala Arg Ser Thr Lys Thr Gly Thr Ala Tyr Thr Phe Phe Thr Pro Asn
        435                 440                 445

Asn Ile Lys Gln Val Ser Asp Leu Ile Ser Val Leu Arg Glu Ala Asn
    450                 455                 460

Gln Ala Ile Asn Pro Lys Leu Leu Gln Leu Val Glu Asp Arg Gly Ser
465                 470                 475                 480

Gly Arg Ser Arg Gly Arg Gly Gly Met Lys Asp Asp Arg Arg Asp Arg
                485                 490                 495

Tyr Ser Ala Gly Lys Arg Gly Gly Phe Asn Thr Phe Arg Asp Arg Glu
            500                 505                 510

Asn Tyr Asp Arg Gly Tyr Ser Ser Leu Leu Lys Arg Asp Phe Gly Ala
        515                 520                 525

Lys Thr Gln Asn Gly Val Tyr Ser Ala Ala Asn Tyr Thr Asn Gly Ser
    530                 535                 540

Phe Gly Ser Asn Phe Val Ser Ala Gly Ile Gln Thr Ser Phe Arg Thr
545                 550                 555                 560

Gly Asn Pro Thr Gly Thr Tyr Gln Asn Gly Tyr Asp Ser Thr Gln Gln
                565                 570                 575

Tyr Gly Ser Asn Val Pro Asn Met His Asn Gly Met Asn Gln Gln Ala
            580                 585                 590

Tyr Ala Tyr Pro Ala Thr Ala Ala Ala Pro Met Ile Gly Tyr Pro Met
        595                 600                 605

Pro Thr Gly Tyr Ser Gln
    610

<210> 76

<211>  261

<212>  PRT

<213>  Homo sapiens

<300>

<308>  sw_hum/las1_human

<309>  1998-07-15

<400>  76

Met Asn Pro Asn Cys Ala Arg Cys Gly Lys Ile Val Tyr Pro Thr Glu
1               5                   10                  15

Lys Val Asn Cys Leu Asp Lys Phe Trp His Lys Ala Cys Phe His Cys
            20                  25                  30

Glu Thr Cys Lys Met Thr Leu Asn Met Lys Asn Tyr Lys Gly Tyr Glu
        35                  40                  45

Lys Lys Pro Tyr Cys Asn Ala His Tyr Pro Lys Gln Ser Phe Thr Met
    50                  55                  60

Val Ala Asp Thr Pro Glu Asn Leu Arg Leu Lys Gln Gln Ser Glu Leu
65                  70                  75                  80

Gln Ser Gln Val Arg Tyr Lys Glu Glu Phe Glu Lys Asn Lys Gly Lys
                85                  90                  95

Gly Phe Ser Val Val Ala Asp Thr Pro Glu Leu Gln Arg Ile Lys Lys
            100                 105                 110

Thr Gln Asp Gln Ile Ser Asn Ile Lys Tyr His Glu Glu Phe Glu Lys
        115                 120                 125

Ser Arg Met Gly Pro Ser Gly Gly Glu Gly Met Glu Pro Glu Arg Arg
    130                 135                 140

Asp Ser Gln Asp Gly Ser Ser Tyr Arg Arg Pro Leu Glu Gln Gln Gln
145                 150                 155                 160

Pro His His Ile Pro Thr Ser Ala Pro Val Tyr Gln Gln Pro Gln Gln
                165                 170                 175

Gln Pro Val Ala Gln Ser Tyr Gly Gly Tyr Lys Glu Pro Ala Ala Pro
            180                 185                 190

Val Ser Ile Gln Arg Ser Ala Pro Gly Gly Gly Gly Lys Arg Tyr Arg
        195                 200                 205

Ala Val Tyr Asp Tyr Ser Ala Ala Asp Glu Asp Glu Val Ser Phe Gln
    210                 215                 220

Asp Gly Asp Thr Ile Val Asn Val Gln Gln Ile Asp Asp Gly Trp Met
225                 230                 235                 240

Tyr Gly Thr Val Glu Arg Thr Gly Asp Thr Gly Met Leu Pro Ala Asn
                245                 250                 255

Tyr Val Glu Ala Ile
            260

<210>  77

<211>  528

<212>  PRT

<213>  Homo sapiens

<300>

<308>  sw_hum/syy_human

<309>  1996-10-01

<400>  77

<210>  78

<211>  418

<212>  PRT

<213>  Homo sapiens

<300>

<308>  sw_hum/sep7_human

<309>  1997-11-01

<400>  78

Met Val Ala Gln Gln Lys Asn Leu Glu Gly Tyr Val Gly Phe Ala Asn
1               5                   10                  15

Leu Pro Asn Gln Val Tyr Arg Lys Ser Val Lys Arg Gly Phe Glu Phe
            20                  25                  30

Thr Leu Met Val Val Gly Glu Ser Gly Leu Gly Lys Ser Thr Leu Ile
        35                  40                  45

Asn Ser Leu Phe Leu Thr Asp Leu Tyr Ser Pro Glu Tyr Pro Gly Pro
    50                  55                  60

Ser His Arg Ile Lys Lys Thr Val Gln Val Glu Gln Ser Lys Val Leu
65                  70                  75                  80

Ile Lys Glu Gly Gly Val Gln Leu Leu Leu Thr Ile Val Asp Thr Pro
                85                  90                  95

Gly Phe Gly Asp Ala Val Asp Asn Ser Asn Cys Trp Gln Pro Val Ile
            100                 105                 110

Asp Tyr Ile Asp Ser Lys Phe Glu Asp Tyr Leu Asn Ala Glu Ser Arg
        115                 120                 125

Val Asn Arg Arg Gln Met Pro Asp Asn Arg Val Gln Cys Cys Leu Tyr
    130                 135                 140

Phe Ile Ala Pro Ser Gly His Gly Leu Lys Pro Leu Asp Ile Glu Phe
145                 150                 155                 160

Met Lys Arg Leu His Glu Lys Val Asn Ile Ile Pro Leu Ile Ala Lys
                165                 170                 175

Ala Asp Thr Leu Thr Pro Glu Glu Cys Gln Gln Phe Lys Lys Gln Ile
            180                 185                 190

Met Lys Glu Ile Gln Glu His Lys Ile Lys Ile Tyr Glu Phe Pro Glu
        195                 200                 205

Thr Asp Asp Glu Glu Glu Asn Lys Leu Val Lys Lys Ile Lys Asp Arg
    210                 215                 220

Leu Pro Leu Ala Val Val Gly Ser Asn Thr Ile Ile Glu Val Asn Gly
225                 230                 235                 240

Lys Arg Val Arg Gly Arg Gln Tyr Pro Trp Gly Ile Ala Glu Val Glu
                245                 250                 255

Asn Gly Glu His Cys Asp Phe Thr Ile Leu Arg Asn Met Lys Ile Arg
            260                 265                 270

Thr His Met Gln Asp Leu Lys Asp Val Thr Asn Asn Val His Tyr Glu
        275                 280                 285

Asn Tyr Arg Ser Arg Lys Leu Ala Ala Val Thr Tyr Asn Gly Val Asp
    290                 295                 300

Asn Asn Lys Asn Lys Gly Gln Leu Thr Lys Ser Pro Leu Ala Gln Met
305                 310                 315                 320

Glu Glu Glu Arg Arg Glu His Val Ala Lys Met Lys Lys Met Glu Met
                325                 330                 335

Glu Met Glu Gln Val Phe Glu Met Lys Val Lys Glu Lys Val Gln Lys
            340                 345                 350

Leu Lys Asp Ser Glu Ala Glu Leu Gln Arg Arg His Glu Gln Met Lys
        355                 360                 365

Lys Asn Leu Glu Ala Gln His Lys Glu Leu Glu Glu Lys Arg Arg Gln
    370                 375                 380

Phe Glu Asp Glu Lys Ala Asn Trp Glu Ala Gln Gln Arg Ile Leu Glu
385                 390                 395                 400

Gln Gln Asn Ser Ser Arg Thr Leu Glu Lys Asn Lys Lys Lys Gly Lys
                405                 410                 415

Ile Phe

<210>  79

<211>  213

<212>  PRT

<213>  Homo sapiens

<300>

<308>  sw_hum/atpo_human

<309>  1996-02-01

<400>  79

Met Ala Ala Pro Ala Val Ser Gly Leu Ser Arg Gln Val Arg Cys Phe
1               5                   10                  15

Ser Thr Ser Val Val Arg Pro Phe Ala Lys Leu Val Arg Pro Pro Val
            20                  25                  30

Gln Val Tyr Gly Ile Glu Gly Arg Tyr Ala Thr Ala Leu Tyr Ser Ala
        35                  40                  45

Ala Ser Lys Gln Asn Lys Leu Glu Gln Val Glu Lys Glu Leu Leu Arg
    50                  55                  60

Val Ala Gln Ile Leu Lys Glu Pro Lys Val Ala Ala Ser Val Leu Asn
65                  70                  75                  80

Pro Tyr Val Lys Arg Ser Ile Lys Val Lys Ser Leu Asn Asp Ile Thr
                85                  90                  95

Ala Lys Glu Arg Phe Ser Pro Leu Thr Thr Asn Leu Ile Asn Leu Leu
            100                 105                 110

Ala Glu Asn Gly Arg Leu Ser Asn Thr Gln Gly Val Val Ser Ala Phe
        115                 120                 125

Ser Thr Met Met Ser Val His Arg Gly Glu Val Pro Cys Thr Val Thr
    130                 135                 140

Ser Ala Ser Pro Leu Glu Glu Ala Thr Leu Ser Glu Leu Lys Thr Val
145                 150                 155                 160

Leu Lys Ser Phe Leu Ser Gln Gly Gln Val Leu Lys Leu Glu Ala Lys
                165                 170                 175

Thr Asp Pro Ser Ile Leu Gly Gly Met Ile Val Arg Ile Gly Glu Lys
            180                 185                 190

Tyr Val Asp Met Ser Val Lys Thr Lys Ile Gln Lys Leu Gly Arg Ala
        195                 200                 205

Met Arg Glu Ile Val
    210

<210>  80

<211>  808

<212>  PRT

<213>  Homo sapiens


<300>

<308>  sw_hum/mcm3_human

<309>  1992-05-01


<400>  80

Met Ala Gly Thr Val Val Leu Asp Asp Val Glu Leu Arg Glu Ala Gln
1               5                   10                  15

Arg Asp Tyr Leu Asp Phe Leu Asp Asp Glu Glu Asp Gln Gly Ile Tyr
            20                  25                  30

Gln Ser Lys Val Arg Glu Leu Ile Ser Asp Asn Gln Tyr Arg Leu Ile
        35                  40                  45

Val Asn Val Asn Asp Leu Arg Arg Lys Asn Glu Lys Arg Ala Asn Arg
    50                  55                  60

Leu Leu Asn Asn Ala Phe Glu Glu Leu Val Ala Phe Gln Arg Ala Leu
65                  70                  75                  80

Lys Asp Phe Val Ala Ser Ile Asp Ala Thr Tyr Ala Lys Gln Tyr Glu
                85                  90                  95

Glu Phe Tyr Val Gly Leu Glu Gly Ser Phe Gly Ser Lys His Val Ser
            100                 105                 110

Pro Arg Thr Leu Thr Ser Cys Phe Leu Ser Cys Val Val Cys Val Glu
        115                 120                 125

Gly Ile Val Thr Lys Cys Ser Leu Val Arg Pro Lys Val Val Arg Ser
    130                 135                 140

Val His Tyr Cys Pro Ala Thr Lys Lys Thr Ile Glu Arg Arg Tyr Ser
145                 150                 155                 160

Asp Leu Thr Thr Leu Val Ala Phe Pro Ser Ser Ser Val Tyr Pro Thr
                165                 170                 175

Lys Asp Glu Glu Asn Asn Pro Leu Glu Thr Glu Tyr Gly Leu Ser Val
            180                 185                 190

Tyr Lys Asp His Gln Thr Ile Thr Ile Gln Glu Met Pro Glu Lys Ala
        195                 200                 205

Pro Ala Gly Gln Leu Pro Arg Ser Val Asp Val Ile Leu Asp Asp Asp
    210                 215                 220

Leu Val Asp Lys Ala Lys Pro Gly Asp Arg Val Gln Val Val Gly Thr
225                 230                 235                 240

Tyr Arg Cys Leu Pro Gly Lys Lys Gly Gly Tyr Thr Ser Gly Thr Phe
                245                 250                 255

Arg Thr Val Leu Ile Ala Cys Asn Val Lys Gln Met Ser Lys Asp Ala
            260                 265                 270

Gln Pro Ser Phe Ser Ala Glu Asp Ile Ala Lys Ile Lys Lys Phe Ser
        275                 280                 285

Lys Thr Arg Ser Lys Asp Ile Phe Asp Gln Leu Ala Lys Ser Leu Ala
    290                 295                 300

Pro Ser Ile His Gly His Asp Tyr Val Lys Lys Ala Ile Leu Cys Leu

305                 310                 315                 320

Leu Leu Gly Gly Val Glu Arg Asp Leu Glu Asn Gly Ser His Ile Arg
                325                 330                 335

Gly Asp Ile Asn Ile Leu Leu Ile Gly Asp Pro Ser Val Ala Lys Ser
            340                 345                 350

Gln Leu Leu Arg Tyr Val Leu Cys Thr Ala Pro Arg Ala Ile Pro Thr
        355                 360                 365

Thr Gly Arg Gly Ser Ser Gly Val Gly Leu Thr Ala Ala Val Thr Thr
    370                 375                 380

Asp Gln Glu Thr Gly Glu Arg Arg Leu Glu Ala Gly Ala Met Val Leu
385                 390                 395                 400

Ala Asp Arg Gly Val Val Cys Ile Asp Glu Phe Asp Lys Met Ser Asp
                405                 410                 415

Met Asp Arg Thr Ala Ile His Glu Val Met Glu Gln Gly Arg Val Thr
            420                 425                 430

Ile Ala Lys Ala Gly Ile His Ala Arg Leu Asn Ala Arg Cys Ser Val
        435                 440                 445

Leu Ala Ala Ala Asn Pro Val Tyr Gly Arg Tyr Asp Gln Tyr Lys Thr
    450                 455                 460

Pro Met Glu Asn Ile Gly Leu Gln Asp Ser Leu Leu Ser Arg Phe Asp
465                 470                 475                 480

Leu Leu Phe Ile Met Leu Asp Gln Met Asp Pro Glu Gln Asp Arg Glu
                485                 490                 495

Ile Ser Asp His Val Leu Arg Met His Arg Tyr Arg Ala Pro Gly Glu
            500                 505                 510

Gln Asp Gly Asp Ala Met Pro Leu Gly Ser Ala Val Asp Ile Leu Ala
        515                 520                 525

Thr Asp Asp Pro Asn Phe Ser Gln Glu Asp Gln Gln Asp Thr Gln Ile
    530                 535                 540

Tyr Glu Lys His Asp Asn Leu Leu His Gly Thr Lys Lys Lys Lys Glu
545                 550                 555                 560

Lys Met Val Ser Ala Ala Phe Met Lys Lys Tyr Ile His Val Ala Lys
                565                 570                 575

Ile Ile Lys Pro Val Leu Thr Gln Glu Ser Ala Thr Tyr Ile Ala Glu
            580                 585                 590

Glu Tyr Ser Arg Leu Arg Ser Gln Asp Ser Met Ser Ser Asp Thr Ala
        595                 600                 605

Arg Thr Ser Pro Val Thr Ala Arg Thr Leu Glu Thr Leu Ile Arg Leu
    610                 615                 620

Ala Thr Ala His Ala Lys Ala Arg Met Ser Lys Thr Val Asp Leu Gln
625                 630                 635                 640

Asp Ala Glu Glu Ala Val Glu Leu Val Gln Tyr Ala Tyr Phe Lys Lys
                645                 650                 655

Val Leu Glu Lys Glu Lys Lys Arg Lys Lys Arg Ser Glu Asp Glu Ser
            660                 665                 670

Glu Thr Glu Asp Glu Glu Glu Lys Ser Gln Glu Asp Gln Glu Gln Lys
        675                 680                 685

Arg Lys Arg Arg Lys Thr Arg Gln Pro Asp Ala Lys Asp Gly Asp Ser
    690                 695                 700

Tyr Asp Pro Tyr Asp Phe Ser Asp Thr Glu Glu Glu Met Pro Gln Val
705                 710                 715                 720

His Thr Pro Lys Thr Ala Asp Ser Gln Glu Thr Lys Glu Ser Gln Lys
                725                 730                 735

Val Glu Leu Ser Glu Ser Arg Leu Lys Ala Phe Lys Val Ala Leu Leu
            740                 745                 750

Asp Val Phe Arg Glu Ala His Ala Gln Ser Ile Gly Met Asn Arg Leu
        755                 760                 765

Thr Glu Ser Ile Asn Arg Asp Ser Glu Glu Pro Phe Ser Ser Val Glu
    770                 775                 780

Ile Gln Ala Ala Leu Ser Lys Met Gln Asp Asp Asn Gln Val Met Val
785                 790                 795                 800

Ser Glu Gly Ile Ile Phe Leu Ile
                805

<210>  81

<211>  165

<212>  PRT

<213>  Homo sapiens

<300>

<308>  sw_hum/rl12_human

<309>  1993-04-01

<400>  81

<210>  82

<211>  918

<212>  PRT

<213>  Homo sapiens


<220>

<221>  MISC_FEATURE

<222>  (614)..(614)

<223>  x


<220>

<221>  MISC_FEATURE

<222>  (648)..(648)

<223>  x


<300>

<308>  tr_hum/q9y280

<309>  1999-11-01


<400>  82

Met Ala Ser Leu Ser Arg Ala Leu Arg Val Ala Ala Ala His Pro Arg
1               5                   10                  15

Gln Ser Pro Thr Arg Gly Met Gly Pro Cys Asn Leu Ser Ser Ala Ala
            20                  25                  30

Gly Pro Thr Ala Glu Lys Ser Val Pro Tyr Gln Arg Thr Leu Lys Glu
        35                  40                  45

Gly Gln Gly Thr Ser Val Val Ala Gln Gly Pro Ser Arg Pro Leu Pro
    50                  55                  60

Ser Thr Ala Asn Val Val Val Ile Gly Gly Gly Ser Leu Gly Cys Gln
65                  70                  75                  80

Thr Leu Tyr His Leu Ala Lys Leu Gly Met Ser Gly Ala Val Leu Leu
                85                  90                  95

Glu Arg Glu Arg Leu Thr Ser Gly Thr Thr Trp His Thr Ala Gly Leu
            100                 105                 110

Leu Trp Gln Leu Arg Pro Ser Asp Val Glu Val Glu Leu Leu Ala His
        115                 120                 125

Thr Arg Arg Val Val Ser Arg Glu Leu Glu Glu Glu Thr Gly Leu His
    130                 135                 140

Thr Gly Trp Ile Gln Asn Gly Gly Leu Phe Ile Ala Ser Asn Arg Gln
145                 150                 155                 160

Arg Leu Asp Glu Tyr Lys Arg Leu Met Ser Leu Gly Lys Ala Tyr Gly
                165                 170                 175

Val Glu Ser His Val Leu Ser Pro Ala Glu Thr Lys Thr Leu Tyr Pro
            180                 185                 190

Leu Met Asn Val Asp Asp Leu Tyr Gly Thr Leu Tyr Val Pro His Asp
        195                 200                 205

Gly Thr Met Asp Pro Ala Gly Thr Cys Thr Thr Leu Ala Arg Ala Ala
    210                 215                 220

Ser Ala Arg Gly Ala Gln Val Ile Glu Asn Cys Pro Val Thr Gly Ile
225                 230                 235                 240

Arg Val Trp Thr Asp Asp Phe Gly Val Arg Arg Val Ala Gly Val Glu
                245                 250                 255

Thr Gln His Gly Ser Ile Gln Thr Pro Cys Val Val Asn Cys Ala Gly
            260                 265                 270

Val Trp Ala Ser Ala Val Gly Arg Met Ala Gly Val Lys Val Pro Leu
        275                 280                 285

Val Ala Met His His Ala Tyr Val Val Thr Glu Arg Ile Glu Gly Ile
    290                 295                 300

Gln Asn Met Pro Asn Val Arg Asp His Asp Ala Ser Val Tyr Leu Arg

305                 310                 315                 320

Leu Gln Gly Asp Ala Leu Ser Val Gly Gly Tyr Glu Ala Asn Pro Ile
                325                 330                 335

Phe Trp Glu Glu Val Ser Asp Lys Phe Ala Phe Gly Leu Phe Asp Leu
            340                 345                 350

Asp Trp Glu Val Phe Thr Gln His Ile Glu Gly Ala Ile Asn Arg Val
        355                 360                 365

Pro Val Leu Glu Lys Thr Gly Ile Lys Ser Thr Val Cys Gly Pro Glu
    370                 375                 380

Ser Phe Thr Pro Asp His Lys Pro Leu Met Gly Glu Ala Pro Glu Leu
385                 390                 395                 400

Arg Gly Phe Phe Leu Gly Cys Gly Phe Asn Ser Ala Gly Met Met Leu
                405                 410                 415

Gly Gly Gly Cys Gly Gln Glu Leu Ala His Trp Ile Ile His Gly Arg
            420                 425                 430

Pro Glu Lys Asp Met His Gly Tyr Asp Ile Arg Arg Phe His His Ser
        435                 440                 445

Leu Thr Asp His Pro Arg Trp Ile Arg Glu Arg Ser His Glu Ser Tyr
    450                 455                 460

Ala Lys Asn Tyr Ser Val Val Phe Pro His Asp Glu Pro Leu Ala Gly
465                 470                 475                 480

Arg Asn Met Arg Arg Asp Pro Leu His Glu Glu Leu Leu Gly Gln Gly
                485                 490                 495

Cys Val Phe Gln Glu Arg His Gly Trp Glu Arg Pro Gly Trp Phe His
            500                 505                 510

Pro Arg Gly Pro Ala Pro Val Leu Glu Tyr Asp Tyr Tyr Gly Ala Tyr
        515                 520                 525

Gly Ser Arg Ala His Glu Asp Tyr Ala Tyr Arg Arg Leu Leu Ala Asp
    530                 535                 540

Glu Tyr Thr Phe Ala Phe Pro Pro His His Asp Thr Ile Lys Lys Glu
545                 550                 555                 560

Cys Leu Ala Cys Arg Gly Ala Ala Ala Val Phe Asp Met Ser Tyr Phe
                565                 570                 575

Gly Lys Phe Tyr Leu Val Gly Leu Asp Ala Arg Lys Ala Ala Asp Trp
            580                 585                 590

Leu Phe Ser Ala Asp Val Ser Arg Pro Pro Gly Ser Thr Val Tyr Thr
        595                 600                 605

Cys Met Leu Asn His Xaa Gly Gly Thr Glu Ser Asp Leu Thr Val Ser
    610                 615                 620

Arg Leu Ala Pro Ser His Gln Ala Ser Pro Leu Ala Pro Ala Phe Glu
625                 630                 635                 640

Gly Asp Gly Tyr Tyr Leu Ala Xaa Gly Gly Ala Val Ala Gln His Asn
                645                 650                 655

Trp Ser His Ile Thr Thr Val Leu Gln Asp Gln Lys Ser Gln Cys Gln
            660                 665                 670

Leu Ile Asp Ser Ser Glu Asp Leu Gly Met Ile Ser Ile Gln Gly Pro
        675                 680                 685

Ala Ser Arg Ala Ile Leu Gln Glu Val Leu Asp Ala Asp Leu Ser Asn
    690                 695                 700

Glu Ala Phe Pro Phe Ser Thr His Lys Leu Leu Arg Ala Ala Gly His
705                 710                 715                 720

Leu Val Arg Ala Met Arg Leu Ser Phe Val Gly Glu Leu Gly Trp Glu
                725                 730                 735

Leu His Ile Pro Lys Ala Ser Cys Val Pro Val Tyr Arg Ala Val Met
            740                 745                 750

Ala Ala Gly Ala Lys His Gly Leu Ile Asn Ala Gly Tyr Arg Ala Ile
        755                 760                 765

Asp Ser Leu Ser Ile Glu Lys Gly Tyr Arg His Trp His Ala Asp Leu
    770                 775                 780

Arg Pro Asp Asp Ser Pro Leu Glu Ala Gly Leu Ala Phe Thr Cys Lys
785                 790                 795                 800

Leu Lys Ser Pro Val Pro Phe Leu Gly Arg Glu Ala Leu Glu Gln Gln
                805                 810                 815

Arg Ala Ala Gly Leu Arg Arg Arg Leu Val Cys Phe Thr Met Glu Asp
            820                 825                 830

Lys Val Pro Met Phe Gly Leu Glu Ala Ile Trp Arg Asn Gly Gln Val
        835                 840                 845

Val Gly His Val Arg Arg Ala Asp Phe Gly Phe Ala Ile Asp Lys Thr
    850                 855                 860

Ile Ala Tyr Gly Tyr Ile His Asp Pro Ser Gly Gly Pro Val Ser Leu
865                 870                 875                 880

Asp Phe Val Lys Ser Gly Asp Tyr Ala Leu Glu Arg Met Gly Val Thr
                885                 890                 895

Tyr Gly Ala Gln Ala His Leu Lys Ser Pro Phe Asp Pro Asn Asn Lys
            900                 905                 910

Arg Val Lys Gly Ile Tyr
        915

<210> 83

<211> 357

<212> PRT

<213> Homo sapiens

<300>

<308> sw_hum/amrp_human

<309> 1993-04-01

<400> 83

Met Ala Pro Arg Arg Val Arg Ser Phe Leu Arg Gly Leu Pro Ala Leu
1               5                   10                  15

Leu Leu Leu Leu Leu Phe Leu Gly Pro Trp Pro Ala Ala Ser His Gly
            20                  25                  30

Gly Lys Tyr Ser Arg Glu Lys Asn Gln Pro Lys Pro Ser Pro Lys Arg
        35                  40                  45

Glu Ser Gly Glu Glu Phe Arg Met Glu Lys Leu Asn Gln Leu Trp Glu
    50                  55                  60

Lys Ala Gln Arg Leu His Leu Pro Pro Val Arg Leu Ala Glu Leu His
65                  70                  75                  80

Ala Asp Leu Lys Ile Gln Glu Arg Asp Glu Leu Ala Trp Lys Lys Leu
                85                  90                  95

Lys Leu Asp Gly Leu Asp Glu Asp Gly Glu Lys Glu Ala Arg Leu Ile
            100                 105                 110

Arg Asn Leu Asn Val Ile Leu Ala Lys Tyr Gly Leu Asp Gly Lys Lys
        115                 120                 125

Asp Ala Arg Gln Val Thr Ser Asn Ser Leu Ser Gly Thr Gln Glu Asp
    130                 135                 140

Gly Leu Asp Asp Pro Arg Leu Glu Lys Leu Trp His Lys Ala Lys Thr
145                 150                 155                 160

Ser Gly Lys Phe Ser Gly Glu Glu Leu Asp Lys Leu Trp Arg Glu Phe
                165                 170                 175

Leu His His Lys Glu Lys Val His Glu Tyr Asn Val Leu Leu Glu Thr
            180                 185                 190

Leu Ser Arg Thr Glu Glu Ile His Glu Asn Val Ile Ser Pro Ser Asp
        195                 200                 205

Leu Ser Asp Ile Lys Gly Ser Val Leu His Ser Arg His Thr Glu Leu
    210                 215                 220

Lys Glu Lys Leu Arg Ser Ile Asn Gln Gly Leu Asp Arg Leu Arg Arg
225                 230                 235                 240

Val Ser His Gln Gly Tyr Ser Thr Glu Ala Glu Phe Glu Glu Pro Arg
                245                 250                 255

Val Ile Asp Leu Trp Asp Leu Ala Gln Ser Ala Asn Leu Thr Asp Lys
            260                 265                 270

Glu Leu Glu Ala Phe Arg Glu Glu Leu Lys His Phe Glu Ala Lys Ile
        275                 280                 285

Glu Lys His Asn His Tyr Gln Lys Gln Leu Glu Ile Ala His Glu Lys
    290                 295                 300

Leu Arg His Ala Glu Ser Val Gly Asp Gly Glu Arg Val Ser Arg Ser
305                 310                 315                 320

Arg Glu Lys His Ala Leu Leu Glu Gly Arg Thr Lys Glu Leu Gly Tyr
                325                 330                 335

Thr Val Lys Lys His Leu Gln Asp Leu Ser Gly Arg Ile Ser Arg Ala
            340                 345                 350

Arg His Asn Glu Leu
        355

<210>  84

<211>  354

<212>  PRT

<213>  Homo sapiens


<300>

<308>  sw_hum/arg2_human

<309>  1997-11-01


<400>  84

Met Ser Leu Arg Gly Ser Leu Ser Arg Leu Leu Gln Thr Arg Val His
1               5                   10                  15

Ser Ile Leu Lys Lys Ser Val His Ser Val Ala Val Ile Gly Ala Pro
            20                  25                  30

Phe Ser Gln Gly Gln Lys Arg Lys Gly Val Glu His Gly Pro Ala Ala
        35                  40                  45

Ile Arg Glu Ala Gly Leu Met Lys Arg Leu Ser Ser Leu Gly Cys His
    50                  55                  60

Leu Lys Asp Phe Gly Asp Leu Ser Phe Thr Pro Val Pro Lys Asp Asp
65                  70                  75                  80

Leu Tyr Asn Asn Leu Ile Val Asn Pro Arg Ser Val Gly Leu Ala Asn

85                  90                  95

Gln Glu Leu Ala Glu Val Val Ser Arg Ala Val Ser Asp Gly Tyr Ser
            100                 105                 110

Cys Val Thr Leu Gly Gly Asp His Ser Leu Ala Ile Gly Thr Ile Ser
        115                 120                 125

Gly His Ala Arg His Cys Pro Asp Leu Cys Val Val Trp Val Asp Ala
    130                 135                 140

His Ala Asp Ile Asn Thr Pro Leu Thr Thr Ser Ser Gly Asn Leu His
145                 150                 155                 160

Gly Gln Pro Val Ser Phe Leu Leu Arg Glu Leu Gln Asp Lys Val Pro
                165                 170                 175

Gln Leu Pro Gly Phe Ser Trp Ile Lys Pro Cys Ile Ser Ser Ala Ser
            180                 185                 190

Ile Val Tyr Ile Gly Leu Arg Asp Val Asp Pro Pro Glu His Phe Ile
        195                 200                 205

Leu Lys Asn Tyr Asp Ile Gln Tyr Phe Ser Met Arg Asp Ile Asp Arg
    210                 215                 220

Leu Gly Ile Gln Lys Val Met Glu Arg Thr Phe Asp Leu Leu Ile Gly
225                 230                 235                 240

Lys Arg Gln Arg Pro Ile His Leu Ser Phe Asp Ile Asp Ala Phe Asp
                245                 250                 255

Pro Thr Leu Ala Pro Ala Thr Gly Thr Pro Val Val Gly Gly Leu Thr
            260                 265                 270

Tyr Arg Glu Gly Met Tyr Ile Ala Glu Glu Ile His Asn Thr Gly Leu
        275                 280                 285

Leu Ser Ala Leu Asp Leu Val Glu Val Asn Pro Gln Leu Ala Thr Ser
    290                 295                 300

Glu Glu Glu Ala Lys Thr Thr Ala Asn Leu Ala Val Asp Val Ile Ala
305                 310                 315                 320

Ser Ser Phe Gly Gln Thr Arg Glu Gly Gly His Ile Val Tyr Asp Gln
                325                 330                 335

Leu Pro Thr Pro Ser Ser Pro Asp Glu Ser Glu Asn Gln Ala Arg Val
            340                 345                 350

Arg Ile

<210>  85

<211>  333

<212>  PRT

<213>  Homo sapiens

<300>

<308>  sw_hum/if2b_human

<309>  1991-02-01

<400>  85

Met Ser Gly Asp Glu Met Ile Phe Asp Pro Thr Met Ser Lys Lys Lys
1               5                   10                  15

Lys Lys Lys Lys Lys Pro Phe Met Leu Asp Glu Glu Gly Asp Thr Gln
            20                  25                  30

Thr Glu Glu Thr Gln Pro Ser Glu Thr Lys Glu Val Glu Pro Glu Pro
        35                  40                  45

Thr Glu Asp Lys Asp Leu Glu Ala Asp Glu Glu Asp Thr Arg Lys Lys
    50                  55                  60

Asp Ala Ser Asp Asp Leu Asp Asp Leu Asn Phe Phe Asn Gln Lys Lys
65                  70                  75                  80

Lys Lys Lys Lys Thr Lys Lys Ile Phe Asp Ile Asp Glu Ala Glu Glu
                85                  90                  95

Gly Val Lys Asp Leu Lys Ile Glu Ser Asp Val Gln Glu Pro Thr Glu
            100                 105                 110

Pro Glu Asp Asp Leu Asp Ile Met Leu Gly Asn Lys Lys Lys Lys Lys
        115                 120                 125

Lys Asn Val Lys Phe Pro Asp Glu Asp Glu Ile Leu Glu Lys Asp Glu
    130                 135                 140

Ala Leu Glu Asp Glu Asp Asn Lys Lys Asp Asp Gly Ile Ser Phe Ser
145                 150                 155                 160

Asn Gln Thr Gly Pro Ala Trp Ala Gly Ser Glu Arg Asp Tyr Thr Tyr
                165                 170                 175

Glu Glu Leu Leu Asn Arg Val Phe Asn Ile Met Arg Glu Lys Asn Pro
            180                 185                 190

Asp Met Val Ala Gly Glu Lys Arg Lys Phe Val Met Lys Pro Pro Gln
        195                 200                 205

Val Val Arg Val Gly Thr Lys Lys Thr Ser Phe Val Asn Phe Thr Asp
    210                 215                 220

Ile Cys Lys Leu Leu His Arg Gln Pro Lys His Leu Leu Ala Phe Leu
225                 230                 235                 240

Leu Ala Glu Leu Gly Thr Ser Gly Ser Ile Asp Gly Asn Asn Gln Leu
                245                 250                 255

Val Ile Lys Gly Arg Phe Gln Gln Lys Gln Ile Glu Asn Val Leu Arg
            260                 265                 270

Arg Tyr Ile Lys Glu Tyr Val Thr Cys His Thr Cys Arg Ser Pro Asp
        275                 280                 285

Thr Ile Leu Gln Lys Asp Thr Arg Leu Tyr Phe Leu Gln Cys Glu Thr
    290                 295                 300

Cys His Ser Arg Cys Ser Val Ala Ser Ile Lys Thr Gly Phe Gln Ala
305                 310                 315                 320

Val Thr Gly Lys Arg Ala Gln Leu Arg Ala Lys Ala Asn
                325                 330

<210> 86

<211> 391

<212> PRT

<213> Homo sapiens

<300>

<308> sw_hum/rog_human

<309> 1994-10-01

<400> 86

Met Val Glu Ala Asp Arg Pro Gly Lys Leu Phe Ile Gly Gly Leu Asn
1               5                   10                  15

Thr Glu Thr Asn Glu Lys Ala Leu Glu Ala Val Phe Gly Lys Tyr Gly
            20                  25                  30

Arg Ile Val Glu Val Leu Leu Met Lys Asp Arg Glu Thr Asn Lys Ser
        35                  40                  45

Arg Gly Phe Ala Phe Val Thr Phe Glu Ser Pro Ala Asp Ala Lys Asp
    50                  55                  60

Ala Ala Arg Asp Met Asn Gly Lys Ser Leu Asp Gly Lys Ala Ile Lys
65                  70                  75                  80

Val Glu Gln Ala Thr Lys Pro Ser Phe Glu Ser Gly Arg Arg Gly Pro
                85                  90                  95

Pro Pro Pro Pro Arg Ser Arg Gly Pro Pro Arg Gly Leu Arg Gly Gly
            100                 105                 110

Arg Gly Gly Ser Gly Gly Thr Arg Gly Pro Pro Ser Arg Gly Gly His
        115                 120                 125

Met Asp Asp Gly Gly Tyr Ser Met Asn Phe Asn Met Ser Ser Ser Arg
    130                 135                 140

Gly Pro Leu Pro Val Lys Arg Gly Pro Pro Pro Arg Ser Gly Gly Pro
145                 150                 155                 160

Pro Pro Lys Arg Ser Ala Pro Ser Gly Pro Val Arg Ser Ser Ser Gly
                165                 170                 175

Met Gly Gly Arg Ala Pro Val Ser Arg Gly Arg Asp Ser Tyr Gly Gly
            180                 185                 190

Pro Pro Arg Arg Glu Pro Leu Pro Ser Arg Arg Asp Val Tyr Leu Ser
        195                 200                 205

Pro Arg Asp Asp Gly Tyr Ser Thr Lys Asp Ser Tyr Ser Ser Arg Asp
    210                 215                 220

Tyr Pro Ser Ser Arg Asp Thr Arg Asp Tyr Ala Pro Pro Pro Arg Asp
225                 230                 235                 240

Tyr Thr Tyr Arg Asp Tyr Gly His Ser Ser Ser Arg Asp Asp Tyr Pro
                245                 250                 255

Ser Arg Gly Tyr Ser Asp Arg Asp Gly Tyr Gly Arg Asp Arg Asp Tyr
            260                 265                 270

Ser Asp His Pro Ser Gly Gly Ser Tyr Arg Asp Ser Tyr Glu Ser Tyr
        275                 280                 285

Gly Asn Ser Arg Ser Ala Pro Pro Thr Arg Gly Pro Pro Pro Ser Tyr
    290                 295                 300

Gly Gly Ser Ser Arg Tyr Asp Asp Tyr Ser Ser Ser Arg Asp Gly Tyr
305                 310                 315                 320

Gly Gly Ser Arg Asp Ser Tyr Ser Ser Ser Arg Ser Asp Leu Tyr Ser
                325                 330                 335

Ser Gly Arg Asp Arg Val Gly Arg Gln Glu Arg Gly Leu Pro Pro Ser
            340                 345                 350

Met Glu Arg Gly Tyr Pro Pro Pro Arg Asp Ser Tyr Ser Ser Ser Ser
        355                 360                 365

Arg Gly Ala Pro Arg Gly Gly Gly Arg Gly Gly Ser Arg Ser Asp Arg
    370                 375                 380

Gly Gly Gly Arg Ser Arg Tyr
385                 390

<210>  87

<211>  669

<212>  PRT

<213>  Homo sapiens

<300>

<308>  humangp/chr11-075932

<309>  2003-04-22

<400>  87

Met Lys Ile Phe Val Gly Asn Val Asp Gly Ala Asp Thr Thr Pro Glu
1               5                   10                  15

Glu Leu Ala Ala Leu Phe Ala Pro Tyr Gly Thr Val Met Ser Cys Ala
            20                  25                  30

Val Met Lys Gln Phe Ala Phe Val His Met Arg Glu Asn Ala Gly Ala
        35                  40                  45

Leu Arg Ala Ile Glu Ala Leu His Gly His Glu Leu Arg Pro Gly Arg
    50                  55                  60

Ala Leu Val Val Glu Met Ser Arg Pro Arg Pro Leu Asn Thr Trp Lys
65                  70                  75                  80

Ile Phe Val Gly Asn Val Ser Ala Ala Cys Thr Ser Gln Glu Leu Arg
                85                  90                  95

Ser Leu Phe Glu Arg Arg Gly Arg Val Ile Glu Cys Asp Val Val Lys
            100                 105                 110

Asp Tyr Ala Phe Val His Met Glu Lys Glu Ala Asp Ala Lys Ala Ala
        115                 120                 125

Ile Ala Gln Leu Asn Gly Lys Glu Val Lys Gly Lys Arg Ile Asn Val
    130                 135                 140

Glu Leu Ser Thr Lys Gly Gln Lys Lys Gly Pro Gly Leu Ala Val Gln
145                 150                 155                 160

Ser Gly Asp Lys Thr Lys Lys Pro Gly Ala Gly Asp Thr Ala Phe Pro
                165                 170                 175

Gly Thr Gly Gly Phe Ser Ala Thr Phe Asp Tyr Gln Gln Ala Phe Gly
            180                 185                 190

Asn Ser Thr Gly Gly Phe Asp Gly Gln Ala Arg Gln Pro Thr Pro Pro
        195                 200                 205

Phe Phe Gly Arg Asp Arg Ser Pro Leu Arg Arg Ser Pro Pro Arg Ala
    210                 215                 220

Ser Tyr Val Ala Pro Leu Thr Ala Gln Pro Ala Thr Tyr Arg Ala Gln
225                 230                 235                 240

Pro Ser Val Ser Leu Gly Ala Ala Tyr Arg Ala Gln Pro Ser Ala Ser
                245                 250                 255

Leu Gly Val Gly Tyr Arg Thr Gln Pro Met Thr Ala Gln Ala Ala Ser
            260                 265                 270

Tyr Arg Ala Gln Pro Ser Val Ser Leu Gly Ala Pro Tyr Arg Gly Gln

275                 280                 285

Leu Ala Ser Pro Ser Ser Gln Ser Ala Ala Ala Ser Ser Leu Gly Pro
    290                 295                 300

Tyr Gly Gly Ala Gln Pro Ser Ala Ser Ala Leu Ser Ser Tyr Gly Gly
305                 310                 315                 320

Gln Ala Ala Ala Ala Ser Ser Leu Asn Ser Tyr Gly Ala Gln Gly Ser
                325                 330                 335

Ser Leu Ala Ser Tyr Gly Asn Gln Pro Ser Ser Tyr Gly Ala Gln Ala
            340                 345                 350

Ala Ser Ser Tyr Gly Val Arg Ala Ala Ala Ser Ser Tyr Asn Thr Gln
        355                 360                 365

Gly Ala Ala Ser Ser Leu Gly Ser Tyr Gly Ala Gln Ala Ala Ser Tyr
    370                 375                 380

Gly Ala Gln Ser Ala Ala Ser Ser Leu Ala Tyr Gly Ala Gln Ala Ala
385                 390                 395                 400

Ser Tyr Asn Ala Gln Pro Ser Ala Ser Tyr Asn Ala Gln Ser Ala Pro
                405                 410                 415

Tyr Ala Ala Gln Gln Ala Ala Ser Tyr Ser Ser Gln Pro Ala Ala Tyr
            420                 425                 430

Val Ala Gln Pro Ala Thr Ala Ala Ala Tyr Ala Ser Gln Pro Ala Ala
        435                 440                 445

Tyr Ala Ala Gln Ala Thr Thr Pro Met Ala Gly Ser Tyr Gly Ala Gln
    450                 455                 460

Pro Val Val Gln Thr Gln Leu Asn Ser Tyr Gly Ala Gln Ala Ser Met
465                 470                 475                 480

Gly Leu Ser Gly Ser Tyr Gly Ala Gln Ser Ala Ala Ala Ala Thr Gly
                485                 490                 495

Ser Tyr Gly Ala Ala Ala Ala Tyr Gly Ala Gln Pro Ser Ala Thr Leu
            500                 505                 510

Ala Ala Pro Tyr Arg Thr Gln Ser Ser Ala Ser Leu Ala Ala Ser Tyr
        515                 520                 525

Ala Ala Gln Gln His Pro Gln Ala Ala Ala Ser Tyr Arg Gly Gln Pro
    530                 535                 540

Gly Asn Ala Tyr Asp Gly Ala Gly Gln Pro Ser Ala Ala Tyr Leu Ser
545                 550                 555                 560

Met Ser Gln Gly Ala Val Ala Asn Ala Asn Ser Thr Pro Pro Pro Tyr
                565                 570                 575

Glu Arg Thr Arg Leu Ser Pro Pro Arg Ala Ser Tyr Asp Asp Pro Tyr
            580                 585                 590

Lys Lys Ala Val Ala Met Ser Lys Arg Tyr Gly Ser Asp Arg Arg Leu
        595                 600                 605

Ala Glu Leu Ser Asp Tyr Arg Arg Leu Ser Glu Ser Gln Leu Ser Phe
    610                 615                 620

Arg Arg Ser Pro Thr Lys Ser Ser Leu Asp Tyr Arg Arg Leu Pro Asp
625                 630                 635                 640

Ala His Ser Asp Tyr Ala Arg Tyr Ser Gly Ser Tyr Asn Asp Tyr Leu
                645                 650                 655

Arg Ala Ala Gln Met His Ser Gly Tyr Gln Arg Arg Met
            660                 665

<210>  88

<211>  539

<212>  PRT

<213>  Homo sapiens

<300>

<308>  sw_hum/tcpd_human

<309>  1996-10-01

<400>  88

Met Pro Glu Asn Val Ala Pro Arg Ser Gly Ala Thr Ala Gly Ala Ala
1               5                   10                  15

Gly Gly Arg Gly Lys Gly Ala Tyr Gln Asp Arg Asp Lys Pro Ala Gln
            20                  25                  30

Ile Arg Phe Ser Asn Ile Ser Ala Ala Lys Ala Val Ala Asp Ala Ile
        35                  40                  45

Arg Thr Ser Leu Gly Pro Lys Gly Met Asp Lys Met Ile Gln Asp Gly
    50                  55                  60

Lys Gly Asp Val Thr Ile Thr Asn Asp Gly Ala Thr Ile Leu Lys Gln
65                  70                  75                  80

Met Gln Val Leu His Pro Ala Ala Arg Met Leu Val Glu Leu Ser Lys
                85                  90                  95

Ala Gln Asp Ile Glu Ala Gly Asp Gly Thr Thr Ser Val Val Ile Ile
            100                 105                 110

Ala Gly Ser Leu Leu Asp Ser Cys Thr Lys Leu Leu Gln Lys Gly Ile
        115                 120                 125

His Pro Thr Ile Ile Ser Glu Ser Phe Gln Lys Ala Leu Glu Lys Gly
    130                 135                 140

Ile Glu Ile Leu Thr Asp Met Ser Arg Pro Val Glu Leu Ser Asp Arg
145                 150                 155                 160

Glu Thr Leu Leu Asn Ser Ala Thr Thr Ser Leu Asn Ser Lys Val Val
                165                 170                 175

Ser Gln Tyr Ser Ser Leu Leu Ser Pro Met Ser Val Asn Ala Val Met
            180                 185                 190

Lys Val Ile Asp Pro Ala Thr Ala Thr Ser Val Asp Leu Arg Asp Ile
        195                 200                 205

Lys Ile Val Lys Lys Leu Gly Gly Thr Ile Asp Asp Cys Glu Leu Val
    210                 215                 220

Glu Gly Leu Val Leu Thr Gln Lys Val Ser Asn Ser Gly Ile Thr Arg
225                 230                 235                 240

Val Glu Lys Ala Lys Ile Gly Leu Ile Gln Phe Cys Leu Ser Ala Pro
                245                 250                 255

Lys Thr Asp Met Asp Asn Gln Ile Val Val Ser Asp Tyr Ala Gln Met
            260                 265                 270

Asp Arg Val Leu Arg Glu Glu Arg Ala Tyr Ile Leu Asn Leu Val Lys
        275                 280                 285

Gln Ile Lys Lys Thr Gly Cys Asn Val Leu Leu Ile Gln Lys Ser Ile
    290                 295                 300

Leu Arg Asp Ala Leu Ser Asp Leu Ala Leu His Phe Leu Asn Lys Met
305                 310                 315                 320

Lys Ile Met Val Ile Lys Asp Ile Glu Arg Glu Asp Ile Glu Phe Ile
                325                 330                 335

Cys Lys Thr Ile Gly Thr Lys Pro Val Ala His Ile Asp Gln Phe Thr
            340                 345                 350

Ala Asp Met Leu Gly Ser Ala Glu Leu Ala Glu Glu Val Asn Leu Asn
        355                 360                 365

Gly Ser Gly Lys Leu Leu Lys Ile Thr Gly Cys Ala Ser Pro Gly Lys
    370                 375                 380

Thr Val Thr Ile Val Val Arg Gly Ser Asn Lys Leu Val Ile Glu Glu
385                 390                 395                 400

Ala Glu Arg Ser Ile His Asp Ala Leu Cys Val Ile Arg Cys Leu Val
                405                 410                 415

Lys Lys Arg Ala Leu Ile Ala Gly Gly Gly Ala Pro Glu Ile Glu Leu
            420                 425                 430

Ala Leu Ala Leu Thr Glu Tyr Ser Arg Thr Leu Ser Gly Met Glu Ser
        435                 440                 445

Tyr Cys Val Arg Ala Phe Ala Asp Ala Met Glu Val Ile Pro Ser Thr
    450                 455                 460

Leu Ala Glu Asn Ala Gly Leu Asn Pro Ile Ser Thr Val Thr Glu Leu
465                 470                 475                 480

Arg Asn Arg His Ala Gln Gly Glu Lys Thr Ala Gly Ile Asn Val Arg
                485                 490                 495

Lys Gly Gly Ile Ser Asn Ile Leu Glu Glu Leu Val Val Gln Pro Leu
            500                 505                 510

Leu Val Ser Val Ser Ala Leu Thr Leu Ala Thr Glu Thr Val Arg Ser
        515                 520                 525

Ile Leu Lys Ile Asp Asp Val Val Asn Thr Arg
    530                 535

<210>  89

<211>  306

<212>  PRT

<213>  Homo sapiens


<220>

<221>  MISC_FEATURE

<222>  (30)..(30)

<223>  x


<220>

<221>  MISC_FEATURE

<222>  (114)..(114)

<223>  x


<220>

<221>  MISC_FEATURE

<222>  (117)..(117)

<223>  x


<220>

<221>  MISC_FEATURE

<222>  (126)..(126)

<223>  x


<220>

<221>  MISC_FEATURE
<222>  (131)..(131)
<223>  x

<220>
<221>  MISC_FEATURE
<222>  (132)..(132)
<223>  x

<220>
<221>  MISC_FEATURE
<222>  (139)..(139)
<223>  x

<220>
<221>  MISC_FEATURE
<222>  (141)..(141)
<223>  x

<220>
<221>  MISC_FEATURE
<222>  (152)..(152)
<223>  x

<220>
<221>  MISC_FEATURE
<222>  (157)..(157)
<223>  x

<220>
<221>  MISC_FEATURE
<222>  (172)..(172)
<223>  x

<220>
<221>  MISC_FEATURE
<222>  (174)..(174)
<223>  x

<220>

<221>  MISC_FEATURE

<222>  (175)..(175)

<223>  x

<220>

<221>  MISC_FEATURE

<222>  (176)..(176)

<223>  x

<220>

<221>  MISC_FEATURE

<222>  (177)..(177)

<223>  x

<220>

<221>  MISC_FEATURE

<222>  (179)..(179)

<223>  x

<220>

<221>  MISC_FEATURE

<222>  (189)..(189)

<223>  x

<220>

<221>  MISC_FEATURE

<222>  (193)..(193)

<223>  x

<220>

<221>  MISC_FEATURE

<222>  (198)..(198)

<223>  x

<220>

<221>  MISC_FEATURE

<222>  (199)..(199)
<223>  x

<220>
<221>  MISC_FEATURE
<222>  (201)..(201)
<223>  x

<220>
<221>  MISC_FEATURE
<222>  (202)..(202)
<223>  x

<220>
<221>  MISC_FEATURE
<222>  (204)..(204)
<223>  x

<220>
<221>  MISC_FEATURE
<222>  (206)..(206)
<223>  x

<220>
<221>  MISC_FEATURE
<222>  (209)..(209)
<223>  x

<220>
<221>  MISC_FEATURE
<222>  (211)..(211)
<223>  x

<220>
<221>  MISC_FEATURE
<222>  (214)..(214)
<223>  x

<220>

<221>  MISC_FEATURE

<222>  (217)..(217)

<223>  x


<220>

<221>  MISC_FEATURE

<222>  (234)..(234)

<223>  x


<300>

<308>  hsugp/075546-8-0

<309>  2003-02-16


<400>  89

Arg Trp Pro Leu Pro Pro Pro Pro Pro Gly Cys Arg Gln Lys Glu Asp
1               5                   10                  15

Ala Asp Leu Glu Glu Gln Leu Ser Asp Glu Glu Lys Val Xaa Tyr Arg
            20                  25                  30

Ala Lys Phe Ile Ile His Ala Pro Pro Gly Glu Phe Asn Glu Val Phe
        35                  40                  45

Asn Asp Val Arg Leu Leu Leu Asn Asn Asp Asn Leu Leu Arg Glu Gly
    50                  55                  60

Ala Ala His Ala Phe Ala Gln Tyr Asn Leu Asp Gln Phe Thr Pro Val
65                  70                  75                  80

Lys Ile Glu Gly Tyr Glu Asp Gln Val Leu Ile Thr Asn Met Gly Asp
                85                  90                  95

Leu Gly Asn Gly Lys Val Phe Gly Ile Gln Arg Thr Glu Ser Val Leu
            100                 105                 110

Asn Xaa Asp His Xaa Lys Glu Gly Gly Gln Leu Ile Gln Xaa Pro Cys
        115                 120                 125

Glu Val Xaa Xaa Met Gln Leu Asn His Gly Xaa Thr Xaa Ser Arg Asn
    130                 135                 140

Cys Ser Glu Ser Leu Arg Lys Xaa Asn Ile Thr Arg Xaa Gly Val Cys
145                 150                 155                 160

Thr Val Tyr Gly Lys Lys Ile Asp Gly Gln Gln Xaa Ile Xaa Xaa Xaa
                165                 170                 175

Xaa Ile Xaa Lys Pro Ser Val Pro Ser Lys Lys Phe Xaa Asn Gly Arg
            180                 185                 190

Xaa Arg Ser Glu Trp Xaa Xaa Thr Xaa Xaa Pro Xaa Asn Xaa Ser Ser
        195                 200                 205

Xaa Trp Xaa Leu Glu Xaa Pro Val Xaa Tyr Tyr Asp Asp Gly Tyr Val
    210                 215                 220

Gln Leu Val Ser His Asn Gly Phe Gln Xaa Ser Leu Thr Val Ser Asn
225                 230                 235                 240

Gly Val His Gln Pro Lys Asn Tyr Lys Val Val Thr Val Arg Met Asn
                245                 250                 255

Ser Met Leu Gly Glu Phe Arg His Val Ala Tyr Ser Ser Leu Val Lys
            260                 265                 270

Gly Arg Pro Gln Arg Trp Glu Asp Phe Asn Gly Ser Ala Leu Lys Arg
        275                 280                 285

Arg Ser Ser Thr Asn Lys Gly Arg Gly Thr Lys Arg Thr Pro Leu Pro
    290                 295                 300

Asp Phe
305

<210>  90

<211>  531

<212>  PRT

<213>  Homo sapiens

<300>

<308>  humangp/chr3-075191

<309>  2003-04-22

<400>  90

Met Ala Glu His Ala Pro Arg Arg Cys Cys Leu Gly Trp Asp Phe Ser
1               5                   10                  15

Thr Gln Gln Val Lys Val Val Ala Val Asp Ala Glu Leu Asn Val Phe
            20                  25                  30

Tyr Glu Glu Ser Val His Phe Asp Arg Asp Leu Pro Glu Phe Gly Tyr
        35                  40                  45

Val Leu Asp Val His Val Thr Gln Gly Gly Val His Val His Lys Asp
    50                  55                  60

Gly Leu Thr Val Thr Ser Pro Val Leu Met Trp Val Gln Ala Leu Asp
65                  70                  75                  80

Ile Ile Leu Glu Lys Met Lys Ala Ser Gly Phe Asp Phe Ser Gln Val
                85                  90                  95

Leu Ala Leu Ser Gly Ala Gly Gln Gln His Gly Ser Ile Tyr Trp Lys
            100                 105                 110

Ala Gly Ala Gln Gln Ala Leu Thr Ser Leu Ser Pro Asp Leu Arg Leu
        115                 120                 125

His Gln Gln Leu Gln Asp Cys Phe Ser Ile Ser Asp Cys Pro Val Trp
    130                 135                 140

Met Asp Ser Ser Thr Thr Ala Gln Cys Arg Gln Leu Glu Ala Ala Val
145                 150                 155                 160

Gly Gly Ala Gln Ala Leu Ser Cys Leu Thr Gly Ser Arg Ala Tyr Glu
                165                 170                 175

Arg Phe Thr Gly Asn Gln Ile Ala Lys Ile Tyr Gln Gln Asn Pro Glu
            180                 185                 190

Ala Tyr Ser His Thr Glu Arg Ile Ser Leu Val Ser Ser Phe Ala Ala
        195                 200                 205

Ser Leu Phe Leu Gly Ser Tyr Ser Pro Ile Asp Tyr Ser Asp Gly Ser
    210                 215                 220

Gly Met Asn Leu Leu Gln Ile Gln Asp Lys Val Trp Ser Gln Ala Cys
225                 230                 235                 240

Leu Gly Ala Cys Ala Pro His Leu Glu Glu Lys Leu Ser Pro Pro Val
                245                 250                 255

Pro Ser Cys Ser Val Val Gly Ala Ile Ser Ser Tyr Tyr Val Gln Arg
            260                 265                 270

Tyr Gly Phe Pro Pro Gly Cys Lys Val Val Ala Phe Thr Gly Asp Asn
        275                 280                 285

Pro Ala Ser Leu Ala Gly Met Arg Leu Glu Glu Gly Asp Ile Ala Val
    290                 295                 300

Ser Leu Gly Thr Ser Asp Thr Leu Phe Leu Trp Leu Gln Glu Pro Met
305                 310                 315                 320

Pro Ala Leu Glu Gly His Ile Phe Cys Asn Pro Val Asp Ser Gln His
                325                 330                 335

Tyr Met Ala Leu Leu Cys Phe Lys Asn Gly Ser Leu Met Arg Glu Lys
            340                 345                 350

Ile Arg Asn Glu Ser Val Ser Arg Ser Trp Ser Asp Phe Ser Lys Ala
        355                 360                 365

Leu Gln Ser Thr Glu Met Gly Asn Gly Gly Asn Leu Gly Phe Tyr Phe
    370                 375                 380

Asp Val Met Glu Ile Thr Pro Glu Ile Ile Gly Arg His Arg Phe Asn
385                 390                 395                 400

Thr Glu Asn His Lys Val Ala Ala Phe Pro Gly Asp Val Glu Val Arg

                405                 410                 415

Ala Leu Ile Glu Gly Gln Phe Met Ala Lys Arg Ile His Ala Glu Gly
            420                 425                 430

Leu Gly Tyr Arg Val Met Ser Lys Thr Lys Ile Leu Ala Thr Gly Gly
        435                 440                 445

Ala Ser His Asn Arg Glu Ile Leu Gln Val Leu Ala Asp Val Phe Asp
    450                 455                 460

Ala Pro Val Tyr Val Ile Asp Thr Ala Asn Ser Ala Cys Val Gly Ser
465                 470                 475                 480

Ala Tyr Arg Ala Phe His Gly Leu Ala Gly Gly Thr Asp Val Pro Phe
                485                 490                 495

Ser Glu Val Val Lys Leu Ala Pro Asn Pro Arg Leu Ala Ala Thr Pro
            500                 505                 510

Ser Pro Gly Ala Ser Gln Val Arg Asp His Arg Asn Leu Phe Val Ala
        515                 520                 525

Phe Ala Leu
    530

<210> 91

<211> 384

<212> PRT

<213> Homo sapiens

<300>

<308> sw_hum/dus9_human

<309> 1998-12-15

<400> 91

Met Glu Gly Leu Gly Arg Ser Cys Leu Trp Leu Arg Arg Glu Leu Ser
1               5                   10                  15

Pro Pro Arg Pro Arg Leu Leu Leu Leu Asp Cys Arg Ser Arg Glu Leu
            20                  25                  30

Tyr Glu Ser Ala Arg Ile Gly Gly Ala Leu Ser Val Ala Leu Pro Ala
        35                  40                  45

Leu Leu Leu Arg Arg Leu Arg Arg Gly Ser Leu Ser Val Arg Ala Leu
    50                  55                  60

Leu Pro Gly Pro Pro Leu Gln Pro Pro Pro Pro Ala Pro Val Leu Leu
65                  70                  75                  80

Tyr Asp Gln Gly Gly Gly Arg Arg Arg Arg Gly Glu Ala Glu Ala Glu
                85                  90                  95

Ala Glu Glu Trp Glu Ala Glu Ser Val Leu Gly Thr Leu Leu Gln Lys
            100                 105                 110

Leu Arg Glu Glu Gly Tyr Leu Ala Tyr Tyr Leu Gln Gly Gly Phe Ser
        115                 120                 125

Arg Phe Gln Ala Glu Cys Pro His Leu Cys Glu Thr Ser Leu Ala Gly
    130                 135                 140

Arg Ala Gly Ser Ser Met Ala Pro Val Pro Gly Pro Val Pro Val Val
145                 150                 155                 160

Gly Leu Gly Ser Leu Cys Leu Gly Ser Asp Cys Ser Asp Ala Glu Ser
                165                 170                 175

Glu Ala Asp Arg Asp Ser Met Ser Cys Gly Leu Asp Ser Glu Gly Ala
            180                 185                 190

Thr Pro Pro Pro Val Gly Leu Arg Ala Ser Phe Pro Val Gln Ile Leu
        195                 200                 205

Pro Asn Leu Tyr Leu Gly Ser Ala Arg Asp Ser Ala Asn Leu Glu Ser
    210                 215                 220

Leu Ala Lys Leu Gly Ile Arg Tyr Ile Leu Asn Val Thr Pro Asn Leu
225                 230                 235                 240

Pro Asn Phe Phe Glu Lys Asn Gly Asp Phe His Tyr Lys Gln Ile Pro
                245                 250                 255

Ile Ser Asp His Trp Ser Gln Asn Leu Ser Arg Phe Phe Pro Glu Ala
            260                 265                 270

Ile Glu Phe Ile Asp Glu Ala Leu Ser Gln Asn Cys Gly Val Leu Val
        275                 280                 285

His Cys Leu Ala Gly Val Ser Arg Ser Val Thr Val Thr Val Ala Tyr
    290                 295                 300

Leu Met Gln Lys Leu His Leu Ser Leu Asn Asp Ala Tyr Asp Leu Val
305                 310                 315                 320

Lys Arg Lys Lys Ser Asn Ile Ser Pro Asn Phe Asn Phe Met Gly Gln
                325                 330                 335

Leu Leu Asp Phe Glu Arg Ser Leu Arg Leu Glu Glu Arg His Ser Gln
            340                 345                 350

Glu Gln Gly Ser Gly Gly Gln Ala Ser Ala Ala Ser Asn Pro Pro Ser
        355                 360                 365

Phe Phe Thr Thr Pro Thr Ser Asp Gly Ala Phe Glu Leu Ala Pro Thr
    370                 375                 380

<210>  92

<211>  218

<212>  PRT

<213>  Homo sapiens

<300>

<308>  sw_hum/gtm4_human

<309>  1995-11-01

<400>  92

Met Ser Met Thr Leu Gly Tyr Trp Asp Ile Arg Gly Leu Ala His Ala
1               5                   10                  15

Ile Arg Leu Leu Leu Glu Tyr Thr Asp Ser Ser Tyr Glu Glu Lys Lys
            20                  25                  30

Tyr Thr Met Gly Asp Ala Pro Asp Tyr Asp Arg Ser Gln Trp Leu Asn
        35                  40                  45

Glu Lys Phe Lys Leu Gly Leu Asp Phe Pro Asn Leu Pro Tyr Leu Ile
    50                  55                  60

Asp Gly Ala His Lys Ile Thr Gln Ser Asn Ala Ile Leu Cys Tyr Ile
65                  70                  75                  80

Ala Arg Lys His Asn Leu Cys Gly Glu Thr Glu Glu Glu Lys Ile Arg
                85                  90                  95

Val Asp Ile Leu Glu Asn Gln Ala Met Asp Val Ser Asn Gln Leu Ala
            100                 105                 110

Arg Val Cys Tyr Ser Pro Asp Phe Glu Lys Leu Lys Pro Glu Tyr Leu
        115                 120                 125

Glu Glu Leu Pro Thr Met Met Gln His Phe Ser Gln Phe Leu Gly Lys
    130                 135                 140

Arg Pro Trp Phe Val Gly Asp Lys Ile Thr Phe Val Asp Phe Leu Ala
145                 150                 155                 160

Tyr Asp Val Leu Asp Leu His Arg Ile Phe Glu Pro Asn Cys Leu Asp
                165                 170                 175

Ala Phe Pro Asn Leu Lys Asp Phe Ile Ser Arg Phe Glu Gly Leu Glu
            180                 185                 190

Lys Ile Ser Ala Tyr Met Lys Ser Ser Arg Phe Leu Pro Lys Pro Leu
        195                 200                 205

Tyr Thr Arg Val Ala Val Trp Gly Asn Lys
    210                 215

<210>  93

<211>  207

<212>  PRT

<213>  Homo sapiens

<300>

<308>  sw_hum/rs8_humam

<309>  1988-11-01

<400>  93

Gly Ile Ser Arg Asp Asn Trp His Lys Arg Arg Lys Thr Gly Gly Lys
1               5                   10                  15

Arg Lys Pro Tyr His Lys Lys Arg Lys Tyr Glu Leu Gly Arg Pro Ala
            20                  25                  30

Ala Asn Thr Lys Ile Gly Pro Arg Arg Ile His Thr Val Arg Val Arg
        35                  40                  45

Gly Gly Asn Lys Lys Tyr Arg Ala Leu Arg Leu Asp Val Gly Asn Phe
    50                  55                  60

Ser Trp Gly Ser Glu Cys Cys Thr Arg Lys Thr Arg Ile Ile Asp Val
65                  70                  75                  80

Val Tyr Asn Ala Ser Asn Asn Glu Leu Val Arg Thr Lys Thr Leu Val
                85                  90                  95

Lys Asn Cys Ile Val Leu Ile Asp Ser Thr Pro Tyr Arg Gln Trp Tyr
            100                 105                 110

Glu Ser His Tyr Ala Leu Pro Leu Gly Arg Lys Lys Gly Ala Lys Leu
        115                 120                 125

Thr Pro Glu Glu Glu Glu Ile Leu Asn Lys Lys Arg Ser Lys Lys Ile
    130                 135                 140

Gln Lys Lys Tyr Asp Glu Arg Lys Lys Asn Ala Lys Ile Ser Ser Leu
145                 150                 155                 160

Leu Glu Glu Gln Phe Gln Gln Gly Lys Leu Leu Ala Cys Ile Ala Ser
                165                 170                 175

Arg Pro Gly Gln Cys Gly Arg Ala Asp Gly Tyr Val Leu Glu Gly Lys
            180                 185                 190

Glu Leu Glu Phe Tyr Leu Arg Lys Ile Lys Ala Arg Lys Gly Lys
        195                 200                 205

<210>  94

<211>  299

<212>  PRT

<213>  Homo sapiens

<300>

<308>  sw_hum/sm30_human

<309>  1998-07-15

<400>  94

Met Ser Ser Ile Lys Ile Glu Cys Val Leu Pro Glu Asn Cys Arg Cys
1               5                   10                  15

Gly Glu Ser Pro Val Trp Glu Glu Val Ser Asn Ser Leu Leu Phe Val
            20                  25                  30

Asp Ile Pro Ala Lys Lys Val Cys Arg Trp Asp Ser Phe Thr Lys Gln
        35                  40                  45

Val Gln Arg Val Thr Met Asp Ala Pro Val Ser Ser Val Ala Leu Arg
    50                  55                  60

Gln Ser Gly Gly Tyr Val Ala Thr Ile Gly Thr Lys Phe Cys Ala Leu
65                  70                  75                  80

Asn Trp Lys Glu Gln Ser Ala Val Val Leu Ala Thr Val Asp Asn Asp
                85                  90                  95

Lys Lys Asn Asn Arg Phe Asn Asp Gly Lys Val Asp Pro Ala Gly Arg
            100                 105                 110

Tyr Phe Ala Gly Thr Met Ala Glu Glu Thr Ala Pro Ala Val Leu Glu
        115                 120                 125

Arg His Gln Gly Ala Leu Tyr Ser Leu Phe Pro Asp His His Val Lys
    130                 135                 140

Lys Tyr Phe Asp Gln Val Asp Ile Ser Asn Gly Leu Asp Trp Ser Leu
145                 150                 155                 160

Asp His Lys Ile Phe Tyr Tyr Ile Asp Ser Leu Ser Tyr Ser Val Asp
                165                 170                 175

Ala Phe Asp Tyr Asp Leu Gln Thr Gly Gln Ile Ser Asn Arg Arg Ser
            180                 185                 190

Val Tyr Lys Leu Glu Lys Glu Glu Gln Ile Pro Asp Gly Met Cys Ile
        195                 200                 205

Asp Ala Glu Gly Lys Leu Trp Val Ala Cys Tyr Asn Gly Gly Arg Val
    210                 215                 220

Ile Arg Leu Asp Pro Val Thr Gly Lys Arg Leu Gln Thr Val Lys Leu
225                 230                 235                 240

Pro Val Asp Lys Thr Thr Ser Cys Cys Phe Gly Gly Lys Asn Tyr Ser
                245                 250                 255

Glu Met Tyr Val Thr Cys Ala Arg Asp Gly Met Asp Pro Glu Gly Leu
            260                 265                 270

Leu Arg Gln Pro Glu Ala Gly Gly Ile Phe Lys Ile Thr Gly Leu Gly
        275                 280                 285

Val Lys Gly Ile Ala Pro Tyr Ser Tyr Ala Gly
    290                 295

<210> 95

<211> 253

<212> PRT

<213> Homo sapiens

<220>

<221> MISC_FEATURE

<222> (76)..(76)

<223> x

<220>

<221> MISC_FEATURE

<222> (114)..(114)

<223> x

<300>

<308> hsugp/013561-2-0

<309> 2003-02-16

<400> 95

Cys Val Val Asp Asn Pro Tyr Tyr Pro Pro Phe Glu Trp Ser Leu Lys
1               5                   10                  15

Pro Val Arg Pro Gln Pro Glu Arg Pro Glu Pro Ser Glu Phe Arg Glu
            20                  25                  30

Arg Gly Lys Arg Lys Glu Pro Pro Ile Arg Gln Pro Ala Ser Pro Arg
        35                  40                  45

Gly Val Ala Gly Leu Ile Asn Ala Asp Gly His Asp Arg Phe Pro Asp
    50                  55                  60

Trp Lys Pro Ala Ser Glu Arg Asn Ala Ile Asn Xaa Glu Leu Ala His
65                  70                  75                  80

Ser Leu Gly Thr Pro Gly Phe Thr Leu Tyr Ala Ser Gly Ser Tyr Val
                85                  90                  95

Val Trp Asn Cys Glu Arg Ile Thr Ile Ser Thr Gly Asn Ser Tyr Asp
            100                 105                 110

Met Xaa Tyr Glu Phe Asn Thr Thr His Tyr Arg Glu Phe Gly Pro Arg
        115                 120                 125

Asp Gln Glu Phe Gly Thr Arg Gly Thr Asn Met Asp Asn Arg Leu Pro
    130                 135                 140

Leu Glu Asn Gly Arg Phe Ile Ala Gly Thr Gly Cys Leu Val Arg Ala
145                 150                 155                 160

Val Glu Met Ala Ala Gln Arg Gln Ala Asp Ile Ile Gly Lys Pro Ser
                165                 170                 175

Arg Phe Ile Phe Asp Cys Val Ser Gln Glu Tyr Gly Ile Asn Pro Glu
            180                 185                 190

Arg Thr Val Met Val Gly Asp Arg Leu Asp Thr Asp Ile Leu Leu Gly
        195                 200                 205

Ala Thr Cys Gly Leu Lys Thr Ile Leu Thr Leu Thr Gly Val Ser Thr
    210                 215                 220

Leu Gly Asp Val Lys Asn Asn Gln Glu Ser Asp Cys Val Ser Lys Lys
225                 230                 235                 240

Lys Met Val Pro Asp Phe Tyr Val Asp Ser Ile Ala Asp
                245                 250

<210>  96

<211>  236

<212>  PRT

<213>  Homo sapiens

<300>

<308>  humangp/chr2-q9uqe6

<309>  2003-01-07

<400>  96

Met Ala Ala Glu Arg Gly Ala Gly Gln Gln Gln Ser Gln Glu Met Met
1               5                   10                  15

Glu Glu Glu His Glu Leu Pro Val Asp Met Glu Thr Ile Asn Leu Asp
            20                  25                  30

Arg Asp Ala Glu Asp Val Asp Leu Asn His Tyr Arg Ile Gly Lys Ile
        35                  40                  45

Glu Gly Phe Glu Val Leu Lys Lys Val Lys Thr Leu Cys Leu Arg Gln
    50                  55                  60

Asn Leu Ile Lys Cys Ile Glu Asn Leu Glu Glu Leu Gln Ser Leu Arg

65                  70                  75                  80

Glu Leu Asp Leu Tyr Asp Asn Gln Ile Lys Lys Ile Glu Asn Leu Glu
                85                  90                  95

Ala Leu Thr Glu Leu Glu Ile Leu Asp Ile Ser Phe Asn Leu Leu Arg
            100                 105                 110

Asn Ile Glu Gly Val Asp Lys Leu Thr Arg Leu Lys Lys Leu Phe Leu
        115                 120                 125

Val Asn Asn Lys Ile Ser Lys Ile Glu Asn Leu Ser Asn Leu His Gln
    130                 135                 140

Leu Gln Met Leu Glu Leu Gly Ser Asn Arg Ile Arg Ala Ile Glu Asn
145                 150                 155                 160

Ile Asp Thr Leu Thr Asn Leu Glu Ser Leu Phe Leu Gly Lys Asn Lys
                165                 170                 175

Ile Thr Lys Leu Gln Asn Leu Asp Ala Leu Thr Asn Leu Thr Val Leu
            180                 185                 190

Ser Met Gln Ser Asn Arg Leu Thr Lys Ile Glu Gly Leu Gln Asn Leu
        195                 200                 205

Val Asn Leu Arg Glu Leu Tyr Leu Ser His Asn Gly Ile Glu Val Ile
    210                 215                 220

Glu Gly Leu Glu Asn Asn Val Arg His Pro Leu Ser
225                 230                 235

<210>  97

<211>  718

<212>  PRT

<213>  Homo sapiens

<300>

<308>  humangp/chr7-q9unh0

<309>  2003-01-07

<400>  97

Met Glu Asp Leu Gly Glu Asn Thr Met Val Leu Ser Thr Leu Arg Ser
1               5                   10                  15

Leu Asn Asn Phe Ile Ser Gln Arg Val Glu Gly Gly Ser Gly Leu Asp
            20                  25                  30

Ile Ser Thr Ser Ala Pro Gly Ser Leu Gln Met Gln Tyr Gln Gln Ser
        35                  40                  45

Met Gln Leu Glu Glu Arg Ala Glu Gln Ile Arg Ser Lys Ser His Leu
    50                  55                  60

Ile Gln Val Glu Arg Glu Lys Met Gln Met Glu Leu Ser His Lys Arg
65                  70                  75                  80

Ala Arg Val Glu Leu Glu Arg Ala Ala Ser Thr Ser Ala Arg Asn Tyr
                85                  90                  95

Glu Arg Glu Val Asp Arg Asn Gln Glu Leu Leu Thr Arg Ile Arg Gln
            100                 105                 110

Leu Gln Glu Arg Glu Ala Gly Ala Glu Glu Lys Met Gln Glu Gln Leu
        115                 120                 125

Glu Arg Asn Arg Gln Cys Gln Gln Asn Leu Asp Ala Ala Ser Lys Arg
    130                 135                 140

Leu Arg Glu Lys Glu Asp Ser Leu Ala Gln Ala Gly Glu Thr Ile Asn
145                 150                 155                 160

Ala Leu Lys Gly Arg Ile Ser Glu Leu Gln Trp Ser Val Met Asp Gln
                165                 170                 175

Glu Met Arg Val Lys Arg Leu Glu Ser Glu Lys Gln Glu Leu Gln Glu
            180                 185                 190

Gln Leu Asp Leu Gln His Lys Lys Cys Gln Glu Ala Asn Gln Lys Ile
        195                 200                 205

Gln Glu Leu Gln Ala Ser Gln Glu Ala Arg Ala Asp His Glu Gln Gln
    210                 215                 220

Ile Lys Asp Leu Glu Gln Lys Leu Ser Leu Gln Glu Gln Asp Ala Ala
225                 230                 235                 240

Ile Val Lys Asn Met Lys Ser Glu Leu Val Arg Leu Pro Arg Leu Glu
                245                 250                 255

Arg Glu Leu Lys Gln Leu Arg Glu Glu Ser Ala His Leu Arg Glu Met
            260                 265                 270

Arg Glu Thr Asn Gly Leu Leu Gln Glu Glu Leu Glu Gly Leu Gln Arg
        275                 280                 285

Lys Leu Gly Arg Gln Glu Lys Met Gln Glu Thr Leu Val Gly Leu Glu
    290                 295                 300

Leu Glu Asn Glu Arg Leu Leu Ala Lys Leu Gln Ser Trp Glu Arg Leu
305                 310                 315                 320

Asp Gln Thr Met Gly Leu Ser Ile Arg Thr Pro Glu Asp Leu Ser Arg
                325                 330                 335

Phe Val Val Glu Leu Gln Gln Arg Glu Leu Ala Leu Lys Asp Lys Asn
            340                 345                 350

Ser Ala Val Thr Ser Ser Ala Arg Gly Leu Glu Lys Ala Arg Gln Gln
        355                 360                 365

Leu Gln Glu Glu Leu Arg Gln Val Ser Gly Gln Leu Leu Glu Glu Arg
    370                 375                 380

Lys Lys Arg Glu Thr His Glu Ala Leu Ala Arg Arg Leu Gln Lys Arg
385                 390                 395                 400

Val Leu Leu Leu Thr Lys Glu Arg Asp Gly Met Arg Ala Ile Leu Gly
                405                 410                 415

Ser Tyr Asp Ser Glu Leu Thr Pro Ala Glu Tyr Ser Pro Gln Leu Thr
            420                 425                 430

Arg Arg Met Arg Glu Ala Glu Asp Met Val Gln Lys Val His Ser His
        435                 440                 445

Ser Ala Glu Met Glu Ala Gln Leu Ser Gln Ala Leu Glu Glu Leu Gly
    450                 455                 460

Gly Gln Lys Gln Arg Ala Asp Met Leu Glu Met Glu Leu Lys Met Leu
465                 470                 475                 480

Lys Ser Gln Ser Ser Ser Ala Glu Gln Ser Phe Leu Phe Ser Arg Glu
                485                 490                 495

Glu Ala Asp Thr Leu Arg Leu Lys Val Glu Glu Leu Glu Gly Glu Arg
            500                 505                 510

Ser Arg Leu Glu Glu Glu Lys Arg Met Leu Glu Ala Gln Leu Glu Arg
        515                 520                 525

Arg Ala Leu Gln Gly Asp Tyr Asp Gln Ser Arg Thr Lys Val Leu His
    530                 535                 540

Met Ser Leu Asn Pro Thr Ser Val Ala Arg Gln Arg Leu Arg Glu Asp
545                 550                 555                 560

His Ser Gln Leu Gln Ala Glu Cys Glu Arg Leu Arg Gly Leu Leu Arg
                565                 570                 575

Ala Met Glu Arg Gly Gly Thr Val Pro Ala Asp Leu Glu Ala Ala Ala
            580                 585                 590

Ala Ser Leu Pro Ser Ser Lys Glu Val Ala Glu Leu Lys Lys Gln Val
        595                 600                 605

Glu Ser Ala Glu Leu Lys Asn Gln Arg Leu Lys Glu Val Phe Gln Thr
    610                 615                 620

Lys Ile Gln Glu Phe Arg Lys Ala Cys Tyr Thr Leu Thr Gly Tyr Gln
625                 630                 635                 640

Ile Asp Ile Thr Thr Glu Asn Gln Tyr Arg Leu Thr Ser Leu Tyr Ala
                645                 650                 655

Glu His Pro Gly Asp Cys Leu Ile Phe Lys Ala Thr Ser Pro Ser Gly
            660                 665                 670

Ser Lys Met Gln Leu Leu Glu Thr Glu Phe Ser His Thr Val Gly Glu
        675                 680                 685

Leu Ile Glu Val His Leu Arg Arg Gln Asp Ser Ile Pro Ala Phe Leu
    690                 695                 700

Ser Ser Leu Thr Leu Glu Leu Phe Ser Arg Gln Thr Val Ala
705                 710                 715

<210>  98

<211>  182

<212>  PRT

<213>  Homo sapiens

<300>

<308>  sw_hum/pebb_human

<309>  1997-11-01

<400>  98

Met Pro Arg Val Val Pro Asp Gln Arg Ser Lys Phe Glu Asn Glu Glu
1               5                   10                  15

Phe Phe Arg Lys Leu Ser Arg Glu Cys Glu Ile Lys Tyr Thr Gly Phe
            20                  25                  30

Arg Asp Arg Pro His Glu Glu Arg Gln Ala Arg Phe Gln Asn Ala Cys
        35                  40                  45

Arg Asp Gly Arg Ser Glu Ile Ala Phe Val Ala Thr Gly Thr Asn Leu
    50                  55                  60

Ser Leu Gln Phe Phe Pro Ala Ser Trp Gln Gly Glu Gln Arg Gln Thr
65                  70                  75                  80

Pro Ser Arg Glu Tyr Val Asp Leu Glu Arg Glu Ala Gly Lys Val Tyr
                85                  90                  95

Leu Lys Ala Pro Met Ile Leu Asn Gly Val Cys Val Ile Trp Lys Gly
            100                 105                 110

Trp Ile Asp Leu Gln Arg Leu Asp Gly Met Gly Cys Leu Glu Phe Asp
        115                 120                 125

Glu Glu Arg Ala Gln Gln Glu Asp Ala Leu Ala Gln Gln Ala Phe Glu
    130                 135                 140

Glu Ala Arg Arg Arg Thr Arg Glu Phe Glu Asp Arg Asp Arg Ser His
145                 150                 155                 160

Arg Glu Glu Met Glu Val Arg Val Ser Gln Leu Leu Ala Val Thr Gly
                165                 170                 175

Lys Lys Thr Thr Arg Pro

            180

<210> 99

<211> 737

<212> PRT

<213> Homo sapiens

<220>

<221> MISC_FEATURE

<222> (98)..(98)

<223> x

<220>

<221> MISC_FEATURE

<222> (242)..(242)

<223> x

<220>

<221> MISC_FEATURE

<222> (259)..(259)

<223> x

<220>

<221> MISC_FEATURE

<222> (262)..(262)

<223> x

<220>

<221> MISC_FEATURE

<222> (263)..(263)

<223> x

<220>

<221> MISC_FEATURE

<222> (264)..(264)

<223> x

<220>

<221> MISC_FEATURE

<222>  (269)..(269)
<223>  x

<220>
<221>  MISC_FEATURE
<222>  (282)..(282)
<223>  x

<220>
<221>  MISC_FEATURE
<222>  (283)..(283)
<223>  x

<220>
<221>  MISC_FEATURE
<222>  (284)..(284)
<223>  x

<220>
<221>  MISC_FEATURE
<222>  (337)..(337)
<223>  x

<220>
<221>  MISC_FEATURE
<222>  (368)..(368)
<223>  x

<220>
<221>  MISC_FEATURE
<222>  (418)..(418)
<223>  x

<220>
<221>  MISC_FEATURE
<222>  (419)..(419)
<223>  x

<220>
<221> MISC_FEATURE
<222> (425)..(425)
<223> x

<220>
<221> MISC_FEATURE
<222> (428)..(428)
<223> x

<220>
<221> MISC_FEATURE
<222> (429)..(429)
<223> x

<220>
<221> MISC_FEATURE
<222> (430)..(430)
<223> x

<220>
<221> MISC_FEATURE
<222> (432)..(432)
<223> x

<220>
<221> MISC_FEATURE
<222> (439)..(439)
<223> x

<220>
<221> MISC_FEATURE
<222> (443)..(443)
<223> x

<220>
<221> MISC_FEATURE
<222> (499)..(499)
<223> x

<220>

<221>  MISC_FEATURE

<222>  (500)..(500)

<223>  x


<220>

<221>  MISC_FEATURE

<222>  (502)..(502)

<223>  x


<220>

<221>  MISC_FEATURE

<222>  (506)..(506)

<223>  x


<220>

<221>  MISC_FEATURE

<222>  (507)..(507)

<223>  x


<220>

<221>  MISC_FEATURE

<222>  (511)..(511)

<223>  x


<220>

<221>  MISC_FEATURE

<222>  (513)..(513)

<223>  x


<300>

<308>  hsugp/075309-23-0

<309>  2003-02-16


<400>  99

Leu Val Gln Leu Glu Val Leu Met Val Thr Pro His Arg Thr Ser Cys
1               5                   10                  15

Leu Leu Glu Ile Ile Tyr Pro Arg Val Pro Phe Pro Phe Leu Gly Gln
            20                  25                  30

Lys Pro Leu Arg Ala Met Tyr Pro Asn Lys Pro Leu Asn Ala Phe Val
        35                  40                  45

Lys Ile Ser Leu Asp Ile Glu Phe Pro Ser Glu Glu Met Glu Lys Val
    50                  55                  60

Leu Gly Val Pro Ser Arg Leu Pro Leu Pro Asp Arg Pro Ile Lys Ser
65                  70                  75                  80

Leu Leu Arg Gly Arg Val Cys Cys Leu Leu Phe Leu Glu Ile His Asp
                85                  90                  95

Arg Xaa Lys His Leu Glu Asp Gly Val Gln Asp Gln Leu Ala Glu Gly
            100                 105                 110

Ala Gly Ser Phe Phe Pro Ser Gly Leu Val Ala Asp Leu Leu Asn Leu
        115                 120                 125

Pro Leu Ala Gly Ser Lys Tyr Leu Ser Pro His Ser Phe Phe Ile Met
    130                 135                 140

Ser Ser Thr Phe Leu Ala Arg Ser Ala Gly Pro Lys Ala Pro Asp Leu
145                 150                 155                 160

Ala Gly Leu Ser His Val Pro Leu Val Leu Leu Gly Gln Val Ala Arg
                165                 170                 175

Pro Leu Leu Glu Leu Leu Thr Gly Gly His Leu Ala Phe Ile Asp Val
            180                 185                 190

Leu Gly Gln Ala Ser Gly Asp Gln Pro Arg Glu Asp Ser Ser Lys Gly
        195                 200                 205

Val Glu Pro Thr Phe Val Gly Gly Trp His His Phe Gly Asn Ile His
    210                 215                 220

His Lys Arg Ala Phe Gly Val Thr Ala Phe Asn Ala His Gly Ser Leu
225                 230                 235                 240

Val Xaa Ser Gly Gly Pro Phe Asp Thr Gly Ala Arg Ser Arg Tyr Phe
                245                 250                 255

Trp Ala Xaa Glu Thr Xaa Xaa Xaa Glu Arg Arg Cys Xaa Arg Asp Ser
            260                 265                 270

Leu Gln Val Arg Leu Arg Ile Arg Gly Xaa Xaa Xaa Glu Asp Val Leu
        275                 280                 285

Leu Trp Val Leu Arg Val Pro Gly Ala Ala Gly Ala Ala Gly Arg Ala
    290                 295                 300

Ala Tyr Asn Leu Ser Arg Lys Leu Ser Arg Ala Gly Met Pro Ser Phe
305                 310                 315                 320

Arg Pro Leu Arg Leu Arg Val Ser Ala Thr Thr Trp Leu Gly Arg Leu

                325                 330                 335

Xaa Val Val Glu Gly Val Ser Gly Gln Asp Leu Pro Val Val Lys His
            340                 345                 350

Thr Leu Gly Glu Arg Leu Ala Ala Arg Val Gly Pro Gln Val Ser Xaa
        355                 360                 365

Asp Pro Lys Gly Pro Leu Met Met Tyr Ile Ser Lys Met Val Pro Thr
    370                 375                 380

Ser Asp Lys Gly Arg Phe Tyr Ala Phe Glu Arg Val Phe Ser Gly Leu
385                 390                 395                 400

Val Ser Thr Gly Leu Lys Val Arg Ile Met Gly Pro Asn Tyr Thr Pro
                405                 410                 415

Gly Xaa Xaa Glu Asp Leu Tyr Leu Xaa Ala Asn Xaa Xaa Xaa Thr Xaa
            420                 425                 430

Leu Asp Asp Gly Pro Leu Xaa Gly Ala His Xaa Gly Cys Ala Leu Gly
        435                 440                 445

Ala Ile Thr Phe Val Ala Ser Val Ser Val Asp His Val Pro Gly Glu
    450                 455                 460

Asp Gly Ser Ile Thr Thr Phe Glu Lys Ala Arg Lys Met Arg Val Met
465                 470                 475                 480

Lys Phe Arg Val Asn Pro Val Val Arg Arg Gly Arg Glu Thr Ala Lys
                485                 490                 495

Asn Pro Xaa Xaa Thr Xaa Pro Lys Leu Xaa Xaa Gly Ala Glu Xaa Gly
            500                 505                 510

Xaa Ala Lys Ser Asp Pro Met Val Gln Cys Ile Ile Glu Glu Ser Gly
        515                 520                 525

Glu His Ile Ile Ala Gly Ala Gly Glu Leu His Leu Glu Ile Cys Leu
    530                 535                 540

Lys Asp Leu Glu Glu Asp His Ala Cys Ile Pro Ile Lys Lys Ser Asp
545                 550                 555                 560

Pro Val Val Ser Tyr Arg Glu Thr Val Ser Glu Glu Ser Asn Val Leu
                565                 570                 575

Cys Leu Ser Lys Ser Pro Asn Lys His Asn Arg Leu Tyr Met Lys Ala
            580                 585                 590

Arg Pro Phe Pro Asp Gly Leu Ala Glu Asp Ile Asp Lys Gly Glu Val
        595                 600                 605

Ser Ala Arg Gln Glu Leu Lys Gln Arg Ala Arg Tyr Leu Ala Glu Lys
    610                 615                 620

Tyr Glu Trp Asp Val Ala Glu Ala Arg Lys Ile Trp Cys Phe Gly Pro
625                 630                 635                 640

Asp Gly Thr Glu His Gln Thr Ile His Ser Pro Thr Tyr Thr Lys Gly
                645                 650                 655

Cys Ser Lys Tyr Leu Asn Glu Ile Lys Ala Gln Lys Trp Ile Gly Gln
            660                 665                 670

Ala Tyr Gln Trp Gly Asn His Arg Arg Ala His Cys Cys Glu Arg Glu
        675                 680                 685

His Cys Val Gly Val Arg Tyr Glu Tyr His Glu Ser Lys Pro Leu Gln
    690                 695                 700

Ala Glu Ala Ile His Thr Glu Gly Ala Lys Ile Ile Thr Arg Gly Gly
705                 710                 715                 720

Ser Cys Leu Tyr Ala Ser Val Leu Asp Ala Gln Pro Arg Leu Met Asp
                725                 730                 735

Pro

<210>  100

<211>  268

<212>  PRT

<213>  Homo sapiens

<220>

<221>  MISC_FEATURE

<222>  (209)..(209)

<223>  x

<220>

<221>  MISC_FEATURE

<222>  (221)..(221)

<223>  x

<220>

<221>  MISC_FEATURE

<222>  (233)..(233)

<223>  x

<220>

<221>  MISC_FEATURE

<222>  (240)..(240)

<223>  x

<220>

<221>  MISC_FEATURE

<222>  (243)..(243)

<223>  x


<220>

<221>  MISC_FEATURE

<222>  (244)..(244)

<223>  x


<220>

<221>  MISC_FEATURE

<222>  (245)..(245)

<223>  x


<220>

<221>  MISC_FEATURE

<222>  (246)..(246)

<223>  x


<220>

<221>  MISC_FEATURE

<222>  (247)..(247)

<223>  x


<220>

<221>  MISC_FEATURE

<222>  (268)..(268)

<223>  x


<300>

<308>  hsugp/075309-24-0

<309>  2003-02-16


<400>  100

Lys Lys Ser Ser Ala Val Val Ala Ala Ile Leu Gly Ala Thr Arg Phe
1               5                   10                  15

Phe Arg Phe Tyr Leu Gly Glu Ser Thr Ala Ile Arg His Leu Val Asn
        20                  25                  30

Phe Thr Val Asp Gln Ile Arg Ala Ile Met Asp Lys Lys Ala Asn Ile
        35                  40                  45

Arg Asn Met Ser Val Ile Ala His Val Asp His Gly Lys Ser Thr Leu
    50                  55                  60

Thr Asp Ser Leu Val Cys Lys Ala Gly Ile Ile Ala Ser Ala Arg Ala
65                  70                  75                  80

Gly Glu Thr Arg Phe Thr Asp Thr Arg Lys Asp Glu Gln Glu Arg Cys
                85                  90                  95

Ile Thr Ile Lys Ser Thr Ala Ile Ser Leu Phe Tyr Glu Leu Ser Glu
            100                 105                 110

Asn Asp Leu Asn Phe Ile Lys Gln Ser Lys Asp Gly Ala Gly Phe Leu
        115                 120                 125

Ile Asn Leu Ile Asp Ser Pro Gly His Val Asp Phe Ser Ser Glu Val
    130                 135                 140

Thr Ala Ala Leu Arg Val Thr Asp Gly Ala Leu Val Val Val Asp Cys
145                 150                 155                 160

Val Ser Gly Val Cys Val Gln Thr Glu Thr Val Leu Arg Gln Ala Ile
                165                 170                 175

Ala Glu Arg Ile Lys Pro Val Leu Met Met Asn Lys Met Asp Arg Ala
            180                 185                 190

Leu Leu Glu Leu Gln Leu Glu Pro Glu Glu Leu Asn Arg Leu Ser Ser
        195                 200                 205

Xaa Ile Val Glu Asn Val Asn Val Ile Ile Ser Thr Xaa Arg Arg Gly
    210                 215                 220

Ala Lys Ser Arg Pro Pro Trp Gly Xaa His His Glu Ser Ile Leu Xaa
225                 230                 235                 240

Leu Arg Xaa Xaa Xaa Xaa Xaa Trp Asp Phe Gly Pro Pro Thr Gly Trp
                245                 250                 255

Gly Pro Cys His Pro Arg Lys Ala Val Phe Pro Xaa
            260                 265

<210>  101

<211>  470

<212>  PRT

<213>  Homo sapiens

<300>

<308>  sw_hum/nr54_human

<309>  1993-07-01

<400>  101

Gln Ser Asn Lys Thr Phe Asn Leu Glu Lys Gln Asn His Thr Pro Arg
1               5                   10                  15

Lys His His Gln His His His Gln Gln Gln His His Gln Gln Gln Gln
            20                  25                  30

Gln Gln Pro Pro Pro Pro Pro Ile Pro Ala Asn Gly Gln Gln Ala Ser
        35                  40                  45

Ser Gln Asn Glu Gly Leu Thr Ile Asp Leu Lys Asn Phe Arg Lys Pro
    50                  55                  60

Gly Glu Lys Thr Phe Thr Gln Arg Ser Arg Leu Phe Val Gly Asn Leu
65                  70                  75                  80

Pro Pro Asp Ile Thr Glu Glu Glu Met Arg Lys Leu Phe Glu Lys Tyr
                85                  90                  95

Gly Lys Ala Gly Glu Val Phe Ile His Lys Asp Lys Gly Phe Gly Phe
            100                 105                 110

Ile Arg Leu Glu Thr Arg Thr Leu Ala Glu Ile Ala Lys Val Glu Leu
        115                 120                 125

Asp Asn Met Pro Leu Arg Gly Lys Gln Leu Arg Val Arg Phe Ala Cys
    130                 135                 140

His Ser Ala Ser Leu Thr Val Arg Asn Leu Pro Gln Tyr Val Ser Asn
145                 150                 155                 160

Glu Leu Leu Glu Glu Ala Phe Ser Val Phe Gly Gln Val Glu Arg Ala
                165                 170                 175

Val Val Ile Val Asp Asp Arg Gly Arg Pro Ser Gly Lys Gly Ile Val
            180                 185                 190

Glu Phe Ser Gly Lys Pro Ala Ala Arg Lys Ala Leu Asp Arg Cys Ser
        195                 200                 205

Glu Gly Ser Phe Leu Leu Thr Thr Phe Pro Arg Pro Val Thr Val Glu
    210                 215                 220

Pro Met Asp Gln Leu Asp Asp Glu Glu Gly Leu Pro Glu Lys Leu Val
225                 230                 235                 240

Ile Lys Asn Gln Gln Phe His Lys Glu Arg Glu Gln Pro Pro Arg Phe
                245                 250                 255

Ala Gln Pro Gly Ser Phe Glu Tyr Glu Tyr Ala Met Arg Trp Lys Ala
            260                 265                 270

Leu Ile Glu Met Glu Lys Gln Gln Gln Asp Gln Val Asp Arg Asn Ile

275                 280                 285

Lys Glu Ala Arg Glu Lys Leu Glu Met Glu Met Glu Ala Ala Arg His
    290                 295                 300

Glu His Gln Val Met Leu Met Arg Gln Asp Leu Met Arg Arg Gln Glu
305                 310                 315                 320

Glu Leu Arg Arg Met Glu Glu Leu His Asn Gln Glu Val Gln Lys Arg
                325                 330                 335

Lys Gln Leu Glu Leu Arg Gln Glu Glu Glu Arg Arg Arg Arg Glu Glu
            340                 345                 350

Glu Met Arg Arg Gln Gln Glu Glu Met Met Arg Arg Gln Gln Glu Gly
        355                 360                 365

Phe Lys Gly Thr Phe Pro Asp Ala Arg Glu Gln Glu Ile Arg Met Gly
    370                 375                 380

Gln Met Ala Met Gly Gly Ala Met Gly Ile Asn Asn Arg Gly Ala Met
385                 390                 395                 400

Pro Pro Ala Pro Val Pro Ala Gly Thr Pro Ala Pro Pro Gly Pro Ala
                405                 410                 415

Thr Met Met Pro Asp Gly Thr Leu Gly Leu Thr Pro Pro Thr Thr Glu
            420                 425                 430

Arg Phe Gly Gln Ala Ala Thr Met Glu Gly Ile Gly Ala Ile Gly Gly
        435                 440                 445

Thr Pro Pro Ala Phe Asn Arg Ala Ala Pro Gly Ala Glu Phe Ala Pro
    450                 455                 460

Asn Lys Arg Arg Arg Tyr
465                 470

<210>  102

<211>  660

<212>  PRT

<213>  Homo sapiens

<300>

<308>  sw_hum/ddxy_human

<309>  1999-07-15

<400>  102

<210> 103

<211> 339

<212> PRT

<213> Homo sapiens

<300>

<308> humangp/chr8-094905

<309> 2003-04-22

<400> 103

Met Ala Gln Leu Gly Ala Val Val Ala Val Ala Ser Ser Phe Phe Cys
1               5                   10                  15

Ala Ser Leu Phe Ser Ala Val His Lys Ile Glu Glu Gly His Ile Gly
            20                  25                  30

Val Tyr Tyr Arg Gly Gly Ala Leu Leu Thr Ser Thr Ser Gly Pro Gly
        35                  40                  45

Phe His Leu Met Leu Pro Phe Ile Thr Ser Tyr Lys Ser Val Gln Thr
    50                  55                  60

Thr Leu Gln Thr Asp Glu Val Lys Asn Val Pro Cys Gly Thr Ser Gly
65                  70                  75                  80

Gly Val Met Ile Tyr Phe Asp Arg Ile Glu Val Val Asn Phe Leu Val
                85                  90                  95

Pro Asn Ala Val Tyr Asp Ile Val Lys Asn Tyr Thr Ala Asp Tyr Asp
            100                 105                 110

Lys Ala Leu Ile Phe Asn Lys Ile His His Glu Leu Asn Gln Phe Cys
        115                 120                 125

Ser Val His Thr Leu Gln Glu Val Tyr Ile Glu Leu Phe Asp Gln Ile
    130                 135                 140

Asp Glu Asn Leu Lys Leu Ala Leu Gln Gln Asp Leu Thr Ser Met Ala
145                 150                 155                 160

Pro Gly Leu Val Ile Gln Ala Val Arg Val Thr Lys Pro Asn Ile Pro
                165                 170                 175

Glu Ala Ile Arg Arg Asn Tyr Glu Leu Met Glu Ser Glu Lys Thr Lys
            180                 185                 190

Leu Leu Ile Ala Ala Gln Lys Gln Lys Val Val Glu Lys Glu Ala Glu

195                    200                    205

Thr Glu Arg Lys Lys Ala Leu Ile Glu Ala Glu Lys Val Ala Gln Val
    210                    215                    220

Ala Glu Ile Thr Tyr Gly Gln Lys Val Met Glu Lys Glu Thr Glu Lys
225                    230                    235                    240

Lys Ile Ser Glu Ile Glu Asp Ala Ala Phe Leu Ala Arg Glu Lys Ala
                245                    250                    255

Lys Ala Asp Ala Glu Cys Tyr Thr Ala Met Lys Ile Ala Glu Ala Asn
            260                    265                    270

Lys Leu Lys Leu Thr Pro Glu Tyr Leu Gln Leu Met Lys Tyr Lys Ala
        275                    280                    285

Ile Ala Ser Asn Ser Lys Ile Tyr Phe Gly Lys Asp Ile Pro Asn Met
    290                    295                    300

Phe Met Asp Ser Ala Gly Ser Val Ser Lys Gln Phe Glu Gly Leu Ala
305                    310                    315                    320

Asp Lys Leu Ser Phe Gly Leu Glu Asp Glu Pro Leu Glu Thr Ala Thr
                325                    330                    335

Lys Glu Asn

<210>  104

<211>  511

<212>  PRT

<213>  Homo sapiens

<300>

<308>  sw_hum/copd_human

<309>  1996-02-01

<400>  104

Met Val Leu Leu Ala Ala Ala Val Cys Thr Lys Ala Gly Lys Ala Ile
1               5                   10                  15

Val Ser Arg Gln Phe Val Glu Met Thr Arg Thr Arg Ile Glu Gly Leu
            20                  25                  30

Leu Ala Ala Phe Pro Lys Leu Met Asn Thr Gly Lys Gln His Thr Phe
        35                  40                  45

Val Glu Thr Glu Ser Val Arg Tyr Val Tyr Gln Pro Met Glu Lys Leu
    50                  55                  60

Tyr Met Val Leu Ile Thr Thr Lys Asn Ser Asn Ile Leu Glu Asp Leu
65                  70                  75                  80

Glu Thr Leu Arg Leu Phe Ser Arg Val Ile Pro Glu Tyr Cys Arg Ala
                85                  90                  95

Leu Glu Glu Asn Glu Ile Ser Glu His Cys Phe Asp Leu Ile Phe Ala
            100                 105                 110

Phe Asp Glu Ile Val Ala Leu Gly Tyr Arg Glu Asn Val Asn Leu Ala
        115                 120                 125

Gln Ile Arg Thr Phe Thr Glu Met Asp Ser His Glu Glu Lys Val Phe
    130                 135                 140

Arg Ala Val Arg Glu Thr Gln Glu Arg Glu Ala Lys Ala Glu Met Arg
145                 150                 155                 160

Arg Lys Ala Lys Glu Leu Gln Gln Ala Arg Arg Asp Ala Glu Arg Gln
                165                 170                 175

Gly Lys Lys Ala Pro Gly Phe Gly Gly Phe Gly Ser Ser Ala Val Ser
            180                 185                 190

Gly Gly Ser Thr Ala Ala Met Ile Thr Glu Thr Ile Ile Glu Thr Asp
        195                 200                 205

Lys Pro Lys Val Ala Pro Ala Pro Ala Arg Pro Ser Gly Pro Ser Lys
    210                 215                 220

Ala Leu Lys Leu Gly Ala Lys Gly Lys Glu Val Asp Asn Phe Val Asp
225                 230                 235                 240

Lys Leu Lys Ser Glu Gly Glu Thr Ile Met Ser Ser Ser Met Gly Lys
                245                 250                 255

Arg Thr Ser Glu Ala Thr Lys Met His Ala Pro Pro Ile Asn Met Glu
            260                 265                 270

Ser Val His Met Lys Ile Glu Glu Lys Ile Thr Leu Thr Cys Gly Arg
        275                 280                 285

Asp Gly Gly Leu Gln Asn Met Glu Leu His Gly Met Ile Met Leu Arg
    290                 295                 300

Ile Ser Asp Asp Lys Tyr Gly Arg Ile Arg Leu His Val Glu Asn Glu
305                 310                 315                 320

Asp Lys Lys Gly Val Gln Leu Gln Thr His Pro Asn Val Asp Lys Lys
                325                 330                 335

Leu Phe Thr Ala Glu Ser Leu Ile Gly Leu Lys Asn Pro Glu Lys Ser
            340                 345                 350

Phe Pro Val Asn Ser Asp Val Gly Val Leu Lys Trp Arg Leu Gln Thr
        355                 360                 365

Thr Glu Glu Ser Phe Ile Pro Leu Thr Ile Asn Cys Trp Pro Ser Glu
    370                 375                 380

Ser Gly Asn Gly Cys Asp Val Asn Ile Glu Tyr Glu Leu Gln Glu Asp
385                 390                 395                 400

Asn Leu Glu Leu Asn Asp Val Val Ile Thr Ile Pro Leu Pro Ser Gly
                405                 410                 415

Val Gly Ala Pro Val Ile Gly Glu Ile Asp Gly Glu Tyr Arg His Asp
            420                 425                 430

Ser Arg Arg Asn Thr Leu Glu Trp Cys Leu Pro Val Ile Asp Ala Lys
        435                 440                 445

Asn Lys Ser Gly Ser Leu Glu Phe Ser Ile Ala Gly Gln Pro Asn Asp
    450                 455                 460

Phe Phe Pro Val Gln Val Ser Phe Val Ser Lys Lys Asn Tyr Cys Asn
465                 470                 475                 480

Ile Gln Val Thr Lys Val Thr Gln Val Asp Gly Asn Ser Pro Val Arg
                485                 490                 495

Phe Ser Thr Glu Thr Thr Phe Leu Val Asp Lys Tyr Glu Ile Leu
            500                 505                 510

<210>  105

<211>  277

<212>  PRT

<213>  Homo sapiens

<300>

<308>  sw_jum/capb_human

<309>  1996-02-01

<400>  105

Met Ser Asp Gln Gln Leu Asp Cys Ala Leu Asp Leu Met Arg Arg Leu
1               5                   10                  15

Pro Pro Gln Gln Ile Glu Lys Asn Leu Ser Asp Leu Ile Asp Leu Val
            20                  25                  30

Pro Ser Leu Cys Glu Asp Leu Leu Ser Ser Val Asp Gln Pro Leu Lys
        35                  40                  45

Ile Ala Arg Asp Lys Val Val Gly Lys Asp Tyr Leu Leu Cys Asp Tyr
    50                  55                  60

Asn Arg Asp Gly Asp Ser Tyr Arg Ser Pro Trp Ser Asn Lys Tyr Asp
65                  70                  75                  80

Pro Pro Leu Glu Asp Gly Ala Met Pro Ser Ala Arg Leu Arg Lys Leu
                85                  90                  95

Glu Val Glu Ala Asn Asn Ala Phe Asp Gln Tyr Arg Asp Leu Tyr Phe
            100                 105                 110

Glu Gly Gly Val Ser Ser Val Tyr Leu Trp Asp Leu Asp His Gly Phe
        115                 120                 125

Ala Gly Val Ile Leu Ile Lys Lys Ala Gly Asp Gly Ser Lys Lys Ile
    130                 135                 140

Lys Gly Cys Trp Asp Ser Ile His Val Val Glu Val Gln Glu Lys Ser
145                 150                 155                 160

Ser Gly Arg Thr Ala His Tyr Lys Leu Thr Ser Thr Val Met Leu Trp
                165                 170                 175

Leu Gln Thr Asn Lys Ser Gly Ser Gly Thr Met Asn Leu Gly Gly Ser
            180                 185                 190

Leu Thr Arg Gln Met Glu Lys Asp Glu Thr Val Ser Asp Cys Ser Pro
        195                 200                 205

His Ile Ala Asn Ile Gly Arg Leu Val Glu Asp Met Glu Asn Lys Ile
    210                 215                 220

Arg Ser Thr Leu Asn Glu Ile Tyr Phe Gly Lys Thr Lys Asp Ile Val
225                 230                 235                 240

Asn Gly Leu Arg Ser Ile Asp Ala Ile Pro Asp Asn Gln Lys Phe Lys
                245                 250                 255

Gln Leu Gln Arg Glu Leu Ser Gln Val Leu Thr Gln Arg Gln Ile Tyr
            260                 265                 270

Ile Gln Pro Asp Asn
        275

<210>  106

<211>  226

<212>  PRT

<213>  Homo sapiens


<300>

<308>  sw_hum/vate_human

<309>  1994-06-01


<400>  106

<210>  107

<211>  1101

<212>  PRT

<213>  Homo sapiens

<300>

<308>  sw_hum/acly_human

<309>  1996-10-01

<400>  107

<210>  108

<211>  197

<212>  PRT

<213>  Homo sapiens



<220>

<221>  MISC_FEATURE

<222>  (21)..(21)

<223>  x



<220>

<221>  MISC_FEATURE

<222>  (75)..(75)

<223>  x



<220>

<221>  MISC_FEATURE

<222>  (91)..(91)

<223>  x

<220>

<221>  MISC_FEATURE

<222>  (104)..(104)

<223>  x

<220>

<221>  MISC_FEATURE

<222>  (106)..(106)

<223>  x

<220>

<221>  MISC_FEATURE

<222>  (117)..(117)

<223>  x

<220>

<221>  MISC_FEATURE

<222>  (133)..(133)

<223>  x

<220>

<221>  MISC_FEATURE

<222>  (152)..(152)

<223>  x

<300>

<308>  hsugp/008768-9-0

<309>  2002-12-05

<400>  108

Val Ser Gly Ile Leu Thr Ile Val Leu Pro Val Lys Thr Asp Ser Ile
1               5                   10                  15

Asp Ile Lys Leu Xaa Leu Tyr Asp Asp Ser Lys Glu Ser Phe Lys Ser
            20                  25                  30

Gln Glu Asn Val Lys Gly Gly Leu Asn Leu Pro Leu Val Ser Ala Ala
        35                  40                  45

Ala Thr Gly Val Ser Gln Glu Val Thr Asn Ser Arg Ala Glu Ser Asp
    50                  55                  60

Pro Leu Ala His Asp Ile Lys Thr Leu Phe Xaa Lys Lys Pro Phe Met
65                  70                  75                  80

Asn Gly Phe Arg Phe Gly Leu Arg Val Ala Xaa Gly Val Asn Trp Ile
                85                  90                  95

Gly Ile Trp Ile His Phe Thr Xaa Lys Xaa Phe Ile Ile Leu Asn Phe
            100                 105                 110

Glu Glu Thr Ser Xaa Pro Leu Ala Ala Leu Pro Asn Val Ser Phe Trp
        115                 120                 125

Glu Arg Glu Phe Xaa Val Thr Gly Leu Arg Ser Leu Thr Thr Gln Asn
    130                 135                 140

Arg Lys Ile Ile Leu Thr Ile Xaa Asp Phe Arg Met Leu Ser Ile Lys
145                 150                 155                 160

Ser His Glu Asn Cys Leu Gln Arg Val Leu Lys Arg His Gln Pro Val
                165                 170                 175

Phe Phe Glu Thr Pro Ser Val Gly Val Glu Asp Ala Thr Gly Asp Asn
            180                 185                 190

Arg Phe Leu Gln Ala
        195

<210>  109

<211>  56

<212>  PRT

<213>  Homo sapiens


<300>

<308>  hsugp/075546-6-0

<309>  2003-02-16


<400>  109

Leu Leu Glu Ala Ala Glu Asn Glu Tyr Gln Thr Ala Ile Ser Glu Asn
1               5                   10                  15

Tyr Gln Thr Met Ser Asp Thr Thr Phe Lys Ala Leu Arg Arg Gln Leu
            20                  25                  30

Pro Val Thr Arg Thr Lys Ile Asp Trp Asn Lys Ile Leu Ser Tyr Lys
        35                  40                  45

Ile Gly Lys Glu Met Gln Asn Ala
    50                  55

<210>  110

<211>  650

<212>  PRT

<213>  Homo sapiens

<300>

<308>  sw_hum/dd17_human

<309>  1998-07-15

<400>  110

Met Arg Gly Gly Gly Phe Gly Asp Arg Asp Arg Asp Arg Asp Arg Gly
1               5                   10                  15

Gly Phe Gly Ala Arg Gly Gly Gly Gly Leu Pro Pro Lys Lys Phe Gly
            20                  25                  30

Asn Pro Gly Glu Arg Leu Arg Lys Lys Lys Trp Asp Leu Ser Glu Leu
        35                  40                  45

Pro Lys Phe Glu Lys Asn Phe Tyr Val Glu His Pro Glu Val Ala Arg
    50                  55                  60

Leu Thr Pro Tyr Glu Val Asp Glu Leu Arg Arg Lys Lys Glu Ile Thr
65                  70                  75                  80

Val Arg Gly Gly Asp Val Cys Pro Lys Pro Val Phe Ala Phe His His
                85                  90                  95

Ala Asn Phe Pro Gln Tyr Val Met Asp Val Leu Met Asp Gln His Phe
            100                 105                 110

Thr Glu Pro Thr Pro Ile Gln Cys Gln Gly Phe Pro Leu Ala Leu Ser
        115                 120                 125

Gly Arg Asp Met Val Gly Ile Ala Gln Thr Gly Ser Gly Lys Thr Leu
    130                 135                 140

Ala Tyr Leu Leu Pro Ala Ile Val His Ile Asn His Gln Pro Tyr Leu
145                 150                 155                 160

Glu Arg Gly Asp Gly Pro Ile Cys Leu Val Leu Ala Pro Thr Arg Glu
                165                 170                 175

Leu Ala Gln Gln Val Gln Gln Val Ala Asp Asp Tyr Gly Lys Cys Ser
            180                 185                 190

Arg Leu Lys Ser Thr Cys Ile Tyr Gly Gly Ala Pro Lys Gly Pro Gln
        195                 200                 205

Ile Arg Asp Leu Glu Arg Gly Val Glu Ile Cys Ile Ala Thr Pro Gly
    210                 215                 220

Arg Leu Ile Asp Phe Leu Glu Ser Gly Lys Thr Asn Leu Arg Arg Cys

225                 230                 235                 240

Thr Tyr Leu Val Leu Asp Glu Ala Asp Arg Met Leu Asp Met Gly Phe
                245                 250                 255

Glu Pro Gln Ile Arg Lys Ile Val Asp Gln Ile Arg Pro Asp Arg Gln
            260                 265                 270

Thr Leu Met Trp Ser Ala Thr Trp Pro Lys Glu Val Arg Gln Leu Ala
        275                 280                 285

Glu Asp Phe Leu Arg Asp Tyr Thr Gln Ile Asn Val Gly Asn Leu Glu
    290                 295                 300

Leu Ser Ala Asn His Asn Ile Leu Gln Ile Val Asp Val Cys Met Glu
305                 310                 315                 320

Ser Glu Lys Asp His Lys Leu Ile Gln Leu Met Glu Glu Ile Met Ala
                325                 330                 335

Glu Lys Glu Asn Lys Thr Ile Ile Phe Val Glu Thr Lys Arg Arg Cys
            340                 345                 350

Asp Asp Leu Thr Arg Arg Met Arg Arg Asp Gly Trp Pro Ala Met Cys
        355                 360                 365

Ile His Gly Asp Lys Ser Gln Pro Glu Arg Asp Trp Val Leu Asn Glu
    370                 375                 380

Phe Arg Ser Gly Lys Ala Pro Ile Leu Ile Ala Thr Asp Val Ala Ser
385                 390                 395                 400

Arg Gly Leu Asp Val Glu Asp Val Lys Phe Val Ile Asn Tyr Asp Tyr
                405                 410                 415

Pro Asn Ser Ser Glu Asp Tyr Val His Arg Ile Gly Arg Thr Ala Arg
            420                 425                 430

Ser Thr Asn Lys Gly Thr Ala Tyr Thr Phe Phe Thr Pro Gly Asn Leu
        435                 440                 445

Lys Gln Ala Arg Glu Leu Ile Lys Val Leu Glu Glu Ala Asn Gln Ala
    450                 455                 460

Ile Asn Pro Lys Leu Met Gln Leu Val Asp His Arg Gly Gly Gly Gly
465                 470                 475                 480

Gly Gly Gly Gly Arg Ser Arg Tyr Arg Thr Thr Ser Ser Ala Asn Asn
                485                 490                 495

Pro Asn Leu Met Tyr Gln Asp Glu Cys Asp Arg Arg Leu Arg Gly Val
            500                 505                 510

Lys Asp Gly Gly Arg Arg Asp Ser Ala Ser Tyr Arg Asp Arg Ser Glu
        515                 520                 525

Thr Asp Arg Ala Gly Tyr Ala Asn Gly Ser Gly Tyr Gly Ser Pro Asn
    530                 535                 540

Ser Ala Phe Gly Ala Gln Ala Gly Gln Tyr Thr Tyr Gly Gln Gly Thr
545                 550                 555                 560

Tyr Gly Ala Ala Ala Tyr Gly Thr Ser Ser Tyr Thr Ala Gln Glu Tyr
                565                 570                 575

Gly Ala Gly Thr Tyr Gly Ala Ser Ser Thr Thr Ser Thr Gly Arg Ser
            580                 585                 590

Ser Gln Ser Ser Ser Gln Gln Phe Ser Gly Ile Gly Arg Ser Gly Gln
        595                 600                 605

Gln Pro Gln Pro Leu Met Ser Gln Gln Phe Ala Gln Pro Pro Gly Ala
    610                 615                 620

Thr Asn Met Ile Gly Tyr Met Gly Gln Thr Ala Tyr Gln Tyr Pro Pro
625                 630                 635                 640

Pro Pro Pro Pro Pro Pro Pro Ser Arg Lys
                645                 650

<210>  111

<211>  348

<212>  PRT

<213>  Homo sapiens

<300>

<308>  sw_hum/capg_human

<309>  1995-02-01

<400>  111

Met Tyr Thr Ala Ile Pro Gln Ser Gly Ser Pro Phe Pro Gly Ser Val
1               5                   10                  15

Gln Asp Pro Gly Leu His Val Trp Arg Val Glu Lys Leu Lys Pro Val
            20                  25                  30

Pro Val Ala Gln Glu Asn Gln Gly Val Phe Phe Ser Gly Asp Ser Tyr
        35                  40                  45

Leu Val Leu His Asn Gly Pro Glu Glu Val Ser His Leu His Leu Trp
    50                  55                  60

Ile Gly Gln Gln Ser Ser Arg Asp Glu Gln Gly Ala Cys Ala Val Leu
65                  70                  75                  80

Ala Val His Leu Asn Thr Leu Leu Gly Glu Arg Pro Val Gln His Arg
                85                  90                  95

Glu Val Gln Gly Asn Glu Ser Asp Leu Phe Met Ser Tyr Phe Pro Arg
            100                 105                 110

Gly Leu Lys Tyr Gln Glu Gly Gly Val Glu Ser Ala Phe His Lys Thr
        115                 120                 125

Ser Thr Gly Ala Pro Ala Ala Ile Lys Lys Leu Tyr Gln Val Lys Gly
    130                 135                 140

Lys Lys Asn Ile Arg Ala Thr Glu Arg Ala Leu Asn Trp Asp Ser Phe
145                 150                 155                 160

Asn Thr Gly Asp Cys Phe Ile Leu Asp Leu Gly Gln Asn Ile Phe Ala
                165                 170                 175

Trp Cys Gly Gly Lys Ser Asn Ile Leu Glu Arg Asn Lys Ala Arg Asp
            180                 185                 190

Leu Ala Leu Ala Ile Arg Asp Ser Glu Arg Gln Gly Lys Ala Gln Val
        195                 200                 205

Glu Ile Val Thr Asp Gly Glu Glu Pro Ala Glu Met Ile Gln Val Leu
    210                 215                 220

Gly Pro Lys Pro Ala Leu Lys Glu Gly Asn Pro Glu Glu Asp Leu Thr
225                 230                 235                 240

Ala Asp Lys Ala Asn Ala Gln Ala Ala Ala Leu Tyr Lys Val Ser Asp
                245                 250                 255

Ala Thr Gly Gln Met Asn Leu Thr Lys Val Ala Asp Ser Ser Pro Phe
            260                 265                 270

Ala Leu Glu Leu Leu Ile Ser Asp Asp Cys Phe Val Leu Asp Asn Gly
        275                 280                 285

Leu Cys Gly Lys Ile Tyr Ile Trp Lys Gly Arg Lys Ala Asn Glu Lys
    290                 295                 300

Glu Arg Gln Ala Ala Leu Gln Val Ala Glu Gly Phe Ile Ser Arg Met
305                 310                 315                 320

Gln Tyr Ala Pro Asn Thr Gln Val Glu Ile Leu Pro Gln Gly Arg Glu
                325                 330                 335

Ser Pro Ile Phe Lys Gln Phe Phe Lys Asp Trp Lys
            340                 345

<210>  112

<211>  513

<212>  PRT

<213>  Homo sapiens


<300>

<308>  sw_hum/vab1_human

<309>  1990-04-01

<400> 112

Met Ala Met Glu Ile Asp Ser Arg Pro Gly Gly Leu Pro Gly Ser Ser
1               5                   10                  15

Cys Asn Leu Gly Ala Ala Arg Glu His Met Gln Ala Val Thr Arg Asn
            20                  25                  30

Tyr Ile Thr His Pro Arg Val Thr Tyr Arg Thr Val Cys Ser Val Asn
        35                  40                  45

Gly Pro Leu Val Val Leu Asp Arg Val Lys Phe Ala Gln Tyr Ala Glu
    50                  55                  60

Ile Val His Phe Thr Leu Pro Asp Gly Thr Gln Arg Ser Gly Gln Val
65                  70                  75                  80

Leu Glu Val Ala Gly Thr Lys Ala Ile Val Gln Val Phe Glu Gly Thr
                85                  90                  95

Ser Gly Ile Asp Ala Arg Lys Thr Thr Cys Glu Phe Thr Gly Asp Ile
            100                 105                 110

Leu Arg Thr Pro Val Ser Glu Asp Met Leu Gly Arg Val Phe Asn Gly
        115                 120                 125

Ser Gly Lys Pro Ile Asp Lys Gly Pro Val Val Met Ala Glu Asp Phe
    130                 135                 140

Leu Asp Ile Asn Gly Gln Pro Ile Asn Pro His Ser Arg Ile Tyr Pro
145                 150                 155                 160

Glu Glu Met Ile Gln Thr Gly Ile Ser Pro Ile Asp Val Met Asn Ser
                165                 170                 175

Ile Ala Arg Gly Gln Lys Ile Pro Ile Phe Ser Ala Ala Gly Leu Pro
            180                 185                 190

His Asn Glu Ile Ala Ala Gln Ile Cys Arg Gln Ala Gly Leu Val Lys
        195                 200                 205

Lys Ser Lys Ala Val Leu Asp Tyr His Asp Asp Asn Phe Ala Ile Val
    210                 215                 220

Phe Ala Ala Met Gly Val Asn Met Glu Thr Ala Arg Phe Phe Lys Ser
225                 230                 235                 240

Asp Phe Glu Gln Asn Gly Thr Met Gly Asn Val Cys Leu Phe Leu Asn
                245                 250                 255

Leu Ala Asn Asp Pro Thr Ile Glu Arg Ile Ile Thr Pro Arg Leu Ala
            260                 265                 270

Leu Thr Thr Ala Glu Phe Leu Ala Tyr Gln Cys Glu Lys His Val Leu
        275                 280                 285

Val Ile Leu Thr Asp Met Ser Ser Tyr Ala Glu Ala Leu Arg Glu Val

290                 295                 300

Ser Ala Ala Arg Glu Glu Val Pro Gly Arg Arg Gly Phe Pro Gly Tyr
305                 310                 315                 320

Met Tyr Thr Asp Leu Ala Thr Ile Tyr Glu Arg Ala Gly Arg Val Glu
                325                 330                 335

Gly Arg Gly Gly Ser Ile Thr Gln Ile Pro Ile Leu Thr Met Pro Asn
            340                 345                 350

Asp Asp Ile Thr His Pro Ile Pro Asp Leu Thr Gly Phe Ile Thr Glu
        355                 360                 365

Gly Gln Ile Tyr Val Asp Arg Gln Leu His Asn Arg Gln Ile Tyr Pro
    370                 375                 380

Pro Ile Asn Val Leu Pro Ser Leu Ser Arg Leu Met Lys Ser Ala Ile
385                 390                 395                 400

Gly Glu Gly Met Thr Arg Lys Asp His Gly Asp Val Ser Asn Gln Leu
                405                 410                 415

Tyr Ala Cys Tyr Ala Ile Gly Lys Asp Val Gln Ala Met Lys Ala Val
            420                 425                 430

Val Gly Glu Glu Ala Leu Thr Ser Glu Asp Leu Leu Tyr Leu Glu Phe
        435                 440                 445

Leu Gln Lys Phe Glu Lys Asn Phe Ile Asn Gln Gly Pro Tyr Glu Asn
    450                 455                 460

Arg Ser Met Phe Glu Ser Leu Asp Leu Ser Trp Lys Leu Leu Arg Ile
465                 470                 475                 480

Phe Pro Lys Glu Met Leu Lys Arg Ile Pro Gln Ala Val Ile Asp Glu
                485                 490                 495

Phe Tyr Ser Arg Glu Gly Arg Leu Gln Asp Leu Ala Pro Asp Thr Ala
            500                 505                 510

Leu

<210>  113

<211>  858

<212>  PRT

<213>  Homo sapiens

<300>

<308>  sw_hum/ubp5_human

<309>  1996-02-01

<400> 113

Met Ala Glu Leu Ser Glu Glu Ala Leu Leu Ser Val Leu Pro Thr Ile
1               5                   10                  15

Arg Val Pro Lys Ala Gly Asp Arg Val His Lys Asp Glu Cys Ala Phe
            20                  25                  30

Ser Phe Asp Thr Pro Glu Ser Glu Gly Gly Leu Tyr Ile Cys Met Asn
        35                  40                  45

Thr Phe Leu Gly Phe Gly Lys Gln Tyr Val Glu Arg His Phe Asn Lys
    50                  55                  60

Thr Gly Gln Arg Val Tyr Leu His Leu Arg Arg Thr Arg Arg Pro Lys
65                  70                  75                  80

Glu Glu Asp Pro Ala Thr Gly Thr Gly Asp Pro Pro Arg Lys Lys Pro
                85                  90                  95

Thr Arg Leu Ala Ile Gly Val Glu Gly Gly Phe Asp Leu Ser Glu Glu
            100                 105                 110

Lys Phe Glu Leu Asp Glu Asp Val Lys Ile Val Ile Leu Pro Asp Tyr
        115                 120                 125

Leu Glu Ile Ala Arg Asp Gly Leu Gly Gly Leu Pro Asp Ile Val Arg
    130                 135                 140

Asp Arg Val Thr Ser Ala Val Glu Ala Leu Leu Ser Ala Asp Ser Ala
145                 150                 155                 160

Ser Arg Lys Gln Glu Val Gln Ala Trp Asp Gly Glu Val Arg Gln Val
                165                 170                 175

Ser Lys His Ala Phe Ser Leu Lys Gln Leu Asp Asn Pro Ala Arg Ile
            180                 185                 190

Pro Pro Cys Gly Trp Lys Cys Ser Lys Cys Asp Met Arg Glu Asn Leu
        195                 200                 205

Trp Leu Asn Leu Thr Asp Gly Ser Ile Leu Cys Gly Arg Arg Tyr Phe
    210                 215                 220

Asp Gly Ser Gly Gly Asn Asn His Ala Val Glu His Tyr Arg Glu Thr
225                 230                 235                 240

Gly Tyr Pro Leu Ala Val Lys Leu Gly Thr Ile Thr Pro Asp Gly Ala
                245                 250                 255

Asp Val Tyr Ser Tyr Asp Glu Asp Asp Met Val Leu Asp Pro Ser Leu
            260                 265                 270

Ala Glu His Leu Ser His Phe Gly Ile Asp Met Leu Lys Met Gln Lys
        275                 280                 285

Thr Asp Lys Thr Met Thr Glu Leu Glu Ile Asp Met Asn Gln Arg Ile
    290                 295                 300

Pro Gly Glu Glu Glu Leu Pro Asp Ile Ala Pro Pro Leu Val Thr Pro
    610                 615                 620

Asp Glu Pro Lys Gly Ser Leu Gly Phe Tyr Gly Asn Glu Asp Glu Asp
625                 630                 635                 640

Ser Phe Cys Ser Pro His Phe Ser Ser Pro Thr Ser Pro Met Leu Asp
                645                 650                 655

Glu Ser Val Ile Ile Gln Leu Val Glu Met Gly Phe Pro Met Asp Ala
            660                 665                 670

Cys Arg Lys Ala Val Tyr Tyr Thr Gly Asn Ser Gly Ala Glu Ala Ala
        675                 680                 685


    690                 695                 700


705                 710                 715                 720


                725                 730                 735


            740                 745                 750


        755                 760                 765


    770                 775                 780


785                 790                 795                 800


                805                 810                 815


            820                 825                 830


        835                 840                 845


    850                 855

<210>  114

<211>  324

<212>  PRT

<213>  Homo sapiens


<300>

<308>  tr_hum/q9bvh9

<309>  2001-06-01

<400>  114

Met Glu Asp Ala Lys Val Tyr Val Ala Lys Val Asp Cys Thr Ala His
1               5                   10                  15

Ser Asp Val Cys Ser Ala Gln Gly Val Arg Gly Tyr Pro Thr Leu Lys
            20                  25                  30

Leu Phe Lys Pro Gly Gln Glu Ala Val Lys Tyr Gln Gly Pro Arg Asp
        35                  40                  45

Phe Gln Thr Leu Glu Asn Trp Met Leu Gln Thr Leu Asn Glu Glu Pro
    50                  55                  60

Val Thr Pro Glu Pro Glu Val Glu Pro Pro Ser Ala Pro Glu Leu Lys
65                  70                  75                  80

Gln Gly Leu Tyr Glu Leu Ser Ala Ser Asn Phe Glu Leu His Val Ala
                85                  90                  95

Gln Gly Asp His Phe Ile Lys Phe Phe Ala Pro Trp Cys Gly His Cys
            100                 105                 110

Lys Ala Leu Ala Pro Thr Trp Glu Gln Leu Ala Leu Gly Leu Glu His
        115                 120                 125

Ser Glu Thr Val Lys Ile Gly Lys Val Asp Cys Thr Gln His Tyr Glu
    130                 135                 140

Leu Cys Ser Gly Asn Gln Val Arg Gly Tyr Pro Thr Leu Leu Trp Phe
145                 150                 155                 160

Arg Asp Gly Lys Lys Val Asp Gln Tyr Lys Gly Lys Arg Asp Leu Glu
                165                 170                 175

Ser Leu Arg Glu Tyr Val Glu Ser Gln Leu Gln Arg Thr Glu Thr Gly
            180                 185                 190

Ala Thr Glu Thr Val Thr Pro Ser Glu Ala Pro Val Leu Ala Ala Glu
        195                 200                 205

Pro Glu Ala Asp Lys Gly Thr Val Leu Ala Leu Thr Glu Asn Asn Phe
    210                 215                 220

Asp Asp Thr Ile Ala Glu Gly Ile Thr Phe Ile Lys Phe Tyr Ala Pro
225                 230                 235                 240

Trp Cys Gly His Cys Lys Thr Leu Ala Pro Thr Trp Glu Glu Leu Ser
                245                 250                 255

Lys Lys Glu Phe Pro Gly Leu Ala Gly Val Lys Ile Ala Glu Val Asp
            260                 265                 270

Cys Thr Ala Glu Arg Asn Ile Cys Ser Lys Tyr Ser Val Arg Gly Tyr

275                    280                    285

Pro Thr Leu Leu Leu Phe Arg Gly Gly Lys Lys Val Ser Glu His Ser
    290                    295                    300

Gly Gly Arg Asp Leu Asp Ser Leu His Arg Phe Val Leu Ser Gln Ala
305                    310                    315                    320

Lys Asp Glu Leu

<210>  115

<211>  475

<212>  PRT

<213>  Homo sapiens

<300>

<308>  sw_hum/cgl1_human

<309>  2003-02-28

<400>  115

Met Ala Ala Leu Thr Thr Leu Phe Lys Tyr Ile Asp Glu Asn Gln Asp
1               5                   10                  15

Arg Tyr Ile Lys Lys Leu Ala Lys Trp Val Ala Ile Gln Ser Val Ser
            20                  25                  30

Ala Trp Pro Glu Lys Arg Gly Glu Ile Arg Arg Met Met Glu Val Ala
        35                  40                  45

Ala Ala Asp Val Lys Gln Leu Gly Gly Ser Val Glu Leu Val Asp Ile
    50                  55                  60

Gly Lys Gln Lys Leu Pro Asp Gly Ser Glu Ile Pro Leu Pro Pro Ile
65                  70                  75                  80

Leu Leu Gly Arg Leu Gly Ser Asp Pro Gln Lys Lys Thr Val Cys Ile
                85                  90                  95

Tyr Gly His Leu Asp Val Gln Pro Ala Ala Leu Glu Asp Gly Trp Asp
            100                 105                 110

Ser Glu Pro Phe Thr Leu Val Glu Arg Asp Gly Lys Leu Tyr Gly Arg
        115                 120                 125

Gly Ser Thr Asp Asp Lys Gly Pro Val Ala Gly Trp Ile Asn Ala Leu
    130                 135                 140

Glu Ala Tyr Gln Lys Thr Gly Gln Glu Ile Pro Val Asn Val Arg Phe
145                 150                 155                 160

Cys Leu Glu Gly Met Glu Glu Ser Gly Ser Glu Gly Leu Asp Glu Leu
                165                 170                 175

Ile Phe Ala Arg Lys Asp Thr Phe Phe Lys Asp Val Asp Tyr Val Cys
            180                 185                 190

Ile Ser Asp Asn Tyr Trp Leu Gly Lys Lys Lys Pro Cys Ile Thr Tyr
        195                 200                 205

Gly Leu Arg Gly Ile Cys Tyr Phe Phe Ile Glu Val Glu Cys Ser Asn
    210                 215                 220

Lys Asp Leu His Ser Gly Val Tyr Gly Gly Ser Val His Glu Ala Met
225                 230                 235                 240

Thr Asp Leu Ile Leu Leu Met Gly Ser Leu Val Asp Lys Arg Gly Asn
                245                 250                 255

Ile Leu Ile Pro Gly Ile Asn Glu Ala Val Ala Ala Val Thr Glu Glu
            260                 265                 270

Glu His Lys Leu Tyr Asp Asp Ile Asp Phe Asp Ile Glu Glu Phe Ala
        275                 280                 285

Lys Asp Val Gly Ala Gln Ile Leu Leu His Ser His Lys Lys Asp Ile
    290                 295                 300

Leu Met His Arg Trp Arg Tyr Pro Ser Leu Ser Leu His Gly Ile Glu
305                 310                 315                 320

Gly Ala Phe Ser Gly Ser Gly Ala Lys Thr Val Ile Pro Arg Lys Val
                325                 330                 335

Val Gly Lys Phe Ser Ile Arg Leu Val Pro Asn Met Thr Pro Glu Val
            340                 345                 350

Val Gly Glu Gln Val Thr Ser Tyr Leu Thr Lys Lys Phe Ala Glu Leu
        355                 360                 365

Arg Ser Pro Asn Glu Phe Lys Val Tyr Met Gly His Gly Gly Lys Pro
    370                 375                 380

Trp Val Ser Asp Phe Ser His Pro His Tyr Leu Ala Gly Arg Arg Ala
385                 390                 395                 400

Met Lys Thr Val Phe Gly Val Glu Pro Asp Leu Thr Arg Glu Gly Gly
                405                 410                 415

Ser Ile Pro Val Thr Leu Thr Phe Gln Glu Ala Thr Gly Lys Asn Val
            420                 425                 430

Met Leu Leu Pro Val Gly Ser Ala Asp Asp Gly Ala His Ser Gln Asn
        435                 440                 445

Glu Lys Leu Asn Arg Tyr Asn Tyr Ile Glu Gly Thr Lys Met Leu Ala
    450                 455                 460

Ala Tyr Leu Tyr Glu Val Ser Gln Leu Lys Asp
465                 470                 475

<210>  116

<211>  465

<212>  PRT

<213>  Homo sapiens

<300>

<308>  sw_hum/vime_human

<309>  1988-01-01

<400>  116

Ser Thr Arg Ser Val Ser Ser Ser Ser Tyr Arg Arg Met Phe Gly Gly
1               5                   10                  15

Pro Gly Thr Ala Ser Arg Pro Ser Ser Ser Arg Ser Tyr Val Thr Thr
            20                  25                  30

Ser Thr Arg Thr Tyr Ser Leu Gly Ser Ala Leu Arg Pro Ser Thr Ser
        35                  40                  45

Arg Ser Leu Tyr Ala Ser Ser Pro Gly Gly Val Tyr Ala Thr Arg Ser
    50                  55                  60

Ser Ala Val Arg Leu Arg Ser Ser Val Pro Gly Val Arg Leu Leu Gln
65                  70                  75                  80

Asp Ser Val Asp Phe Ser Leu Ala Asp Ala Ile Asn Thr Glu Phe Lys
                85                  90                  95

Asn Thr Arg Thr Asn Glu Lys Val Glu Leu Gln Glu Leu Asn Asp Arg
            100                 105                 110

Phe Ala Asn Tyr Ile Asp Lys Val Arg Phe Leu Glu Gln Gln Asn Lys
        115                 120                 125

Ile Leu Leu Ala Glu Leu Glu Gln Leu Lys Gly Gln Gly Lys Ser Arg
    130                 135                 140

Leu Gly Asp Leu Tyr Glu Glu Glu Met Arg Glu Leu Arg Arg Gln Val
145                 150                 155                 160

Asp Gln Leu Thr Asn Asp Lys Ala Arg Val Glu Val Glu Arg Asp Asn
                165                 170                 175

Leu Ala Glu Asp Ile Met Arg Leu Arg Glu Lys Leu Gln Glu Glu Met
            180                 185                 190

Leu Gln Arg Glu Glu Ala Glu Asn Thr Leu Gln Ser Phe Arg Gln Asp
        195                 200                 205

Val Asp Asn Ala Ser Leu Ala Arg Leu Asp Leu Glu Arg Lys Val Glu
    210                 215                 220

Ser Leu Gln Glu Glu Ile Ala Phe Leu Lys Lys Leu His Glu Glu Glu
225                 230                 235                 240

Ile Gln Glu Leu Gln Ala Gln Ile Gln Glu Gln His Val Gln Ile Asp
                245                 250                 255

Val Asp Val Ser Lys Pro Asp Leu Thr Ala Ala Leu Arg Asp Val Arg
            260                 265                 270

Gln Gln Tyr Glu Ser Val Ala Ala Lys Asn Leu Gln Glu Ala Glu Glu
        275                 280                 285

Trp Tyr Lys Ser Lys Phe Ala Asp Leu Ser Glu Ala Ala Asn Arg Asn
    290                 295                 300

Asn Asp Ala Leu Arg Gln Ala Lys Gln Glu Ser Thr Glu Tyr Arg Arg
305                 310                 315                 320

Gln Val Gln Ser Leu Thr Cys Glu Val Asp Ala Leu Lys Gly Thr Asn
                325                 330                 335

Glu Ser Leu Glu Arg Gln Met Arg Glu Met Glu Glu Asn Phe Ala Val
            340                 345                 350

Glu Ala Ala Asn Tyr Gln Asp Thr Ile Gly Arg Leu Gln Asp Glu Ile
        355                 360                 365

Gln Asn Met Lys Glu Glu Met Ala Arg His Leu Arg Glu Tyr Gln Asp
    370                 375                 380

Leu Leu Asn Val Lys Met Ala Leu Asp Ile Glu Ile Ala Thr Tyr Arg
385                 390                 395                 400

Lys Leu Leu Glu Gly Glu Glu Ser Arg Ile Ser Leu Pro Leu Pro Asn
                405                 410                 415

Phe Ser Ser Leu Asn Leu Arg Glu Thr Asn Leu Asp Ser Leu Pro Leu
            420                 425                 430

Val Asp Thr His Ser Lys Arg Thr Phe Leu Ile Lys Thr Val Glu Thr
        435                 440                 445

Arg Asp Gly Gln Val Ile Asn Glu Thr Ser Gln His His Asp Asp Leu
    450                 455                 460

Glu
465

<210>  117

<211>  139

<212>  PRT

<213>  Homo sapiens

<300>

<308>  sw_hum/pro1_human

<309>  1988-08-01

<400>  117

<210>  118

<211>  243

<212>  PRT

<213>  Homo sapiens

<300>

<308>  sw_hum/rs3_human

<309>  1991-11-01

<400>  118

Met Ala Val Gln Ile Ser Lys Lys Arg Lys Phe Val Ala Asp Gly Ile
1               5                   10                  15

Phe Lys Ala Glu Leu Asn Glu Phe Leu Thr Arg Glu Leu Ala Glu Asp
            20                  25                  30

Gly Tyr Ser Gly Val Glu Val Arg Val Thr Pro Thr Arg Thr Glu Ile
        35                  40                  45

Ile Ile Leu Ala Thr Arg Thr Gln Asn Val Leu Gly Glu Lys Gly Arg

50                  55                  60

Arg Ile Arg Glu Leu Thr Ala Val Val Gln Lys Arg Phe Gly Phe Pro
65                  70                  75                  80

Glu Gly Ser Val Glu Leu Tyr Ala Glu Lys Val Ala Thr Arg Gly Leu
                85                  90                  95

Cys Ala Ile Ala Gln Ala Glu Ser Leu Arg Tyr Lys Leu Leu Gly Gly
            100                 105                 110

Leu Ala Val Arg Arg Ala Cys Tyr Gly Val Leu Arg Phe Ile Met Glu
        115                 120                 125

Ser Gly Ala Lys Gly Cys Glu Val Val Val Ser Gly Lys Leu Arg Gly
    130                 135                 140

Gln Arg Ala Lys Ser Met Lys Phe Val Asp Gly Leu Met Ile His Ser
145                 150                 155                 160

Gly Asp Pro Val Asn Tyr Tyr Val Asp Thr Ala Val Arg His Val Leu
                165                 170                 175

Leu Arg Gln Gly Val Leu Gly Ile Lys Val Lys Ile Met Leu Pro Trp
            180                 185                 190

Asp Pro Thr Gly Lys Ile Gly Pro Lys Lys Pro Leu Pro Asp His Val
        195                 200                 205

Ser Ile Val Glu Pro Lys Asp Glu Ile Leu Pro Thr Thr Pro Ile Ser
    210                 215                 220

Glu Gln Lys Gly Gly Lys Pro Glu Pro Pro Ala Met Pro Gln Pro Val
225                 230                 235                 240

Pro Thr Ala

<210>  119

<211>  297

<212>  PRT

<213>  Homo sapiens

<300>

<308>  sw_hum/nadc_human

<309>  1997-11-01

<400>  119

Met Asp Ala Glu Gly Leu Ala Leu Leu Leu Pro Pro Val Thr Leu Ala
1               5                   10                  15

Ala Leu Val Asp Ser Trp Leu Arg Glu Asp Cys Pro Gly Leu Asn Tyr
            20                  25                  30

Ala Ala Leu Val Ser Gly Ala Gly Pro Ser Gln Ala Ala Leu Trp Ala
        35                  40                  45

Lys Ser Pro Gly Val Leu Ala Gly Gln Pro Phe Phe Asp Ala Ile Phe
    50                  55                  60

Thr Gln Leu Asn Cys Gln Val Ser Trp Phe Leu Pro Glu Gly Ser Lys
65                  70                  75                  80

Leu Val Pro Val Ala Arg Val Ala Glu Val Arg Gly Pro Ala His Cys
                85                  90                  95

Leu Leu Leu Gly Glu Arg Val Ala Leu Asn Thr Leu Ala Arg Cys Ser
            100                 105                 110

Gly Ile Ala Ser Ala Ala Ala Ala Ala Val Glu Ala Ala Arg Gly Ala
        115                 120                 125

Gly Trp Thr Gly His Val Ala Gly Thr Arg Lys Thr Thr Pro Gly Phe
    130                 135                 140

Arg Leu Val Glu Lys Tyr Gly Leu Leu Val Gly Gly Ala Ala Ser His
145                 150                 155                 160

Arg Tyr Asp Leu Gly Gly Leu Val Met Val Lys Asp Asn His Val Val
                165                 170                 175

Ala Ala Gly Gly Val Glu Lys Ala Val Arg Ala Ala Arg Gln Ala Ala
            180                 185                 190

Asp Phe Ala Leu Lys Val Glu Val Glu Cys Ser Ser Leu Gln Glu Ala
        195                 200                 205

Val Gln Ala Ala Glu Ala Gly Ala Asp Leu Val Leu Leu Asp Asn Phe
    210                 215                 220

Lys Pro Glu Glu Leu His Pro Thr Ala Thr Val Leu Lys Ala Gln Phe
225                 230                 235                 240

Pro Ser Val Ala Val Glu Ala Ser Gly Gly Ile Thr Leu Asp Asn Leu
                245                 250                 255

Pro Gln Phe Cys Gly Pro His Ile Asp Val Ile Ser Met Gly Met Leu
            260                 265                 270

Thr Gln Ala Ala Pro Ala Leu Asp Phe Ser Leu Lys Leu Phe Ala Lys
        275                 280                 285

Glu Val Ala Pro Val Pro Lys Ile His
    290                 295

<210>  120

<211>  279

<212>  PRT

<213>  Homo sapiens

<300>

<308>  humangp/chr5-q96nm0

<309>  2003-04-22

<400>  120

Met Leu Leu Ala Ala Ser Lys Val Leu Asp Arg Leu Lys Pro Val Ile
1               5                   10                  15

Gly Val Asn Thr Asp Pro Glu Arg Ser Glu Gly His Leu Cys Leu Pro
            20                  25                  30

Val Arg Tyr Thr His Ser Phe Pro Glu Ala Leu Gln Lys Phe Tyr Arg
        35                  40                  45

Gly Glu Phe Arg Trp Leu Trp Arg Gln Arg Ile Arg Leu Tyr Leu Glu
    50                  55                  60

Gly Thr Gly Ile Asn Pro Val Pro Val Asp Leu His Glu Gln Gln Leu
65                  70                  75                  80

Ser Leu Asn Gln His Asn Arg Ala Leu Asn Ile Glu Arg Ala His Asp
                85                  90                  95

Glu Arg Ser Glu Ala Ser Gly Pro Gln Leu Leu Pro Val Arg Ala Leu
            100                 105                 110

Asn Glu Val Phe Ile Gly Glu Ser Leu Ser Ser Arg Ala Ser Tyr Tyr
        115                 120                 125

Glu Ile Ser Val Asp Asp Gly Pro Trp Glu Lys Gln Lys Ser Ser Gly
    130                 135                 140

Leu Asn Leu Cys Thr Gly Thr Gly Ser Lys Ala Trp Ser Phe Asn Ile
145                 150                 155                 160

Asn Arg Val Ala Thr Gln Ala Val Glu Asp Val Leu Asn Ile Ala Lys
                165                 170                 175

Arg Gln Gly Asn Leu Ser Leu Pro Leu Asn Arg Glu Leu Val Glu Lys
            180                 185                 190

Val Thr Asn Glu Tyr Asn Glu Ser Leu Leu Tyr Ser Pro Glu Glu Pro
        195                 200                 205

Lys Ile Leu Phe Ser Ile Arg Glu Pro Ile Ala Asn Arg Val Phe Ser
    210                 215                 220

Ser Ser Arg Gln Arg Cys Phe Ser Ser Lys Val Cys Val Arg Ser Arg
225                 230                 235                 240

Cys Trp Asp Ala Cys Met Val Val Asp Gly Gly Thr Ser Phe Glu Phe
                245                 250                 255

Asn Asp Gly Ala Ile Ala Ser Met Met Ile Asn Lys Glu Asp Glu Leu
            260                 265                 270

Arg Thr Val Leu Leu Glu Gln
        275

<210>  121

<211>  346

<212>  PRT

<213>  Homo sapiens

<300>

<308>  humangp/chr19-095717

<309>  2003-04-22

<400>  121

Met Val Glu Lys Glu Glu Ala Gly Gly Gly Ile Ser Glu Glu Glu Ala
1               5                   10                  15

Ala Gln Tyr Asp Arg Gln Ile Arg Leu Trp Gly Leu Glu Ala Gln Lys
            20                  25                  30

Arg Leu Arg Ala Ser Arg Val Leu Leu Val Gly Leu Lys Gly Leu Gly
        35                  40                  45

Ala Glu Ile Ala Lys Asn Leu Ile Leu Ala Gly Val Lys Gly Leu Thr
    50                  55                  60

Met Leu Asp His Glu Gln Val Thr Pro Glu Asp Pro Gly Ala Gln Phe
65                  70                  75                  80

Leu Ile Arg Thr Gly Ser Val Gly Arg Asn Arg Ala Glu Ala Ser Leu
                85                  90                  95

Glu Arg Ala Gln Asn Leu Asn Pro Met Val Asp Val Lys Val Asp Thr
            100                 105                 110

Glu Asp Ile Glu Lys Lys Pro Glu Ser Phe Phe Thr Gln Phe Asp Ala
        115                 120                 125

Val Cys Leu Thr Cys Cys Ser Arg Asp Val Ile Val Lys Val Asp Gln
    130                 135                 140

Ile Cys His Lys Asn Ser Ile Lys Phe Phe Thr Gly Asp Val Phe Gly
145                 150                 155                 160

Tyr His Gly Tyr Thr Phe Ala Asn Leu Gly Glu His Glu Phe Val Glu
                165                 170                 175

Glu Lys Thr Lys Val Ala Lys Val Ser Gln Gly Val Glu Asp Gly Pro
            180                 185                 190

Asp Thr Lys Arg Ala Lys Leu Asp Ser Ser Glu Thr Thr Met Val Lys
        195                 200                 205

Lys Lys Val Val Phe Cys Pro Val Lys Glu Ala Leu Glu Val Asp Trp
    210                 215                 220

Ser Ser Glu Lys Ala Lys Ala Ala Leu Lys Arg Thr Thr Ser Asp Tyr
225                 230                 235                 240

Phe Leu Leu Gln Val Leu Leu Lys Phe Arg Thr Asp Lys Gly Arg Asp
                245                 250                 255

Pro Ser Ser Asp Thr Tyr Glu Glu Asp Ser Glu Leu Leu Leu Gln Ile
            260                 265                 270

Arg Asn Asp Val Leu Asp Ser Leu Gly Ile Ser Pro Asp Leu Leu Pro
        275                 280                 285

Glu Asp Phe Val Arg Tyr Cys Phe Ser Glu Met Ala Pro Val Cys Ala
    290                 295                 300

Val Val Gly Gly Ile Leu Ala Gln Glu Ile Val Lys Ala Leu Ser Gln
305                 310                 315                 320

Arg Asp Pro Pro His Asn Asn Phe Phe Phe Phe Asp Gly Met Lys Gly
                325                 330                 335

Asn Gly Ile Val Glu Cys Leu Gly Pro Lys
            340                 345

<210>  122

<211>  289

<212>  PRT

<213>  Homo sapiens

<300>

<308>  sw_hum/gnpi_human

<309>  1995-11-01

<400>  122

Met Lys Leu Ile Ile Leu Glu His Tyr Ser Gln Ala Ser Glu Trp Ala
1               5                   10                  15

Ala Lys Tyr Ile Arg Asn Arg Ile Ile Gln Phe Asn Pro Gly Pro Glu
            20                  25                  30

Lys Tyr Phe Thr Leu Gly Leu Pro Thr Gly Ser Thr Pro Leu Gly Cys
        35                  40                  45

Tyr Lys Lys Leu Ile Glu Tyr Tyr Lys Asn Gly Asp Leu Ser Phe Lys
    50                  55                  60

Tyr Val Lys Thr Phe Asn Met Asp Glu Tyr Val Gly Leu Pro Arg Asp

65                  70                  75                  80

His Pro Glu Ser Tyr His Ser Phe Met Trp Asn Asn Phe Phe Lys His
                85                  90                  95

Ile Asp Ile His Pro Glu Asn Thr His Ile Leu Asp Gly Asn Ala Val
            100                 105                 110

Asp Leu Gln Ala Glu Cys Asp Ala Phe Glu Glu Lys Ile Lys Ala Ala
        115                 120                 125

Gly Gly Ile Glu Leu Phe Val Gly Gly Ile Gly Pro Asp Gly His Ile
    130                 135                 140

Ala Phe Asn Glu Pro Gly Ser Ser Leu Val Ser Arg Thr Arg Val Lys
145                 150                 155                 160

Thr Leu Ala Met Asp Thr Ile Leu Ala Asn Ala Arg Phe Phe Asp Gly
                165                 170                 175

Glu Leu Thr Lys Val Pro Thr Met Ala Leu Thr Val Gly Val Gly Thr
            180                 185                 190

Val Met Asp Ala Arg Glu Val Met Ile Leu Ile Thr Gly Ala His Lys
        195                 200                 205

Ala Phe Ala Leu Tyr Lys Ala Ile Glu Glu Gly Val Asn His Met Trp
    210                 215                 220

Thr Val Ser Ala Phe Gln Gln His Pro Arg Thr Val Phe Val Cys Asp
225                 230                 235                 240

Glu Asp Ala Thr Leu Glu Leu Lys Val Lys Thr Val Lys Tyr Phe Lys
                245                 250                 255

Gly Leu Met Leu Val His Asn Lys Leu Val Asp Pro Leu Tyr Ser Ile
            260                 265                 270

Lys Glu Lys Glu Thr Glu Lys Ser Gln Ser Ser Lys Lys Pro Tyr Ser
        275                 280                 285

Asp

<210>  123

<211>  344

<212>  PRT

<213>  Homo sapiens

<300>

<308>  sw_hum/nagk_human

<309>  2003-02-28

<400>  123

Met Ala Ala Ile Tyr Gly Gly Val Glu Gly Gly Gly Thr Arg Ser Glu
1               5                   10                  15

Val Leu Leu Val Ser Glu Asp Gly Lys Ile Leu Ala Glu Ala Asp Gly
            20                  25                  30

Leu Ser Thr Asn His Trp Leu Ile Gly Thr Asp Lys Cys Val Glu Arg
        35                  40                  45

Ile Asn Glu Met Val Asn Arg Ala Lys Arg Lys Ala Gly Val Asp Pro
    50                  55                  60

Leu Val Pro Leu Arg Ser Leu Gly Leu Ser Leu Ser Gly Gly Asp Gln
65                  70                  75                  80

Glu Asp Ala Gly Arg Ile Leu Ile Glu Glu Leu Arg Asp Arg Phe Pro
                85                  90                  95

Tyr Leu Ser Glu Ser Tyr Leu Ile Thr Thr Asp Ala Ala Gly Ser Ile
            100                 105                 110

Ala Thr Ala Thr Pro Asp Gly Gly Val Val Leu Ile Ser Gly Thr Gly
        115                 120                 125

Ser Asn Cys Arg Leu Ile Asn Pro Asp Gly Ser Glu Ser Gly Cys Gly
    130                 135                 140

Gly Trp Gly His Met Met Gly Asp Glu Gly Ser Ala Tyr Trp Ile Ala
145                 150                 155                 160

His Gln Ala Val Lys Ile Val Phe Asp Ser Ile Asp Asn Leu Glu Ala
                165                 170                 175

Ala Pro His Asp Ile Gly Tyr Val Lys Gln Ala Met Phe His Tyr Phe
            180                 185                 190

Gln Val Pro Asp Arg Leu Gly Ile Leu Thr His Leu Tyr Arg Asp Phe
        195                 200                 205

Asp Lys Cys Arg Phe Ala Gly Phe Cys Arg Lys Ile Ala Glu Gly Ala
    210                 215                 220

Gln Gln Gly Asp Pro Leu Ser Arg Tyr Ile Phe Arg Lys Ala Gly Glu
225                 230                 235                 240

Met Leu Gly Arg His Ile Val Ala Val Leu Pro Glu Ile Asp Pro Val
                245                 250                 255

Leu Phe Gln Gly Lys Ile Gly Leu Pro Ile Leu Cys Val Gly Ser Val
            260                 265                 270

Trp Lys Ser Trp Glu Leu Leu Lys Glu Gly Phe Leu Leu Ala Leu Thr
        275                 280                 285

Gln Gly Arg Glu Ile Gln Ala Gln Asn Phe Phe Ser Ser Phe Thr Leu
    290                 295                 300

Met Lys Leu Arg His Ser Ser Ala Leu Gly Gly Ala Ser Leu Gly Ala
305                 310                 315                 320

Arg His Ile Gly His Leu Leu Pro Met Asp Tyr Ser Ala Asn Ala Ile
                325                 330                 335

Ala Phe Tyr Ser Tyr Thr Phe Ser
            340

<210>  124

<211>  222

<212>  PRT

<213>  Homo sapiens

<300>

<308>  sw_hum/sodm_human

<309>  1987-03-20

<400>  124

<210> 125

<211> 282

<212> PRT

<213> Homo sapiens

<300>

<308> sw_hum/estd_human

<309> 1989-07-01

<400> 125

Met Ala Leu Lys Gln Ile Ser Ser Asn Lys Cys Phe Gly Gly Leu Gln
1               5                   10                  15

Lys Val Phe Glu His Asp Ser Val Glu Leu Asn Cys Lys Met Lys Phe
            20                  25                  30

Ala Val Tyr Leu Pro Pro Lys Ala Glu Thr Gly Lys Cys Pro Ala Leu
        35                  40                  45

Tyr Trp Leu Ser Gly Leu Thr Cys Thr Glu Gln Asn Phe Ile Ser Lys
    50                  55                  60

Ser Gly Tyr His Gln Ser Ala Ser Glu His Gly Leu Val Val Ile Ala
65                  70                  75                  80

Pro Asp Thr Ser Pro Arg Gly Cys Asn Ile Lys Gly Glu Asp Glu Ser
                85                  90                  95

Trp Asp Phe Gly Thr Gly Ala Gly Phe Tyr Val Asp Ala Thr Glu Asp
            100                 105                 110

Pro Trp Lys Thr Asn Tyr Arg Met Tyr Ser Tyr Val Thr Glu Glu Leu
        115                 120                 125

Pro Gln Leu Ile Asn Ala Asn Phe Pro Val Asp Pro Gln Arg Met Ser
    130                 135                 140

Ile Phe Gly His Ser Met Gly Gly His Gly Ala Leu Ile Cys Ala Leu
145                 150                 155                 160

Lys Asn Pro Gly Lys Tyr Lys Ser Val Ser Ala Phe Ala Pro Ile Cys
                165                 170                 175

Asn Pro Val Leu Cys Pro Trp Gly Lys Lys Ala Phe Ser Gly Tyr Leu

180                 185                 190

Gly Thr Asp Gln Ser Lys Trp Lys Ala Tyr Asp Ala Thr His Leu Val
        195                 200                 205

Lys Ser Tyr Pro Gly Ser Gln Leu Asp Ile Leu Ile Asp Gln Gly Lys
    210                 215                 220

Asp Asp Gln Phe Leu Leu Asp Gly Gln Leu Leu Pro Asp Asn Phe Ile
225                 230                 235                 240

Ala Ala Cys Thr Glu Lys Lys Ile Pro Val Val Phe Arg Leu Gln Glu
                245                 250                 255

Gly Tyr Asp His Ser Tyr Tyr Phe Ile Ala Thr Phe Ile Thr Asp His
            260                 265                 270

Ile Arg His His Ala Lys Tyr Leu Asn Ala
        275                 280

<210>  126

<211>  504

<212>  PRT

<213>  Homo sapiens

<300>

<308>  sw_hum/glym_human

<309>  1994-02-01

<400>  126

<210>  127

<211>  452

<212>  PRT

<213>  Homo sapiens

<300>

<308>  sw_hum/idhp_human

<309>  1996-02-01

<400>  127

<210>  128

<211>  263

<212>  PRT

<213>  Homo sapiens

<300>

<308>  sw_hum/psa1_human

<309>  1992-05-01

<400>  128

Met Phe Arg Asn Gln Tyr Asp Asn Asp Val Thr Val Trp Ser Pro Gln
1               5                   10                  15

Gly Arg Ile His Gln Ile Glu Tyr Ala Met Glu Ala Val Lys Gln Gly
            20                  25                  30

Ser Ala Thr Val Gly Leu Lys Ser Lys Thr His Ala Val Leu Val Ala
        35                  40                  45

Leu Lys Arg Ala Gln Ser Glu Leu Ala Ala His Gln Lys Lys Ile Leu
    50                  55                  60

His Val Asp Asn His Ile Gly Ile Ser Ile Ala Gly Leu Thr Ala Asp
65                  70                  75                  80

Ala Arg Leu Leu Cys Asn Phe Met Arg Gln Glu Cys Leu Asp Ser Arg
                85                  90                  95

Phe Val Phe Asp Arg Pro Leu Pro Val Ser Arg Leu Val Ser Leu Ile
            100                 105                 110

Gly Ser Lys Thr Gln Ile Pro Thr Gln Arg Tyr Gly Arg Arg Pro Tyr
        115                 120                 125

Gly Val Gly Leu Leu Ile Ala Gly Tyr Asp Asp Met Gly Pro His Ile
    130                 135                 140

Phe Gln Thr Cys Pro Ser Ala Asn Tyr Phe Asp Cys Arg Ala Met Ser
145                 150                 155                 160

Ile Gly Ala Arg Ser Gln Ser Ala Arg Thr Tyr Leu Glu Arg His Met
                165                 170                 175

Ser Glu Phe Met Glu Cys Asn Leu Asn Glu Leu Val Lys His Gly Leu
            180                 185                 190

Arg Ala Leu Arg Glu Thr Leu Pro Ala Glu Gln Asp Leu Thr Thr Lys
        195                 200                 205

Asn Val Ser Ile Gly Ile Val Gly Lys Asp Leu Glu Phe Thr Ile Tyr
    210                 215                 220

Asp Asp Asp Asp Val Ser Pro Phe Leu Glu Gly Leu Glu Glu Arg Pro
225                 230                 235                 240

Gln Arg Lys Ala Gln Pro Ala Gln Pro Ala Asp Glu Pro Ala Glu Lys
                245                 250                 255

Ala Asp Glu Pro Met Glu His
            260

<210>  129

<211>  740

<212>  PRT

<213>  Homo sapiens

<300>

<308>  sw_hum/k6a3_human

<309>  1996-10-01

<400>  129

<210>  130

<211>  194

<212>  PRT

<213>  Homo sapiens

<300>

<308>  humangp/chr20-014878

<309>  2003-04-22

<400>  130

<210>  131

<211>  524

<212>  PRT

<213>  Homo sapiens

<300>

<308>  sw_hum/pak2_human

<309>  1997-11-01

<400>  131

Met Ser Asp Asn Gly Glu Leu Glu Asp Lys Pro Pro Ala Pro Pro Val
1               5                   10                  15

Arg Met Ser Ser Thr Ile Phe Ser Thr Gly Gly Lys Asp Pro Leu Ser
            20                  25                  30

Ala Asn His Ser Leu Lys Pro Leu Pro Ser Val Pro Glu Glu Lys Lys
        35                  40                  45

Pro Arg His Lys Ile Ile Ser Ile Phe Ser Gly Thr Glu Lys Gly Ser
    50                  55                  60

Lys Lys Lys Glu Lys Glu Arg Pro Glu Ile Ser Pro Pro Ser Asp Phe
65                  70                  75                  80

Glu His Thr Ile His Val Gly Phe Asp Ala Val Thr Gly Glu Phe Thr
                85                  90                  95

Gly Met Pro Glu Gln Trp Ala Arg Leu Leu Gln Thr Ser Asn Ile Thr
            100                 105                 110

Lys Leu Glu Gln Lys Lys Asn Pro Gln Ala Val Leu Asp Val Leu Lys
        115                 120                 125

Phe Tyr Asp Ser Asn Thr Val Lys Gln Lys Tyr Leu Ser Phe Thr Pro
    130                 135                 140

Pro Glu Lys Asp Gly Leu Pro Ser Gly Thr Pro Ala Leu Asn Ala Lys
145                 150                 155                 160

Gly Thr Glu Ala Pro Ala Val Val Thr Glu Glu Glu Asp Asp Asp Glu
                165                 170                 175

Glu Thr Ala Pro Pro Val Ile Ala Pro Arg Pro Asp His Thr Lys Ser
            180                 185                 190

Ile Tyr Thr Arg Ser Val Ile Asp Pro Val Pro Ala Pro Val Gly Asp
        195                 200                 205

Ser His Val Asp Gly Ala Ala Lys Ser Leu Asp Lys Gln Lys Lys Lys
    210                 215                 220

Pro Lys Met Thr Asp Glu Glu Ile Met Glu Lys Leu Arg Thr Ile Val
225                 230                 235                 240

Ser Ile Gly Asp Pro Lys Lys Lys Tyr Thr Arg Tyr Glu Lys Ile Gly
                245                 250                 255

Gln Gly Ala Ser Gly Thr Val Phe Thr Ala Thr Asp Val Ala Leu Gly
            260                 265                 270

Gln Glu Val Ala Ile Lys Gln Ile Asn Leu Gln Lys Gln Pro Lys Lys

275                 280                 285

Glu Leu Ile Ile Asn Glu Ile Leu Val Met Lys Glu Leu Lys Asn Pro
    290                 295                 300

Asn Ile Val Asn Phe Leu Asp Ser Tyr Leu Val Gly Asp Glu Leu Phe
305                 310                 315                 320

Val Val Met Glu Tyr Leu Ala Gly Gly Ser Leu Thr Asp Val Val Thr
                325                 330                 335

Glu Thr Cys Met Asp Glu Ala Gln Ile Ala Ala Val Cys Arg Glu Cys
            340                 345                 350

Leu Gln Ala Leu Glu Phe Leu His Ala Asn Gln Val Ile His Arg Asp
        355                 360                 365

Ile Lys Ser Asp Asn Val Leu Leu Gly Met Glu Gly Ser Val Lys Leu
    370                 375                 380

Thr Asp Phe Gly Phe Cys Ala Gln Ile Thr Pro Glu Gln Ser Lys Arg
385                 390                 395                 400

Ser Thr Met Val Gly Thr Pro Tyr Trp Met Ala Pro Glu Val Val Thr
                405                 410                 415

Arg Lys Ala Tyr Gly Pro Lys Val Asp Ile Trp Ser Leu Gly Ile Met
            420                 425                 430

Ala Ile Glu Met Val Glu Gly Glu Pro Pro Tyr Leu Asn Glu Asn Pro
        435                 440                 445

Leu Arg Ala Leu Tyr Leu Ile Ala Thr Asn Gly Thr Pro Glu Leu Gln
    450                 455                 460

Asn Pro Glu Lys Leu Ser Pro Ile Phe Arg Asp Phe Leu Asn Arg Cys
465                 470                 475                 480

Leu Glu Met Asp Val Glu Lys Arg Gly Ser Ala Lys Glu Leu Leu Gln
                485                 490                 495

His Pro Phe Leu Lys Leu Ala Lys Pro Leu Ser Ser Leu Thr Pro Leu
            500                 505                 510

Ile Met Ala Ala Lys Glu Ala Met Lys Ser Asn Arg
        515                 520

<210>  132

<211>  688

<212>  PRT

<213>  Homo sapiens


<300>

<308>  sw_hum/mepd_human

<309>  1996-10-01

<400>  132

Lys Pro Pro Ala Ala Cys Ala Gly Asp Met Ala Asp Ala Ala Ser Pro
1               5                   10                  15

Cys Ser Val Val Asn Asp Leu Arg Trp Asp Leu Ser Ala Gln Gln Ile
            20                  25                  30

Glu Glu Arg Thr Arg Glu Leu Ile Glu Gln Thr Lys Arg Val Tyr Asp
        35                  40                  45

Gln Val Gly Thr Gln Glu Phe Glu Asp Val Ser Tyr Glu Ser Thr Leu
    50                  55                  60

Lys Ala Leu Ala Asp Val Glu Val Thr Tyr Thr Val Gln Arg Asn Ile
65                  70                  75                  80

Leu Asp Phe Pro Gln His Val Ser Pro Ser Lys Asp Ile Arg Thr Ala
                85                  90                  95

Ser Thr Glu Ala Asp Lys Lys Leu Ser Glu Phe Asp Val Glu Met Ser
            100                 105                 110

Met Arg Glu Asp Val Tyr Gln Arg Ile Val Trp Leu Gln Glu Lys Val
        115                 120                 125

Gln Lys Asp Ser Leu Arg Pro Glu Ala Ala Arg Tyr Leu Glu Arg Leu
    130                 135                 140

Ile Lys Leu Gly Arg Arg Asn Gly Leu His Leu Pro Arg Glu Thr Gln
145                 150                 155                 160

Glu Asn Ile Lys Arg Ile Lys Lys Lys Leu Ser Leu Leu Cys Ile Asp
                165                 170                 175

Phe Asn Lys Asn Leu Asn Glu Asp Thr Thr Phe Leu Pro Phe Thr Leu
            180                 185                 190

Gln Glu Leu Gly Gly Leu Pro Glu Asp Phe Leu Asn Ser Leu Glu Lys
        195                 200                 205

Met Glu Asp Gly Lys Leu Lys Val Thr Leu Lys Tyr Pro His Tyr Phe
    210                 215                 220

Pro Leu Leu Lys Lys Cys His Val Pro Glu Thr Arg Arg Lys Val Glu
225                 230                 235                 240

Glu Ala Phe Asn Cys Arg Cys Lys Glu Glu Asn Cys Ala Ile Leu Lys
                245                 250                 255

Glu Leu Val Thr Leu Arg Ala Gln Lys Ser Arg Leu Leu Gly Phe His
            260                 265                 270

Thr His Ala Asp Tyr Val Leu Glu Met Asn Met Ala Lys Thr Ser Gln
        275                 280                 285

Thr Val Ala Thr Phe Leu Asp Glu Leu Ala Gln Lys Leu Lys Pro Leu
    290                 295                 300

Gly Glu Gln Glu Arg Ala Val Ile Leu Glu Leu Lys Arg Ala Glu Cys
305                 310                 315                 320

Glu Arg Arg Gly Leu Pro Phe Asp Gly Arg Ile Arg Ala Trp Asp Met
                325                 330                 335

Arg Tyr Tyr Met Asn Gln Val Glu Glu Thr Arg Tyr Cys Val Asp Gln
            340                 345                 350

Asn Leu Leu Lys Glu Tyr Phe Pro Val Gln Val Val Thr His Gly Leu
        355                 360                 365

Leu Gly Ile Tyr Gln Glu Leu Leu Gly Leu Ala Phe His His Glu Glu
    370                 375                 380

Gly Ala Ser Ala Trp His Glu Asp Val Arg Leu Tyr Thr Ala Arg Asp
385                 390                 395                 400

Ala Ala Ser Gly Glu Val Val Gly Lys Phe Tyr Leu Asp Leu Tyr Pro
                405                 410                 415

Arg Glu Gly Lys Tyr Gly His Ala Ala Cys Phe Gly Leu Gln Pro Gly
            420                 425                 430

Cys Leu Arg Gln Asp Gly Ser Arg Gln Ile Ala Ile Ala Ala Met Val
        435                 440                 445

Ala Asn Phe Thr Lys Pro Thr Ala Asp Ala Pro Ser Leu Leu Gln His
    450                 455                 460

Asp Glu Val Glu Thr Tyr Phe His Glu Phe Gly His Val Met His Gln
465                 470                 475                 480

Leu Cys Ser Gln Ala Glu Phe Ala Met Phe Ser Gly Thr His Val Glu
                485                 490                 495

Arg Asp Phe Val Glu Ala Pro Ser Gln Met Leu Glu Asn Trp Val Trp
            500                 505                 510

Glu Gln Glu Pro Leu Leu Arg Met Ser Arg His Tyr Arg Thr Gly Ser
        515                 520                 525

Ala Val Pro Arg Glu Leu Leu Glu Lys Leu Ile Glu Ser Arg Gln Ala
    530                 535                 540

Asn Thr Gly Leu Phe Asn Leu Arg Gln Ile Val Leu Ala Lys Val Asp
545                 550                 555                 560

Gln Ala Leu His Thr Gln Thr Asp Ala Asp Pro Ala Glu Glu Tyr Ala
                565                 570                 575

Arg Leu Cys Gln Glu Ile Leu Gly Val Pro Ala Thr Pro Gly Thr Asn
            580                 585                 590

Met Pro Ala Thr Phe Gly His Leu Ala Gly Gly Tyr Asp Ala Gln Tyr
        595                 600                 605

Tyr Gly Tyr Leu Trp Ser Glu Val Tyr Ser Met Asp Met Phe His Thr
    610                 615                 620

Arg Phe Lys Gln Glu Gly Val Leu Asn Ser Lys Val Gly Met Asp Tyr
625                 630                 635                 640

Arg Ser Cys Ile Leu Arg Pro Gly Gly Ser Glu Asp Ala Ser Ala Met
                645                 650                 655

Leu Arg Arg Phe Leu Gly Arg Asp Pro Lys Gln Asp Ala Phe Leu Leu
            660                 665                 670

Ser Lys Gly Leu Gln Val Gly Gly Cys Glu Pro Glu Pro Gln Val Cys
        675                 680                 685

<210>  133

<211>  208

<212>  PRT

<213>  Homo sapiens

<300>

<308>  sw_hum/psb5_human

<309>  1992-08-01

<400>  133

<210>  134

<211>  360

<212>  PRT

<213>  Homo sapiens

<300>

<308>  sw_hum/mk01_human

<309>  1992-12-01

<400>  134

<210>  135

<211>  563

<212>  PRT

<213>  Homo sapiens

<300>

<308>  sw_hum/mccb_human

<309>  2003-02-28

<400>  135

<210>  136

<211>  1410

<212>  PRT

<213>  Homo sapiens

<300>

<308>  sw_hum/rrb1_human

<309>  2003-02-28

<400>  136

Met Asp Ile Tyr Asp Thr Gln Thr Leu Gly Val Val Val Phe Gly Gly
1               5                   10                  15

Phe Met Val Val Ser Ala Ile Gly Ile Phe Leu Val Ser Thr Phe Ser
            20                  25                  30

Met Lys Glu Thr Ser Tyr Glu Glu Ala Leu Ala Asn Gln Arg Lys Glu
        35                  40                  45

Met Ala Lys Thr His His Gln Lys Val Glu Lys Lys Lys Lys Glu Lys
    50                  55                  60

Thr Val Glu Lys Lys Gly Lys Thr Lys Lys Lys Glu Glu Lys Pro Asn
65                  70                  75                  80

Gly Lys Ile Pro Asp His Asp Pro Ala Pro Asn Val Thr Val Leu Leu
                85                  90                  95

Arg Glu Pro Val Arg Ala Pro Ala Val Ala Val Ala Pro Thr Pro Val
            100                 105                 110

Gln Pro Pro Ile Ile Val Ala Pro Val Ala Thr Val Pro Ala Met Pro
        115                 120                 125

Gln Glu Lys Leu Ala Ser Ser Pro Lys Asp Lys Lys Lys Lys Glu Lys
    130                 135                 140

Lys Val Ala Lys Val Glu Pro Ala Val Ser Ser Val Val Asn Ser Ile
145                 150                 155                 160

Gln Val Leu Thr Ser Lys Ala Ala Ile Leu Glu Thr Ala Pro Lys Glu
                165                 170                 175

Val Pro Met Val Val Val Pro Pro Val Gly Ala Lys Gly Asn Thr Pro
            180                 185                 190

Ala Thr Gly Thr Thr Gln Gly Lys Lys Ala Glu Gly Thr Gln Asn Gln
        195                 200                 205

Ser Lys Lys Ala Glu Gly Ala Pro Asn Gln Gly Arg Lys Ala Glu Gly
    210                 215                 220

Thr Pro Asn Gln Gly Lys Lys Thr Glu Gly Thr Pro Asn Gln Gly Lys
225                 230                 235                 240

Lys Ala Glu Gly Thr Pro Asn Gln Gly Lys Lys Ala Glu Gly Thr Pro
                245                 250                 255

Asn Gln Gly Lys Lys Ala Glu Gly Ala Gln Asn Gln Gly Lys Lys Val
            260                 265                 270

Asp Thr Thr Pro Asn Gln Gly Lys Lys Val Glu Gly Ala Pro Thr Gln

275                 280                 285

Gly Arg Lys Ala Glu Gly Ala Gln Asn Gln Ala Lys Lys Val Glu Gly
    290                 295                 300

Ala Gln Asn Gln Gly Lys Lys Ala Glu Gly Ala Gln Asn Gln Gly Lys
305                 310                 315                 320

Lys Gly Glu Gly Ala Gln Asn Gln Gly Lys Lys Ala Glu Gly Ala Gln
                325                 330                 335

Asn Gln Gly Lys Lys Ala Glu Gly Ala Gln Asn Gln Gly Lys Lys Ala
            340                 345                 350

Glu Gly Ala Gln Asn Gln Gly Lys Lys Ala Glu Gly Ala Gln Asn Gln
        355                 360                 365

Gly Lys Lys Ala Glu Gly Ala Gln Asn Gln Gly Lys Lys Ser Glu Gly
    370                 375                 380

Ala Gln Asn Gln Gly Lys Lys Val Glu Gly Ala Gln Asn Gln Gly Lys
385                 390                 395                 400

Lys Ala Glu Gly Ala Gln Asn Gln Gly Lys Lys Ala Glu Gly Ala Gln
                405                 410                 415

Asn Gln Gly Lys Lys Ala Glu Gly Ala Gln Asn Gln Gly Lys Lys Ala
            420                 425                 430

Glu Gly Ala Gln Asn Gln Gly Lys Lys Ala Glu Gly Ala Gln Asn Gln
        435                 440                 445

Gly Lys Lys Ala Glu Gly Ala Gln Asn Gln Gly Lys Lys Ala Glu Gly
    450                 455                 460

Ala Gln Asn Gln Gly Lys Lys Val Glu Gly Ala Gln Asn Gln Gly Lys
465                 470                 475                 480

Lys Ala Glu Gly Ala Gln Asn Gln Gly Lys Lys Ala Glu Gly Ala Gln
                485                 490                 495

Asn Gln Gly Lys Lys Ala Glu Gly Ala Gln Asn Gln Gly Gln Lys Gly
            500                 505                 510

Glu Gly Ala Gln Asn Gln Gly Lys Lys Thr Glu Gly Ala Gln Gly Lys
        515                 520                 525

Lys Ala Glu Arg Ser Pro Asn Gln Gly Lys Lys Gly Glu Gly Ala Pro
    530                 535                 540

Ile Gln Gly Lys Lys Ala Asp Ser Val Ala Asn Gln Gly Thr Lys Val
545                 550                 555                 560

Glu Gly Ile Thr Asn Gln Gly Lys Lys Ala Glu Gly Ser Pro Ser Glu
                565                 570                 575

Gly Lys Lys Ala Glu Gly Ser Pro Asn Gln Gly Lys Lys Ala Asp Ala
            580                 585                 590

Ala Ala Asn Gln Gly Lys Lys Thr Glu Ser Ala Ser Val Gln Gly Arg
        595                 600                 605

Asn Thr Asp Val Ala Gln Ser Pro Glu Ala Pro Lys Gln Glu Ala Pro
    610                 615                 620

Ala Lys Lys Lys Ser Gly Ser Lys Lys Lys Gly Glu Pro Gly Pro Pro
625                 630                 635                 640

Asp Ala Asp Gly Pro Leu Tyr Leu Pro Tyr Lys Thr Leu Val Ser Thr
                645                 650                 655

Val Gly Ser Met Val Phe Asn Glu Gly Glu Ala Gln Arg Leu Ile Glu
            660                 665                 670

Ile Leu Ser Glu Lys Ala Gly Ile Ile Gln Asp Thr Trp His Lys Ala
        675                 680                 685

Thr Gln Lys Gly Asp Pro Val Ala Ile Leu Lys Arg Gln Leu Glu Glu
    690                 695                 700

Lys Glu Lys Leu Leu Ala Thr Glu Gln Glu Asp Ala Ala Val Ala Lys
705                 710                 715                 720

Ser Lys Leu Arg Glu Leu Asn Lys Glu Met Ala Ala Glu Lys Ala Lys
                725                 730                 735

Ala Ala Ala Gly Glu Ala Lys Val Lys Lys Gln Leu Val Ala Arg Glu
            740                 745                 750

Gln Glu Ile Thr Ala Val Gln Ala Arg Met Gln Ala Ser Tyr Arg Glu
        755                 760                 765

His Val Lys Glu Val Gln Gln Leu Gln Gly Lys Ile Arg Thr Leu Gln
    770                 775                 780

Glu Gln Leu Glu Asn Gly Pro Asn Thr Gln Leu Ala Arg Leu Gln Gln
785                 790                 795                 800

Glu Asn Ser Ile Leu Arg Asp Ala Leu Asn Gln Ala Thr Ser Gln Val
                805                 810                 815

Glu Ser Lys Gln Asn Ala Glu Leu Ala Lys Leu Arg Gln Glu Leu Ser
            820                 825                 830

Lys Val Ser Lys Glu Leu Val Glu Lys Ser Glu Ala Val Arg Gln Asp
        835                 840                 845

Glu Gln Gln Arg Lys Ala Leu Glu Ala Lys Ala Ala Ala Phe Glu Lys
    850                 855                 860

Gln Val Leu Gln Leu Gln Ala Ser His Arg Glu Ser Glu Glu Ala Leu
865                 870                 875                 880

Gln Lys Arg Leu Asp Glu Val Ser Arg Glu Leu Cys His Thr Gln Ser
                885                 890                 895

Ser His Ala Ser Leu Arg Ala Asp Ala Glu Lys Ala Gln Glu Gln Gln
            900                 905                 910

Gln Gln Met Ala Glu Leu His Ser Lys Leu Gln Ser Ser Glu Ala Glu
        915                 920                 925

Val Arg Ser Lys Cys Glu Glu Leu Ser Gly Leu His Gly Gln Leu Gln
    930                 935                 940

Glu Ala Arg Ala Glu Asn Ser Gln Leu Thr Glu Arg Ile Arg Ser Ile
945                 950                 955                 960

Glu Ala Leu Leu Glu Ala Gly Gln Ala Arg Asp Ala Gln Asp Val Gln
                965                 970                 975

Ala Ser Gln Ala Glu Ala Asp Gln Gln Gln Thr Arg Leu Lys Glu Leu
            980                 985                 990

Glu Ser Gln Val Ser Gly Leu Glu Lys Glu Ala Ile Glu Leu Arg Glu
        995                 1000                1005

Ala Val Glu Gln Gln Lys Val Lys Asn Asn Asp Leu Arg Glu Lys
    1010                1015                1020

Asn Trp Lys Ala Met Glu Ala Leu Ala Thr Ala Glu Gln Ala Cys
    1025                1030                1035

Lys Glu Lys Leu Leu Ser Leu Thr Gln Ala Lys Glu Glu Ser Glu
    1040                1045                1050

Lys Gln Leu Cys Leu Ile Glu Ala Gln Thr Met Glu Ala Leu Leu
    1055                1060                1065

Ala Leu Leu Pro Glu Leu Ser Val Leu Ala Gln Gln Asn Tyr Thr
    1070                1075                1080

Glu Trp Leu Gln Asp Leu Lys Glu Lys Gly Pro Thr Leu Leu Lys
    1085                1090                1095

His Pro Pro Ala Pro Ala Glu Pro Ser Ser Asp Leu Ala Ser Lys
    1100                1105                1110

Leu Arg Glu Ala Glu Glu Thr Gln Ser Thr Leu Gln Ala Glu Cys
    1115                1120                1125

Asp Gln Tyr Arg Ser Ile Leu Ala Glu Thr Glu Gly Met Leu Arg
    1130                1135                1140

Asp Leu Gln Lys Ser Val Glu Glu Glu Glu Gln Val Trp Arg Ala
    1145                1150                1155

Lys Val Gly Ala Ala Glu Glu Glu Leu Gln Lys Ser Arg Val Thr
    1160                1165                1170

Val Lys His Leu Glu Glu Ile Val Glu Lys Leu Lys Gly Glu Leu
    1175                1180                1185

Glu Ser Ser Asp Gln Val Arg Glu His Thr Ser His Leu Glu Ala
    1190                1195                1200

Glu Leu Glu Lys His Met Ala Ala Ala Ser Ala Glu Cys Gln Asn
    1205                1210                1215

Tyr Ala Lys Glu Val Ala Gly Leu Arg Gln Leu Leu Leu Glu Ser
    1220                1225                1230

Gln Ser Gln Leu Asp Ala Ala Lys Ser Glu Ala Gln Lys Gln Ser
    1235                1240                1245

Asp Glu Leu Ala Leu Val Arg Gln Gln Leu Ser Glu Met Lys Ser
    1250                1255                1260

His Val Glu Asp Gly Asp Ile Ala Gly Ala Pro Ala Ser Ser Pro
    1265                1270                1275

Glu Ala Pro Pro Ala Glu Gln Asp Pro Val Gln Leu Lys Thr Gln
    1280                1285                1290

Leu Glu Trp Thr Glu Ala Ile Leu Glu Asp Glu Gln Thr Gln Arg
    1295                1300                1305

Gln Lys Leu Thr Ala Glu Phe Glu Glu Ala Gln Thr Ser Ala Cys
    1310                1315                1320

Arg Leu Gln Glu Glu Leu Glu Lys Leu Arg Thr Ala Gly Pro Leu
    1325                1330                1335

Glu Ser Ser Glu Thr Glu Glu Ala Ser Gln Leu Lys Glu Arg Leu
    1340                1345                1350

Glu Lys Glu Lys Lys Leu Thr Ser Asp Leu Gly Arg Ala Ala Thr
    1355                1360                1365

Arg Leu Gln Glu Leu Leu Lys Thr Thr Gln Glu Gln Leu Ala Arg
    1370                1375                1380

Glu Lys Asp Thr Val Lys Lys Leu Gln Glu Gln Leu Glu Lys Ala
    1385                1390                1395

Glu Asp Gly Ser Ser Ser Lys Glu Gly Thr Ser Val
    1400                1405                1410

<210> 137

<211> 360

<212> PRT

<213> Homo sapiens


<300>

<308> tr_hum/q96e67

<309> 2001-12-01

<400> 137

Met Cys Lys Ala Gly Phe Ala Gly Asp Asp Ala Pro Arg Ala Val Phe
1               5                   10                  15

Pro Ser Ile Val Gly Arg Pro Arg His Gln Gly Val Met Val Gly Met
            20                  25                  30

Gly Gln Lys Asp Ser Tyr Val Gly Asp Glu Ala Gln Ser Lys Arg Gly
        35                  40                  45

Ile Leu Thr Leu Lys Tyr Pro Ile Glu His Gly Ile Val Thr Asn Trp
    50                  55                  60

Asp Asp Met Glu Lys Ile Trp His His Thr Phe Tyr Asn Glu Leu Arg
65                  70                  75                  80

Val Ala Pro Glu Glu His Pro Val Leu Leu Thr Glu Ala Pro Leu Asn
                85                  90                  95

Pro Lys Ala Asn Leu Glu Lys Met Thr Gln Ile Met Phe Glu Thr Phe
            100                 105                 110

Asn Thr Pro Ala Met Tyr Val Ala Ile Gln Ala Val Leu Ser Leu Tyr
        115                 120                 125

Ala Ser Gly Arg Thr Thr Gly Ile Val Met Asp Ser Gly Asp Gly Val
    130                 135                 140

Thr His Thr Val Pro Ile Tyr Glu Gly Tyr Ala Leu Pro His Ala Ile
145                 150                 155                 160

Leu Arg Leu Asp Leu Ala Gly Arg Asp Leu Thr Asp Tyr Leu Met Lys
                165                 170                 175

Ile Leu Thr Glu Arg Gly Tyr Ser Phe Thr Thr Thr Ala Glu Arg Glu
            180                 185                 190

Ile Val Arg Asp Ile Lys Glu Lys Leu Cys Tyr Val Ala Leu Asp Phe
        195                 200                 205

Glu Gln Glu Met Ala Thr Ala Ala Ser Ser Ser Ser Leu Glu Lys Ser
    210                 215                 220

Tyr Glu Leu Pro Asp Gly Gln Val Ile Thr Ile Gly Asn Glu Arg Phe
225                 230                 235                 240

Arg Cys Pro Glu Ala Leu Phe Gln Pro Ser Phe Leu Gly Met Glu Ser
                245                 250                 255

Cys Gly Ile His Glu Thr Thr Phe Asn Ser Ile Met Lys Cys Asp Val
            260                 265                 270

Asp Ile Arg Lys Asp Leu Tyr Ala Asn Thr Val Leu Ser Gly Gly Thr
        275                 280                 285

Thr Met Tyr Pro Gly Ile Ala Asp Arg Met Gln Lys Glu Ile Thr Ala

290                 295                 300

Leu Ala Pro Ser Thr Met Lys Ile Lys Ile Ile Ala Pro Pro Glu Arg
305                 310                 315                 320

Lys Tyr Ser Val Trp Ile Gly Gly Ser Ile Leu Ala Ser Leu Ser Thr
                325                 330                 335

Phe Gln Gln Met Trp Ile Ser Lys Gln Glu Tyr Asp Glu Ser Gly Pro
            340                 345                 350

Ser Ile Val His Arg Lys Cys Phe
        355                 360

<210>  138

<211>  445

<212>  PRT

<213>  Homo sapiens

<300>

<308>  sw_hum/hgd_human

<309>  1997-11-01

<400>  138

Met Ala Glu Leu Lys Tyr Ile Ser Gly Phe Gly Asn Glu Cys Ser Ser
1               5                   10                  15

Glu Asp Pro Arg Cys Pro Gly Ser Leu Pro Glu Gly Gln Asn Asn Pro
            20                  25                  30

Gln Val Cys Pro Tyr Asn Leu Tyr Ala Glu Gln Leu Ser Gly Ser Ala
        35                  40                  45

Phe Thr Cys Pro Arg Ser Thr Asn Lys Arg Ser Trp Leu Tyr Arg Ile
    50                  55                  60

Leu Pro Ser Val Ser His Lys Pro Phe Glu Ser Ile Asp Glu Gly His
65                  70                  75                  80

Val Thr His Asn Trp Asp Glu Val Asp Pro Asp Pro Asn Gln Leu Arg
                85                  90                  95

Trp Lys Pro Phe Glu Ile Pro Lys Ala Ser Gln Lys Lys Val Asp Phe
            100                 105                 110

Val Ser Gly Leu His Thr Leu Cys Gly Ala Gly Asp Ile Lys Ser Asn
        115                 120                 125

Asn Gly Leu Ala Ile His Ile Phe Leu Cys Asn Thr Ser Met Glu Asn
    130                 135                 140

Arg Cys Phe Tyr Asn Ser Asp Gly Asp Phe Leu Ile Val Pro Gln Lys
145                 150                 155                 160

Gly Asn Leu Leu Ile Tyr Thr Glu Phe Gly Lys Met Leu Val Gln Pro
                165                 170                 175

Asn Glu Ile Cys Val Ile Gln Arg Gly Met Arg Phe Ser Ile Asp Val
            180                 185                 190

Phe Glu Glu Thr Arg Gly Tyr Ile Leu Glu Val Tyr Gly Val His Phe
        195                 200                 205

Glu Leu Pro Asp Leu Gly Pro Ile Gly Ala Asn Gly Leu Ala Asn Pro
    210                 215                 220

Arg Asp Phe Leu Ile Pro Ile Ala Trp Tyr Glu Asp Arg Gln Val Pro
225                 230                 235                 240

Gly Gly Tyr Thr Val Ile Asn Lys Tyr Gln Gly Lys Leu Phe Ala Ala
                245                 250                 255

Lys Gln Asp Val Ser Pro Phe Asn Val Val Ala Trp His Gly Asn Tyr
            260                 265                 270

Thr Pro Tyr Lys Tyr Asn Leu Lys Asn Phe Met Val Ile Asn Ser Val
        275                 280                 285

Ala Phe Asp His Ala Asp Pro Ser Ile Phe Thr Val Leu Thr Ala Lys
    290                 295                 300

Ser Val Arg Pro Gly Val Ala Ile Ala Asp Phe Val Ile Phe Pro Pro
305                 310                 315                 320

Arg Trp Gly Val Ala Asp Lys Thr Phe Arg Pro Pro Tyr Tyr His Arg
                325                 330                 335

Asn Cys Met Ser Glu Phe Met Gly Leu Ile Arg Gly His Tyr Glu Ala
            340                 345                 350

Lys Gln Gly Gly Phe Leu Pro Gly Gly Gly Ser Leu His Ser Thr Met
        355                 360                 365

Thr Pro His Gly Pro Asp Ala Asp Cys Phe Glu Lys Ala Ser Lys Val
    370                 375                 380

Lys Leu Ala Pro Glu Arg Ile Ala Asp Gly Thr Met Ala Phe Met Phe
385                 390                 395                 400

Glu Ser Ser Leu Ser Leu Ala Val Thr Lys Trp Gly Leu Lys Ala Ser
                405                 410                 415

Arg Cys Leu Asp Glu Asn Tyr His Lys Cys Trp Glu Pro Leu Lys Ser
            420                 425                 430

His Phe Thr Pro Asn Ser Arg Asn Pro Ala Glu Pro Asn
        435                 440                 445

<210>  139

<211>  482

<212>  PRT

<213>  Homo sapiens

<300>

<308>  sw_hum/6pgd_human

<309>  1996-10-01

<400>  139

Ala Gln Ala Asp Ile Ala Leu Ile Gly Leu Ala Val Met Gly Gln Asn
1               5                   10                  15

Leu Ile Leu Asn Met Asn Asp His Gly Phe Val Val Cys Ala Phe Asn
            20                  25                  30

Arg Thr Val Ser Lys Val Asp Asp Phe Leu Ala Asn Glu Ala Lys Gly
        35                  40                  45

Thr Lys Val Val Gly Ala Gln Ser Leu Lys Glu Met Val Ser Lys Leu
    50                  55                  60

Lys Lys Pro Arg Arg Ile Ile Leu Leu Val Lys Ala Gly Gln Ala Val
65                  70                  75                  80

Asp Asp Phe Ile Glu Lys Leu Val Pro Leu Leu Asp Thr Gly Asp Ile
                85                  90                  95

Ile Ile Asp Gly Gly Asn Ser Glu Tyr Arg Asp Thr Thr Arg Arg Cys
            100                 105                 110

Arg Asp Leu Lys Ala Lys Gly Ile Leu Phe Val Gly Ser Gly Val Ser
        115                 120                 125

Gly Gly Glu Glu Gly Ala Arg Tyr Gly Pro Ser Leu Met Pro Gly Gly
    130                 135                 140

Asn Lys Glu Ala Trp Pro His Ile Lys Thr Ile Phe Gln Gly Ile Ala
145                 150                 155                 160

Ala Lys Val Gly Thr Gly Glu Pro Cys Cys Asp Trp Val Gly Asp Glu
                165                 170                 175

Gly Ala Gly His Phe Val Lys Met Val His Asn Gly Ile Glu Tyr Gly
            180                 185                 190

Asp Met Gln Leu Ile Cys Glu Ala Tyr His Leu Met Lys Asp Val Leu
        195                 200                 205

Gly Met Ala Gln Asp Glu Met Ala Gln Ala Phe Glu Asp Trp Asn Lys
    210                 215                 220

Thr Glu Leu Asp Ser Phe Leu Ile Glu Ile Thr Ala Asn Ile Leu Lys
225                 230                 235                 240

Phe Gln Asp Thr Asp Gly Lys His Leu Leu Pro Lys Ile Arg Asp Ser
                245                 250                 255

Ala Gly Gln Lys Gly Thr Gly Lys Trp Thr Ala Ile Ser Ala Leu Glu
            260                 265                 270

Tyr Gly Val Pro Val Thr Leu Ile Gly Glu Ala Val Phe Ala Arg Cys
        275                 280                 285

Leu Ser Ser Leu Lys Asp Glu Arg Ile Gln Ala Ser Lys Lys Leu Lys
    290                 295                 300

Gly Pro Gln Lys Phe Gln Phe Asp Gly Asp Lys Lys Ser Phe Leu Glu
305                 310                 315                 320

Asp Ile Arg Lys Ala Leu Tyr Ala Ser Lys Ile Ile Ser Tyr Ala Gln
                325                 330                 335

Gly Phe Met Leu Leu Arg Gln Ala Ala Thr Glu Phe Gly Trp Thr Leu
            340                 345                 350

Asn Tyr Gly Gly Ile Ala Leu Met Trp Arg Gly Gly Cys Ile Ile Arg
        355                 360                 365

Ser Val Phe Leu Gly Lys Ile Lys Asp Ala Phe Asp Arg Asn Pro Glu
    370                 375                 380

Leu Gln Asn Leu Leu Leu Asp Asp Phe Phe Lys Ser Ala Val Glu Asn
385                 390                 395                 400

Cys Gln Asp Ser Trp Arg Arg Ala Val Ser Thr Gly Val Gln Ala Gly
                405                 410                 415

Ile Pro Met Pro Cys Phe Thr Thr Ala Leu Ser Phe Tyr Asp Gly Tyr
            420                 425                 430

Arg His Glu Met Leu Pro Ala Ser Leu Ile Gln Ala Gln Arg Asp Tyr
        435                 440                 445

Phe Gly Ala His Thr Tyr Glu Leu Leu Ala Lys Pro Gly Gln Phe Ile
    450                 455                 460

His Thr Asn Trp Thr Gly His Gly Gly Thr Val Ser Ser Ser Ser Tyr
465                 470                 475                 480

Asn Ala

<210>  140

<211>  411

<212>  PRT

<213>  Homo sapiens

<300>

<308>  sw_hum/if4n_human

<309>  1995-02-01

<400>  140

Met Ala Thr Thr Ala Thr Met Ala Thr Ser Gly Ser Ala Arg Lys Arg
1               5                   10                  15

Leu Leu Lys Glu Glu Asp Met Thr Lys Val Glu Phe Glu Thr Ser Glu
            20                  25                  30

Glu Val Asp Val Thr Pro Thr Phe Asp Thr Met Gly Leu Arg Glu Asp
        35                  40                  45

Leu Leu Arg Gly Ile Tyr Ala Tyr Gly Phe Glu Lys Pro Ser Ala Ile
    50                  55                  60

Gln Gln Arg Ala Ile Lys Gln Ile Ile Lys Gly Arg Asp Val Ile Ala
65                  70                  75                  80

Gln Ser Gln Ser Gly Thr Gly Lys Thr Ala Thr Phe Ser Ile Ser Val
                85                  90                  95

Leu Gln Cys Leu Asp Ile Gln Val Arg Glu Thr Gln Ala Leu Ile Leu
            100                 105                 110

Ala Pro Thr Arg Glu Leu Ala Val Gln Ile Gln Lys Gly Leu Leu Ala
        115                 120                 125

Leu Gly Asp Tyr Met Asn Val Gln Cys His Ala Cys Ile Gly Gly Thr
    130                 135                 140

Asn Val Gly Glu Asp Ile Arg Lys Leu Asp Tyr Gly Gln His Val Val
145                 150                 155                 160

Ala Gly Thr Pro Gly Arg Val Phe Asp Met Ile Arg Arg Arg Ser Leu
                165                 170                 175

Arg Thr Arg Ala Ile Lys Met Leu Val Leu Asp Glu Ala Asp Glu Met
            180                 185                 190

Leu Asn Lys Gly Phe Lys Glu Gln Ile Tyr Asp Val Tyr Arg Tyr Leu
        195                 200                 205

Pro Pro Ala Thr Gln Val Val Leu Ile Ser Ala Thr Leu Pro His Glu
    210                 215                 220

Ile Leu Glu Met Thr Asn Lys Phe Met Thr Asp Pro Ile Arg Ile Leu
225                 230                 235                 240

Val Lys Arg Asp Glu Leu Thr Leu Glu Gly Ile Lys Gln Phe Phe Val
                245                 250                 255

Ala Val Glu Arg Glu Glu Trp Lys Phe Asp Thr Leu Cys Asp Leu Tyr
            260                 265                 270

Asp Thr Leu Thr Ile Thr Gln Ala Val Ile Phe Cys Asn Thr Lys Arg

275                 280                 285

Lys Val Asp Trp Leu Thr Glu Lys Met Arg Glu Ala Asn Phe Thr Val
    290                 295                 300

Ser Ser Met His Gly Asp Met Pro Gln Lys Glu Arg Glu Ser Ile Met
305                 310                 315                 320

Lys Glu Phe Arg Ser Gly Ala Ser Arg Val Leu Ile Ser Thr Asp Val
                325                 330                 335

Trp Ala Arg Gly Leu Asp Val Pro Gln Val Ser Leu Ile Ile Asn Tyr
            340                 345                 350

Asp Leu Pro Asn Asn Arg Glu Leu Tyr Ile His Arg Ile Gly Arg Ser
        355                 360                 365

Gly Arg Tyr Gly Arg Lys Gly Val Ala Ile Asn Phe Val Lys Asn Asp
    370                 375                 380

Asp Ile Arg Ile Leu Arg Asp Ile Glu Gln Tyr Tyr Ser Thr Gln Ile
385                 390                 395                 400

Asp Glu Met Pro Met Asn Val Ala Asp Leu Ile
                405                 410

<210>  141

<211>  453

<212>  PRT

<213>  Homo sapiens

<300>

<308>  sw_hum/ucr2_human

<309>  1991-08-01

<400>  141

<210>  142

<211>  245

<212>  PRT

<213>  Homo sapiens

<300>

<308>  s_hum/143z_human

<309>  1992-12-01

<400>  142

Met Asp Lys Asn Glu Leu Val Gln Lys Ala Lys Leu Ala Glu Gln Ala
1               5                   10                  15

Glu Arg Tyr Asp Asp Met Ala Ala Cys Met Lys Ser Val Thr Glu Gln
            20                  25                  30

Gly Ala Glu Leu Ser Asn Glu Glu Arg Asn Leu Leu Ser Val Ala Tyr
        35                  40                  45

Lys Asn Val Val Gly Ala Arg Arg Ser Ser Trp Arg Val Val Ser Ser
    50                  55                  60

Ile Glu Gln Lys Thr Glu Gly Ala Glu Lys Lys Gln Gln Met Ala Arg
65                  70                  75                  80

Glu Tyr Arg Glu Lys Ile Glu Thr Glu Leu Arg Asp Ile Cys Asn Asp
                85                  90                  95

Val Leu Ser Leu Leu Glu Lys Phe Leu Ile Pro Asn Ala Ser Gln Ala
            100                 105                 110

Glu Ser Lys Val Phe Tyr Leu Lys Met Lys Gly Asp Tyr Tyr Arg Tyr
        115                 120                 125

Leu Ala Glu Val Ala Ala Gly Asp Asp Lys Lys Gly Ile Val Asp Gln
    130                 135                 140

Ser Gln Gln Ala Tyr Gln Glu Ala Phe Glu Ile Ser Lys Lys Glu Met
145                 150                 155                 160

Gln Pro Thr His Pro Ile Arg Leu Gly Leu Ala Leu Asn Phe Ser Val
                165                 170                 175

Phe Tyr Tyr Glu Ile Leu Asn Ser Pro Glu Lys Ala Cys Ser Leu Ala
            180                 185                 190

Lys Thr Ala Phe Asp Glu Ala Ile Ala Glu Leu Asp Thr Leu Ser Glu
        195                 200                 205

Glu Ser Tyr Lys Asp Ser Thr Leu Ile Met Gln Leu Leu Arg Asp Asn
    210                 215                 220

Leu Thr Leu Trp Thr Ser Asp Thr Gln Gly Asp Glu Ala Glu Ala Gly
225                 230                 235                 240

Glu Gly Gly Glu Asn
                245

<210>  143

<211>  337

<212>  PRT

<213>  Homo sapiens

<300>

<308>  sw_hum/tal1_human

<309>  1994-10-01

<400>  143

<210>  144

<211>  284

<212>  PRT

<213>  Homo sapiens

<300>

<308>  sw_hum/nps1_human

<309>  2001-10-16

<400>  144
   Met Ala Pro Arg Leu Cys Ser Ile Ser Val Thr Ala Arg Arg Leu Leu
   1               5                   10                  15
   Gly Gly Pro Gly Pro Arg Ala Gly Asp Val Ala Ser Ala Ala Ala Ala
               20                  25                  30
   Arg Phe Tyr Ser Lys Asp Asn Glu Gly Ser Trp Phe Arg Ser Leu Phe
           35                  40                  45
   Val His Lys Val Asp Pro Arg Lys Asp Ala His Ser Thr Leu Leu Ser
       50                  55                  60
   Lys Lys Glu Thr Ser Asn Leu Tyr Lys Ile Gln Phe His Asn Val Lys
   65                  70                  75                  80
   Pro Glu Tyr Leu Asp Ala Tyr Asn Ser Leu Thr Glu Ala Val Leu Pro
                   85                  90                  95
   Lys Leu His Leu Asp Glu Asp Tyr Pro Cys Ser Leu Val Gly Asn Trp
               100                 105                 110
   Asn Thr Trp Tyr Gly Glu Gln Asp Gln Ala Val His Leu Trp Arg Phe
           115                 120                 125
   Ser Gly Gly Tyr Pro Ala Leu Met Asp Cys Met Asn Lys Leu Lys Asn
       130                 135                 140
   Asn Lys Glu Tyr Leu Glu Phe Arg Arg Glu Arg Ser Gln Met Leu Leu
   145                 150                 155                 160
   Ser Arg Arg Asn Gln Leu Leu Leu Glu Phe Ser Phe Trp Asn Glu Pro
                   165                 170                 175
   Gln Pro Arg Met Gly Pro Asn Ile Tyr Glu Leu Arg Thr Tyr Lys Leu
               180                 185                 190
   Lys Pro Gly Thr Met Ile Glu Trp Gly Asn Asn Trp Ala Arg Ala Ile
           195                 200                 205
   Lys Tyr Arg Gln Glu Asn Gln Glu Ala Val Gly Gly Phe Phe Ser Gln
       210                 215                 220
   Ile Gly Glu Leu Tyr Val Val His His Leu Trp Ala Tyr Lys Asp Leu
   225                 230                 235                 240
   Gln Ser Arg Glu Glu Thr Arg Asn Ala Ala Trp Arg Lys Arg Gly Trp
                   245                 250                 255
   Asp Glu Asn Val Tyr Tyr Thr Val Pro Leu Val Arg His Met Glu Ser
               260                 265                 270
   Arg Ile Met Ile Pro Leu Lys Ile Ser Pro Leu Gln
           275                 280

<210>  145

<211>  367

<212>  PRT

<213>  Homo sapiens

<300>

<308>  sw_hum/dcup_human

<309>  1988-01-01

<400>  145

<210>  146

<211>  430

<212>  PRT

<213>  Homo sapiens

<300>

<308>  sw_hum/aatm_human

<309>  1986-07-21

<400>  146

Met Ala Leu Leu His Ser Gly Arg Val Leu Pro Gly Ile Ala Ala Ala
1               5                   10                  15

Phe His Pro Gly Leu Ala Ala Ala Ala Ser Ala Arg Ala Ser Ser Trp
          20                  25                  30

Trp Thr His Val Glu Met Gly Pro Pro Asp Pro Ile Leu Gly Val Thr
      35                  40                  45

Glu Ala Phe Lys Arg Asp Thr Asn Ser Lys Lys Met Asn Leu Gly Val
    50                  55                  60

Gly Ala Tyr Arg Asp Asp Asn Gly Lys Pro Tyr Val Leu Pro Ser Val
65                  70                  75                  80

Arg Lys Ala Glu Ala Gln Ile Ala Ala Lys Asn Leu Asp Lys Glu Tyr
                85                  90                  95

Leu Pro Ile Gly Gly Leu Ala Glu Phe Cys Lys Ala Ser Ala Glu Leu
          100                 105                 110

Ala Leu Gly Glu Asn Ser Glu Val Leu Lys Ser Gly Arg Phe Val Thr
        115                 120                 125

Val Gln Thr Ile Ser Gly Thr Gly Ala Leu Arg Ile Gly Ala Ser Phe
    130                 135                 140

Leu Gln Arg Phe Phe Lys Phe Ser Arg Asp Val Phe Leu Pro Lys Pro
145                 150                 155                 160

Thr Trp Gly Asn His Thr Pro Ile Phe Arg Asp Ala Gly Met Gln Leu
                165                 170                 175

Gln Gly Tyr Arg Tyr Tyr Asp Pro Lys Thr Cys Gly Phe Asp Phe Thr
            180                 185                 190

Gly Ala Val Glu Asp Ile Ser Lys Ile Pro Glu Gln Ser Val Leu Leu
        195                 200                 205

Leu His Ala Cys Ala His Asn Pro Thr Gly Val Asp Pro Arg Pro Glu
    210                 215                 220

Gln Trp Lys Glu Ile Ala Thr Val Val Lys Lys Arg Asn Leu Phe Ala
225                 230                 235                 240

Phe Phe Asp Met Ala Tyr Gln Gly Phe Ala Ser Gly Asp Gly Asp Lys
                245                 250                 255

Asp Ala Trp Ala Val Arg His Phe Ile Glu Gln Gly Ile Asn Val Cys
            260                 265                 270

Leu Cys Gln Ser Tyr Ala Lys Asn Met Gly Leu Tyr Gly Glu Arg Val
        275                 280                 285

Gly Ala Phe Thr Met Val Cys Lys Asp Ala Asp Glu Ala Lys Arg Val
    290                 295                 300

Glu Ser Gln Leu Lys Ile Leu Ile Arg Pro Met Tyr Ser Asn Pro Pro
305                 310                 315                 320

Leu Asn Gly Ala Arg Ile Ala Ala Ala Ile Leu Asn Thr Pro Asp Leu
                325                 330                 335

Arg Lys Gln Trp Leu Gln Glu Val Lys Gly Met Ala Asp Arg Ile Ile
            340                 345                 350

Gly Met Arg Thr Gln Leu Val Ser Asn Leu Lys Lys Glu Gly Ser Thr
        355                 360                 365

His Asn Trp Gln His Ile Thr Asp Gln Ile Gly Met Phe Cys Phe Thr
    370                 375                 380

Gly Leu Lys Pro Glu Gln Val Glu Arg Leu Ile Lys Glu Phe Ser Ile
385                 390                 395                 400

Tyr Met Thr Lys Asp Gly Arg Ile Ser Val Ala Gly Val Thr Ser Ser
                405                 410                 415

Asn Val Gly Tyr Leu Ala His Ala Ile His Gln Val Thr Lys

420                    425                    430

<210>  147

<211>  233

<212>  PRT

<213>  Homo sapiens

<300>

<308>  sw_hum/psa2_human

<309>  1992-05-01

<400>  147

Ala Glu Arg Gly Tyr Ser Phe Ser Leu Thr Thr Phe Ser Pro Ser Gly
1               5                   10                  15

Lys Leu Val Gln Ile Glu Tyr Ala Leu Ala Ala Val Ala Gly Gly Ala
            20                  25                  30

Pro Ser Val Gly Ile Lys Ala Ala Asn Gly Val Val Leu Ala Thr Glu
        35                  40                  45

Lys Lys Gln Lys Ser Ile Leu Tyr Asp Glu Arg Ser Val His Lys Val
    50                  55                  60

Glu Pro Ile Thr Lys His Ile Gly Leu Val Tyr Ser Gly Met Gly Pro
65                  70                  75                  80

Asp Tyr Arg Val Leu Val His Arg Ala Arg Lys Leu Ala Gln Gln Tyr
                85                  90                  95

Tyr Leu Val Tyr Gln Glu Pro Ile Pro Thr Ala Gln Leu Val Gln Arg
            100                 105                 110

Val Ala Ser Val Met Gln Glu Tyr Thr Gln Ser Gly Gly Val Arg Pro
        115                 120                 125

Phe Gly Val Ser Leu Leu Ile Cys Gly Trp Asn Glu Gly Arg Pro Tyr
    130                 135                 140

Leu Phe Gln Ser Asp Pro Ser Gly Ala Tyr Phe Ala Trp Lys Ala Thr
145                 150                 155                 160

Ala Met Gly Lys Asn Tyr Val Asn Gly Lys Thr Phe Leu Glu Lys Arg
                165                 170                 175

Tyr Asn Glu Asp Leu Glu Leu Glu Asp Ala Ile His Thr Ala Ile Leu
            180                 185                 190

Thr Leu Lys Glu Ser Phe Glu Gly Gln Met Thr Glu Asp Asn Ile Glu
        195                 200                 205

Val Gly Ile Cys Asn Glu Ala Gly Phe Arg Arg Leu Thr Pro Thr Glu
    210                 215                 220

Val Lys Asp Tyr Leu Ala Ala Ile Ala
225                 230

<210>  148

<211>  530

<212>  PRT

<213>  Homo sapiens

<300>

<308>  sw_hum/kpy1_human

<309>  1990-04-01

<400>  148

Ser Lys Pro His Ser Glu Ala Gly Thr Ala Phe Ile Gln Thr Gln Gln
1               5                   10                  15

Leu His Ala Ala Met Ala Asp Thr Phe Leu Glu His Met Cys Arg Leu
            20                  25                  30

Asp Ile Asp Ser Pro Pro Ile Thr Ala Arg Asn Thr Gly Ile Ile Cys
        35                  40                  45

Thr Ile Gly Pro Ala Ser Arg Ser Val Glu Thr Leu Lys Glu Met Ile
    50                  55                  60

Lys Ser Gly Met Asn Val Ala Arg Leu Asn Phe Ser His Gly Thr His
65                  70                  75                  80

Glu Tyr His Ala Glu Thr Ile Lys Asn Val Arg Thr Ala Thr Glu Ser
                85                  90                  95

Phe Ala Ser Asp Pro Ile Leu Tyr Arg Pro Val Ala Val Ala Leu Asp
            100                 105                 110

Thr Lys Gly Pro Glu Ile Arg Thr Gly Leu Ile Lys Gly Ser Gly Thr
        115                 120                 125

Ala Glu Val Glu Leu Lys Lys Gly Ala Thr Leu Lys Ile Thr Leu Asp
    130                 135                 140

Asn Ala Tyr Met Glu Lys Cys Asp Glu Asn Ile Leu Trp Leu Asp Tyr
145                 150                 155                 160

Lys Asn Ile Cys Lys Val Val Glu Val Gly Ser Lys Ile Tyr Val Asp
                165                 170                 175

Asp Gly Leu Ile Ser Leu Gln Val Lys Gln Lys Gly Ala Asp Phe Leu
            180                 185                 190

Val Thr Glu Val Glu Asn Gly Gly Ser Leu Gly Ser Lys Lys Gly Val
        195                 200                 205

Asn Leu Pro Gly Ala Ala Val Asp Leu Pro Ala Val Ser Glu Lys Asp
    210                 215                 220

Ile Gln Asp Leu Lys Phe Gly Val Glu Gln Asp Val Asp Met Val Phe
225                 230                 235                 240

Ala Ser Phe Ile Arg Lys Ala Ser Asp Val His Glu Val Arg Lys Val
                245                 250                 255

Leu Gly Glu Lys Gly Lys Asn Ile Lys Ile Ile Ser Lys Ile Glu Asn
            260                 265                 270

His Glu Gly Val Arg Arg Phe Asp Glu Ile Leu Glu Ala Ser Asp Gly
        275                 280                 285

Ile Met Val Ala Arg Gly Asp Leu Gly Ile Glu Ile Pro Ala Glu Lys
    290                 295                 300

Val Phe Leu Ala Gln Lys Met Met Ile Gly Arg Cys Asn Arg Ala Gly
305                 310                 315                 320

Lys Pro Val Ile Cys Ala Thr Gln Met Leu Glu Ser Met Ile Lys Lys
                325                 330                 335

Pro Arg Pro Thr Arg Ala Glu Gly Ser Asp Val Ala Asn Ala Val Leu
            340                 345                 350

Asp Gly Ala Asp Cys Ile Met Leu Ser Gly Glu Thr Ala Lys Gly Asp
        355                 360                 365

Tyr Pro Leu Glu Ala Val Arg Met Gln His Leu Ile Ala Arg Glu Ala
    370                 375                 380

Glu Ala Ala Ile Tyr His Leu Gln Leu Phe Glu Glu Leu Arg Arg Leu
385                 390                 395                 400

Ala Pro Ile Thr Ser Asp Pro Thr Glu Ala Thr Ala Val Gly Ala Val
                405                 410                 415

Glu Ala Ser Phe Lys Cys Cys Ser Gly Ala Ile Ile Val Leu Thr Lys
            420                 425                 430

Ser Gly Arg Ser Ala His Gln Val Ala Arg Tyr Arg Pro Arg Ala Pro
        435                 440                 445

Ile Ile Ala Val Thr Arg Asn Pro Gln Thr Ala Arg Gln Ala His Leu
    450                 455                 460

Tyr Arg Gly Ile Phe Pro Val Leu Cys Lys Asp Pro Val Gln Glu Ala
465                 470                 475                 480

Trp Ala Glu Asp Val Asp Leu Arg Val Asn Phe Ala Met Asn Val Gly
                485                 490                 495

Lys Ala Arg Gly Phe Phe Lys Lys Gly Asp Val Val Ile Val Leu Thr
            500                 505                 510

Gly Trp Arg Pro Gly Ser Gly Phe Thr Asn Thr Met Arg Val Val Pro
        515                 520                 525

Val Pro
    530

<210>  149

<211>  412

<212>  PRT

<213>  Homo sapiens

<300>

<308>  sw_hum/aatc_human

<309>  1990-08-01

<400>  149

Ala Pro Pro Ser Val Phe Ala Glu Val Pro Gln Ala Gln Pro Val Leu
1               5                   10                  15

Val Phe Lys Leu Thr Ala Asp Phe Arg Glu Asp Pro Asp Pro Arg Lys
            20                  25                  30

Val Asn Leu Gly Val Gly Ala Tyr Arg Thr Asp Asp Cys His Pro Trp
        35                  40                  45

Val Leu Pro Val Val Lys Lys Val Glu Gln Lys Ile Ala Asn Asp Asn
    50                  55                  60

Ser Leu Asn His Glu Tyr Leu Pro Ile Leu Gly Leu Ala Glu Phe Arg
65                  70                  75                  80

Ser Cys Ala Ser Arg Leu Ala Leu Gly Asp Asp Ser Pro Ala Leu Lys
                85                  90                  95

Glu Lys Arg Val Gly Gly Val Gln Ser Leu Gly Gly Thr Gly Ala Leu
            100                 105                 110

Arg Ile Gly Ala Asp Phe Leu Ala Arg Trp Tyr Asn Gly Thr Asn Asn
        115                 120                 125

Lys Asn Thr Pro Val Tyr Val Ser Ser Pro Thr Trp Glu Asn His Asn
    130                 135                 140

Ala Val Phe Ser Ala Ala Gly Phe Lys Asp Ile Arg Ser Tyr Arg Tyr
145                 150                 155                 160

Trp Asp Ala Glu Lys Arg Gly Leu Asp Leu Gln Gly Phe Leu Asn Asp
                165                 170                 175

Leu Glu Asn Ala Pro Glu Phe Ser Ile Val Val Leu His Ala Cys Ala
            180                 185                 190

His Asn Pro Thr Gly Ile Asp Pro Thr Pro Glu Gln Trp Lys Gln Ile

195                     200                     205

Ala Ser Val Met Lys His Arg Phe Leu Phe Pro Phe Phe Asp Ser Ala
    210                     215                     220

Tyr Gln Gly Phe Ala Ser Gly Asn Leu Glu Arg Asp Ala Trp Ala Ile
225                     230                     235                     240

Arg Tyr Phe Val Ser Glu Gly Phe Glu Phe Phe Cys Ala Gln Ser Phe
                245                     250                     255

Ser Lys Asn Phe Gly Leu Tyr Asn Glu Arg Val Gly Asn Leu Thr Val
            260                     265                     270

Val Gly Lys Glu Pro Glu Ser Ile Leu Gln Val Leu Ser Gln Met Glu
        275                     280                     285

Lys Ile Val Arg Ile Thr Trp Ser Asn Pro Pro Ala Gln Gly Ala Arg
    290                     295                     300

Ile Val Ala Ser Thr Leu Ser Asn Pro Glu Leu Phe Glu Glu Trp Thr
305                     310                     315                     320

Gly Asn Val Lys Thr Met Ala Asp Arg Ile Leu Thr Met Arg Ser Glu
                325                     330                     335

Leu Arg Ala Arg Leu Glu Ala Leu Lys Thr Pro Gly Thr Trp Asn His
            340                     345                     350

Ile Thr Asp Gln Ile Gly Met Phe Ser Phe Thr Gly Leu Asn Pro Lys
        355                     360                     365

Gln Val Glu Tyr Leu Val Asn Glu Lys His Ile Tyr Leu Leu Pro Ser
    370                     375                     380

Gly Arg Ile Asn Val Ser Gly Leu Thr Thr Lys Asn Leu Asp Tyr Val
385                     390                     395                     400

Ala Thr Ser Ile His Glu Ala Val Thr Lys Ile Gln
                405                     410

<210>  150

<211>  553

<212>  PRT

<213>  Homo sapiens

<300>

<308>  sw_hum/atpa_human

<309>  1992-05-01

<400>  150

<210>  151

<211>  452

<212>  PRT

<213>  Homo sapiens


<300>

<308>  sw_hum/e2bg_human

<309>  2001-10-16

<400> 151

<210>  152

<211>  524

<212>  PRT

<213>  Homo sapiens

<300>

<308>  sw_hum/glpk_human

<309>  1993-10-01

<400>  152

Met Ala Ala Ser Lys Lys Ala Val Leu Gly Pro Leu Val Gly Ala Val
1               5                   10                  15

Asp Gln Gly Thr Ser Ser Thr Arg Phe Leu Val Phe Asn Ser Lys Thr
            20                  25                  30

Ala Glu Leu Leu Ser His His Gln Val Glu Ile Lys Gln Glu Phe Pro
        35                  40                  45

Arg Glu Gly Trp Val Glu Gln Asp Pro Lys Glu Ile Leu His Ser Val
    50                  55                  60

Tyr Glu Cys Ile Glu Lys Thr Cys Glu Lys Leu Gly Gln Leu Asn Ile
65                  70                  75                  80

Asp Ile Ser Asn Ile Lys Ala Ile Gly Val Ser Asn Gln Arg Glu Thr
                85                  90                  95

Thr Val Val Trp Asp Lys Ile Thr Gly Glu Pro Leu Tyr Asn Ala Val
            100                 105                 110

Val Trp Leu Asp Leu Arg Thr Gln Ser Thr Val Glu Ser Leu Ser Lys
        115                 120                 125

Arg Ile Pro Gly Asn Asn Asn Phe Val Lys Ser Lys Thr Gly Leu Pro
    130                 135                 140

Leu Ser Thr Tyr Phe Ser Ala Val Lys Leu Arg Trp Leu Leu Asp Asn
145                 150                 155                 160

Val Arg Lys Val Gln Lys Ala Val Glu Glu Lys Arg Ala Leu Phe Gly
                165                 170                 175

Thr Ile Asp Ser Trp Leu Ile Trp Ser Leu Thr Gly Gly Val Asn Gly
            180                 185                 190

Gly Val His Cys Thr Asp Val Thr Asn Ala Ser Arg Thr Met Leu Phe
        195                 200                 205

Asn Ile His Ser Leu Glu Trp Asp Lys Gln Leu Cys Glu Phe Phe Gly
    210                 215                 220

Ile Pro Met Glu Ile Leu Pro Asn Val Arg Ser Ser Ser Glu Ile Tyr
225                 230                 235                 240

Gly Leu Met Lys Ala Gly Ala Leu Glu Gly Val Pro Ile Ser Gly Cys
                245                 250                 255

Leu Gly Asp Gln Ser Ala Ala Leu Val Gly Gln Met Cys Phe Gln Ile
            260                 265                 270

Gly Gln Ala Lys Asn Thr Tyr Gly Thr Gly Cys Phe Leu Leu Cys Asn
        275                 280                 285

Thr Gly His Lys Cys Val Phe Ser Asp His Gly Leu Leu Thr Thr Val
    290                 295                 300

Ala Tyr Lys Leu Gly Arg Asp Lys Pro Val Tyr Tyr Ala Leu Glu Gly
305                 310                 315                 320

Ser Val Ala Ile Ala Gly Ala Val Ile Arg Trp Leu Arg Asp Asn Leu
                325                 330                 335

Gly Ile Ile Lys Thr Ser Glu Glu Ile Glu Lys Leu Ala Lys Glu Val
            340                 345                 350

Gly Thr Ser Tyr Gly Cys Tyr Phe Val Pro Ala Phe Ser Gly Leu Tyr
        355                 360                 365

Ala Pro Tyr Trp Glu Pro Ser Ala Arg Gly Ile Ile Cys Gly Leu Thr
    370                 375                 380

Gln Phe Thr Asn Lys Cys His Ile Ala Phe Ala Ala Leu Glu Ala Val
385                 390                 395                 400

Cys Phe Gln Thr Arg Glu Ile Leu Asp Ala Met Asn Arg Asp Cys Gly
                405                 410                 415

Ile Pro Leu Ser His Leu Gln Val Asp Gly Gly Met Thr Ser Asn Lys
            420                 425                 430

Ile Leu Met Gln Leu Gln Ala Asp Ile Leu Tyr Ile Pro Val Val Lys
        435                 440                 445

Pro Ser Met Pro Glu Thr Thr Ala Leu Gly Ala Ala Met Ala Ala Gly
    450                 455                 460

Ala Ala Glu Gly Val Gly Val Trp Ser Leu Glu Pro Glu Asp Leu Ser
465                 470                 475                 480

Ala Val Thr Met Glu Arg Phe Glu Pro Gln Ile Asn Ala Glu Glu Ser
                485                 490                 495

Glu Ile Arg Tyr Ser Thr Trp Lys Lys Ala Val Met Lys Ser Met Gly
            500                 505                 510

Trp Val Thr Thr Gln Ser Pro Glu Ser Gly Ile Pro
        515                 520

<210>  153

<211>  293

<212>  PRT

<213>  Homo sapiens


<300>

<308>  sw_hum/rs2_human

<309>  1990-04-01


<400>  153

<210>  154

<211>  508

<212>  PRT

<213>  Homo sapiens


<300>

<308>  sw_hum/syfa_human

<309>  2001-10-16

<400> 154

Met Ala Asp Gly Gln Val Ala Glu Leu Leu Leu Arg Arg Leu Glu Ala
1               5                   10                  15

Ser Asp Gly Gly Leu Asp Ser Ala Glu Leu Ala Ala Glu Leu Gly Met
            20                  25                  30

Glu His Gln Ala Val Val Gly Ala Val Lys Ser Leu Gln Ala Leu Gly
        35                  40                  45

Glu Val Ile Glu Ala Glu Leu Arg Ser Thr Lys His Trp Glu Leu Thr
    50                  55                  60

Ala Glu Gly Glu Glu Ile Ala Arg Glu Gly Ser His Glu Ala Arg Val
65                  70                  75                  80

Phe Arg Ser Ile Pro Pro Glu Gly Leu Ala Gln Ser Glu Leu Met Arg
                85                  90                  95

Leu Pro Ser Gly Lys Val Gly Phe Ser Lys Ala Met Ser Asn Lys Trp
            100                 105                 110

Ile Arg Val Asp Lys Ser Ala Ala Asp Gly Pro Arg Val Phe Arg Val
        115                 120                 125

Val Asp Ser Met Glu Asp Glu Val Gln Arg Arg Leu Gln Leu Val Arg
    130                 135                 140

Gly Gly Gln Ala Glu Lys Leu Gly Glu Lys Glu Arg Ser Glu Leu Arg
145                 150                 155                 160

Lys Arg Lys Leu Leu Ala Glu Val Thr Leu Lys Thr Tyr Trp Val Ser
                165                 170                 175

Lys Gly Ser Ala Phe Ser Thr Ser Ile Ser Lys Gln Glu Thr Glu Leu
            180                 185                 190

Ser Pro Glu Met Ile Ser Ser Gly Ser Trp Arg Asp Arg Pro Phe Lys
        195                 200                 205

Pro Tyr Asn Phe Leu Ala His Gly Val Leu Pro Asp Ser Gly His Leu
    210                 215                 220

His Pro Leu Leu Lys Val Arg Ser Gln Phe Arg Gln Ile Phe Leu Glu
225                 230                 235                 240

Met Gly Phe Thr Glu Met Pro Thr Asp Asn Phe Ile Glu Ser Ser Phe
                245                 250                 255

Trp Asn Phe Asp Ala Leu Phe Gln Pro Gln Gln His Pro Ala Arg Asp
            260                 265                 270

Gln His Asp Thr Phe Phe Leu Arg Asp Pro Ala Glu Ala Leu Gln Leu
        275                 280                 285

Pro Met Asp Tyr Val Gln Arg Val Lys Arg Thr His Ser Gln Gly Gly

290                 295                 300

Tyr Gly Ser Gln Gly Tyr Lys Tyr Asn Trp Lys Leu Asp Glu Ala Arg
305                 310                 315                 320

Lys Asn Leu Leu Arg Thr His Thr Thr Ser Ala Ser Ala Arg Ala Leu
                325                 330                 335

Tyr Arg Leu Ala Gln Lys Lys Pro Phe Thr Pro Val Lys Tyr Phe Ser
            340                 345                 350

Ile Asp Arg Val Phe Arg Asn Glu Thr Leu Asp Ala Thr His Leu Ala
        355                 360                 365

Glu Phe His Gln Ile Glu Gly Val Val Ala Asp His Gly Leu Thr Leu
    370                 375                 380

Gly His Leu Met Gly Val Leu Arg Glu Phe Phe Thr Lys Leu Gly Ile
385                 390                 395                 400

Thr Gln Leu Arg Phe Lys Pro Ala Tyr Asn Pro Tyr Thr Glu Pro Ser
                405                 410                 415

Met Glu Val Phe Ser Tyr His Gln Gly Leu Lys Lys Trp Val Glu Val
            420                 425                 430

Gly Asn Ser Gly Val Phe Arg Pro Glu Met Leu Leu Pro Met Gly Leu
        435                 440                 445

Pro Glu Asn Val Ser Val Ile Ala Trp Gly Leu Ser Leu Glu Arg Pro
    450                 455                 460

Thr Met Ile Lys Tyr Gly Ile Asn Asn Ile Arg Glu Leu Val Gly His
465                 470                 475                 480

Lys Val Asn Leu Gln Met Val Tyr Asp Ser Pro Leu Cys Arg Leu Asp
                485                 490                 495

Ala Glu Pro Arg Pro Pro Pro Thr Gln Glu Ala Ala
            500                 505

<210>  155

<211>  397

<212>  PRT

<213>  Homo sapiens

<300>

<308>  sw_hum/dja1_human

<309>  1993-07-01

<400>  155

Met Val Lys Glu Thr Thr Tyr Tyr Asp Val Leu Gly Val Lys Pro Asn
1               5                   10                  15

Ala Thr Gln Glu Glu Leu Lys Lys Ala Tyr Arg Lys Leu Ala Leu Lys
            20                  25                  30

Tyr His Pro Asp Lys Asn Pro Asn Glu Gly Glu Lys Phe Lys Gln Ile
        35                  40                  45

Ser Gln Ala Tyr Glu Val Leu Ser Asp Ala Lys Lys Arg Glu Leu Tyr
    50                  55                  60

Asp Lys Gly Gly Glu Gln Ala Ile Lys Glu Gly Gly Ala Gly Gly Gly
65                  70                  75                  80

Phe Gly Ser Pro Met Asp Ile Phe Asp Met Phe Phe Gly Gly Gly Gly
                85                  90                  95

Arg Met Gln Arg Glu Arg Arg Gly Lys Asn Val Val His Gln Leu Ser
            100                 105                 110

Val Thr Leu Glu Asp Leu Tyr Asn Gly Ala Thr Arg Lys Leu Ala Leu
        115                 120                 125

Gln Lys Asn Val Ile Cys Asp Lys Cys Glu Gly Arg Gly Gly Lys Lys
    130                 135                 140

Gly Ala Val Glu Cys Cys Pro Asn Cys Arg Gly Thr Gly Met Gln Ile
145                 150                 155                 160

Arg Ile His Gln Ile Gly Pro Gly Met Val Gln Gln Ile Gln Ser Val
                165                 170                 175

Cys Met Glu Cys Gln Gly His Gly Glu Arg Ile Ser Pro Lys Asp Arg
            180                 185                 190

Cys Lys Ser Cys Asn Gly Arg Lys Ile Val Arg Glu Lys Lys Ile Leu
        195                 200                 205

Glu Val His Ile Asp Lys Gly Met Lys Asp Gly Gln Lys Ile Thr Phe
    210                 215                 220

His Gly Glu Gly Asp Gln Glu Pro Gly Leu Glu Pro Gly Asp Ile Ile
225                 230                 235                 240

Ile Val Leu Asp Gln Lys Asp His Ala Val Phe Thr Arg Arg Gly Glu
                245                 250                 255

Asp Leu Phe Met Cys Met Asp Ile Gln Leu Val Glu Ala Leu Cys Gly
            260                 265                 270

Phe Gln Lys Pro Ile Ser Thr Leu Asp Asn Arg Thr Ile Val Ile Thr
        275                 280                 285

Ser His Pro Gly Gln Ile Val Lys His Gly Asp Ile Lys Cys Val Leu
    290                 295                 300

Asn Glu Gly Met Pro Ile Tyr Arg Arg Pro Tyr Glu Lys Gly Arg Leu
305                 310                 315                 320

Ile Ile Glu Phe Lys Val Asn Phe Pro Glu Asn Gly Phe Leu Ser Pro
                325                 330                 335

Asp Lys Leu Ser Leu Leu Glu Lys Leu Leu Pro Glu Arg Lys Glu Val
            340                 345                 350

Glu Glu Thr Asp Glu Met Asp Gln Val Glu Leu Val Asp Phe Asp Pro
        355                 360                 365

Asn Gln Glu Arg Arg Arg His Tyr Asn Gly Glu Ala Tyr Glu Asp Asp
    370                 375                 380

Glu His His Pro Arg Gly Gly Val Gln Cys Gln Thr Ser
385                 390                 395

<210>  156

<211>  518

<212>  PRT

<213>  Homo sapiens

<300>

<308>  sw_hum/sysm_human

<309>  2003-02-28

<400>  156

<210>  157

<211>  279

<212>  PRT

<213>  Homo sapiens

<300>

<308>  sw_hum/hax1_human

<309>  1998-07-15

<400>  157

Met Ser Leu Phe Asp Leu Phe Arg Gly Phe Phe Gly Phe Pro Gly Pro
1               5                   10                  15

Arg Ser His Arg Asp Pro Phe Phe Gly Gly Met Thr Arg Asp Glu Asp
            20                  25                  30

Asp Asp Glu Glu Glu Glu Glu Glu Gly Gly Ser Trp Gly Arg Gly Asn
        35                  40                  45

Pro Arg Phe His Ser Pro Gln His Pro Pro Glu Glu Phe Gly Phe Gly
    50                  55                  60

Phe Ser Phe Ser Pro Gly Gly Gly Ile Arg Phe His Asp Asn Phe Gly
65                  70                  75                  80

Phe Asp Asp Leu Val Arg Asp Phe Asn Ser Ile Phe Ser Asp Met Gly
                85                  90                  95

Ala Trp Thr Leu Pro Ser His Pro Pro Glu Leu Pro Gly Pro Glu Ser
            100                 105                 110

Glu Thr Pro Gly Glu Arg Leu Arg Glu Gly Gln Thr Leu Arg Asp Ser
        115                 120                 125

Met Leu Lys Tyr Pro Asp Ser His Gln Pro Arg Ile Phe Gly Gly Val
    130                 135                 140

Leu Glu Ser Asp Ala Arg Ser Glu Ser Pro Gln Pro Ala Pro Asp Trp
145                 150                 155                 160

Gly Ser Gln Arg Pro Phe His Arg Phe Asp Asp Val Trp Pro Met Asp
                165                 170                 175

Pro His Pro Arg Thr Arg Glu Asp Asn Asp Leu Asp Ser Gln Val Ser
            180                 185                 190

Gln Glu Gly Leu Gly Pro Val Leu Gln Pro Gln Pro Lys Ser Tyr Phe
        195                 200                 205

Lys Ser Ile Ser Val Thr Lys Ile Thr Lys Pro Asp Gly Ile Val Glu
    210                 215                 220

Glu Arg Arg Thr Val Val Asp Ser Glu Gly Arg Thr Glu Thr Thr Val
225                 230                 235                 240

Thr Arg His Glu Ala Asp Ser Ser Pro Arg Gly Asp Pro Glu Ser Pro
                245                 250                 255

Arg Pro Pro Ala Leu Asp Asp Ala Phe Ser Ile Leu Asp Leu Phe Leu
            260                 265                 270

Gly Arg Trp Phe Arg Ser Arg
        275

<210>  158

<211>  476

<212>  PRT

<213>  Homo sapiens


<300>

<308>  humangp/chr19-q96g16

<309>  2003-04-22


<400>  158

Met Val Ser Lys Arg Ile Ala Gln Glu Thr Phe Asp Ala Ala Val Arg
1               5                   10                  15

Glu Asn Ile Glu Glu Phe Ala Met Gly Pro Glu Glu Ala Val Lys Glu
            20                  25                  30

Ala Val Glu Gln Phe Glu Ser Gln Gly Val Asp Leu Ser Asn Ile Val
        35                  40                  45

Lys Thr Ala Pro Lys Val Ser Ala Asp Gly Ser Gln Glu Pro Thr His
    50                  55                  60

Asp Ile Leu Gln Met Leu Ser Asp Leu Gln Glu Ser Val Ala Ser Ser
65                  70                  75                  80

Arg Pro Gln Glu Val Ser Ala Tyr Leu Thr Arg Phe Cys Asp Gln Cys
                85                  90                  95

Lys Gln Asp Lys Ala Cys Arg Phe Leu Ala Ala Gln Lys Gly Ala Tyr
            100                 105                 110

Pro Ile Ile Phe Thr Ala Trp Lys Leu Ala Thr Ala Gly Asp Gln Gly
        115                 120                 125

Leu Leu Leu Gln Ser Leu Asn Ala Leu Ser Val Leu Thr Asp Gly Gln
    130                 135                 140

Pro Asp Leu Leu Asp Ala Gln Gly Leu Gln Leu Leu Val Ala Thr Leu
145                 150                 155                 160

Thr Gln Asn Ala Asp Glu Ala Asp Leu Thr Cys Ser Gly Ile Arg Cys
                165                 170                 175

Val Arg His Ala Cys Leu Lys His Glu Gln Asn Arg Gln Asp Leu Val
            180                 185                 190

Lys Ala Gly Val Leu Pro Leu Leu Thr Gly Ala Ile Thr His His Gly
        195                 200                 205

His His Thr Asp Val Val Arg Glu Ala Cys Trp Ala Leu Arg Val Met
    210                 215                 220

Thr Phe Asp Asp Asp Ile Arg Val Pro Phe Gly His Ala His Asn His
225                 230                 235                 240

Ala Lys Met Ile Val Gln Glu Asn Lys Gly Leu Lys Val Leu Ile Glu
                245                 250                 255

Ala Thr Lys Ala Phe Leu Asp Asn Pro Gly Ile Leu Ser Glu Leu Cys
            260                 265                 270

Gly Thr Leu Ser Arg Leu Ala Ile Arg Asn Glu Phe Cys Gln Glu Val
        275                 280                 285

Val Asp Leu Gly Gly Leu Ser Ile Leu Val Ser Leu Leu Ala Asp Cys
    290                 295                 300

Asn Asp His Gln Met Arg Asp Gln Ser Gly Val Gln Glu Leu Val Lys
305                 310                 315                 320

Gln Val Leu Ser Thr Leu Arg Ala Ile Ala Gly Asn Asp Asp Val Lys
                325                 330                 335

Asp Ala Ile Val Arg Ala Gly Gly Thr Glu Ser Ile Val Ala Ala Met
            340                 345                 350

Thr Gln His Leu Thr Ser Pro Gln Val Cys Glu Gln Ser Cys Ala Ala
        355                 360                 365

Leu Cys Phe Leu Ala Leu Arg Lys Pro Asp Asn Ser Arg Ile Ile Val
    370                 375                 380

Glu Gly Gly Gly Ala Val Ala Ala Leu Gln Ala Met Lys Ala His Pro
385                 390                 395                 400

Gln Lys Ala Gly Val Gln Lys Gln Ala Cys Met Leu Ile Arg Asn Leu
                405                 410                 415

Val Ala His Gly Gln Ala Phe Ser Lys Pro Ile Leu Asp Leu Gly Ala
            420                 425                 430

Glu Ala Leu Ile Met Gln Ala Arg Ser Ala His Arg Asp Cys Glu Asp
        435                 440                 445

Val Ala Lys Ala Ala Leu Arg Asp Leu Gly Cys His Val Glu Leu Arg
    450                 455                 460

Glu Leu Trp Thr Gly Gln Arg Gly Asn Leu Ala Pro
465                 470                 475

<210> 159

<211> 319

<212> PRT

<213> Homo sapiens

<300>

<308> human/chr10-q9hbk9

<309> 2003-04-22

<400> 159

Gln Thr Tyr Tyr Gly Gln Val Leu Lys Arg Ser Ala Asp Leu Gln Thr
1               5                   10                  15

Asn Gly Cys Val Thr Thr Ala Arg Pro Val Pro Lys His Ile Arg Glu
            20                  25                  30

Ala Leu Gln Asn Val His Glu Glu Val Ala Leu Arg Tyr Tyr Gly Cys
        35                  40                  45

Gly Leu Val Ile Pro Glu His Leu Glu Asn Cys Trp Ile Leu Asp Leu
    50                  55                  60

Gly Ser Gly Ser Gly Arg Asp Cys Tyr Val Leu Ser Gln Leu Val Gly
65                  70                  75                  80

Glu Lys Gly His Val Thr Gly Ile Asp Met Thr Lys Gly Gln Val Glu
                85                  90                  95

Val Ala Glu Lys Tyr Leu Asp Tyr His Met Glu Lys Tyr Gly Phe Gln
            100                 105                 110

Ala Ser Asn Val Thr Phe Ile His Gly Tyr Ile Glu Lys Leu Gly Glu
        115                 120                 125

Ala Gly Ile Lys Asn Glu Ser His Asp Ile Val Val Ser Asn Cys Val
    130                 135                 140

Ile Asn Leu Val Pro Asp Lys Gln Gln Val Leu Gln Glu Ala Tyr Arg

145                 150                 155                 160

Val Leu Lys His Gly Gly Glu Leu Tyr Phe Ser Asp Val Tyr Thr Ser
                165                 170                 175

Leu Glu Leu Pro Glu Glu Ile Arg Thr His Lys Val Leu Trp Gly Glu
            180                 185                 190

Cys Leu Gly Gly Ala Leu Tyr Trp Lys Glu Leu Ala Val Leu Ala Gln
        195                 200                 205

Lys Ile Gly Phe Cys Pro Pro Arg Leu Val Thr Ala Asn Leu Ile Thr
    210                 215                 220

Ile Gln Asn Lys Glu Leu Glu Arg Val Ile Gly Asp Cys Arg Phe Val
225                 230                 235                 240

Ser Ala Thr Phe Arg Leu Phe Lys His Ser Lys Thr Gly Pro Thr Lys
                245                 250                 255

Arg Cys Gln Val Ile Tyr Asn Gly Gly Ile Thr Gly His Glu Lys Glu
            260                 265                 270

Leu Met Phe Asp Ala Asn Phe Thr Phe Lys Glu Gly Glu Ile Val Glu
        275                 280                 285

Val Asp Glu Glu Thr Ala Ala Ile Leu Lys Asn Ser Arg Phe Ala Gln
    290                 295                 300

Asp Phe Leu Ile Arg Pro Ile Gly Glu Lys Leu Pro Thr Ser Gly
305                 310                 315

<210>  160

<211>  392

<212>  PRT

<213>  Homo sapiens

<300>

<308>  sw_hum/sps1_human

<309>  1996-10-01

<400>  160

Met Ser Thr Arg Glu Ser Phe Asn Pro Glu Ser Tyr Glu Leu Asp Lys
1               5                   10                  15

Ser Phe Arg Leu Thr Arg Phe Thr Glu Leu Lys Gly Thr Gly Cys Lys
            20                  25                  30

Val Pro Gln Asp Val Leu Gln Lys Leu Leu Glu Ser Leu Gln Glu Asn
        35                  40                  45

His Phe Gln Glu Asp Glu Gln Phe Leu Gly Ala Val Met Pro Arg Leu
    50                  55                  60

Gly Ile Gly Met Asp Thr Cys Val Ile Pro Leu Arg His Gly Gly Leu
65                  70                  75                  80

Ser Leu Val Gln Thr Thr Asp Tyr Ile Tyr Pro Ile Val Asp Asp Pro
                85                  90                  95

Tyr Met Met Gly Arg Ile Ala Cys Ala Asn Val Leu Ser Asp Leu Tyr
            100                 105                 110

Ala Met Gly Val Thr Glu Cys Asp Asn Met Leu Met Leu Leu Gly Val
        115                 120                 125

Ser Asn Lys Met Thr Asp Arg Glu Arg Asp Lys Val Met Pro Leu Ile
    130                 135                 140

Ile Gln Gly Phe Lys Asp Ala Ala Glu Glu Ala Gly Thr Ser Val Thr
145                 150                 155                 160

Gly Gly Gln Thr Val Leu Asn Pro Trp Ile Val Leu Gly Gly Val Ala
                165                 170                 175

Thr Thr Val Cys Gln Pro Asn Glu Phe Ile Met Pro Asp Asn Ala Val
            180                 185                 190

Pro Gly Asp Val Leu Val Leu Thr Lys Pro Leu Gly Thr Gln Val Ala
        195                 200                 205

Val Ala Val His Gln Trp Leu Asp Ile Pro Glu Lys Trp Asn Lys Ile
    210                 215                 220

Lys Leu Val Val Thr Gln Glu Asp Val Glu Leu Ala Tyr Gln Glu Ala
225                 230                 235                 240

Met Met Asn Met Ala Arg Leu Asn Arg Thr Ala Ala Gly Leu Met His
                245                 250                 255

Thr Phe Asn Ala His Ala Ala Thr Asp Ile Thr Gly Phe Gly Ile Leu
            260                 265                 270

Gly His Ala Gln Asn Leu Ala Lys Gln Gln Arg Asn Glu Val Ser Phe
        275                 280                 285

Val Ile His Asn Leu Pro Val Leu Ala Lys Met Ala Ala Val Ser Lys
    290                 295                 300

Ala Cys Gly Asn Met Phe Gly Leu Met His Gly Thr Cys Pro Glu Thr
305                 310                 315                 320

Ser Gly Gly Leu Leu Ile Cys Leu Pro Arg Glu Gln Ala Ala Arg Phe
                325                 330                 335

Cys Ala Glu Ile Lys Ser Pro Lys Tyr Gly Glu Gly His Gln Ala Trp
            340                 345                 350

Ile Ile Gly Ile Val Glu Lys Gly Asn Arg Thr Ala Arg Ile Ile Asp
        355                 360                 365

Lys Pro Arg Ile Ile Glu Val Ala Pro Gln Val Ala Thr Gln Asn Val
    370                 375                 380

Asn Pro Thr Pro Gly Ala Thr Ser
385                 390

<210>  161

<211>  814

<212>  PRT

<213>  Homo sapiens

<300>

<308>  sw_hum/if39_human

<309>  1997-11-01

<400>  161

Met Gln Asp Ala Glu Asn Val Ala Val Pro Glu Ala Ala Glu Glu Arg
1               5                   10                  15

Ala Glu Pro Gly Gln Gln Gln Pro Ala Ala Glu Pro Pro Pro Ala Glu
            20                  25                  30

Gly Leu Leu Arg Pro Ala Gly Pro Gly Ala Pro Glu Ala Ala Gly Thr
        35                  40                  45

Glu Ala Ser Ser Glu Glu Val Gly Ile Ala Glu Ala Gly Pro Glu Pro
    50                  55                  60

Glu Val Arg Thr Glu Pro Ala Ala Glu Ala Glu Ala Ala Ser Gly Pro
65                  70                  75                  80

Ser Glu Ser Pro Ser Pro Pro Ala Ala Glu Glu Leu Pro Gly Ser His
                85                  90                  95

Ala Glu Pro Pro Val Pro Ala Gln Gly Glu Ala Pro Gly Glu Gln Ala
            100                 105                 110

Arg Asp Glu Arg Ser Asp Ser Arg Ala Gln Ala Val Ser Glu Asp Ala
        115                 120                 125

Gly Gly Asn Glu Gly Arg Ala Ala Glu Ala Glu Pro Arg Ala Leu Glu
    130                 135                 140

Asn Gly Asp Ala Asp Glu Pro Ser Phe Ser Asp Pro Glu Asp Phe Val
145                 150                 155                 160

Asp Asp Val Ser Glu Glu Glu Leu Leu Gly Asp Val Leu Lys Asp Arg
                165                 170                 175

Pro Gln Glu Ala Asp Gly Ile Asp Ser Val Ile Val Val Asp Asn Val
            180                 185                 190

Pro Gln Val Gly Pro Asp Arg Leu Glu Lys Leu Lys Asn Val Ile His
        195                 200                 205

Lys Ile Phe Ser Lys Phe Gly Lys Ile Thr Asn Asp Phe Tyr Pro Glu
    210                 215                 220

Glu Asp Gly Lys Thr Lys Gly Tyr Ile Phe Leu Glu Tyr Ala Ser Pro
225                 230                 235                 240

Ala His Ala Val Asp Ala Val Lys Asn Ala Asp Gly Tyr Lys Leu Asp
                245                 250                 255

Lys Gln His Thr Phe Arg Val Asn Leu Phe Thr Asp Phe Asp Lys Tyr
            260                 265                 270

Met Thr Ile Ser Asp Glu Trp Asp Ile Pro Glu Lys Gln Pro Phe Lys
        275                 280                 285

Asp Leu Gly Asn Leu Arg Tyr Trp Leu Glu Glu Ala Glu Cys Arg Asp
    290                 295                 300

Gln Tyr Ser Val Ile Phe Glu Ser Gly Asp Arg Thr Ser Ile Phe Trp
305                 310                 315                 320

Asn Asp Val Lys Asp Pro Val Ser Ile Glu Glu Arg Ala Arg Trp Thr
                325                 330                 335

Glu Thr Tyr Val Arg Trp Ser Pro Lys Gly Thr Tyr Leu Ala Thr Phe
            340                 345                 350

His Gln Arg Gly Ile Ala Leu Trp Gly Gly Glu Lys Phe Lys Gln Ile
        355                 360                 365

Gln Arg Phe Ser His Gln Gly Val Gln Leu Ile Asp Phe Ser Pro Cys
    370                 375                 380

Glu Arg Tyr Leu Val Thr Phe Ser Pro Leu Met Asp Thr Gln Asp Asp
385                 390                 395                 400

Pro Gln Ala Ile Ile Ile Trp Asp Ile Leu Thr Gly His Lys Lys Arg
                405                 410                 415

Gly Phe His Cys Glu Ser Ser Ala His Trp Pro Ile Phe Lys Trp Ser
            420                 425                 430

His Asp Gly Lys Phe Phe Ala Arg Met Thr Leu Asp Thr Leu Ser Ile
        435                 440                 445

Tyr Glu Thr Pro Ser Met Gly Leu Leu Asp Lys Lys Ser Leu Lys Ile
    450                 455                 460

Ser Gly Ile Lys Asp Phe Ser Trp Ser Pro Gly Gly Asn Ile Ile Ala
465                 470                 475                 480

Phe Trp Val Pro Glu Asp Lys Asp Ile Pro Ala Arg Val Thr Leu Met
                485                 490                 495

Gln Leu Pro Thr Arg Gln Glu Ile Arg Val Arg Asn Leu Phe Asn Val

500                 505                 510

Val Asp Cys Lys Leu His Trp Gln Lys Asn Gly Asp Tyr Leu Cys Val
        515                 520                 525

Lys Val Asp Arg Thr Pro Lys Gly Thr Gln Gly Val Val Thr Asn Phe
    530                 535                 540

Glu Ile Phe Arg Met Arg Glu Lys Gln Val Pro Val Asp Val Val Glu
545                 550                 555                 560

Met Lys Glu Thr Ile Ile Ala Phe Ala Trp Glu Pro Asn Gly Ser Lys
                565                 570                 575

Phe Ala Val Leu His Gly Glu Ala Pro Arg Ile Ser Val Ser Phe Tyr
            580                 585                 590

His Val Lys Asn Asn Gly Lys Ile Glu Leu Ile Lys Met Phe Asp Lys
        595                 600                 605

Gln Gln Ala Asn Thr Ile Phe Trp Ser Pro Gln Gly Gln Phe Val Val
    610                 615                 620

Leu Ala Gly Leu Arg Ser Met Asn Gly Ala Leu Ala Phe Val Asp Thr
625                 630                 635                 640

Ser Asp Cys Thr Val Met Asn Ile Ala Glu His Tyr Met Ala Ser Asp
                645                 650                 655

Val Glu Trp Asp Pro Thr Gly Arg Tyr Val Val Thr Ser Val Ser Trp
            660                 665                 670

Trp Ser His Lys Val Asp Asn Ala Tyr Trp Leu Trp Thr Phe Gln Gly
        675                 680                 685

Arg Leu Leu Gln Lys Asn Asn Lys Asp Arg Phe Cys Gln Leu Leu Trp
    690                 695                 700

Arg Pro Arg Pro Pro Thr Leu Leu Ser Gln Glu Gln Ile Lys Gln Ile
705                 710                 715                 720

Lys Lys Asp Leu Lys Lys Tyr Ser Lys Ile Phe Glu Gln Lys Asp Arg
                725                 730                 735

Leu Ser Gln Ser Lys Ala Ser Lys Glu Leu Val Glu Arg Arg Arg Thr
            740                 745                 750

Met Met Glu Asp Phe Arg Lys Tyr Arg Lys Met Ala Gln Glu Leu Tyr
        755                 760                 765

Met Glu Gln Lys Asn Glu Arg Leu Glu Leu Arg Gly Gly Val Asp Thr
    770                 775                 780

Asp Glu Leu Asp Ser Asn Val Asp Asp Trp Glu Glu Glu Thr Ile Glu
785                 790                 795                 800

Phe Phe Val Thr Glu Glu Ile Ile Pro Leu Gly Asn Gln Glu
                805                 810

<210>  162

<211>  461

<212>  PRT

<213>  Homo sapiens


<300>

<308>  sw_hum/ncb1_human

<309>  1993-07-01


<400>  162

Met Pro Pro Ser Gly Pro Arg Gly Thr Leu Leu Leu Leu Pro Leu Leu
1               5                   10                  15

Leu Leu Leu Leu Leu Arg Ala Val Leu Ala Val Pro Leu Glu Arg Gly
            20                  25                  30

Ala Pro Asn Lys Glu Glu Thr Pro Ala Thr Glu Ser Pro Asp Thr Gly
        35                  40                  45

Leu Tyr Tyr His Arg Tyr Leu Gln Glu Val Ile Asp Val Leu Glu Thr
    50                  55                  60

Asp Gly His Phe Arg Glu Lys Leu Gln Ala Ala Asn Ala Glu Asp Ile
65                  70                  75                  80

Lys Ser Gly Lys Leu Ser Arg Glu Leu Asp Phe Val Ser His His Val
                85                  90                  95

Arg Thr Lys Leu Asp Glu Leu Lys Arg Gln Glu Val Ser Arg Leu Arg
            100                 105                 110

Met Leu Leu Lys Ala Lys Met Asp Ala Glu Gln Asp Pro Asn Val Gln
        115                 120                 125

Val Asp His Leu Asn Leu Leu Lys Gln Phe Glu His Leu Asp Pro Gln
    130                 135                 140

Asn Gln His Thr Phe Glu Ala Arg Asp Leu Glu Leu Leu Ile Gln Thr
145                 150                 155                 160

Ala Thr Arg Asp Leu Ala Gln Tyr Asp Ala Ala His His Glu Glu Phe
                165                 170                 175

Lys Arg Tyr Glu Met Leu Lys Glu His Glu Arg Arg Arg Tyr Leu Glu
            180                 185                 190

Ser Leu Gly Glu Glu Gln Arg Lys Glu Ala Glu Arg Lys Leu Glu Glu
        195                 200                 205

Gln Gln Arg Arg His Arg Glu His Pro Lys Val Asn Val Pro Gly Ser
    210                 215                 220

Gln Ala Gln Leu Lys Glu Val Trp Glu Glu Leu Asp Gly Leu Asp Pro
225                 230                 235                 240

Asn Arg Phe Asn Pro Lys Thr Phe Phe Ile Leu His Asp Ile Asn Ser
                245                 250                 255

Asp Gly Val Leu Asp Glu Gln Glu Leu Glu Ala Leu Phe Thr Lys Glu
            260                 265                 270

Leu Glu Lys Val Tyr Asp Pro Lys Asn Glu Glu Asp Asp Met Arg Glu
        275                 280                 285

Met Glu Glu Glu Arg Leu Arg Met Arg Glu His Val Met Lys Asn Val
    290                 295                 300

Asp Thr Asn Gln Asp Arg Leu Val Thr Leu Glu Glu Phe Leu Ala Ser
305                 310                 315                 320

Thr Gln Arg Lys Glu Phe Gly Asp Thr Gly Glu Gly Trp Glu Thr Val
                325                 330                 335

Glu Met His Pro Ala Tyr Thr Glu Glu Glu Leu Arg Arg Phe Glu Glu
            340                 345                 350

Glu Leu Ala Ala Arg Glu Ala Glu Leu Asn Ala Lys Ala Gln Arg Leu
        355                 360                 365

Ser Gln Glu Thr Glu Ala Leu Gly Arg Ser Gln Gly Arg Leu Glu Ala
    370                 375                 380

Lys Lys Arg Glu Leu Leu Leu Ala Val Leu His Met Glu Gln Gln Lys
385                 390                 395                 400

Gln Gln Gln Gln Gln Gln Gln Gly His Lys Ala Pro Ala Ala His Pro
                405                 410                 415

Glu Gly Gln Leu Lys Phe His Pro Asp Thr Asp Asp Val Pro Val Pro
            420                 425                 430

Ala Pro Ala Gly Asp Gln Lys Glu Val Asp Thr Ser Glu Lys Lys Leu
        435                 440                 445

Leu Glu Arg Leu Pro Glu Val Glu Val Pro Gln His Leu
    450                 455                 460

<210>  163

<211>  297

<212>  PRT

<213>  Homo sapiens


<300>

<308>  sw_hum/y33k_human

<309>  1996-10-01

<400> 163

Met Ala Glu Leu Thr Ala Leu Glu Ser Leu Ile Glu Met Gly Phe Pro
1               5                   10                  15

Arg Gly Arg Ala Glu Lys Ala Leu Ala Leu Thr Gly Asn Gln Gly Ile
            20                  25                  30

Glu Ala Ala Met Asp Trp Leu Met Glu His Glu Asp Asp Pro Asp Val
        35                  40                  45

Asp Glu Pro Leu Glu Thr Pro Leu Gly His Ile Leu Gly Arg Glu Pro
    50                  55                  60

Thr Ser Ser Glu Gln Gly Gly Leu Glu Gly Ser Gly Ser Ala Ala Gly
65                  70                  75                  80

Glu Gly Lys Pro Ala Leu Ser Glu Glu Glu Arg Gln Glu Gln Thr Lys
                85                  90                  95

Arg Met Leu Glu Leu Val Ala Gln Lys Gln Arg Glu Arg Glu Glu Arg
            100                 105                 110

Glu Glu Arg Glu Ala Leu Glu Arg Glu Arg Gln Arg Arg Arg Gln Gly
        115                 120                 125

Gln Glu Leu Ser Ala Ala Arg Gln Arg Leu Gln Glu Asp Glu Met Arg
    130                 135                 140

Arg Ala Ala Glu Glu Arg Arg Arg Glu Lys Ala Glu Glu Leu Ala Ala
145                 150                 155                 160

Arg Gln Arg Val Arg Glu Lys Ile Glu Arg Asp Lys Ala Glu Arg Ala
                165                 170                 175

Lys Lys Tyr Gly Gly Ser Val Gly Ser Gln Pro Pro Pro Val Ala Pro
            180                 185                 190

Glu Pro Gly Pro Val Pro Ser Ser Pro Ser Gln Glu Pro Pro Thr Lys
        195                 200                 205

Arg Glu Tyr Asp Gln Cys Arg Ile Gln Val Arg Leu Pro Asp Gly Thr
    210                 215                 220

Ser Leu Thr Gln Thr Phe Arg Ala Arg Glu Gln Leu Ala Ala Val Arg
225                 230                 235                 240

Leu Tyr Val Glu Leu His Arg Gly Glu Glu Leu Gly Gly Gly Gln Asp
                245                 250                 255

Pro Val Gln Leu Leu Ser Gly Phe Pro Arg Arg Ala Phe Ser Glu Ala
            260                 265                 270

Asp Met Glu Arg Pro Leu Gln Glu Leu Gly Leu Val Pro Ser Ala Val
        275                 280                 285

Leu Ile Val Ala Lys Lys Cys Pro Ser

290                    295

<210>  164

<211>  354

<212>  PRT

<213>  Homo sapiens

<300>

<308>  sw_hum/hem6_human

<309>  1994-06-01

<400>  164

Met Leu Pro Lys Thr Ser Gly Thr Arg Ala Thr Ser Leu Gly Arg Pro
1               5                   10                  15

Glu Glu Glu Glu Asp Glu Leu Ala His Arg Cys Ser Ser Phe Met Ala
            20                  25                  30

Pro Pro Val Thr Asp Leu Gly Glu Leu Arg Arg Arg Pro Gly Asp Met
        35                  40                  45

Lys Thr Lys Met Glu Leu Leu Ile Leu Glu Thr Gln Ala Gln Val Cys
    50                  55                  60

Gln Ala Leu Ala Gln Val Asp Gly Gly Ala Asn Phe Ser Val Asp Arg
65                  70                  75                  80

Trp Glu Arg Lys Glu Gly Gly Gly Gly Ile Ser Cys Val Leu Gln Asp
                85                  90                  95

Gly Cys Val Phe Glu Lys Ala Gly Val Ser Ile Ser Val Val His Gly
            100                 105                 110

Asn Leu Ser Glu Glu Ala Ala Lys Gln Met Arg Ser Arg Gly Lys Val
        115                 120                 125

Leu Lys Thr Lys Asp Gly Lys Leu Pro Phe Cys Ala Met Gly Val Ser
    130                 135                 140

Ser Val Thr His Pro Lys Asn Pro His Ala Pro Thr Ile His Phe Asn
145                 150                 155                 160

Tyr Arg Tyr Phe Glu Val Glu Glu Ala Asp Gly His Lys Gln Trp Trp
                165                 170                 175

Phe Gly Gly Gly Cys Asp Leu Thr Pro Thr Tyr Leu Asn Gln Glu Asp
            180                 185                 190

Ala Val His Phe His Arg Thr Leu Lys Glu Ala Cys Asp Gln His Gly
        195                 200                 205

Pro Asp Leu Tyr Pro Lys Phe Lys Lys Trp Cys Asp Asp Tyr Phe Phe
    210                 215                 220

Ile Ala His Arg Gly Glu Arg Arg Gly Ile Gly Gly Ile Phe Phe Asp
225                 230                 235                 240

Asp Leu Asp Ser Pro Ser Lys Glu Glu Val Phe Arg Phe Val Gln Ser
                245                 250                 255

Cys Ala Arg Ala Val Val Pro Ser Tyr Ile Pro Leu Val Lys Lys His
            260                 265                 270

Cys Asp Asp Ser Phe Thr Pro Gln Glu Lys Leu Trp Gln Gln Leu Arg
        275                 280                 285

Arg Gly Arg Tyr Val Glu Phe Asn Leu Leu Tyr Asp Arg Gly Thr Lys
    290                 295                 300

Phe Gly Leu Phe Thr Pro Gly Ser Arg Ile Glu Ser Ile Leu Met Ser
305                 310                 315                 320

Leu Pro Leu Thr Ala Arg Trp Glu Tyr Met His Ser Pro Ser Glu Asn
                325                 330                 335

Ser Lys Glu Ala Glu Ile Leu Glu Val Leu Arg His Pro Arg Asp Trp
            340                 345                 350

Val Arg

<210>  165

<211>  397

<212>  PRT

<213>  Homo sapiens

<300>

<308>  sw_hum/thim_human

<309>  1995-11-01

<400>  165

Met Arg Leu Leu Arg Gly Val Phe Val Val Ala Ala Lys Arg Thr Pro
1               5                   10                  15

Phe Gly Ala Tyr Gly Gly Leu Leu Lys Asp Phe Thr Ala Thr Asp Leu
            20                  25                  30

Ser Glu Phe Ala Ala Lys Ala Ala Leu Ser Ala Gly Lys Val Ser Pro
        35                  40                  45

Glu Thr Val Asp Ser Val Ile Met Gly Asn Val Leu Gln Ser Ser Ser
    50                  55                  60

Asp Ala Ile Tyr Leu Ala Arg His Val Gly Leu Arg Val Gly Ile Pro
65                  70                  75                  80

Lys Glu Thr Pro Ala Leu Thr Ile Asn Arg Leu Cys Gly Ser Gly Phe
                85                  90                  95

Gln Ser Ile Val Asn Gly Cys Gln Glu Ile Cys Val Lys Glu Ala Glu
            100                 105                 110

Val Val Leu Cys Gly Gly Thr Glu Ser Met Ser Gln Ala Pro Tyr Cys
        115                 120                 125

Val Arg Asn Val Arg Phe Gly Thr Lys Leu Gly Ser Asp Ile Lys Leu
    130                 135                 140

Glu Asp Ser Leu Trp Val Ser Leu Thr Asp Gln His Val Gln Leu Pro
145                 150                 155                 160

Met Ala Met Thr Ala Glu Asn Leu Thr Val Lys His Lys Ile Ser Arg
                165                 170                 175

Glu Glu Cys Asp Lys Tyr Ala Leu Gln Ser Gln Gln Arg Trp Lys Ala
            180                 185                 190

Ala Asn Asp Ala Gly Tyr Phe Asn Asp Glu Met Ala Pro Ile Glu Val
        195                 200                 205

Lys Thr Lys Lys Gly Lys Gln Thr Met Gln Val Asp Glu His Ala Arg
    210                 215                 220

Pro Gln Thr Thr Leu Glu Gln Leu Gln Lys Leu Pro Pro Val Phe Lys
225                 230                 235                 240

Lys Asp Gly Thr Val Thr Ala Gly Asn Ala Ser Gly Val Ala Asp Gly
                245                 250                 255

Ala Gly Ala Val Ile Ile Ala Ser Glu Asp Ala Val Lys Lys His Asn
            260                 265                 270

Phe Thr Pro Leu Ala Arg Ile Val Gly Tyr Phe Val Ser Gly Cys Asp
        275                 280                 285

Pro Ser Ile Met Gly Ile Gly Pro Val Pro Ala Ile Ser Gly Ala Leu
    290                 295                 300

Lys Lys Ala Gly Leu Ser Leu Lys Asp Met Asp Leu Val Glu Val Asn
305                 310                 315                 320

Glu Ala Phe Ala Pro Gln Tyr Leu Ala Val Glu Arg Ser Leu Asp Leu
                325                 330                 335

Asp Ile Ser Lys Thr Asn Val Asn Gly Gly Ala Ile Ala Leu Gly His
            340                 345                 350

Pro Leu Gly Gly Ser Gly Ser Arg Ile Thr Ala His Leu Val His Glu
        355                 360                 365

Leu Arg Arg Arg Gly Gly Lys Tyr Ala Val Gly Ser Ala Cys Ile Gly
    370                 375                 380

Gly Gly Gln Gly Ile Ala Val Ile Ile Gln Ser Thr Ala
385                 390                 395

<210>  166

<211>  550

<212>  PRT

<213>  Homo sapiens

<300>

<308>  sw_hum/src8_human

<309>  1997-11-01

<400>  166

Met Trp Lys Ala Ser Ala Gly His Ala Val Ser Ile Ala Gln Asp Asp
1               5                   10                  15

Ala Gly Ala Asp Asp Trp Glu Thr Asp Pro Asp Phe Val Asn Asp Val
            20                  25                  30

Ser Glu Lys Glu Gln Arg Trp Gly Ala Lys Thr Val Gln Gly Ser Gly
        35                  40                  45

His Gln Glu His Ile Asn Ile His Lys Leu Arg Glu Asn Val Phe Gln
    50                  55                  60

Glu His Gln Thr Leu Lys Glu Lys Glu Leu Glu Thr Gly Pro Lys Ala
65                  70                  75                  80

Ser His Gly Tyr Gly Gly Lys Phe Gly Val Glu Gln Asp Arg Met Asp
                85                  90                  95

Lys Ser Ala Val Gly His Glu Tyr Gln Ser Lys Leu Ser Lys His Cys
            100                 105                 110

Ser Gln Val Asp Ser Val Arg Gly Phe Gly Gly Lys Phe Gly Val Gln
        115                 120                 125

Met Asp Arg Val Asp Gln Ser Ala Val Gly Phe Glu Tyr Gln Gly Lys
    130                 135                 140

Thr Glu Lys His Ala Ser Gln Lys Asp Tyr Ser Ser Gly Phe Gly Gly
145                 150                 155                 160

Lys Tyr Gly Val Gln Ala Asp Arg Val Asp Lys Ser Ala Val Gly Phe
                165                 170                 175

Asp Tyr Gln Gly Lys Thr Glu Lys His Glu Ser Gln Arg Asp Tyr Ser
            180                 185                 190

Lys Gly Phe Gly Gly Lys Tyr Gly Ile Asp Lys Asp Lys Val Asp Lys
        195                 200                 205

Ser Ala Val Gly Phe Glu Tyr Gln Gly Lys Thr Glu Lys His Glu Ser

210                 215                 220

Gln Lys Asp Tyr Val Lys Gly Phe Gly Gly Lys Phe Gly Val Gln Thr
225                 230                 235                 240

Asp Arg Gln Asp Lys Cys Ala Leu Gly Trp Asp His Gln Glu Lys Leu
                245                 250                 255

Gln Leu His Glu Ser Gln Lys Asp Tyr Lys Thr Gly Phe Gly Gly Lys
            260                 265                 270

Phe Gly Val Gln Ser Glu Arg Gln Asp Ser Ala Ala Val Gly Phe Asp
        275                 280                 285

Tyr Lys Glu Lys Leu Ala Lys His Glu Ser Gln Gln Asp Tyr Ser Lys
    290                 295                 300

Gly Phe Gly Gly Lys Tyr Gly Val Gln Lys Asp Arg Met Asp Lys Asn
305                 310                 315                 320

Ala Ser Thr Phe Glu Asp Val Thr Gln Val Ser Ser Ala Tyr Gln Lys
                325                 330                 335

Thr Val Pro Val Glu Ala Val Thr Ser Lys Thr Ser Asn Ile Arg Ala
            340                 345                 350

Asn Phe Glu Asn Leu Ala Lys Glu Lys Glu Gln Glu Asp Arg Arg Lys
        355                 360                 365

Ala Glu Ala Glu Arg Ala Gln Arg Met Ala Lys Glu Arg Gln Glu Gln
    370                 375                 380

Glu Glu Ala Arg Arg Lys Leu Glu Glu Gln Ala Arg Ala Lys Thr Gln
385                 390                 395                 400

Thr Pro Pro Val Ser Pro Ala Pro Gln Pro Thr Glu Glu Arg Leu Pro
                405                 410                 415

Ser Ser Pro Val Tyr Glu Asp Ala Ala Ser Phe Lys Ala Glu Leu Ser
            420                 425                 430

Tyr Arg Gly Pro Val Ser Gly Thr Glu Pro Glu Pro Val Tyr Ser Met
        435                 440                 445

Glu Ala Ala Asp Tyr Arg Glu Ala Ser Ser Gln Gln Gly Leu Ala Tyr
    450                 455                 460

Ala Thr Glu Ala Val Tyr Glu Ser Ala Glu Ala Pro Gly His Tyr Pro
465                 470                 475                 480

Ala Glu Asp Ser Thr Tyr Asp Glu Tyr Glu Asn Asp Leu Gly Tyr Thr
                485                 490                 495

Ala Val Ala Leu Tyr Asp Tyr Gln Ala Ala Gly Asp Asp Glu Ile Ser
            500                 505                 510

Phe Asp Pro Asp Asp Ile Ile Thr Asn Ile Glu Met Ile Asp Asp Gly
        515                 520                 525

Trp Trp Arg Gly Val Cys Lys Gly Arg Tyr Gly Leu Phe Pro Ala Asn
    530                 535                 540

Tyr Val Glu Leu Arg Gln
545                 550

<210>  167

<211>  223

<212>  PRT

<213>  Homo sapiens

<300>

<308>  sw_hum/kad4_human

<309>  1992-08-01

<400>  167

Met Ala Ser Lys Leu Leu Arg Ala Val Ile Leu Gly Pro Pro Gly Ser
1               5                   10                  15

Gly Lys Gly Thr Val Cys Gln Arg Ile Ala Gln Asn Phe Gly Leu Gln
            20                  25                  30

His Leu Ser Ser Gly His Phe Leu Arg Glu Asn Ile Lys Ala Ser Thr
        35                  40                  45

Glu Val Gly Glu Met Ala Lys Gln Tyr Ile Glu Lys Ser Leu Leu Val
    50                  55                  60

Pro Asp His Val Ile Thr Arg Leu Met Met Ser Glu Leu Glu Asn Arg
65                  70                  75                  80

Arg Gly Gln His Trp Leu Leu Asp Gly Phe Pro Arg Thr Leu Gly Gln
                85                  90                  95

Ala Glu Ala Leu Asp Lys Ile Cys Glu Val Asp Leu Val Ile Ser Leu
            100                 105                 110

Asn Ile Pro Phe Glu Thr Leu Lys Asp Arg Leu Ser Arg Arg Trp Ile
        115                 120                 125

His Pro Pro Ser Gly Arg Val Tyr Asn Leu Asp Phe Asn Pro Pro His
    130                 135                 140

Val His Gly Ile Asp Asp Val Thr Gly Glu Pro Leu Val Gln Gln Glu
145                 150                 155                 160

Asp Asp Lys Pro Glu Ala Val Ala Ala Arg Leu Arg Gln Tyr Lys Asp
                165                 170                 175

Val Ala Lys Pro Val Ile Glu Leu Tyr Lys Ser Arg Gly Val Leu His
            180                 185                 190

Gln Phe Ser Gly Thr Glu Thr Asn Lys Ile Trp Pro Tyr Val Tyr Thr
        195                 200                 205

Leu Phe Ser Asn Lys Ile Thr Pro Ile Gln Ser Lys Glu Ala Tyr
    210                 215                 220

<210>  168

<211>  189

<212>  PRT

<213>  Homo sapiens

<300>

<308>  humangp/chr12-q9nrv9

<309>  2003-04-22

<400>  168

Met Leu Gly Met Ile Lys Asn Ser Leu Phe Gly Ser Val Glu Thr Trp
1               5                   10                  15

Pro Trp Gln Val Leu Ser Lys Gly Asp Lys Glu Glu Val Ala Tyr Glu
            20                  25                  30

Glu Arg Ala Cys Glu Gly Gly Lys Phe Ala Thr Val Glu Val Thr Asp
        35                  40                  45

Lys Pro Val Asp Glu Ala Leu Arg Glu Ala Met Pro Lys Val Ala Lys
    50                  55                  60

Tyr Ala Gly Gly Thr Asn Asp Lys Gly Ile Gly Met Gly Met Thr Val
65                  70                  75                  80

Pro Ile Ser Phe Ala Val Phe Pro Asn Glu Asp Gly Ser Leu Gln Lys
                85                  90                  95

Lys Leu Lys Val Trp Phe Arg Ile Pro Asn Gln Phe Gln Ser Asp Pro
            100                 105                 110

Pro Ala Pro Ser Asp Lys Ser Val Lys Ile Glu Glu Arg Glu Gly Ile
        115                 120                 125

Thr Val Tyr Ser Met Gln Phe Gly Gly Tyr Ala Lys Glu Ala Asp Tyr
    130                 135                 140

Val Ala Gln Ala Thr Arg Leu Arg Ala Ala Leu Glu Gly Thr Ala Thr
145                 150                 155                 160

Tyr Arg Gly Asp Ile Tyr Phe Cys Thr Gly Tyr Asp Pro Pro Met Lys
                165                 170                 175

Pro Tyr Gly Arg Arg Asn Glu Ile Trp Leu Leu Lys Thr
            180                 185

<210>  169

<211>  1404

<212>  PRT

<213>  Homo sapiens

<300>

<308>  humangp/chr12-q14221

<309>  2003-04-22

<400>  169

Gln Thr Pro Gly Arg Val Gly Ser Gln Gly Ser Asp Leu Asp Ser Ser
1               5                   10                  15

Ala Thr Pro Ile Asn Thr Val Asp Val Asn Asn Glu Ser Ser Ser Glu
            20                  25                  30

Gly Phe Ile Cys Pro Gln Cys Met Lys Ser Leu Gly Ser Ala Asp Glu
        35                  40                  45

Leu Phe Lys His Tyr Glu Ala Val His Asp Ala Gly Asn Asp Ser Gly
    50                  55                  60

His Gly Gly Glu Ser Asn Leu Ala Leu Lys Arg Asp Asp Val Thr Leu
65                  70                  75                  80

Leu Arg Gln Glu Val Gln Asp Leu Gln Ala Ser Leu Lys Glu Glu Lys
                85                  90                  95

Trp Tyr Ser Glu Glu Leu Lys Lys Glu Leu Glu Lys Tyr Gln Gly Leu
            100                 105                 110

Gln Gln Gln Glu Ala Lys Pro Asp Gly Leu Val Thr Asp Ser Ser Ala
        115                 120                 125

Glu Leu Gln Ser Leu Glu Gln Gln Leu Glu Glu Ala Gln Thr Glu Asn
    130                 135                 140

Phe Asn Ile Lys Gln Met Lys Asp Leu Phe Glu Gln Lys Ala Ala Gln
145                 150                 155                 160

Leu Ala Thr Glu Ile Ala Asp Ile Lys Ser Lys Tyr Asp Glu Glu Arg
                165                 170                 175

Ser Leu Arg Glu Ala Ala Glu Gln Lys Val Thr Arg Leu Thr Glu Glu
            180                 185                 190

Leu Asn Lys Glu Ala Thr Val Ile Gln Asp Leu Lys Thr Glu Leu Leu
        195                 200                 205

Gln Arg Pro Gly Ile Glu Asp Val Ala Val Leu Lys Lys Glu Leu Val
    210                 215                 220

Gln Val Gln Thr Leu Met Asp Asn Met Thr Leu Glu Arg Glu Arg Glu

225                 230                 235                 240

Ser Glu Lys Leu Lys Asp Glu Cys Lys Lys Leu Gln Ser Gln Tyr Ala
                245                 250                 255

Ser Ser Glu Ala Thr Ile Ser Gln Leu Arg Ser Glu Leu Ala Lys Gly
            260                 265                 270

Pro Gln Glu Val Ala Val Tyr Val Gln Glu Leu Gln Lys Leu Lys Ser
        275                 280                 285

Ser Val Asn Glu Leu Thr Gln Lys Asn Gln Thr Leu Thr Glu Asn Leu
    290                 295                 300

Leu Lys Lys Glu Gln Asp Tyr Thr Lys Leu Glu Glu Lys His Asn Glu
305                 310                 315                 320

Glu Ser Val Ser Lys Lys Asn Ile Gln Ala Thr Leu His Gln Lys Asp
                325                 330                 335

Leu Asp Cys Gln Gln Leu Gln Ser Arg Leu Ser Ala Ser Glu Thr Ser
            340                 345                 350

Leu His Arg Ile His Val Glu Leu Ser Glu Lys Gly Glu Ala Thr Gln
        355                 360                 365

Lys Leu Lys Glu Glu Leu Ser Glu Val Glu Thr Lys Tyr Gln His Leu
    370                 375                 380

Lys Ala Glu Phe Lys Gln Leu Gln Gln Gln Arg Glu Glu Lys Glu Gln
385                 390                 395                 400

His Gly Leu Gln Leu Gln Ser Glu Ile Asn Gln Leu His Ser Lys Leu
                405                 410                 415

Leu Glu Thr Glu Arg Gln Leu Gly Glu Ala His Gly Arg Leu Lys Glu
            420                 425                 430

Gln Arg Gln Leu Ser Ser Glu Lys Leu Met Asp Lys Glu Gln Gln Val
        435                 440                 445

Ala Asp Leu Gln Leu Lys Leu Ser Arg Leu Glu Glu Gln Leu Lys Glu
    450                 455                 460

Lys Val Thr Asn Ser Thr Glu Leu Gln His Gln Leu Asp Lys Thr Lys
465                 470                 475                 480

Gln Gln His Gln Glu Gln Gln Ala Leu Gln Gln Ser Thr Thr Ala Lys
                485                 490                 495

Leu Arg Glu Ala Gln Asn Asp Leu Glu Gln Val Leu Arg Gln Ile Gly
            500                 505                 510

Asp Lys Asp Gln Lys Ile Gln Asn Leu Glu Ala Leu Leu Gln Lys Ser
        515                 520                 525

Lys Glu Asn Ile Ser Leu Leu Glu Lys Glu Arg Glu Asp Leu Tyr Ala
    530                 535                 540

Lys Ile Gln Ala Gly Glu Gly Glu Thr Ala Val Leu Asn Gln Leu Gln
545                 550                 555                 560

Glu Lys Asn His Thr Leu Gln Glu Gln Val Thr Gln Leu Thr Glu Lys
                565                 570                 575

Leu Lys Asn Gln Ser Glu Ser His Lys Gln Ala Gln Glu Asn Leu His
            580                 585                 590

Asp Gln Val Gln Glu Gln Lys Ala His Leu Arg Ala Ala Gln Asp Arg
        595                 600                 605

Val Leu Ser Leu Glu Thr Ser Val Asn Glu Leu Asn Ser Gln Leu Asn
    610                 615                 620

Glu Ser Lys Glu Lys Val Ser Gln Leu Asp Ile Gln Ile Lys Ala Lys
625                 630                 635                 640

Thr Glu Leu Leu Leu Ser Ala Glu Ala Ala Lys Thr Ala Gln Arg Ala
                645                 650                 655

Asp Leu Gln Asn His Leu Asp Thr Ala Gln Asn Ala Leu Gln Asp Lys
            660                 665                 670

Gln Gln Glu Leu Asn Lys Ile Thr Thr Gln Leu Asp Gln Val Thr Ala
        675                 680                 685

Lys Leu Gln Asp Lys Gln Glu His Cys Ser Gln Leu Glu Ser His Leu
    690                 695                 700

Lys Glu Tyr Lys Glu Lys Tyr Leu Ser Leu Glu Gln Lys Thr Glu Glu
705                 710                 715                 720

Leu Glu Gly Gln Ile Lys Lys Leu Glu Ala Asp Ser Leu Glu Val Lys
                725                 730                 735

Ala Ser Lys Glu Gln Ala Leu Gln Asp Leu Gln Gln Gln Arg Gln Leu
            740                 745                 750

Asn Thr Asp Leu Glu Leu Arg Ala Thr Glu Leu Ser Lys Gln Leu Glu
        755                 760                 765

Met Glu Lys Glu Ile Val Ser Ser Thr Arg Leu Asp Leu Gln Lys Lys
    770                 775                 780

Ser Glu Ala Leu Glu Ser Ile Lys Gln Lys Leu Thr Lys Gln Glu Glu
785                 790                 795                 800

Glu Lys Lys Ile Leu Lys Gln Asp Phe Glu Thr Leu Ser Gln Glu Thr
                805                 810                 815

Lys Ile Gln His Glu Glu Leu Asn Asn Arg Ile Gln Thr Thr Val Thr
            820                 825                 830

Glu Leu Gln Lys Val Lys Met Glu Lys Glu Ala Leu Met Thr Glu Leu
        835                 840                 845

Ser Thr Val Lys Asp Lys Leu Ser Lys Val Ser Asp Ser Leu Lys Asn
    850                 855                 860

Ser Lys Ser Glu Phe Glu Lys Glu Asn Gln Lys Gly Lys Ala Ala Ile
865                 870                 875                 880

Leu Asp Leu Glu Lys Thr Cys Lys Glu Leu Lys His Gln Leu Gln Val
                885                 890                 895

Gln Met Glu Asn Thr Leu Lys Glu Gln Lys Glu Leu Lys Lys Ser Leu
            900                 905                 910

Glu Lys Glu Lys Glu Ala Ser His Gln Leu Lys Leu Glu Leu Asn Ser
        915                 920                 925

Met Gln Glu Gln Leu Ile Gln Ala Gln Asn Thr Leu Lys Gln Asn Glu
    930                 935                 940

Lys Glu Glu Gln Gln Leu Gln Gly Asn Ile Asn Glu Leu Lys Gln Ser
945                 950                 955                 960

Ser Glu Gln Lys Lys Lys Gln Ile Glu Ala Leu Gln Gly Glu Leu Lys
                965                 970                 975

Ile Ala Val Leu Gln Lys Thr Glu Leu Glu Asn Lys Leu Gln Gln Gln
            980                 985                 990

Leu Thr Gln Ala Ala Gln Glu Leu  Ala Ala Glu Lys Glu  Lys Ile Ser
        995                 1000                 1005

Val Leu  Gln Asn Asn Tyr Glu  Lys Ser Gln Glu Thr  Phe Lys Gln
    1010                 1015                 1020

Leu Gln  Ser Asp Phe Tyr Gly  Arg Glu Ser Glu Leu  Leu Ala Thr
    1025                 1030                 1035

Arg Gln  Asp Leu Lys Ser Val  Glu Glu Lys Leu Ser  Leu Ala Gln
    1040                 1045                 1050

Glu Asp  Leu Ile Ser Asn Arg  Asn Gln Ile Gly Asn  Gln Asn Lys
    1055                 1060                 1065

Leu Ile  Gln Glu Leu Lys Thr  Ala Lys Ala Thr Leu  Glu Gln Asp
    1070                 1075                 1080

Ser Ala  Lys Lys Glu Gln Gln  Leu Gln Glu Arg Cys  Lys Ala Leu
    1085                 1090                 1095

Gln Asp  Ile Gln Lys Glu Lys  Ser Leu Lys Glu Lys  Glu Leu Val
    1100                 1105                 1110

Asn Glu  Lys Ser Lys Leu Ala  Glu Ile Glu Glu Ile  Lys Cys Arg
    1115                 1120                 1125

Gln Glu  Lys Glu Ile Thr Lys  Leu Asn Glu Glu Leu  Lys Ser His
    1130                 1135                 1140

Lys Leu Glu Ser Ile Lys Glu Ile Thr Asn Leu Lys Asp Ala Lys
    1145                1150                1155

Gln Leu Leu Ile Gln Gln Lys Leu Glu Leu Gln Gly Lys Ala Asp
    1160                1165                1170

Ser Leu Lys Ala Ala Val Glu Gln Glu Lys Arg Asn Gln Gln Ile
    1175                1180                1185

Leu Lys Asp Gln Val Lys Lys Glu Glu Glu Glu Leu Lys Lys Glu
    1190                1195                1200

Phe Ile Glu Lys Glu Ala Lys Leu His Ser Glu Ile Lys Glu Lys
    1205                1210                1215

Glu Val Gly Met Lys Lys His Glu Glu Asn Glu Ala Lys Leu Thr
    1220                1225                1230

Met Gln Ile Thr Ala Leu Asn Glu Asn Leu Gly Thr Val Lys Lys
    1235                1240                1245

Glu Trp Gln Ser Ser Gln Arg Arg Val Ser Glu Leu Glu Lys Gln
    1250                1255                1260

Thr Asp Asp Leu Arg Gly Glu Ile Ala Val Leu Glu Ala Thr Val
    1265                1270                1275

Gln Asn Asn Gln Asp Glu Arg Arg Ala Leu Leu Glu Arg Cys Leu
    1280                1285                1290

Lys Gly Glu Gly Glu Ile Glu Lys Leu Gln Thr Lys Val Leu Glu
    1295                1300                1305

Leu Gln Arg Lys Leu Asp Asn Thr Thr Ala Ala Val Gln Glu Leu
    1310                1315                1320

Gly Arg Glu Asn Gln Ser Leu Gln Ile Lys His Thr Gln Ala Leu
    1325                1330                1335

Asn Arg Lys Trp Ala Glu Asp Asn Glu Val Gln Asn Cys Met Ala
    1340                1345                1350

Cys Gly Lys Gly Phe Ser Val Thr Val Arg Arg His His Cys Arg
    1355                1360                1365

Gln Cys Gly Asn Ile Phe Cys Ala Glu Cys Ser Ala Lys Asn Ala
    1370                1375                1380

Leu Thr Pro Ser Ser Lys Lys Pro Val Arg Val Cys Asp Ala Cys
    1385                1390                1395

Phe Asn Asp Leu Gln Gly
    1400

<210> 170

<211> 376

<212>  PRT

<213>  Homo sapiens

<300>

<308>  sw_hum/myg1_human

<309>  2001-10-16

<400>  170

Met Gly His Arg Phe Leu Arg Gly Leu Leu Thr Leu Leu Leu Pro Pro
1               5                   10                  15

Pro Pro Leu Tyr Thr Arg His Arg Met Leu Gly Pro Glu Ser Val Pro
            20                  25                  30

Pro Pro Lys Arg Ser Arg Ser Lys Leu Met Ala Pro Pro Arg Ile Gly
        35                  40                  45

Thr His Asn Gly Thr Phe His Cys Asp Glu Ala Leu Ala Cys Ala Leu
    50                  55                  60

Leu Arg Leu Leu Pro Glu Tyr Arg Asp Ala Glu Ile Val Arg Thr Arg
65                  70                  75                  80

Asp Pro Glu Lys Leu Ala Ser Cys Asp Ile Val Val Asp Val Gly Gly
                85                  90                  95

Glu Tyr Asp Pro Arg Arg His Arg Tyr Asp His His Gln Arg Ser Phe
            100                 105                 110

Thr Glu Thr Met Ser Ser Leu Ser Pro Gly Lys Pro Trp Gln Thr Lys
        115                 120                 125

Leu Ser Ser Ala Gly Leu Ile Tyr Leu His Phe Gly His Lys Leu Leu
    130                 135                 140

Ala Gln Leu Leu Gly Pro Ser Glu Glu Asp Ser Met Val Gly Thr Leu
145                 150                 155                 160

Tyr Asp Lys Met Tyr Glu Asn Phe Val Glu Glu Val Asp Ala Val Asp
                165                 170                 175

Asn Gly Ile Ser Gln Trp Ala Glu Gly Glu Pro Arg Tyr Ala Leu Thr
            180                 185                 190

Thr Thr Leu Ser Ala Arg Val Ala Arg Leu Asn Pro Thr Trp Asn His
        195                 200                 205

Pro Asp Gln Asp Thr Glu Ala Gly Phe Lys Arg Ala Met Asp Leu Val
    210                 215                 220

Gln Glu Glu Phe Leu Gln Arg Leu Asp Phe Tyr Gln His Ser Trp Leu
225                 230                 235                 240

Pro Ala Arg Ala Leu Val Glu Glu Ala Leu Ala Gln Arg Phe Gln Val

245                 250                 255

Asp Pro Ser Gly Glu Ile Val Glu Leu Ala Lys Gly Ala Cys Pro Trp
            260                 265                 270

Lys Glu His Leu Tyr His Leu Glu Ser Gly Leu Ser Pro Pro Val Ala
        275                 280                 285

Ile Phe Phe Val Ile Tyr Thr Asp Gln Ala Gly Gln Trp Arg Ile Gln
    290                 295                 300

Cys Val Pro Lys Glu Pro His Ser Phe Gln Ser Arg Leu Pro Leu Pro
305                 310                 315                 320

Glu Pro Trp Arg Gly Leu Arg Asp Glu Ala Leu Asp Gln Val Ser Gly
                325                 330                 335

Ile Pro Gly Cys Ile Phe Val His Ala Ser Gly Phe Ile Gly Gly His
            340                 345                 350

Pro Thr Arg Glu Gly Ala Leu Ser Met Ala Arg Ala Thr Leu Ala Gln
        355                 360                 365

Arg Ser Tyr Leu Pro Gln Ile Ser
    370                 375

<210>  171

<211>  525

<212>  PRT

<213>  Homo sapiens

<300>

<308>  sw_hum/mppa_human

<309>  1996-10-01

<400>  171

<210> 172

<211> 422

<212> PRT

<213> Homo sapiens

<300>

<308> sw_hum/psdb_human

<309> 2003-02-28

<400> 172

Met Ala Ala Ala Ala Val Val Glu Phe Gln Arg Ala Gln Ser Leu Leu
1               5                   10                  15

Ser Thr Asp Arg Glu Ala Ser Ile Asp Ile Leu His Ser Ile Val Lys
            20                  25                  30

Arg Asp Ile Gln Glu Asn Asp Glu Glu Ala Val Gln Val Lys Glu Gln
        35                  40                  45

Ser Ile Leu Glu Leu Gly Ser Leu Leu Ala Lys Thr Gly Gln Ala Ala
    50                  55                  60

Glu Leu Gly Gly Leu Leu Lys Tyr Val Arg Pro Phe Leu Asn Ser Ile
65                  70                  75                  80

Ser Lys Ala Lys Ala Ala Arg Leu Val Arg Ser Leu Leu Asp Leu Phe
                85                  90                  95

Leu Asp Met Glu Ala Ala Thr Gly Gln Glu Val Glu Leu Cys Leu Glu
            100                 105                 110

Cys Ile Glu Trp Ala Lys Ser Glu Lys Arg Thr Phe Leu Arg Gln Ala
        115                 120                 125

Leu Glu Ala Arg Leu Val Ser Leu Tyr Phe Asp Thr Lys Arg Tyr Gln
    130                 135                 140

Glu Ala Leu His Leu Gly Ser Gln Leu Leu Arg Glu Leu Lys Lys Met
145                 150                 155                 160

Asp Asp Lys Ala Leu Leu Val Glu Val Gln Leu Leu Glu Ser Lys Thr
                165                 170                 175

Tyr His Ala Leu Ser Asn Leu Pro Lys Ala Arg Ala Ala Leu Thr Ser
            180                 185                 190

Ala Arg Thr Thr Ala Asn Ala Ile Tyr Cys Pro Pro Lys Leu Gln Ala
        195                 200                 205

Thr Leu Asp Met Gln Ser Gly Ile Ile His Ala Ala Glu Glu Lys Asp
    210                 215                 220

Trp Lys Thr Ala Tyr Ser Tyr Phe Tyr Glu Ala Phe Glu Gly Tyr Asp
225                 230                 235                 240

Ser Ile Asp Ser Pro Lys Ala Ile Thr Ser Leu Lys Tyr Met Leu Leu
                245                 250                 255

Cys Lys Ile Met Leu Asn Thr Pro Glu Asp Val Gln Ala Leu Val Ser
            260                 265                 270

Gly Lys Leu Ala Leu Arg Tyr Ala Gly Arg Gln Thr Glu Ala Leu Lys
        275                 280                 285

Cys Val Ala Gln Ala Ser Lys Asn Arg Ser Leu Ala Asp Phe Glu Lys
    290                 295                 300

Ala Leu Thr Asp Tyr Arg Ala Glu Leu Arg Asp Asp Pro Ile Ile Ser
305                 310                 315                 320

Thr His Leu Ala Lys Leu Tyr Asp Asn Leu Leu Glu Gln Asn Leu Ile
                325                 330                 335

Arg Val Ile Glu Pro Phe Ser Arg Val Gln Ile Glu His Ile Ser Ser
            340                 345                 350

Leu Ile Lys Leu Ser Lys Ala Asp Val Glu Arg Lys Leu Ser Gln Met
        355                 360                 365

Ile Leu Asp Lys Lys Phe His Gly Ile Leu Asp Gln Gly Glu Gly Val
    370                 375                 380

Leu Ile Ile Phe Asp Glu Pro Pro Val Asp Lys Thr Tyr Glu Ala Ala
385                 390                 395                 400

Leu Glu Thr Ile Gln Asn Met Ser Lys Val Val Asp Ser Leu Tyr Asn

405                 410                 415

Lys Ala Lys Lys Leu Thr
            420

<210>  173

<211>  421

<212>  PRT

<213>  Homo sapiens

<300>

<308>  sw_hum/acdm_human

<309>  1989-07-01

<400>  173

<210>  174

<211>  333

<212>  PRT

<213>  Homo sapiens

<300>

<308>  sw_hum/gipc_human

<309>  2001-10-16

<400>  174

Met Pro Leu Gly Leu Gly Arg Arg Lys Lys Ala Pro Pro Leu Val Glu
1               5                   10                  15

Asn Glu Glu Ala Glu Pro Gly Arg Gly Gly Leu Gly Val Gly Glu Pro
            20                  25                  30

Gly Pro Leu Gly Gly Gly Gly Ser Gly Gly Pro Gln Met Gly Leu Pro
        35                  40                  45

Pro Pro Pro Pro Ala Leu Arg Pro Arg Leu Val Phe His Thr Gln Leu
    50                  55                  60

Ala His Gly Ser Pro Thr Gly Arg Ile Glu Gly Phe Thr Asn Val Lys
65                  70                  75                  80

Glu Leu Tyr Gly Lys Ile Ala Glu Ala Phe Arg Leu Pro Thr Ala Glu
                85                  90                  95

Val Met Phe Cys Thr Leu Asn Thr His Lys Val Asp Met Asp Lys Leu
            100                 105                 110

Leu Gly Gly Gln Ile Gly Leu Glu Asp Phe Ile Phe Ala His Val Lys
        115                 120                 125

Gly Gln Arg Lys Glu Val Glu Val Phe Lys Ser Glu Asp Ala Leu Gly
    130                 135                 140

Leu Thr Ile Thr Asp Asn Gly Ala Gly Tyr Ala Phe Ile Lys Arg Ile
145                 150                 155                 160

Lys Glu Gly Ser Val Ile Asp His Ile His Leu Ile Ser Val Gly Asp
                165                 170                 175

Met Ile Glu Ala Ile Asn Gly Gln Ser Leu Leu Gly Cys Arg His Tyr
            180                 185                 190

Glu Val Ala Arg Leu Leu Lys Glu Leu Pro Arg Gly Arg Thr Phe Thr
        195                 200                 205

Leu Lys Leu Thr Glu Pro Arg Lys Ala Phe Asp Met Ile Ser Gln Arg
    210                 215                 220

Ser Ala Gly Gly Arg Pro Gly Ser Gly Pro Gln Leu Gly Thr Gly Arg
225                 230                 235                 240

Gly Thr Leu Arg Leu Arg Ser Arg Gly Pro Ala Thr Val Glu Asp Leu
                245                 250                 255

Pro Ser Ala Phe Glu Glu Lys Ala Ile Glu Lys Val Asp Asp Leu Leu
            260                 265                 270

Glu Ser Tyr Met Gly Ile Arg Asp Thr Glu Leu Ala Ala Thr Met Val
        275                 280                 285

Glu Leu Gly Lys Asp Lys Arg Asn Pro Asp Glu Leu Ala Glu Ala Leu
    290                 295                 300

Asp Glu Arg Leu Gly Asp Phe Ala Phe Pro Asp Glu Phe Val Phe Asp
305                 310                 315                 320

Val Trp Gly Ala Ile Gly Asp Ala Lys Val Gly Arg Tyr
                325                 330

<210>  175

<211>  248

<212>  PRT

<213>  Homo sapiens

<300>

<308>  humangp/chr1-aah15403

<309>  2003-04-22

<400>  175

Met Ala Gly Ile Thr Thr Ile Glu Ala Val Lys Arg Lys Ile Gln Val
1               5                   10                  15

Leu Gln Gln Gln Ala Asp Asp Ala Glu Glu Arg Ala Glu Arg Leu Gln
            20                  25                  30

Arg Glu Val Glu Gly Glu Arg Arg Ala Arg Glu Gln Ala Glu Ala Glu
        35                  40                  45

Val Ala Ser Leu Asn Arg Arg Ile Gln Leu Val Glu Glu Glu Leu Asp
    50                  55                  60

Arg Ala Gln Glu Arg Leu Ala Thr Ala Leu Gln Lys Leu Glu Glu Ala
65                  70                  75                  80

Glu Lys Ala Ala Asp Glu Ser Glu Arg Gly Met Lys Val Ile Glu Asn
                85                  90                  95

Arg Ala Leu Lys Asp Glu Glu Lys Met Glu Leu Gln Glu Ile Gln Leu
            100                 105                 110

Lys Glu Ala Lys His Ile Ala Glu Glu Ala Asp Arg Lys Tyr Glu Glu
        115                 120                 125

Val Ala Arg Lys Leu Val Ile Ile Glu Gly Asp Leu Glu Arg Thr Glu
    130                 135                 140

Glu Arg Ala Glu Leu Ala Glu Ser Arg Cys Arg Glu Met Asp Glu Gln
145                 150                 155                 160

Ile Arg Leu Met Asp Gln Asn Leu Lys Cys Leu Ser Ala Ala Glu Glu
                165                 170                 175

Lys Tyr Ser Gln Lys Glu Asp Lys Tyr Glu Glu Glu Ile Lys Ile Leu
            180                 185                 190

Thr Asp Lys Leu Lys Glu Ala Glu Thr Arg Ala Glu Phe Ala Glu Arg

195                 200                 205

Ser Val Ala Lys Leu Glu Lys Thr Ile Asp Asp Leu Glu Asp Lys Leu
    210                 215                 220

Lys Cys Thr Lys Glu Glu His Leu Cys Thr Gln Arg Met Leu Asp Gln
225                 230                 235                 240

Thr Leu Leu Asp Leu Asn Glu Met
                245

<210>  176

<211>  423

<212>  PRT

<213>  Homo sapiens

<300>

<308>  sw_gatm_human

<309>  1996-10-01

<400>  176

<210>  177

<211>  254

<212>  PRT

<213>  Homo sapiens

<300>

<308>  sw_hum/pse3_human

<309>  2001-10-16

<400>  177

Met Ala Ser Leu Leu Lys Val Asp Gln Glu Val Lys Leu Lys Val Asp
1               5                   10                  15

Ser Phe Arg Glu Arg Ile Thr Ser Glu Ala Glu Asp Leu Val Ala Asn
            20                  25                  30

Phe Phe Pro Lys Lys Leu Leu Glu Leu Asp Ser Phe Leu Lys Glu Pro
        35                  40                  45

Ile Leu Asn Ile His Asp Leu Thr Gln Ile His Ser Asp Met Asn Leu
    50                  55                  60

Pro Val Pro Asp Pro Ile Leu Leu Thr Asn Ser His Asp Gly Leu Asp
65                  70                  75                  80

Gly Pro Thr Tyr Lys Lys Arg Arg Leu Asp Glu Cys Glu Glu Ala Phe
                85                  90                  95

Gln Gly Thr Lys Val Phe Val Met Pro Asn Gly Met Leu Lys Ser Asn
            100                 105                 110

Gln Gln Leu Val Asp Ile Ile Glu Lys Val Lys Pro Glu Ile Arg Leu
        115                 120                 125

Leu Ile Glu Lys Cys Asn Thr Val Lys Met Trp Val Gln Leu Leu Ile
    130                 135                 140

Pro Arg Ile Glu Asp Gly Asn Asn Phe Gly Val Ser Ile Gln Glu Glu
145                 150                 155                 160

Thr Val Ala Glu Leu Arg Thr Val Glu Ser Glu Ala Ala Ser Tyr Leu
                165                 170                 175

Asp Gln Ile Ser Arg Tyr Tyr Ile Thr Arg Ala Lys Leu Val Ser Lys
            180                 185                 190

Ile Ala Lys Tyr Pro His Val Glu Asp Tyr Arg Arg Thr Val Thr Glu
        195                 200                 205

Ile Asp Glu Lys Glu Tyr Ile Ser Leu Arg Leu Ile Ile Ser Glu Leu
    210                 215                 220

Arg Asn Gln Tyr Val Thr Leu His Asp Met Ile Leu Lys Asn Ile Glu
225                 230                 235                 240

Lys Ile Lys Arg Pro Arg Ser Ser Asn Ala Glu Thr Leu Tyr
                245                 250

<210>  178

<211>  315

<212>  PRT

<213>  Homo sapiens

<300>

<308>  sw_hum/calu_human

<309>  1999-07-15

<400>  178

Met Asp Leu Arg Gln Phe Leu Met Cys Leu Ser Leu Cys Thr Ala Phe
1               5                   10                  15

Ala Leu Ser Lys Pro Thr Glu Lys Lys Asp Arg Val His His Glu Pro
            20                  25                  30

Gln Leu Ser Asp Lys Val His Asn Asp Ala Gln Ser Phe Asp Tyr Asp
        35                  40                  45

His Asp Ala Phe Leu Gly Ala Glu Glu Ala Lys Thr Phe Asp Gln Leu
    50                  55                  60

Thr Pro Glu Glu Ser Lys Glu Arg Leu Gly Lys Ile Val Ser Lys Ile
65                  70                  75                  80

Asp Gly Asp Lys Asp Gly Phe Val Thr Val Asp Glu Leu Lys Asp Trp
                85                  90                  95

Ile Lys Phe Ala Gln Lys Arg Trp Ile Tyr Glu Asp Val Glu Arg Gln
            100                 105                 110

Trp Lys Gly His Asp Leu Asn Glu Asp Gly Leu Val Ser Trp Glu Glu
        115                 120                 125

Tyr Lys Asn Ala Thr Tyr Gly Tyr Val Leu Asp Asp Pro Asp Pro Asp
    130                 135                 140

Asp Gly Phe Asn Tyr Lys Gln Met Met Val Arg Asp Glu Arg Arg Phe
145                 150                 155                 160

Lys Met Ala Asp Lys Asp Gly Asp Leu Ile Ala Thr Lys Glu Glu Phe
                165                 170                 175

Thr Ala Phe Leu His Pro Glu Glu Tyr Asp Tyr Met Lys Asp Ile Val
            180                 185                 190

Val Gln Glu Thr Met Glu Asp Ile Asp Lys Asn Ala Asp Gly Phe Ile
        195                 200                 205

Asp Leu Glu Glu Tyr Ile Gly Asp Met Tyr Ser His Asp Gly Asn Thr
    210                 215                 220

Asp Glu Pro Glu Trp Val Lys Thr Glu Arg Glu Gln Phe Val Glu Phe
225                 230                 235                 240

Arg Asp Lys Asn Arg Asp Gly Lys Met Asp Lys Glu Glu Thr Lys Asp
                245                 250                 255

Trp Ile Leu Pro Ser Asp Tyr Asp His Ala Glu Ala Glu Ala Arg His
            260                 265                 270

Leu Val Tyr Glu Ser Asp Gln Asn Lys Asp Gly Lys Leu Thr Lys Glu
        275                 280                 285

Glu Ile Val Asp Lys Tyr Asp Leu Phe Val Gly Ser Gln Ala Thr Asp
    290                 295                 300

Phe Gly Glu Ala Leu Val Arg His Asp Glu Phe
305                 310                 315

<210>  179

<211>  401

<212>  PRT

<213>  Homo sapiens

<300>

<308>  sw_hum/dct2_human

<309>  1997-11-01

<400>  179

Met Ala Asp Pro Lys Tyr Ala Asp Leu Pro Gly Ile Ala Arg Asn Glu
1               5                   10                  15

Pro Asp Val Tyr Glu Thr Ser Asp Leu Pro Glu Asp Asp Gln Ala Glu
            20                  25                  30

Phe Asp Ala Glu Glu Leu Thr Ser Thr Ser Val Glu His Ile Ile Val
        35                  40                  45

Asn Pro Asn Ala Ala Tyr Asp Lys Phe Lys Asp Lys Arg Val Gly Thr
    50                  55                  60

Lys Gly Leu Asp Phe Ser Asp Arg Ile Gly Lys Thr Lys Arg Thr Gly
65                  70                  75                  80

Tyr Glu Ser Gly Glu Tyr Glu Met Leu Gly Glu Gly Leu Gly Val Lys
                85                  90                  95

Glu Thr Pro Gln Gln Lys Tyr Gln Arg Leu Leu His Glu Val Gln Glu
            100                 105                 110

Leu Thr Thr Glu Val Glu Lys Ile Lys Thr Thr Val Lys Glu Ser Ala
        115                 120                 125

Thr Glu Glu Lys Leu Thr Pro Val Leu Leu Ala Lys Gln Leu Ala Ala
    130                 135                 140

Leu Lys Gln Gln Leu Val Ala Ser His Leu Glu Lys Leu Leu Gly Pro
145                 150                 155                 160

Asp Ala Ala Ile Asn Leu Thr Asp Pro Asp Gly Ala Leu Ala Lys Arg
                165                 170                 175

Leu Leu Leu Gln Leu Glu Ala Thr Lys Asn Ser Lys Gly Gly Ser Gly
            180                 185                 190

Gly Lys Thr Thr Gly Thr Pro Pro Asp Ser Ser Leu Val Thr Tyr Glu
        195                 200                 205

Leu His Ser Arg Pro Glu Gln Asp Lys Phe Ser Gln Ala Ala Lys Val
    210                 215                 220

Ala Glu Leu Glu Lys Arg Leu Thr Glu Leu Glu Thr Ala Val Arg Cys
225                 230                 235                 240

Asp Gln Asp Ala Gln Asn Pro Leu Ser Ala Gly Leu Gln Gly Ala Cys
                245                 250                 255

Leu Met Glu Thr Val Glu Leu Leu Gln Ala Lys Val Ser Ala Leu Asp
            260                 265                 270

Leu Ala Val Leu Asp Gln Val Glu Ala Arg Leu Gln Ser Val Leu Gly
        275                 280                 285

Lys Val Asn Glu Ile Ala Lys His Lys Ala Ser Val Glu Asp Ala Asp
    290                 295                 300

Thr Gln Ser Lys Val His Gln Leu Tyr Glu Thr Ile Gln Arg Trp Ser
305                 310                 315                 320

Pro Ile Ala Ser Thr Leu Pro Glu Leu Val Gln Arg Leu Val Thr Ile
                325                 330                 335

Lys Gln Leu His Glu Gln Ala Met Gln Phe Gly Gln Leu Leu Thr His
            340                 345                 350

Leu Asp Thr Thr Gln Gln Met Ile Ala Asn Ser Leu Lys Asp Asn Thr
        355                 360                 365

Thr Leu Leu Thr Gln Val Gln Thr Thr Met Arg Glu Asn Leu Ala Thr
    370                 375                 380

Val Glu Gly Asn Phe Ala Ser Ile Asp Glu Arg Met Lys Lys Leu Gly
385                 390                 395                 400

Lys

<210>  180

<211>  261

<212>  PRT

<213>  Homo sapiens

<300>

<308>  sw_hum/hcd2_human

<309>  1997-11-01

<400>  180

<210>  181

<211>  312

<212>  PRT

<213>  Homo sapiens


<300>

<308>  sw_hum/pdxk_human

<309>  1997-11-01


<400>  181

<210>  182

<211>  245

<212>  PRT

<213>  Homo sapiens

<300>

<308>  sw_hum/if6_human

<309>  1998-07-15

<400>  182

Met Ala Val Arg Ala Ser Phe Glu Asn Asn Cys Glu Ile Gly Cys Phe
1               5                   10                  15

Ala Lys Leu Thr Asn Thr Tyr Cys Leu Val Ala Ile Gly Gly Ser Glu
            20                  25                  30

Asn Phe Tyr Ser Val Phe Glu Gly Glu Leu Ser Asp Thr Ile Pro Val
        35                  40                  45

Val His Ala Ser Ile Ala Gly Cys Arg Ile Ile Gly Arg Met Cys Val
    50                  55                  60

Gly Asn Arg His Gly Leu Leu Val Pro Asn Asn Thr Thr Asp Gln Glu
65                  70                  75                  80

Leu Gln His Ile Arg Asn Ser Leu Pro Asp Thr Val Gln Ile Arg Arg
                85                  90                  95

Val Glu Glu Arg Leu Ser Ala Leu Gly Asn Val Thr Thr Cys Asn Asp
            100                 105                 110

Tyr Val Ala Leu Val His Pro Asp Leu Asp Arg Glu Thr Glu Glu Ile
        115                 120                 125

Leu Ala Asp Val Leu Lys Val Glu Val Phe Arg Gln Thr Val Ala Asp
    130                 135                 140

Gln Val Leu Val Gly Ser Tyr Cys Val Phe Ser Asn Gln Gly Gly Leu
145                 150                 155                 160

Val His Pro Lys Thr Ser Ile Glu Asp Gln Asp Glu Leu Ser Ser Leu
                165                 170                 175

Leu Gln Val Pro Leu Val Ala Gly Thr Val Asn Arg Gly Ser Glu Val
            180                 185                 190

Ile Ala Ala Gly Met Val Val Asn Asp Trp Cys Ala Phe Cys Gly Leu
        195                 200                 205

Asp Thr Thr Ser Thr Glu Leu Ser Val Val Glu Ser Val Phe Lys Leu
    210                 215                 220

Asn Glu Ala Gln Pro Ser Thr Ile Ala Thr Ser Met Arg Asp Ser Leu
225                 230                 235                 240

Ile Asp Ser Leu Thr
                245

<210>  183

<211>  214

<212>  PRT

<213>  Homo sapiens

<300>

<308>  sw_hum/pdx5_human

<309>  1993-04-01

<400>  183

Met Gly Leu Ala Gly Val Cys Ala Leu Arg Arg Ser Ala Gly Tyr Ile
1               5                   10                  15

Leu Val Gly Gly Ala Gly Gly Gln Ser Ala Ala Ala Ala Ala Arg Arg
            20                  25                  30

Cys Ser Glu Gly Glu Trp Ala Ser Gly Gly Val Arg Ser Phe Ser Arg
        35                  40                  45

Ala Ala Ala Ala Met Ala Pro Ile Lys Val Gly Asp Ala Ile Pro Ala
    50                  55                  60

Val Glu Val Phe Glu Gly Glu Pro Gly Asn Lys Val Asn Leu Ala Glu
65                  70                  75                  80

Leu Phe Lys Gly Lys Lys Gly Val Leu Phe Gly Val Pro Gly Ala Phe
                85                  90                  95

Thr Pro Gly Cys Ser Lys Thr His Leu Pro Gly Phe Val Glu Gln Ala
            100                 105                 110

Glu Ala Leu Lys Ala Lys Gly Val Gln Val Val Ala Cys Leu Ser Val
        115                 120                 125

Asn Asp Ala Phe Val Thr Gly Glu Trp Gly Arg Ala His Lys Ala Glu
    130                 135                 140

Gly Lys Val Arg Leu Leu Ala Asp Pro Thr Gly Ala Phe Gly Lys Glu
145                 150                 155                 160

Thr Asp Leu Leu Leu Asp Asp Ser Leu Val Ser Ile Phe Gly Asn Arg
                165                 170                 175

Arg Leu Lys Arg Phe Ser Met Val Val Gln Asp Gly Ile Val Lys Ala
            180                 185                 190

Leu Asn Val Glu Pro Asp Gly Thr Gly Leu Thr Cys Ser Leu Ala Pro
        195                 200                 205

Asn Ile Ile Ser Gln Leu
    210

<210> 184

<211> 323

<212> PRT

<213> Homo sapiens

<300>

<308> sw_hum/pp1g_human

<309> 1994-06-01

<400> 184

Met Ala Asp Leu Asp Lys Leu Asn Ile Asp Ser Ile Ile Gln Arg Leu
1               5                   10                  15

Leu Glu Val Arg Gly Ser Lys Pro Gly Lys Asn Val Gln Leu Gln Glu
            20                  25                  30

Asn Glu Ile Arg Gly Leu Cys Leu Lys Ser Arg Glu Ile Phe Leu Ser
        35                  40                  45

Gln Pro Ile Leu Leu Glu Leu Glu Ala Pro Leu Lys Ile Cys Gly Asp
    50                  55                  60

Ile His Gly Gln Tyr Tyr Asp Leu Leu Arg Leu Phe Glu Tyr Gly Gly
65                  70                  75                  80

Phe Pro Pro Glu Ser Asn Tyr Leu Phe Leu Gly Asp Tyr Val Asp Arg
                85                  90                  95

Gly Lys Gln Ser Leu Glu Thr Ile Cys Leu Leu Leu Ala Tyr Lys Ile

            100                 105                 110

Lys Tyr Pro Glu Asn Phe Phe Leu Leu Arg Gly Asn His Glu Cys Ala
        115                 120                 125

Ser Ile Asn Arg Ile Tyr Gly Phe Tyr Asp Glu Cys Lys Arg Arg Tyr
    130                 135                 140

Asn Ile Lys Leu Trp Lys Thr Phe Thr Asp Cys Phe Asn Cys Leu Pro
145                 150                 155                 160

Ile Ala Ala Ile Val Asp Glu Lys Ile Phe Cys Cys His Gly Gly Leu
                165                 170                 175

Ser Pro Asp Leu Gln Ser Met Glu Gln Ile Arg Arg Ile Met Arg Pro
            180                 185                 190

Thr Asp Val Pro Asp Gln Gly Leu Leu Cys Asp Leu Leu Trp Ser Asp
        195                 200                 205

Pro Asp Lys Asp Val Leu Gly Trp Gly Glu Asn Asp Arg Gly Val Ser
    210                 215                 220

Phe Thr Phe Gly Ala Glu Val Val Ala Lys Phe Leu His Lys His Asp
225                 230                 235                 240

Leu Asp Leu Ile Cys Arg Ala His Gln Val Val Glu Asp Gly Tyr Glu
                245                 250                 255

Phe Phe Ala Lys Arg Gln Leu Val Thr Leu Phe Ser Ala Pro Asn Tyr
            260                 265                 270

Cys Gly Glu Phe Asp Asn Ala Gly Ala Met Met Ser Val Asp Glu Thr
        275                 280                 285

Leu Met Cys Ser Phe Gln Ile Leu Lys Pro Ala Glu Lys Lys Lys Pro
    290                 295                 300

Asn Ala Thr Arg Pro Val Thr Pro Pro Arg Gly Met Ile Thr Lys Gln
305                 310                 315                 320

Ala Lys Lys

<210> 185

<211> 226

<212> PRT

<213> Homo sapiens

<300>

<308> sw_hum/pimt_human

<309> 1991-08-01

<400>  185

Ala Trp Lys Ser Gly Gly Ala Ser His Ser Glu Leu Ile His Asn Leu
1               5                   10                  15

Arg Lys Asn Gly Ile Ile Lys Thr Asp Lys Val Phe Glu Val Met Leu
            20                  25                  30

Ala Thr Asp Arg Ser His Tyr Ala Lys Cys Asn Pro Tyr Met Asp Ser
        35                  40                  45

Pro Gln Ser Ile Gly Phe Gln Ala Thr Ile Ser Ala Pro His Met His
    50                  55                  60

Ala Tyr Ala Leu Glu Leu Leu Phe Asp Gln Leu His Glu Gly Ala Lys
65                  70                  75                  80

Ala Leu Asp Val Gly Ser Gly Ser Gly Ile Leu Thr Ala Cys Phe Ala
                85                  90                  95

Arg Met Val Gly Cys Thr Gly Lys Val Ile Gly Ile Asp His Ile Lys
            100                 105                 110

Glu Leu Val Asp Asp Ser Ile Asn Asn Val Arg Lys Asp Asp Pro Thr
        115                 120                 125

Leu Leu Ser Ser Gly Arg Val Gln Leu Val Val Gly Asp Gly Arg Met
    130                 135                 140

Gly Tyr Ala Glu Glu Ala Pro Tyr Asp Ala Ile His Val Gly Ala Ala
145                 150                 155                 160

Ala Pro Val Val Pro Gln Ala Leu Ile Asp Gln Leu Lys Pro Gly Gly
                165                 170                 175

Arg Leu Ile Leu Pro Val Gly Pro Ala Gly Gly Asn Gln Met Leu Glu
            180                 185                 190

Gln Tyr Asp Lys Leu Gln Asp Gly Ser Ile Lys Met Lys Pro Leu Met
        195                 200                 205

Gly Val Ile Tyr Val Pro Leu Thr Asp Lys Glu Lys Gln Trp Ser Arg
    210                 215                 220

Trp Lys
225

<210>  186

<211>  548

<212>  PRT

<213>  Homo sapiens

<300>

<308>  sw_hum/tcpq_human

<309>  1996-10-01

<400>  186

Met Ala Leu His Val Pro Lys Ala Pro Gly Phe Ala Gln Met Leu Lys
1               5                   10                  15

Glu Gly Ala Lys His Phe Ser Gly Leu Glu Glu Ala Val Tyr Arg Asn
            20                  25                  30

Ile Gln Ala Cys Lys Glu Leu Ala Gln Thr Thr Arg Thr Ala Tyr Gly
        35                  40                  45

Pro Asn Gly Met Asn Lys Met Val Ile Asn His Leu Glu Lys Leu Phe
    50                  55                  60

Val Thr Asn Asp Ala Ala Thr Ile Leu Arg Glu Leu Glu Val Gln His
65                  70                  75                  80

Pro Ala Ala Lys Met Ile Val Met Ala Ser His Met Gln Glu Gln Glu
                85                  90                  95

Val Gly Asp Gly Thr Asn Phe Val Leu Val Phe Ala Gly Ala Leu Leu
            100                 105                 110

Glu Leu Ala Glu Glu Leu Leu Arg Ile Gly Leu Ser Val Ser Glu Val
        115                 120                 125

Ile Glu Gly Tyr Glu Ile Ala Cys Arg Lys Ala His Glu Ile Leu Pro
    130                 135                 140

Asn Leu Val Cys Cys Ser Ala Lys Asn Leu Arg Asp Ile Asp Glu Val
145                 150                 155                 160

Ser Ser Leu Leu Arg Thr Ser Ile Met Ser Lys Gln Tyr Gly Asn Glu
                165                 170                 175

Val Phe Leu Ala Lys Leu Ile Ala Gln Ala Cys Val Ser Ile Phe Pro
            180                 185                 190

Asp Ser Gly His Phe Asn Val Asp Asn Ile Arg Val Cys Lys Ile Leu
        195                 200                 205

Gly Ser Gly Ile Ser Ser Ser Ser Val Leu His Gly Met Val Phe Lys
    210                 215                 220

Lys Glu Thr Glu Gly Asp Val Thr Ser Val Lys Asp Ala Lys Ile Ala
225                 230                 235                 240

Val Tyr Ser Cys Pro Phe Asp Gly Met Ile Thr Glu Thr Lys Gly Thr
                245                 250                 255

Val Leu Ile Lys Thr Ala Glu Glu Leu Met Asn Phe Ser Lys Gly Glu
            260                 265                 270

Glu Asn Leu Met Asp Ala Gln Val Lys Ala Ile Ala Asp Thr Gly Ala
        275                 280                 285

Asn Val Val Val Thr Gly Gly Lys Val Ala Asp Met Ala Leu His Tyr
    290                 295                 300

Ala Asn Lys Tyr Asn Ile Met Leu Val Arg Leu Asn Ser Lys Trp Asp
305                 310                 315                 320

Leu Arg Arg Leu Cys Lys Thr Val Gly Ala Thr Ala Leu Pro Arg Leu
                325                 330                 335

Thr Pro Pro Val Leu Glu Glu Met Gly His Cys Asp Ser Val Tyr Leu
            340                 345                 350

Ser Glu Val Gly Asp Thr Gln Val Val Val Phe Lys His Glu Lys Glu
        355                 360                 365

Asp Gly Ala Ile Ser Thr Ile Val Leu Arg Gly Ser Thr Asp Asn Leu
    370                 375                 380

Met Asp Asp Ile Glu Arg Ala Val Asp Asp Gly Val Asn Thr Phe Lys
385                 390                 395                 400

Val Leu Thr Arg Asp Lys Arg Leu Val Pro Gly Gly Gly Ala Thr Glu
                405                 410                 415

Ile Glu Leu Ala Lys Gln Ile Thr Ser Tyr Gly Glu Thr Cys Pro Gly
            420                 425                 430

Leu Glu Gln Tyr Ala Ile Lys Lys Phe Ala Glu Ala Phe Glu Ala Ile
        435                 440                 445

Pro Arg Ala Leu Ala Glu Asn Ser Gly Val Lys Ala Asn Glu Val Ile
    450                 455                 460

Ser Lys Leu Tyr Ala Val His Gln Glu Gly Asn Lys Asn Val Gly Leu
465                 470                 475                 480

Asp Ile Glu Ala Glu Val Pro Ala Val Lys Asp Met Leu Glu Ala Gly
                485                 490                 495

Ile Leu Asp Thr Tyr Leu Gly Lys Tyr Trp Ala Ile Lys Leu Ala Thr
            500                 505                 510

Asn Ala Ala Val Thr Val Leu Arg Val Asp Gln Ile Ile Met Ala Lys
        515                 520                 525

Pro Ala Gly Gly Pro Lys Pro Pro Ser Gly Lys Lys Asp Trp Asp Asp
    530                 535                 540

Asp Gln Asn Asp
545

<210>  187

<211>  51

<212>  PRT

<213>  Homo sapiens

<300>

<308>  hsugp/333417-11-0

<309>  2002-02-16

<400>  187

Ser Arg Phe Lys Ile Val Met Val Val Leu Tyr Gly Asn Thr Ala Val
1               5                   10                  15

Ile His Pro Met Cys Ala Ile Asp Glu Tyr Ala Met Ile Phe Arg Ser
            20                  25                  30

Trp Val Trp Phe Ser Pro Pro His Pro Pro Ile Lys Ile Asp Asn Met
        35                  40                  45

Ala Ile Val
    50

<210>  188

<211>  205

<212>  PRT

<213>  Homo sapiens

<300>

<308>  sw_hum/psb3_human

<309>  1993-07-01

<400>  188

Met Ser Ile Met Ser Tyr Asn Gly Gly Ala Val Met Ala Met Lys Gly
1               5                   10                  15

Lys Asn Cys Val Ala Ile Ala Ala Asp Arg Arg Phe Gly Ile Gln Ala
            20                  25                  30

Gln Met Val Thr Thr Asp Phe Gln Lys Ile Phe Pro Met Gly Asp Arg
        35                  40                  45

Leu Tyr Ile Gly Leu Ala Gly Leu Ala Thr Asp Val Gln Thr Val Ala
    50                  55                  60

Gln Arg Leu Lys Phe Arg Leu Asn Leu Tyr Glu Leu Lys Glu Gly Arg
65                  70                  75                  80

Gln Ile Lys Pro Tyr Thr Leu Met Ser Met Val Ala Asn Leu Leu Tyr
                85                  90                  95

Glu Lys Arg Phe Gly Pro Tyr Tyr Thr Glu Pro Val Ile Ala Gly Leu
            100                 105                 110

Asp Pro Lys Thr Phe Lys Pro Phe Ile Cys Ser Leu Asp Leu Ile Gly

115                     120                     125

Cys Pro Met Val Thr Asp Asp Phe Val Val Ser Gly Thr Cys Ala Glu
    130                     135                     140

Gln Met Tyr Gly Met Cys Glu Ser Leu Trp Glu Pro Asn Met Asp Pro
145                     150                     155                     160

Asp His Leu Phe Glu Thr Ile Ser Gln Ala Met Leu Asn Ala Val Asp
                165                     170                     175

Arg Asp Ala Val Ser Gly Met Gly Val Ile Val His Ile Ile Glu Lys
            180                     185                     190

Asp Lys Ile Thr Thr Arg Thr Leu Lys Ala Arg Met Asp
        195                     200                     205

<210>  189

<211>  397

<212>  PRT

<213>  Homo sapiens

<300>

<308>  humangp/chr6-q8tdm4

<309>  2003-04-22

<400>  189

Met Asn Ala Gly Ser Asp Pro Val Val Ile Val Ser Ala Ala Arg Thr
1               5                   10                  15

Ile Ile Gly Ser Phe Asn Gly Ala Leu Ala Ala Val Pro Val Gln Asp
            20                  25                  30

Leu Gly Ser Thr Val Ile Lys Glu Val Leu Lys Arg Ala Thr Val Ala
        35                  40                  45

Pro Glu Asp Val Ser Glu Val Ile Phe Gly His Val Leu Ala Ala Gly
    50                  55                  60

Cys Gly Gln Asn Pro Val Arg Gln Ala Ser Val Gly Ala Gly Ile Pro
65                  70                  75                  80

Tyr Ser Val Pro Ala Trp Ser Cys Gln Met Ile Cys Gly Ser Gly Leu
                85                  90                  95

Lys Ala Val Cys Leu Ala Val Gln Ser Ile Gly Ile Gly Asp Ser Ser
            100                 105                 110

Ile Val Val Ala Gly Gly Met Glu Asn Met Ser Lys Ala Pro His Leu
        115                 120                 125

Ala Tyr Leu Arg Thr Gly Val Lys Ile Gly Glu Met Pro Leu Thr Asp
    130                 135                 140

Ser Ile Leu Cys Asp Gly Leu Thr Asp Ala Phe His Asn Cys His Met
145                 150                 155                 160

Gly Ile Thr Ala Glu Asn Val Ala Lys Lys Trp Gln Val Ser Arg Glu
                165                 170                 175

Asp Gln Asp Lys Val Ala Val Leu Ser Gln Asn Arg Thr Glu Asn Ala
            180                 185                 190

Gln Lys Ala Gly His Phe Asp Lys Glu Ile Val Pro Val Leu Val Ser
        195                 200                 205

Thr Arg Lys Gly Leu Ile Glu Val Lys Thr Asp Glu Phe Pro Arg His
    210                 215                 220

Gly Ser Asn Ile Glu Ala Met Ser Lys Leu Lys Pro Tyr Phe Leu Thr
225                 230                 235                 240

Asp Gly Thr Gly Thr Val Thr Pro Ala Asn Ala Ser Gly Ile Asn Asp
                245                 250                 255

Gly Ala Ala Ala Val Val Leu Met Lys Lys Ser Glu Ala Asp Lys Arg
            260                 265                 270

Gly Leu Thr Pro Leu Ala Arg Ile Val Ser Trp Ser Gln Val Gly Val
        275                 280                 285

Glu Pro Ser Ile Met Gly Ile Gly Pro Ile Pro Ala Ile Lys Gln Ala
    290                 295                 300

Val Thr Lys Ala Gly Trp Ser Leu Glu Asp Val Asp Ile Phe Glu Ile
305                 310                 315                 320

Asn Glu Ala Phe Ala Ala Val Ser Ala Ala Ile Val Lys Glu Leu Gly
                325                 330                 335

Leu Asn Pro Glu Lys Val Asn Ile Glu Gly Gly Ala Ile Ala Leu Gly
            340                 345                 350

His Pro Leu Gly Ala Ser Gly Cys Arg Ile Leu Val Thr Leu Leu His
        355                 360                 365

Thr Leu Glu Arg Met Gly Arg Ser Arg Gly Val Ala Ala Leu Cys Ile
    370                 375                 380

Gly Gly Gly Met Gly Ile Ala Met Cys Val Gln Arg Glu
385                 390                 395

<210>  190

<211>  296

<212>  PRT

<213>  Homo sapiens

<300>

<308>  sw_hum/biea_human

<309>  1996-10-01

<400>  190

Met Asn Ala Glu Pro Glu Arg Lys Phe Gly Val Val Val Val Gly Val
1               5                   10                  15

Gly Arg Ala Gly Ser Val Arg Met Arg Asp Leu Arg Asn Pro His Pro
            20                  25                  30

Ser Ser Ala Phe Leu Asn Leu Ile Gly Phe Val Ser Arg Arg Glu Leu
        35                  40                  45

Gly Ser Ile Asp Gly Val Gln Gln Ile Ser Leu Glu Asp Ala Leu Ser
    50                  55                  60

Ser Gln Glu Val Glu Val Ala Tyr Ile Cys Ser Glu Ser Ser Ser His
65                  70                  75                  80

Glu Asp Tyr Ile Arg Gln Phe Leu Asn Ala Gly Lys His Val Leu Val
                85                  90                  95

Glu Tyr Pro Met Thr Leu Ser Leu Ala Ala Ala Gln Glu Leu Trp Glu
            100                 105                 110

Leu Ala Glu Gln Lys Gly Lys Val Leu His Glu Glu His Val Glu Leu
        115                 120                 125

Leu Met Glu Glu Phe Ala Phe Leu Lys Lys Glu Val Val Gly Lys Asp
    130                 135                 140

Leu Leu Lys Gly Ser Leu Leu Phe Thr Ala Gly Pro Leu Glu Glu Glu
145                 150                 155                 160

Arg Phe Gly Phe Pro Ala Phe Ser Gly Ile Ser Arg Leu Thr Trp Leu
                165                 170                 175

Val Ser Leu Phe Gly Glu Leu Ser Leu Val Ser Ala Thr Leu Glu Glu
            180                 185                 190

Arg Lys Glu Asp Gln Tyr Met Lys Met Thr Val Cys Leu Glu Thr Glu
        195                 200                 205

Lys Lys Ser Pro Leu Ser Trp Ile Glu Glu Lys Gly Pro Gly Leu Lys
    210                 215                 220

Arg Asn Arg Tyr Leu Ser Phe His Phe Lys Ser Gly Ser Leu Glu Asn
225                 230                 235                 240

Val Pro Asn Val Gly Val Asn Lys Asn Ile Phe Leu Lys Asp Gln Asn
                245                 250                 255

Ile Phe Val Gln Lys Leu Leu Gly Gln Phe Ser Glu Lys Glu Leu Ala
            260                 265                 270

Ala Glu Lys Lys Arg Ile Leu His Cys Leu Gly Leu Ala Glu Glu Ile
        275                 280                 285

Gln Lys Tyr Cys Cys Ser Arg Lys
    290                 295

<210>  191

<211>  216

<212>  PRT

<213>  Homo sapiens

<300>

<308>  sw_hum/ran_human

<309>  1990-08-01

<400>  191

Met Ala Ala Gln Gly Glu Pro Gln Val Gln Phe Lys Leu Val Leu Val
1               5                   10                  15

Gly Asp Gly Gly Thr Gly Lys Thr Thr Phe Val Lys Arg His Leu Thr
            20                  25                  30

Gly Glu Phe Glu Lys Lys Tyr Val Ala Thr Leu Gly Val Glu Val His
        35                  40                  45

Pro Leu Val Phe His Thr Asn Arg Gly Pro Ile Lys Phe Asn Val Trp
    50                  55                  60

Asp Thr Ala Gly Gln Glu Lys Phe Gly Gly Leu Arg Asp Gly Tyr Tyr
65                  70                  75                  80

Ile Gln Ala Gln Cys Ala Ile Ile Met Phe Asp Val Thr Ser Arg Val
                85                  90                  95

Thr Tyr Lys Asn Val Pro Asn Trp His Arg Asp Leu Val Arg Val Cys
            100                 105                 110

Glu Asn Ile Pro Ile Val Leu Cys Gly Asn Lys Val Asp Ile Lys Asp
        115                 120                 125

Arg Lys Val Lys Ala Lys Ser Ile Val Phe His Arg Lys Lys Asn Leu
    130                 135                 140

Gln Tyr Tyr Asp Ile Ser Ala Lys Ser Asn Tyr Asn Phe Glu Lys Pro
145                 150                 155                 160

Phe Leu Trp Leu Ala Arg Lys Leu Ile Gly Asp Pro Asn Leu Glu Phe
                165                 170                 175

Val Ala Met Pro Ala Leu Ala Pro Pro Glu Val Val Met Asp Pro Ala
            180                 185                 190

Leu Ala Ala Gln Tyr Glu His Asp Leu Glu Val Ala Gln Thr Thr Ala
        195                 200                 205

Leu Pro Asp Glu Asp Asp Asp Leu
    210                 215

<210>  192

<211>  406

<212>  PRT

<213>  Homo sapiens

<300>

<308>  sw_hum/prs8_human

<309>  1996-02-01

<400>  192

Met Ala Leu Asp Gly Pro Glu Gln Met Glu Leu Glu Glu Gly Lys Ala
1               5                   10                  15

Gly Ser Gly Leu Arg Gln Tyr Tyr Leu Ser Lys Ile Glu Glu Leu Gln
            20                  25                  30

Leu Ile Val Asn Asp Lys Ser Gln Asn Leu Arg Arg Leu Gln Ala Gln
        35                  40                  45

Arg Asn Glu Leu Asn Ala Lys Val Arg Leu Leu Arg Glu Glu Leu Gln
    50                  55                  60

Leu Leu Gln Glu Gln Gly Ser Tyr Val Gly Glu Val Val Arg Ala Met
65                  70                  75                  80

Asp Lys Lys Lys Val Leu Val Lys Val His Pro Glu Gly Lys Phe Val
                85                  90                  95

Val Asp Val Asp Lys Asn Ile Asp Ile Asn Asp Val Thr Pro Asn Cys
            100                 105                 110

Arg Val Ala Leu Arg Asn Asp Ser Tyr Thr Leu His Lys Ile Leu Pro
        115                 120                 125

Asn Lys Val Asp Pro Leu Val Ser Leu Met Met Val Glu Lys Val Pro
    130                 135                 140

Asp Ser Thr Tyr Glu Met Ile Gly Gly Leu Asp Lys Gln Ile Lys Glu
145                 150                 155                 160

Ile Lys Glu Val Ile Glu Leu Pro Val Lys His Pro Glu Leu Phe Glu
                165                 170                 175

Ala Leu Gly Ile Ala Gln Pro Lys Gly Val Leu Leu Tyr Gly Pro Pro
            180                 185                 190

Gly Thr Gly Lys Thr Leu Leu Ala Arg Ala Val Ala His His Thr Asp

195                 200                 205

Cys Thr Phe Ile Arg Val Ser Gly Ser Glu Leu Val Gln Lys Phe Ile
    210                 215                 220

Gly Glu Gly Ala Arg Met Val Arg Glu Leu Phe Val Met Ala Arg Glu
225                 230                 235                 240

His Ala Pro Ser Ile Ile Phe Met Asp Glu Ile Asp Ser Ile Gly Ser
                245                 250                 255

Ser Arg Leu Glu Gly Gly Ser Gly Gly Asp Ser Glu Val Gln Arg Thr
            260                 265                 270

Met Leu Glu Leu Leu Asn Gln Leu Asp Gly Phe Glu Ala Thr Lys Asn
        275                 280                 285

Ile Lys Val Ile Met Ala Thr Asn Arg Ile Asp Ile Leu Asp Ser Ala
    290                 295                 300

Leu Leu Arg Pro Gly Arg Ile Asp Arg Lys Ile Glu Phe Pro Pro Pro
305                 310                 315                 320

Asn Glu Glu Ala Arg Leu Asp Ile Leu Lys Ile His Ser Arg Lys Met
                325                 330                 335

Asn Leu Thr Arg Gly Ile Asn Leu Arg Lys Ile Ala Glu Leu Met Pro
            340                 345                 350

Gly Ala Ser Gly Ala Glu Val Lys Gly Val Cys Thr Glu Ala Gly Met
        355                 360                 365

Tyr Ala Leu Arg Glu Arg Arg Val His Val Thr Gln Glu Asp Phe Glu
    370                 375                 380

Met Ala Val Ala Lys Val Met Gln Lys Asp Ser Glu Lys Asn Met Ser
385                 390                 395                 400

Ile Lys Lys Leu Trp Lys
                405

<210>  193

<211>  189

<212>  PRT

<213>  Homo sapiens


<300>

<308>  humangp/chr1-014805

<309>  2003-04-22


<400>  193

<210>  194

<211>  246

<212>  PRT

<213>  Homo sapiens

<300>

<308>  sw_hum/psa6_human

<309>  1994-02-01

<400>  194

Met Ser Arg Gly Ser Ser Ala Gly Phe Asp Arg His Ile Thr Ile Phe
1               5                   10                  15

Ser Pro Glu Gly Arg Leu Tyr Gln Val Glu Tyr Ala Phe Lys Ala Ile
            20                  25                  30

Asn Gln Gly Gly Leu Thr Ser Val Ala Val Arg Gly Lys Asp Cys Ala
        35                  40                  45

Val Ile Val Thr Gln Lys Lys Val Pro Asp Lys Leu Leu Asp Ser Ser
    50                  55                  60

Thr Val Thr His Leu Phe Lys Ile Thr Glu Asn Ile Gly Cys Val Met
65                  70                  75                  80

Thr Gly Met Thr Ala Asp Ser Arg Ser Gln Val Gln Arg Ala Arg Tyr
                85                  90                  95

Glu Ala Ala Asn Trp Lys Tyr Lys Tyr Gly Tyr Glu Ile Pro Val Asp
            100                 105                 110

Met Leu Cys Lys Arg Ile Ala Asp Ile Ser Gln Val Tyr Thr Gln Asn
        115                 120                 125

Ala Glu Met Arg Pro Leu Gly Cys Cys Met Ile Leu Ile Gly Ile Asp
    130                 135                 140

Glu Glu Gln Gly Pro Gln Val Tyr Lys Cys Asp Pro Ala Gly Tyr Tyr
145                 150                 155                 160

Cys Gly Phe Lys Ala Thr Ala Ala Gly Val Lys Gln Thr Glu Ser Thr
                165                 170                 175

Ser Phe Leu Glu Lys Lys Val Lys Lys Lys Phe Asp Trp Thr Phe Glu
            180                 185                 190

Gln Thr Val Glu Thr Ala Ile Thr Cys Leu Ser Thr Val Leu Ser Ile
        195                 200                 205

Asp Phe Lys Pro Ser Glu Ile Glu Val Gly Val Val Thr Val Glu Asn
    210                 215                 220

Pro Lys Phe Arg Ile Leu Thr Glu Ala Glu Ile Asp Ala His Leu Val
225                 230                 235                 240

Ala Leu Ala Glu Arg Asp
                245

<210>  195

<211>  258

<212>  PRT

<213>  Homo sapiens


<300>

<308>  sw_hum/6pgl_human

<309>  2000-05-30


<400>  195

Met Ala Ala Pro Ala Pro Gly Leu Ile Ser Val Phe Ser Ser Ser Gln
1               5                   10                  15

Glu Leu Gly Ala Ala Leu Ala Gln Leu Val Ala Gln Arg Ala Ala Cys
            20                  25                  30

Cys Leu Ala Gly Ala Arg Ala Arg Phe Ala Leu Gly Leu Ser Gly Gly
        35                  40                  45

Ser Leu Val Ser Met Leu Ala Arg Glu Leu Pro Ala Ala Val Ala Pro
    50                  55                  60

Ala Gly Pro Ala Ser Leu Ala Arg Trp Thr Leu Gly Phe Cys Asp Glu
65                  70                  75                  80

Arg Leu Val Pro Phe Asp His Ala Glu Ser Thr Tyr Gly Leu Tyr Arg
                85                  90                  95

Thr His Leu Leu Ser Arg Leu Pro Ile Pro Glu Ser Gln Val Ile Thr
            100                 105                 110

Ile Asn Pro Glu Leu Pro Val Glu Glu Ala Ala Glu Asp Tyr Ala Lys
        115                 120                 125

Lys Leu Arg Gln Ala Phe Gln Gly Asp Ser Ile Pro Val Phe Asp Leu
    130                 135                 140

Leu Ile Leu Gly Val Gly Pro Asp Gly His Thr Cys Ser Leu Phe Pro
145                 150                 155                 160

Asp His Pro Leu Leu Gln Glu Arg Glu Lys Ile Val Ala Pro Ile Ser
                165                 170                 175

Asp Ser Pro Lys Pro Pro Pro Gln Arg Val Thr Leu Thr Leu Pro Val
            180                 185                 190

Leu Asn Ala Ala Arg Thr Val Ile Phe Val Ala Thr Gly Glu Gly Lys
        195                 200                 205

Ala Ala Val Leu Lys Arg Ile Leu Glu Asp Gln Glu Glu Asn Pro Leu
    210                 215                 220

Pro Ala Ala Leu Val Gln Pro His Thr Gly Lys Leu Cys Trp Phe Leu
225                 230                 235                 240

Asp Glu Ala Ala Ala Arg Leu Leu Thr Val Pro Phe Glu Lys His Ser
                245                 250                 255

Thr Leu

<210>  196

<211>  310

<212>  PRT

<213>  Homo sapiens

<300>

<308>  humangp/chr19-q8wvx0

<309>  2003-04-22

<400>  196

Leu Thr Gly Ser Asn Tyr Pro Gly Leu Ser Ile Ser Leu Arg Leu Thr
1               5                   10                  15

Gly Ser Ser Ala Gln Glu Glu Ala Ser Gly Val Ala Leu Gly Glu Ala
            20                  25                  30

Pro Asp His Ser Tyr Glu Ser Leu Arg Val Thr Ser Ala Gln Lys His
        35                  40                  45

Val Leu His Val Gln Leu Asn Arg Pro Asn Lys Arg Asn Ala Met Asn
    50                  55                  60

Lys Val Phe Trp Arg Glu Met Val Glu Cys Phe Asn Lys Ile Ser Arg
65                  70                  75                  80

Asp Ala Asp Cys Arg Ala Val Val Ile Ser Gly Ala Gly Lys Met Phe
                85                  90                  95

Thr Ala Gly Ile Asp Leu Met Asp Met Ala Ser Asp Ile Leu Gln Pro
            100                 105                 110

Lys Gly Asp Asp Val Ala Arg Ile Ser Trp Tyr Leu Arg Asp Ile Ile
        115                 120                 125

Thr Arg Tyr Gln Glu Thr Phe Asn Val Ile Glu Arg Cys Pro Lys Pro
    130                 135                 140

Val Ile Ala Ala Val His Gly Gly Cys Ile Gly Gly Gly Val Asp Leu
145                 150                 155                 160

Val Thr Ala Cys Asp Ile Arg Tyr Cys Ala Gln Asp Ala Phe Phe Gln
                165                 170                 175

Val Lys Glu Val Asp Val Gly Leu Ala Ala Asp Val Gly Thr Leu Gln
            180                 185                 190

Arg Leu Pro Lys Val Ile Gly Asn Gln Ser Leu Val Asn Glu Leu Ala
        195                 200                 205

Phe Thr Ala Arg Lys Met Met Ala Asp Glu Ala Leu Gly Ser Gly Leu
    210                 215                 220

Val Ser Arg Val Phe Pro Asp Lys Glu Val Met Leu Asp Ala Ala Leu
225                 230                 235                 240

Ala Leu Ala Ala Glu Ile Ser Ser Lys Ser Pro Val Ala Val Gln Ser
                245                 250                 255

Thr Lys Val Asn Leu Leu Tyr Ser Arg Asp His Ser Val Ala Glu Ser
            260                 265                 270

Leu Asn Tyr Val Ala Ser Trp Asn Met Ser Met Leu Gln Thr Gln Asp

275                 280                 285

Leu Val Lys Ser Val Gln Ala Thr Thr Glu Asn Lys Glu Leu Lys Thr
    290                 295                 300

Val Thr Phe Ser Lys Leu
305                 310

<210>  197

<211>  780

<212>  PRT

<213>  Homo sapiens

<300>

<308>  sw_hum/acon_human

<309>  1997-11-01

<400>  197

<210>  198

<211>  480

<212>  PRT

<213>  Homo sapiens

<300>

<308>  sw_hum/ucr1_human

<309>  1993-07-01

<400>  198

Met Ala Ala Ser Val Val Cys Arg Ala Ala Thr Ala Gly Ala Gln Val
1               5                   10                  15

Leu Leu Arg Ala Arg Arg Ser Pro Ala Leu Leu Arg Thr Pro Ala Leu
            20                  25                  30

Arg Ser Thr Ala Thr Phe Ala Gln Ala Leu Gln Phe Val Pro Glu Thr
        35                  40                  45

Gln Val Ser Leu Leu Asp Asn Gly Leu Arg Val Ala Ser Glu Gln Ser
    50                  55                  60

Ser Gln Pro Thr Cys Thr Val Gly Val Trp Ile Asp Val Gly Ser Arg
65                  70                  75                  80

Phe Glu Thr Glu Lys Asn Asn Gly Ala Gly Tyr Phe Leu Glu His Leu
                85                  90                  95

Ala Phe Lys Gly Thr Lys Asn Arg Pro Gly Ser Ala Leu Glu Lys Glu
            100                 105                 110

Val Glu Ser Met Gly Ala His Leu Asn Ala Tyr Ser Thr Arg Glu His
        115                 120                 125

Thr Ala Tyr Tyr Ile Lys Ala Leu Ser Lys Asp Leu Pro Lys Ala Val
    130                 135                 140

Glu Leu Leu Gly Asp Ile Val Gln Asn Cys Ser Leu Glu Asp Ser Gln
145                 150                 155                 160

Ile Glu Lys Glu Arg Asp Val Ile Leu Arg Glu Met Gln Glu Asn Asp
                165                 170                 175

Ala Ser Met Arg Asp Val Val Phe Asn Tyr Leu His Ala Thr Ala Phe
            180                 185                 190

Gln Gly Thr Pro Leu Ala Gln Ala Val Glu Gly Pro Ser Glu Asn Val
        195                 200                 205

Arg Lys Leu Ser Arg Ala Asp Leu Thr Glu Tyr Leu Ser Thr His Tyr
    210                 215                 220

Lys Ala Pro Arg Met Val Leu Ala Ala Ala Gly Gly Val Glu His Gln
225                 230                 235                 240

Gln Leu Leu Asp Leu Ala Gln Lys His Leu Gly Gly Ile Pro Trp Thr

245                 250                 255

Tyr Ala Glu Asp Ala Val Pro Thr Leu Thr Pro Cys Arg Phe Thr Gly
            260                 265                 270

Ser Glu Ile Arg His Arg Asp Asp Ala Leu Pro Phe Ala His Val Ala
        275                 280                 285

Ile Ala Val Glu Gly Pro Gly Trp Ala Ser Pro Asp Ser Val Ala Leu
    290                 295                 300

Gln Val Ala Asn Ala Ile Ile Gly His Tyr Asp Cys Thr Tyr Gly Gly
305                 310                 315                 320

Gly Val His Leu Ser Ser Pro Leu Ala Ser Gly Ala Val Ala Asn Lys
                325                 330                 335

Leu Cys Gln Ser Phe Gln Thr Phe Ser Ile Cys Tyr Ala Glu Thr Gly
            340                 345                 350

Leu Leu Gly Ala His Phe Val Cys Asp Arg Met Lys Ile Asp Asp Met
        355                 360                 365

Met Phe Val Leu Gln Gly Gln Trp Met Arg Leu Cys Thr Ser Ala Thr
    370                 375                 380

Glu Ser Glu Val Ala Arg Gly Lys Asn Ile Leu Arg Asn Ala Leu Val
385                 390                 395                 400

Ser His Leu Asp Gly Thr Thr Pro Val Cys Glu Asp Ile Gly Arg Ser
                405                 410                 415

Leu Leu Thr Tyr Gly Arg Arg Ile Pro Leu Ala Glu Trp Glu Ser Arg
            420                 425                 430

Ile Ala Glu Val Asp Ala Ser Val Val Arg Glu Ile Cys Ser Lys Tyr
        435                 440                 445

Ile Tyr Asp Gln Cys Pro Ala Val Ala Gly Tyr Gly Pro Ile Glu Gln
    450                 455                 460

Leu Pro Asp Tyr Asn Arg Ile Arg Ser Gly Met Phe Trp Leu Arg Phe
465                 470                 475                 480

<210>  199

<211>  205

<212>  PRT

<213>  Homo sapiens

<300>

<308>  sw_hum/hs27_human

<309>  1987-08-13

<400> 199


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205

35 40 45

Cys Leu Asp Asn Gly Ala Lys Ser Val Val Leu Met Ser His Leu Gly
50 55 60

Arg Pro Asp Gly Val Pro Met Pro Asp Lys Tyr Ser Leu Glu Pro Val
65 70 75 80

Ala Val Glu Leu Lys Ser Leu Leu Gly Lys Asp Val Leu Phe Leu Lys
85 90 95

Asp Cys Val Gly Pro Glu Val Glu Lys Ala Cys Ala Asn Pro Ala Ala
100 105 110

Gly Ser Val Ile Leu Leu Glu Asn Leu Arg Phe His Val Glu Glu Glu
115 120 125

Gly Lys Gly Lys Asp Ala Ser Gly Asn Lys Val Lys Ala Glu Pro Ala
130 135 140

Lys Ile Glu Ala Phe Arg Ala Ser Leu Ser Lys Leu Gly Asp Val Tyr
145 150 155 160

Val Asn Asp Ala Phe Gly Thr Ala His Arg Ala His Ser Ser Met Val
165 170 175

Gly Val Asn Leu Pro Gln Lys Ala Gly Gly Phe Leu Met Lys Lys Glu
180 185 190

Leu Asn Tyr Phe Ala Lys Ala Leu Glu Ser Pro Glu Arg Pro Phe Leu
195 200 205

Ala Ile Leu Gly Gly Ala Lys Val Ala Asp Lys Ile Gln Leu Ile Asn
210 215 220

Asn Met Leu Asp Lys Val Asn Glu Met Ile Ile Gly Gly Gly Met Ala
225 230 235 240


245 250 255


260 265 270


275 280 285


290 295 300


305 310 315 320

Lys Lys Tyr Ala Glu Ala Val Thr Arg Ala Lys Gln Ile Val Trp Asn
325 330 335

Gly Pro Val Gly Val Phe Glu Trp Glu Ala Phe Ala Arg Gly Thr Lys
340 345 350

Gly Glu Trp Glu Leu Ile Gln Glu Ser Gly Val Pro Leu Lys Pro Leu
305                 310                 315                 320

Phe Gly Pro Gly Tyr Thr Gly Ile Arg Asn Leu Gly Asn Ser Cys Tyr
                325                 330                 335

Leu Asn Ser Val Val Gln Val Leu Phe Ser Ile Pro Asp Phe Gln Arg
            340                 345                 350

Lys Tyr Val Asp Lys Leu Glu Lys Ile Phe Gln Asn Ala Pro Thr Asp
        355                 360                 365

Pro Thr Gln Asp Phe Ser Thr Gln Val Ala Lys Leu Gly His Gly Leu
    370                 375                 380

Leu Ser Gly Glu Tyr Ser Lys Pro Val Pro Glu Ser Gly Asp Gly Glu
385                 390                 395                 400

Arg Val Pro Glu Gln Lys Glu Val Gln Asp Gly Ile Ala Pro Arg Met
                405                 410                 415

Phe Lys Ala Leu Ile Gly Lys Gly His Pro Glu Phe Ser Thr Asn Arg
            420                 425                 430

Gln Gln Asp Ala Gln Glu Phe Phe Leu His Leu Ile Asn Met Val Glu
        435                 440                 445

Arg Asn Cys Arg Ser Ser Glu Asn Pro Asn Glu Val Phe Arg Phe Leu
    450                 455                 460

Val Glu Glu Lys Ile Lys Cys Leu Ala Thr Glu Lys Val Lys Tyr Thr
465                 470                 475                 480

Gln Arg Val Asp Tyr Ile Met Gln Leu Pro Val Pro Met Asp Ala Ala
                485                 490                 495

Leu Asn Lys Glu Glu Leu Leu Glu Tyr Glu Glu Lys Lys Arg Gln Ala
            500                 505                 510

Glu Glu Glu Lys Met Ala Leu Pro Glu Leu Val Arg Ala Gln Val Pro
        515                 520                 525

Phe Ser Ser Cys Leu Glu Ala Tyr Gly Ala Pro Glu Gln Val Asp Asp
    530                 535                 540

Phe Trp Ser Thr Ala Leu Gln Ala Lys Ser Val Ala Val Lys Thr Thr
545                 550                 555                 560

Arg Phe Ala Ser Phe Pro Asp Tyr Leu Val Ile Gln Ile Lys Lys Phe
                565                 570                 575

Thr Phe Gly Leu Asp Trp Val Pro Lys Lys Leu Asp Val Ser Ile Glu
            580                 585                 590

Met Pro Glu Glu Leu Asp Ile Ser Gln Leu Arg Gly Thr Gly Leu Gln
        595                 600                 605